# EUROPEAN PATENT APPLICATION

(11) **EP 1 439 224 A2**
(43) Date of publication of application: **21.07.2004**
(21) Application number: 04005568.3
(22) Date of filing: 27.01.1999
(51) Int. Cl.: C12N 15/00, C07K 14/47

(54) **67 human secreted proteins**

(30) Priority: 30.01.1998 US 73164; 30.01.1998 US 73160; 30.01.1998 US 73165; 30.01.1998 US 73170; 30.01.1998 US 73161; 30.01.1998 US 73162; 30.01.1998 US 73167; 30.01.1998 US 73159
(62) Divisional of application: 99904290.6
(71) Applicant: Human Genome Sciences, Inc., Rockville, MD 20850 (US)
(72) Inventor: Ruben, Steven M., Brookeville MD 20833 (US); Ferrie, Ann M., Painted Post NY 14870 (US); Rosen, Craig A., Laytonsville MD 20882 (US); Florence, Kimberly A., Rockville MD 20851 (US); Carter, Kenneth C., North Potomac MD 20878 (US); Soppet, Daniel R., Centreville VA 20120 (US); Yu, Guo-Liang, Berkeley CA 94705 (US); Florence, Charles, Rockville MD 20851 (US); Young, Paul E., Gaithersburg MD 20878 (US); Ni, Jian, Germantown MD 20874 (US); Feng, Ping, Germantown MD 20874 (US); Endress, Gregory A., Florence, MA 01062 (US); Janat, Fouad, Westerley, RI 02891 (US)
(74) Representative: VOSSIUS & PARTNER

(57) **Abstract**

The present invention relates to novel human secreted proteins and isolated nucleic acids containing the coding regions of the genes encoding such proteins. Also provided are vectors, host cells, antibodies, and recombinant methods for producing human secreted protein. The invention further relates to diagnostic and therapeutic methods useful for diagnosing and treating disorders related to these novel human secreted proteins.

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

In specific embodiments, the polynucleotides of the invention are less than 300 kb, 200 kb, 100 kb, 50 kb, 15 kb, 10 kb, or 7.5 kb in length. In a further embodiment, polynucleotides of the invention comprise at least 15 contiguous nucleotides of the coding sequence, but do not comprise all or a portion of any intron. In another embodiment, the nucleic acid comprising the coding sequence does not contain coding sequences of a genomic flanking gene (i.e., 5' or 3' to the gene in the genome).

As used herein , a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

The gene encoding the disclosed cDNA is thought to reside on the X chromosome. Accordingly, polynucleotides related to this invention are useful as a marker in linkage analysis for the X chromosome. When tested against U937 Myeloid cell lines, supernatants removed from cells containing this gene activated the GAS assay. Thus, it is likely that this gene activates myeloid cells, or more generally, immune or hematopoietic cells, in addition to other cells or cell-types, through the JAK-STAT signal transduction pathway. The gamma activating sequence (GAS) is a promoter element found upstream of many genes which are involved in the JAK-STAT pathway. The JAK-STAT pathway is a large, signal transduction pathway involved in the differentiation and proliferation of cells. Therefore, activation of the JAK-STAT pathway, reflected by the binding of the GAS element, can be used to indicate proteins involved in the proliferation and differentiation of cells. In specific embodiments, polypeptides of the invention comprise the following amino acid sequence: GSFLGSTNRDRESLAFQFCAG (SEQ ID NO:147). Polynucleotides encoding these polypeptides are also encompassed by the invention.

This gene is expressed primarily in larynx carcinoma II, T-cell lymphoma, thymus, and to a lesser extent in a broad range of cancerous tissues.

Therefore, polynucleotides and polypeptides of the invention are useful as reagents for differential identification of the tissue(s) or cell type(s) present in a biological sample and for diagnosis of diseases and conditions which include, but are not limited to, cancers, uncontrolled cell growth and/or differentiation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in a number of immune and cancerous tissues, in conjunction with the biological activity data, indicates that polynucleotides and polypeptides corresponding to this gene are useful for the diagnosis and/or treatment of various cancers, particularly those arising within immune tissues, as well as cancers of other tissues where expression has been observed. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:11 and may have been publicly available prior to conception of the present invention. Preferably, such related polynucleotides are specifically excluded from the scope of the present invention. To list every related sequence would be cumbersome. Accordingly, preferably excluded-from the present invention are one or more polynucleotides comprising a nucleotide sequence described by the general formula of a-b, where a is any integer between 1 to 1065 of SEQ ID NO:11, b is an integer of 15 to 1079, where both a and b correspond to the positions of nucleotide residues shown in SEQ ID NO:11, and where b is greater than or equal to a + 14.

### FEATURES OF PROTEIN ENCODED BY GENE NO: 2

The translation product of this gene shares sequence homology with the conserved golgi complexed alpha-mannosidase gene family members (from mouse, rabbit, C.elegans and yeast), which are thought to be important in catalyzing the hydrolysis of terminal, D-mannose residues of mannosides (particularly in glycoproteins). Thus, based on the sequence similarity, the translation product of this clone is expected to share biological activities with glycoprotein synthases, and more generally, glycoproteins. Such activities are known in the art and described elsewhere herein. The gene encoding the disclosed cDNA is thought to reside on chromosome 20. Accordingly, polynucleotides related to this invention are useful as a marker in linkage analysis for chromosome 20. When tested against U937 Myeloid cell lines and Jurkat T-cell cell lines, supernatants removed from cells containing this gene activated the GAS assay. Thus, it is likely that this gene activates both myeloid cells and T-cells, or more generally, other immune or hematopoietic cells, in addition to other cells or cell-types, through the JAK-STAT signal transduction pathway.

The gamma activating sequence (GAS) is a promoter element found upstream of many genes which are involved in the JAK-STAT pathway. The JAK-STAT pathway is a large, signal transduction pathway involved in the differentiation and proliferation of cells. Therefore, activation of the JAK-STAT pathway, reflected by the binding of the GAS element, can be used to indicate proteins involved in the proliferation and differentiation of cells.

This gene is expressed primarily in stomach and colon cancer, kidney, and cerebellum tissue, and to a lesser extent in whole brain tissue.

Therefore, polynucleotides and polypeptides of the invention are useful as reagents for differential identification of the tissue(s) or cell type(s) present in a biological sample and for diagnosis of diseases and conditions which include, but are not limited to, mannosidosis and cancer. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues or cell types (e.g., nervous, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 80 as residues: Pro-23 to His-34, Thr-64 to Trp-71.

The tissue distribution in nervous system tissues such as brain and cerebellum tissue, and the homology to alpha-mannosidase, indicates that polynucleotides and polypeptides corresponding to this gene are useful for the diagnosis and/or treatment of mannosidosis, which is associated with mental retardation, Kyphosis and vacuolated lymphocytes, with the accumulation of mannose in tissue, and with autosomal recessive inheritance. Furthermore, the tissue distribution in stomach and colon cancerous tissues indicates that the translation product of this gene is useful in the detection and/or treatment of colon and stomach cancer, as well as cancers of other tissues where expression has been observed. Protein, as well as, antibodies directed against the protein may show utility as a tissue-specific marker and/or immunotherapy target for the above listed tissues.

Many polynucleotide sequences, such as EST sequences, are publicly available and accessible through sequence databases. Some of these sequences are related to SEQ ID NO:12 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1918 of SEQ ID NO:12, b is an integer of 15 to 1932, where both a and b correspond to the positions of nucleotide residues shown in SEQ ID NO:12, and where b is greater than or equal to a + 14.

### FEATURES OF PROTEIN ENCODED BY GENE NO: 3

When tested against U937 Myeloid cell lines, supernatants removed from cells containing this gene activated the GAS assay. Thus, it is likely that this gene activates myeloid cells, or more generally, immune or hematopoietic cells, in addition to other cells or cell-types, through the JAK-STAT signal transduction pathway. The gamma activating sequence (GAS) is a promoter element found upstream of many genes which are involved in the JAK-STAT pathway. The JAK-STAT pathway is a large, signal transduction pathway involved in the differentiation and proliferation of cells. Therefore, activation of the JAK-STAT pathway, reflected by the binding of the GAS element, can be used to indicate proteins involved in the proliferation and differentiation of cells.

This gene is expressed primarily in fetal liver/spleen and other hematopoietic tissues, and to a lesser extent in endothelial cells.

Therefore, polynucleotides and polypeptides of the invention are useful as reagents for differential identification of the tissue(s) or cell type(s) present in a biological sample and for diagnosis of diseases and conditions which include, but are not limited to, hematopoietic disorders; immune dysfunction; autoimmunity; impaired immunity; aberrant angiogenesis. Similarly, polypeptides and antibodies directed to these polypeptides are useful in providing immunological probes for differential identification of the tissue(s) or cell type(s). For a number of disorders of the above tissues or cells, particularly of the immune and circulatory systems, expression of this gene at significantly higher or lower levels may be routinely detected in certain tissues or cell types (e.g., immune, circulatory, cancerous and wounded tissues) or bodily fluids (e.g., lymph, serum, plasma, urine, amniotic fluid, bile, synovial fluid and spinal fluid) or another tissue or cell sample taken from an individual having such a disorder, relative to the standard gene expression level, i.e., the expression level in healthy tissue or bodily fluid from an individual not'having the disorder.

Preferred epitopes include those comprising a sequence shown in SEQ ID NO. 81 as residues: Glu-57 to Cys-64, Pro-66 to Val-73, Thr-76 to Leu-82.

The tissue distribution in immune tissues and endothelial tissues, in conjunction with the biological activity data, indicates that polynucleotides and polypeptides corresponding to this gene are useful for the diagnosis and treatment of a variety of human disorders. Elevated expression of this gene product in hematopoietic tissues and endothelial cells indicates possible roles in both of these tissues and systems. In particular, elevated expression in sites of active hematopoiesis such as fetal liver and spleen suggest that this gene product may play critical roles in the proliferation, differentiation, and/or survival of several hematopoietic lineages, including hematopoietic stem cells.

Expression in the vasculature indicates possible roles in vascular development, particularly angiogenesis. Thus, this gene product could be useful in manipulating the numbers of hematopoietic stem cells; in increasing specific blood cell lineages; in the regulation of angiogenesis; and in the coordination of immune responses. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:13 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1813 of SEQ ID NO:13, b is an integer of 15 to 1827, where both a and b correspond to the positions of nucleotide residues shown in SEQ ID NO:13, and where b is greater than or equal to a + 14.

### FEATURES OF PROTEIN ENCODED BY GENE NO: 4

In specific embodiments, polypeptides of the invention comprise the following amino acid sequence: HEVEEKFNSPLMQTEGDIQ (SEQ ID NO:148). Polynucleotides encoding these polypeptides are also encompassed by the invention.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, neutropenia, leukemia and other blood-related and immune disorders and diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 82 as residues: Arg-42 to Leu-47.

The tissue distribution in neutrophils indicates that polynucleotides and polypeptides corresponding to this gene are useful for the diagnosis and/or treatment of blood-related diseases such as leukemia and neutropeania. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis.

In addition, this gene product may have commercial utility in the expansion of stem cells and committed progenitors of various blood lineages, and in the differentiation and/or proliferation of various cell types. Expression of this gene product in neutrophils also strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:14 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 682 of SEQ ID NO:14, b is an integer of 15 to 696, where both a and b correspond to the positions of nucleotide residues shown in SEQ ID NO:14, and where b is greater than or equal to a + 14.

### FEATURES OF PROTEIN ENCODED BY GENE NO: 5

In specific embodiments, polypeptides of the invention comprise the following amino acid sequence:
INFSEMTLQELVHKAASCYMDRVAVCFDECNNQLPVYYTYKTVVNAASELS NFLLLHCDFQGIREIGLYCQPGIDLPSWILGILQVPAAYVPIEPDSPPSLSTHFM KKCNLKYILVEKKQINKFKSFHETLLNYDTFTVEHNDLVLFRLHWKNTEVNL MLNDGKEKYEKEKIKSISSEHVNEEKAEEHMDLRXKHCLAYVLHTSGTTGIP KIVRX PHKCIVPNIQHFRVLFDITQEDVLFLXSPLTFDPSVVEIFLALSSGASLLIVPTSV KLLPSKLASVLFSHHRVTVLQATPTLLRRFGSQLIKSTVLSATTSLRVLALGGE AFPSLTVLRSWRGEGNKTQIFNVYGITEVSSWATIXRIPEKTLNSTLKCELPXQ LGFPLLGTVVEVRDTNGFTIQEGSGQVFLGCFIFVDWEFFFQEK (SEQ ID NO:149), INFSEMTLQELVHKAASCYMDRVAVCFDECNNQLPVYYTYKTVV (SEQ ID NO:150),
NAASELSNFLLLHCDFQGIREIGLYCQPGIDLPSWILGILQVPAAYV (SEQ ID NO:151), PIEPDSPPSLSTHFMKKCNLKYILVEKKQINKFKSFHETLL NYDTF (SEQ ID NO:152), TVEHNDLVLFRLHWKNTEVNLMLNDGKEKYEKE KIKSISSEHVNEEK (SEQ ID NO:153), AEEHMDLRXKHCLAYVLHTSGTTGIPK IVRXPHKCIVPNIQHFRVL (SEQ ID NO:154), FDITQEDVLFLXSPLTFDPSVVE IFLALSSGASLLIVPTSVKLLPSKL (SEQ ID NO:155), ASVLFSHHRVTVLQATP TLLRRFGSQLIKSTVLSATTSLRVLALGG (SEQ ID NO:156), EAFPSLTVLRSW RGEGNKTQIFNVYGITEVSSWATIXRIPEKTLNST (SEQ ID NO:157), and/or LKCELPXQLGFPLLGTVVEVRDTNGFTIQEGSGQVFLGCFIFVDWEFFFQEK (SEQ ID NO:158). Polynucleotides encoding these polypeptides are also encompassed by the invention.

This gene is expressed primarily in T cells, most notably helper T cells, as well as in fetal liver/spleen.

Therefore, polynucleotides and polypeptides of the invention are useful as reagents for differential identification of the tissue(s) or cell type(s) present in a biological sample and for diagnosis of diseases and conditions which include, but are not limited to, T cell lymphoma, impaired immune function; autoimmunity; hematopoietic disorders; impaired immune surveillance; inflammation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, amniotic fluid, bile, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in T-cells and fetal liver/spleen tissue indicates that polynucleotides and polypeptides corresponding to this gene are useful for the diagnosis and/or treatment of disorders of the immune system. Elevated levels of expression of this gene product in T cell lineages indicates that it may play an active role in normal T cell function and in the regulation of the immune response. For example, this gene product may be involved in T cell activation, in the activation or control of differentiation of other hematopoietic cell lineages, in antigen recognition, or in T cell proliferation.

Similarly, expression of this gene product in active sites of hematopoiesis, such as fetal liver and spleen likewise suggest a role in the control of proliferation, differentiation, and survival of hematopoietic cell lineages, including the hematopoietic stem cell. Therefore, this gene product may have clinical utility in the control of hematopoietic cell lineages; in stem cell self renewal; in stem cell expansion and mobilization; in the treatment of immune dysfunction; in the correction of autoimmunity; in immune modulation; and in the control of inflammation. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:15 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1670 of SEQ ID NO:15, b is an integer of 15 to 1684, where both a and b correspond to the positions of nucleotide residues shown in SEQ ID NO:15, and where b is greater than or equal to a + 14.

### FEATURES OF PROTEIN ENCODED BY GENE NO: 6

The translation product of this gene shares sequence homology with the mouse 19.5 protein, which is thought to be important in the development of T-cells (See for example: W09116430). The 19.5 protein, or "Lov" protein, is thought to be useful for the regulation of T-cell development and tumorigenic phenotypes, and to block T-cell activation in autoimmune diseases. The 19.5 gene encoding this protein is also referred to as "Lov" (Lymphoid and Ovarian Cellular expression). It is inducible in SL 12.4 cells after co-cultivation on thymic epithelial monolayers. The Lov gene has been mapped to murine chromosome 16. The Lov gene product is developmentally regulated and plays a role in T cell development. The protein (32.981 kD) has four highly hydrophobic, potential transmembrane spanning regions. In specific embodiments, polypeptides of the invention comprise the following amino acid sequence: EAKAQFWLLHSYLFCHSSNVPDLLRPRMTNDSEGKMGFKHPKI (SEQ ID NO:159). Polynucleotides encoding these polypeptides are also encompassed by the invention.

This gene is expressed primarily in healing-groin wound, as well as vascular tissue and smooth muscle tissue.

Therefore, polynucleotides and polypeptides of the invention are useful as reagents for differential identification of the tissue(s) or cell type(s) present in a biological sample and for diagnosis of diseases and conditions which include, but are not limited to, infection, muscle repair, HIV, leukemia, vascular disorders or cancer. Similarly, polypeptides and antibodies directed to these polypeptides are useful in providing immunological probes for differential identification of the tissue(s) or cell type(s). For a number of disorders of the above tissues or cells, particularly of the vascular and immune systems, expression of this gene at significantly higher or lower levels may be routinely detected in certain tissues or cell types (e.g., vascular, reproductive, muscular, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 84 as residues: Cys-31 to Arg-36, Asp-81 to His-86, Asn-264 to Met-275.

The tissue distribution in healing groin wound, combined with the homology to mouse 19.5 protein indicate that the protein product of this gene is expected to share some activities with the 19.5 protein, and be useful for the treatment or diagnosis of diseases, particularly those related to the activation of T-cells, for example, which occurs frequently at the site of an infection or wound.

Furthermore, the tissue distribution in smooth muscle tissue indicates that the protein product of this gene is useful for the diagnosis and treatment of conditions and pathologies of the cardiovascular system, such as heart disease, restenosis, atherosclerosis, stoke, angina, thrombosis, and wound healing. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:16 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nuclebtide sequence described by the general formula of a-b, where a is any integer between 1 to 1509 of SEQ ID NO:16, b is an integer of 15 to 1523, where both a and b correspond to the positions of nucleotide residues shown in SEQ ID NO:16, and where b is greater than or equal to a + 14.

### FEATURES OF PROTEIN ENCODED BY GENE NO: 7

This gene is expressed primarily in lung and placenta.

Therefore, polynucleotides and polypeptides of the invention are useful as reagents for differential identification of the tissue(s) or cell type(s) present in a biological sample and for diagnosis of diseases and conditions which include, but are not limited to, respiratory or vascular disorders. Similarly, polypeptides and antibodies directed to these polypeptides are useful in providing immunological probes for differential identification of the tissue(s) or cell type(s). For a number of disorders of the above tissues or cells, particularly of the adult and fetal respiratory systems, expression of this gene at significantly higher or lower levels may be routinely detected in certain tissues or cell types (e.g., pulmonary, vascular, endothelial, and cancerous and wounded tissues) or bodily fluids (e.g., lymph, serum, plasma, pulmonary surfactant or sputum, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in placenta and lung tissues indicates that polynucleotides and polypeptides corresponding to this gene are useful for the diagnosis and/or treatment of certain respiratory disorders. Furthermore, the tissue distribution indicates that polynucleotides and polypeptides corresponding to this gene are useful for the detection and treatment of disorders associated with developing lungs, particularly in premature infants where the lungs are the last tissues to develop. The tissue distribution indicates that polynucleotides and polypeptides corresponding to this gene are useful for the diagnosis and intervention of lung tumors, since the gene may be involved in the regulation of cell division, particularly since it is expressed in fetal tissue.

Alternatively, the expression in placenta suggests the protein is useful in the detection, treatment, and/or prevention of vascular conditions, which include, but are not limited to, microvascular disease, vascular leak syndrome, aneurysm, stroke, atherosclerosis, arteriosclerosis, or embolism. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:17 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 587 of SEQ ID NO:17, b is an integer of 15 to 601, where both a and b correspond to the positions of nucleotide residues shown in SEQ ID NO:17, and where b is greater than or equal to a + 14.

### FEATURES OF PROTEIN ENCODED BY GENE NO: 8

The gene encoding the disclosed cDNA is thought to reside on chromosome 2. Accordingly, polynucleotides related to this invention are useful as a marker in linkage analysis for chromosome 2.

This gene is expressed primarily in frontal cortex, amygdala, hypothalmus, and early stage human brain, and to a lesser extent in adrenal gland tumor.

Therefore, polynucleotides and polypeptides of the invention are useful as reagents for differential identification of the tissue(s) or cell type(s) present in a biological sample and for diagnosis of diseases and conditions which include, but are not limited to, neurodegenerative disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g., brain, cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in a wide variety of brain-specific tissues indicates that polynucleotides and polypeptides corresponding to this gene are useful for the diagnosis and/or treatment of neurodegenerative disorders. Furthermore, the tissue distribution in brain tissue indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered behaviors, including disorders in feeding, sleep patterns, balance, and perception. In addition, the gene or gene product may also play a role in the treatment and/or detection of developmental disorders associated with the developing embryo, or sexually-linked disorders.

Elevated expression of this gene product within the frontal cortex of the brain indicates that it may be involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:18 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 2595 of SEQ ID NO:18, b is an integer of 15 to 2609, where both a and b correspond to the positions of nucleotide residues shown in SEQ ID NO:18, and where b is greater than or equal to a + 14.

### FEATURES OF PROTEIN ENCODED BY GENE NO: 9

In specific embodiments, polypeptides of the invention comprise the following amino acid sequence: GTSGDGAKMISGHLLQEPTGSPVVSEEPLDLLPTLDLRQE (SEQ ID NO:160). Polynucleotides encoding these polypeptides are also encompassed by the invention. The translation product of this gene shares sequence homology with a human KIAA0668 protein (See Genbank Accession No. AB014568).

This gene is expressed primarily in osteoarthritis, and to a lesser extent in testes.

Therefore, polynucleotides and polypeptides of the invention are useful as reagents for differential identification of the tissue(s) or cell type(s) present in a biological sample and for diagnosis of diseases and conditions which include, but are not limited to, skeletal, endocrine, and/or reproductive disorders, particularly osteoarthritis and infertility. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues or cell types (e.g., skeletal, reproductive, endocrine, and cancerous and wounded tissues) or bodily fluids (e.g., lymph, serum, plasma, urine, seminal fluid, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 87 as residues: Leu-67 to Glu-73, Arg-83 to Gln-92, Leu-124 to Tyr-134, Gln-146 to Thr-157.

The tissue distribution in osteoarthritic tissue indicates that polynucleotides and polypeptides corresponding to this gene are useful for the diagnosis and/or treatment of osteoarthritis. In addition, the expression of this gene product suggests this protein may play a role in the detection and treatment of disorders and conditions affecting the skeletal system, in particular osteoporosis as well as disorders afflicting connective tissues (e.g., trauma, tendonitis, chrondomalacia and inflammation), such as in the diagnosis or treatment of various autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial arthritis, Atelosteogenesis type II, metaphyseal chondrodysplasia type Schmid). In addition, expression of this gene product in the testis may implicate this gene product in normal testicular function. In addition, this gene product may be useful in the treatment of male infertility, and/or could be used as a male contraceptive. Protein, as well as, antibodies directed against the protein may show utility as a tumor marker and/or immunotherapy targets for the above listed tissues.

Many polynucleotide sequences; such as EST sequences, are publicly available and accessible through sequence databases. Some of these sequences are related to SEQ ID NO:19 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1099 of SEQ ID NO:19, b is an integer of 15 to 1113, where both a and b correspond to the positions of nucleotide residues shown in SEQ ID NO:19, and where b is greater than or equal to a + 14.

### FEATURES OF PROTEIN ENCODED BY GENE NO: 10

This gene is expressed primarily in brain frontal cortex.

Therefore, polynucleotides and polypeptides of the invention are useful as reagents for differential identification of the tissue(s) or cell type(s) present in a biological sample and for diagnosis of diseases and conditions which include, but are not limited to, neurodegenerative disorders; learning disabilities; brain cancer and/or tumors. Similarly, polypeptides and antibodies directed to these polypeptides are useful in providing immunological probes for differential identification of the tissue(s) or cell type(s). For a number of disorders of the above tissues or cells, particularly of the brain or central nervous system, expression of this gene at significantly higher or lower levels may be routinely detected in certain tissues or cell types (e.g., neural, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 88 as residues: Arg-30 to Gly-42, Asp-58 to Ser-63.

The tissue distribution in frontal cortex tissue indicates that polynucleotides and polypeptides corresponding to this gene are useful for the diagnosis and/or treatment of a variety of neurodegenerative disorders. Expression of this gene product at elevated levels in brain frontal cortex indicates that it may play a role in normal neuronal function or in the support of brain activity. This could be effected in a number of ways, including neuronal survival; synapse formation; neurotransmission; neural conductance; proper neuronal pathfinding; etc. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:20 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 933 of SEQ ID NO:20, b is an integer of 15 to 947, where both a and b correspond to the positions of nucleotide residues shown in SEQ ID NO:20, and where b is greater than or equal to a + 14.

### FEATURES OF PROTEIN ENCODED BY GENE NO: 11

This gene is expressed primarily in brain frontal cortex.

Therefore, polynucleotides and polypeptides of the invention are useful as reagents for differential identification of the tissue(s) or cell type(s) present in a biological sample and for diagnosis of diseases and conditions which include, but are not limited to, neurodegenerative disorders; learning disabilities; vertigo; brain cancer and/or tumors. Similarly, polypeptides and antibodies directed to these polypeptides are useful in providing immunological probes for differential identification of the tissue(s) or cell type(s). For a number of disorders of the above tissues or cells, particularly of the brain and/or central nervous system, expression of this gene at significantly higher or lower levels may be routinely detected in certain tissues or cell types (e.g., neural, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 89 as residues: Ser-29 to Gly-37, Arg-39 to Pro-45.

The tissue distribution in frontal cortex tissue indicates that polynucleotides and polypeptides corresponding to this gene are useful for the diagnosis and/or treatment of a variety of neurodegenerative disorders. Expression of this gene product at elevated levels in the brain indicates that it may be involved in the maintenance of normal brain function. For example, it may play a role in a variety of processes including neuronal survival, synapse formation, neurotransmission; axon pathfinding, learning, conductance, etc. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:21 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1671 of SEQ ID NO:21, b is an integer of 15 to 1685, where both a and b correspond to the positions of nucleotide residues shown in SEQ ID NO:21, and where b is greater than or equal to a +14.

### FEATURES OF PROTEIN ENCODED BY GENE NO: 12

In specific embodiments, polypeptides of the invention comprise the following amino acid sequence:
LTTEEXCMLGSALCPFQGNFTIILYGRADEGIQPDPYYGLKYIGVGKGGALELH GXKKLSWTFLNKXLHPGGMAEGGYFFERSWGHRGVIVHVIDPKSGTVIHSDR FDTYRSXKESERLVQYLNAVPDGXILSVAVXDXGSRNLDDMARKAMTKLGSK HFLHLGFRHPWSFLTVKGNPSSSVEDHIEEYHGHRGSAAARVFKLFQTEHGEY XNVSLSSEWVQXVXWTXWFDHDKVSQTKGGEKISDLWKAHPGKICNRPIDIQ ATTMDGVNLSTEVVYKKXQDYRFACYDRGRACRSYRVRFLCGKPVRPKLTVT IDTNVNSTILNLEDNVQSWKPGDTLVIASTDYSMYQAEEFQVLPCRSCAPNQVK VAGKPMYLHIGGRRGRESRVDELTSRRP (SEQ ID NO:161), LTTEEXCMLGSA LCPFQGNFTIILYGRADEGIQPDPYYGLKYIG (SEQ ID NO:162), VGKGGALE LHGXKKLSWTFLNKXLHPGGMAEGGYFFERSWGH (SEQ ID NO:163), RGVI VHVIDPKSGTVIHSDRFDTYRSXKESERLVQYLNAVPDGXIL (SEQ ID NO:164), SVAVXDXGSRNLDDMARKAMTKLGSKHFLHLGFRHPWSFLT (SEQ ID NO:165), VKGNPSSSVEDHIEYHGHRGSAAARVFKLFQTEHGEYXNVSLSS (SEQ ID NO:166), EWVQXVXWTXWFDHDKVSQTKGGEKISDLWKAHPGKI CNRPID (SEQ ID NO:167), IQATTMDGVNLSTEWYKKXQDYRFACYDRGRAC RSYRVRFLC (SEQ ID NO:168), GKPVRPKLTVTIDTNVNSTILNLEDNVQSWK PGDTLVIASTDYSM (SEQ ID NO:169), and/or YQAEEFQVLPCRSCAPNQVK VAGKPMYLHIGGRRGRESRVDELTSRRP (SEQ ID NO:170). Polynucleotides encoding these polypeptides are also encompassed by the invention.

This gene is expressed primarily in endometrial stromal cells and osteoblasts.

Therefore, polynucleotides and polypeptides of the invention are useful as reagents for differential identification of the tissue(s) or cell type(s) present in a biological sample and for diagnosis of diseases and conditions which include, but are not limited to, skeletal, or reproductive disorders, particularly endometrial tumors, osteoblastoma, and/or arthritis. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues or cell types (e.g., skeletal, reproductive, and cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 90 as residues: Pro-37 to Asp-53.

The tissue distribution in endometrial tumor tissue and osteoblasts indicates that polynucleotides and polypeptides corresponding to this gene are useful for treating and/or diagnosing osteoblastoma and endometrial tumors. Furthermore, the tissue distribution indicates that polynucleotides and polypeptides corresponding to this gene are useful for the diagnosis and/or treatment of bone disorders. Elevated levels of expression of this gene product in osteoblastoma indicates that it may play a role in the survival, proliferation, and/or growth of osteoblasts. Therefore, it may be useful in influencing bone mass in such conditions as osteoporosis.

Alternatively, the tissue distribution in endometrial tumor tissue indicates that the translation product of this gene is useful for the diagnosis and/or treatment of endometrial tumors, as well as tumors of other tissues where expression has been observed. Furthermore, the tissue distribution indicates that polynucleotides and polypeptides corresponding to this gene are useful for treating female infertility. The protein product is likely involved in preparation of the endometrium of implantation and could be administered either topically or orally. Alternatively, this gene could be transfected in gene-replacement treatments into the cells of the endometrium and the protein products could be produced. Similarly, these treatments could be performed during artificial insemination for the purpose of increasing the likelyhood of implantation and development of a healthy embryo. In both cases this gene or its gene product could be administered at later stages of pregnancy to promote heathy development of the endometrium.

Moreover, the protein is useful in the detection, treatment, and/or prevention of vascular conditions, which include, but are not limited to, microvascular disease, vascular leak syndrome, aneurysm, stroke, atherosclerosis, arteriosclerosis, or embolism. Protein, as well as, antibodies directed against the protein may show utility as a tumor marker and/or immunotherapy targets for the above listed tissues. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:22 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1823 of SEQ ID NO:22, b is an integer of 15 to 1837, where both a and b correspond to the positions of nucleotide residues shown in SEQ ID NO:22, and where b is greater than or equal to a + 14.

### FEATURES OF PROTEIN ENCODED BY GENE NO: 13

In specific embodiments, polypeptides of the invention comprise the following amino acid sequence: GTRNGWVFFKQLLPQHFDIRYANL (SEQ ID NO:171). Polynucleotides encoding these polypeptides are also encompassed by the invention. The gene encoding the disclosed cDNA is thought to reside on chromosome 1. Accordingly, polynucleotides related to this invention are useful as a marker in linkage analysis for chromosome 1.

This gene is expressed primarily in chronic synovitis, and to a lesser extent in human whole six week old embryo.

Therefore, polynucleotides and polypeptides of the invention are useful as reagents for differential identification of the tissue(s) or cell type(s) present in a biological sample and for diagnosis of diseases and conditions which include, but are not limited to, chronic synovitis. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues or cell types (e.g., skeletal, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 91 as residues: Pro-57 to Trp-62.

The tissue distribution in chronic synovitis tissue indicates that polynucleotides and polypeptides corresponding to this gene are useful for the diagnosis and/or treatment of chronic synovitis. In addition, the expression of this gene product in synovial tissue indicates a role in the detection and treatment of disorders and conditions affecting the skeletal system, in particular osteoporosis as well as disorders afflicting connective tissues (e.g. arthritis, trauma, tendonitis, chrondomalacia and inflammation), such as in the diagnosis or treatment of various autoimmune disorders such as rheumatoid arthritis, lupus, scleroderma, and dermatomyositis as well as dwarfism, spinal deformation, and specific joint abnormalities as well as chondrodysplasias (ie. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:23 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1081 of SEQ ID NO:23, b is an integer of 15 to 1095, where both a and b correspond to the positions of nucleotide residues shown in SEQ ID NO:23, and where b is greater than or equal to a + 14.

### FEATURES OF PROTEIN ENCODED BY GENE NO: 14

This gene is expressed primarily in activated T-cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 92 as residues: Pro-32 to GIn-37.

The tissue distribution in T-cells indicates that polynucleotides and polypeptides corresponding to this gene are useful for the diagnosis and/or treatment of immune disorders involving activated T-cells. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis.

In addition, this gene product may have commercial utility in the expansion of stem cells and committed progenitors of various blood lineages, and in the differentiation and/or proliferation of various cell types. Expression of this gene product in T cells also strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:24 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1025 of SEQ ID NO:24, b is an integer of 15 to 1039, where both a and b correspond to the positions of nucleotide residues shown in SEQ ID NO:24, and where b is greater than or equal to a + 14.

### FEATURES OF PROTEIN ENCODED BY GENE NO: 15

This gene is expressed primarily in tissue from a 12 week old human.

Therefore, polynucleotides and polypeptides of the invention are useful as reagents for differential identification of the tissue(s) or cell type(s) present in a biological sample and for diagnosis of diseases and conditions which include, but are not limited to, developmental and congenital defects or conditions. Similarly, polypeptides and antibodies directed to these polypeptides are useful in providing immunological probes for differential identification of the tissue(s) or cell type(s). For a number of disorders of the above tissues or cells, particularly of the fetal systems, expression of this gene at significantly higher or lower levels may be routinely detected in certain tissues or cell types (e.g., developing, embryonic, cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 93 as residues: Tyr-48 to Ala-53.

The tissue distribution in embryonic tissue indicates that polynucleotides and polypeptides corresponding to this gene are useful for the diagnosis and/or treatment of developmental defects. Furthermore, expression within embryonic tissue and other cellular sources marked by proliferating cells indicates that this protein may play a role in the regulation of cellular division, and may show utility in the diagnosis and treatment of cancer and other proliferative disorders.

Similarly, embryonic development also involves decisions involving cell differentiation and/or apoptosis in pattern formation. Dysregulation of apoptosis can result in inappropriate suppression of cell death, as occurs in the development of some cancers, or in failure to control the extent of cell death, as is believed to occur in acquired immunodeficiency and certain neurodegenerative disorders, such as spinal muscular atrophy (SMA). Therefore, the polynucleotides and polypeptides of the present invention are useful in treating, detecting, and/or preventing said disorders and conditions, in addition to other types of degenerative conditions. Thus, this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:25 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1062 of SEQ ID NO:25, b is an integer of 15 to 1076, where both a and b correspond to the positions of nucleotide residues shown in SEQ ID NO:25, and where b is greater than or equal to a + 14.

### FEATURES OF PROTEIN ENCODED BY GENE NO: 16

In specific embodiments, polypeptides of the invention comprise the following amino acid sequence: GEVEAGQGKRRVSLGESTLGPPCRGTPSTLRPAAQQARR (SEQ ID NO:172). Polynucleotides encoding these polypeptides are also encompassed by the invention. The gene encoding the disclosed cDNA is thought to reside on chromosome 9. Accordingly, polynucleotides related to this invention are useful as a marker in linkage analysis for chromosome 9.

This gene is expressed primarily in fetal liver, and to a lesser extent in early infant brain.

Therefore, polynucleotides and polypeptides of the invention are useful as reagents for differential identification of the tissue(s) or cell type(s) present in a biological sample and for diagnosis of diseases and conditions which include, but are not limited to, hematopoietic disorders; impaired immune function; autoimmunity; neurodegenerative disorders; learning disabilities and/or developmental abnormalities. Similarly, polypeptides and antibodies directed to these polypeptides are useful in providing immunological probes for differential identification of the tissue(s) or cell type(s). For a number of disorders of the above tissues or cells, particularly of the brain, central nervous system, and/or immune system, expression of this gene at significantly higher or lower levels may be routinely detected in certain tissues or cell types (e.g., brain, neural, immune, developing, and cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 94 as residues: Val-55 to Lys-65.

The tissue distribution in brain and immune tissues indicates that polynucleotides and polypeptides corresponding to this gene are useful for the diagnosis and/or treatment of a variety of human disorders. Elevated expression of this gene product in fetal liver and infant brain suggest that it may play a role in the normal processes of hematopoiesis and brain function. In particular, expression in an active site of hematopoiesis such as the fetal liver indicates that this gene product may play a key role in the proliferation, differentiation, and survival of hematopoietic cell lineages, including the hematopoietic stem cell.

Likewise, expression in the infant brain indicates that this gene product may play a key role during the active phase of neural development, and may be involved in neuronal survival; axonal pathfinding; synapse formation; neurotransmission; and learning. The gene product may have important therapeutic uses therefore in regulation of immunity; manipulation of hematopoietic cell lineages; immune modulation; treatment of neurodegenerative disorders; and improvement of brain function. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:26 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 846 of SEQ ID NO:26, b is an integer of 15 to 860, where both a and b correspond to the positions of nucleotide residues shown in SEQ ID NO:26, and where b is greater than or equal to a + 14.

### FEATURES OF PROTEIN ENCODED BY GENE NO: 17

This gene is expressed primarily in adipose tissue.

Therefore, polynucleotides and polypeptides of the invention are useful as reagents for differential identification of the tissue(s) or cell type(s) present in a biological sample and for diagnosis of diseases and conditions which include, but are not limited to, metabolic disorders, particularly obesity. Similarly, polypeptides and antibodies directed to these polypeptides are useful in providing immunological probes for differential identification of the tissue(s) or cell type(s). For a number of disorders of the above tissues or cells, particularly of the metabolic system, expression of this gene at significantly higher or lower levels may be routinely detected in certain tissues or cell types (e.g., metabolic, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 95 as residues: Asp-45 to Ala-50.

The tissue distribution in adipose tissue indicates that polynucleotides and polypeptides corresponding to this gene are useful for the treatment of obesity and other metabolic and endocrine conditions or disorders. Furthermore, the protein product of this gene may show utility in ameliorating conditions which occur secondary to aberrant fatty-acid metabolism (e.g. aberrant myelin sheath development), either directly or indirectly. The protein is useful for the diagnosis, prevention, and/or treatment of various congenital metabolic disorders such as Tay-Sachs disease, phenylkenonuria, galactosemia, hyperlipidemias, porphyrias, and Hurler's syndrome. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:27 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 762 of SEQ ID NO:27, b is an integer of 15 to 776, where both a and b correspond to the positions of nucleotide residues shown in SEQ ID NO:27, and where b is greater than or equal to a + 14.

### FEATURES OF PROTEIN ENCODED BY GENE NO: 18

This gene is expressed primarily in bone marrow, and to a lesser extent in activated monocytes.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in bone marrow and monocytes indicates that polynucleotides and polypeptides corresponding to this gene are useful for the diagnosis and/or treatment of immune system disorders of stem cell origin. Furthermore, the tissue distribution indicates that polynucleotides and polypeptides corresponding to this gene are useful for the treatment and diagnosis of hematopoetic related disorders such as anemia, pancytopenia, leukopenia, thrombocytopenia or leukemia. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc.

In addition, this gene product may have commercial utility in the expansion of stem cells and committed progenitors of various blood lineages, and in the differentiation and/or proliferation of various cell types. This is particularly supported by the expression of this gene product in bone marrow, a primary sites of definitive hematopoiesis. Expression of this gene product in monocytes also strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:28 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b; where a is any integer between 1 to 1060 of SEQ ID NO:28, b is an integer of 15 to 1074, where both a and b correspond to the positions of nucleotide residues shown in SEQ ID NO:28, and where b is greater than or equal to a + 14.

### FEATURES OF PROTEIN ENCODED BY GENE NO: 19

The gene encoding the disclosed cDNA is thought to reside on chromosome 13. Accordingly, polynucleotides related to this invention are useful as a marker in linkage analysis for chromosome 13.

This gene is expressed primarily in placenta and breast tissue, and to a lesser extent in a variety of hematopoietic cells and tissues, including T cells, T cell lymphoma, and spleen.

Therefore, polynucleotides and polypeptides of the invention are useful as reagents for differential identification of the tissue(s) or cell type(s) present in a biological sample and for diagnosis of diseases and conditions which include, but are not limited to, vascular disease; breast cancer; T cell lymphoma; immune dysfunction; autoimmunity; hematopoietic disorders; and/or developmental abnormalities. Similarly, polypeptides and antibodies directed to these polypeptides are useful in providing immunological probes for differential identification of the tissue(s) or cell type(s). For a number of disorders of the above tissues or cells, particularly of the vasculature, circulatory system, and/or immune system, expression of this gene at significantly higher or lower levels may be routinely detected in certain tissues or cell types (e.g., immune, vascular, developmental, and cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in immune, breast and placental tissues indicates that polynucleotides and polypeptides corresponding to this gene are useful for the diagnosis and/or treatment of a variety of pathological conditions. Expression of this gene product at elevated levels in both endothelial cells and hematopoietic cells is consistent with the common ancestry of these two lineages, and indicates roles for the gene product in a variety of processes, including vasculogenesis; angiogenesis; survival, differentiation, and proliferation of blood cell lineages; and normal immune function and immune surveillance. In particular, expression of this gene product in T cell lymphoma indicates that it may play a role in the proliferation of the lymphoid cell lineages, and may be involved in normal antigen recognition and activation of T cells during the immune process.

Furthermore, the tissue distribution indicates that polynucleotides and polypeptides corresponding to this gene are useful for the diagnosis and/or treatment of disorders of the placenta. Specific expression within the placenta indicates that this gene product may play a role in the proper establishment and maintenance of placental function. Alternately, this gene product may be produced by the placenta and then transported to the embryo, where it may play a crucial role in the development and/or survival of the developing embryo or fetus.

Expression of this gene product in a vascular-rich tissue such as the placenta also indicates that this gene product may be produced more generally in endothelial cells or within the circulation. In such instances, it may play more generalized roles in vascular function, such as in angiogenesis. It may also be produced in the vasculature and have effects on other cells within the circulation, such as hematopoietic cells. It may serve to promote the proliferation, survival, activation, and/or differentiation of hematopoietic cells, as well as other cells throughout the body. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:29 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 2735 of SEQ ID NO:29, b is an integer of 15 to 2749, where both a and b correspond to the positions of nucleotide residues shown in SEQ ID NO:29, and where b is greater than or equal to a + 14.

### FEATURES OF PROTEIN ENCODED BY GENE NO: 20

This gene is expressed primarily in helper T cells.

Therefore, polynucleotides and polypeptides of the invention are useful as reagents for differential identification of the tissue(s) or cell type(s) present in a biological sample and for diagnosis of diseases and conditions which include, but are not limited to, immune dysfunction; impaired immune responses; autoimmunity; inflammation; allergy; T cell lymphoma, or other immune or hematopoietic disorders and conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 98 as residues: Ser-50 to Leu-56.

The tissue distribution in helper T-cells indicates that polynucleotides and polypeptides corresponding to this gene are useful for the diagnosis and/or treatment of a variety of disorders of the immune system. Elevated or specific expression of this gene product in T cells, notably helper T cells, indicates that it may play key roles in the regulation and coordination of immune responses. For example, it may be involved in the regulation of the activation state of T cells, or the activation/differentiation of other key hematopoietic lineages, including neutrophils, B cells, monocytes, and macrophages. Therefore, this gene product may have clinical relevance in the treatment of impaired immunity; in the correction of autoimmunity; in immune modulation; in the treatment of allergy; and in the regulation of inflammation. It may also play a role in influencing differentiation of specific hematopoietic lineages, and may even affect the hematopoietic stem cell. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:30 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 590 of SEQ ID NO:30, b is an integer of 15 to 604, where both a and b correspond to the positions of nucleotide residues shown in SEQ ID NO:30, and where b is greater than or equal to a + 14.

### FEATURES OF PROTEIN ENCODED BY GENE NO: 21

In specific embodiments, polypeptides of the invention comprise the following amino acid sequence: QSKTPDPVSKKKFPSSQGVVEAESV (SEQ ID NO:173). Polynucleotides encoding these polypeptides are also encompassed by the invention.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders and conditions, particularly allergy associated illnesses (e.g., rhinosinusitis to allogeneic from transplantation), acute inflammatory response, HIV, and ulcers. Similarly, polypeptides and antibodies directed to these polypeptides are useful in providing immunological probes for differential identification of the tissue(s) or cell type(s). For a number of disorders of the above tissues or cells, particularly of the hemo-lymphoid and/or immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 99 as residues: Cys-27 to Trp-42, Ser-76 to Ser-82.

The tissue distribution in neutrophils indicates that polynucleotides and polypeptides corresponding to this gene are useful for the treatment or diagnosis of tissue/bone rejection from transplantation, allergic responses to external stimuli and other immune system-related conditions. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Expression of this gene product in neutrophils also strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:31 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 734 of SEQ ID NO:31, b is an integer of 15 to 748, where both a and b correspond to the positions of nucleotide residues shown in SEQ ID NO:31, and where b is greater than or equal to a + 14.

### FEATURES OF PROTEIN ENCODED BY GENE NO: 22

This gene is expressed primarily, if not exclusively, in T-Cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders and/or conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The strong tissue distribution in T-cells indicates that polynucleotides and polypeptides corresponding to this gene are useful for the diagnosis and/or treatment of immune disorders involving T-cells. Furthermore, this gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis.

In addition, this gene product may have commercial utility in the expansion of stem cells and committed progenitors of various blood lineages, and in the differentiation and/or proliferation of various cell types. Expression of this gene product in T cells also strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:32 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 929 of SEQ ID NO:32, b is an integer of 15 to 943, where both a and b correspond to the positions of nucleotide residues shown in SEQ ID NO:32, and where b is greater than or equal to a + 14.

### FEATURES OF PROTEIN ENCODED BY GENE NO: 23

In specific embodiments, polypeptides of the invention comprise the following amino acid sequence:
CFCFLLPLLPSRWEPSRREGGEMIAELVSSALGLALYLNTLSADFCYDDSRAI KTNQDLLPETPWTHIFYNDFWGTLLTHSGSHKSYRPLCTLSFRLNHAIGGLNP WSYHLVNVLLHAAVTGLFTSFSKILLGDGYWTFMAGLMFASHPIHTEAVAGI VGRADVGASLFFLLSLLCYIKHCSTRGYSARTWGWFLGSGLCAGCSMLWKE QGVTVLAVSAVYDVFVFHRLKIKQILPTIYKRKNLSLFLSISLLIFWGSSLLGA RLYWMGNKPPSFSNSDNPAADSDSLLTRTLTFFYLPTKNLWLLLXPDTLSFEWS MDAVPLLKTVCDWRNLHTVGLLXWDSFSLA (SEQ ID NO:174), CFCFLLPLLPSR WEPSRREGGGEMIAELVSSALGLALYLNTLS (SEQ ID NO:175), ADFCYDDSR AIKTNQDLLPETPWTHIFYNDFWGTLLTHSGSHKS (SEQ ID NO:176), YRPLCLSFRLNHAIGGLNPWSYHLVNVLLHAAVTGLFTSFSK (SEQ ID NO:177), ILLGDGYWTFMAGLMFASHPIHTEAVAGIVGRADVGASLFFLLS (SEQ ID NO:178), LLCYIKHCSTRGYSARTWGWFLGSGLCAGCSMLWKEQGVTVLA (SEQ ID NO:179), VSAVYDVFVFHRLKIKQILPTIYKRKNLSLFLSISLLIFW GSSLLGA (SEQ ID NO:180), RLYWMGNKPPSFSNSDNPAADSDSLLTRTLTF FYLPTKNLWLL (SEQ ID NO:181), and/or LXPDTLSFEWSMDAVPLLKTVCD WRNLHTVGLLXWDSFSLA (SEQ ID NO:182). Polynucleotides encoding these polypeptides are also encompassed by the invention. The gene encoding the disclosed cDNA is thought to reside on chromosome 12. Accordingly, polynucleotides related to this invention are useful as a marker in linkage analysis for chromosome 12. The translation product of this gene shares sequence homology to TPR domains of C. elegans (See Genbank Accession No. gi|2291234).

This gene is expressed primarily in HL-60, and to a lesser extent in substantia nigra.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders and conditions, particularly promyelocytic leukemia. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 101 as residues: Glu-16 to Gly-34.

The tissue distribution in HL-60 cells, a promylocytic leukemia cell line, indicates that polynucleotides and polypeptides corresponding to this gene are useful for the diagnosis and/or treatment of promyelocytic leukemia. Furthermore, the tissue distribution indicates that polynucleotides and polypeptides corresponding to this gene are useful for the diagnosis and treatment of cancer and other proliferative disorders. Expression within embryonic tissue and other cellular sources marked by proliferating cells indicates that this protein may play a role in the regulation of cellular division.

Additionally, the expression in hematopoietic cells and tissues indicates that this protein may play a role in the proliferation, differentiation, and/or survival of hematopoietic cell lineages. In such an event, this gene may be useful in the treatment of lymphoproliferative disorders, and in the maintenance and differentiation of various hematopoietic lineages from early hematopoietic stem and committed progenitor cells. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:33 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1279 of SEQ ID NO:33, b is an integer of 15 to 1293, where both a and b correspond to the positions of nucleotide residues shown in SEQ ID NO:33, and where b is greater than or equal to a +14.

### FEATURES OF PROTEIN ENCODED BY GENE NO: 24

In specific embodiments, polypeptides of the invention comprise the following amino acid sequence: HNVFKVYSCCSKVRNCFSFKEKVS (SEQ ID NO:183). Polynucleotides encoding these polypeptides are also encompassed by the invention. When tested against U937 Myeloid cell lines, supernatants removed from cells containing this gene activated the GAS assay. Thus, it is likely that this gene activates myeloid cells, or more generally, immune or hematopoietic cells, in addition to other cells or cell-types, through the JAK-STAT signal transduction pathway. The gamma activating sequence (GAS) is a promoter element found upstream of many genes which are involved in the JAK-STAT pathway. The JAK-STAT pathway is a large, signal transduction pathway involved in the differentiation and proliferation of cells. Therefore, activation of the JAK-STAT pathway, reflected by the binding of the GAS element, can be used to indicate proteins involved in the proliferation and differentiation of cells.

This gene is expressed primarily in neutrophils, and to a lesser extent in T-cells.

Therefore, polynucleotides and polypeptides of the invention are useful as reagents for differential identification of the tissue(s) or cell type(s) present in a biological sample and for diagnosis of diseases and conditions which include, but are not limited to, a variety of immune system or hematopoietic disorders and conditions, including AIDS, impaired immune response, autoimmune disorders and various forms of tissue destruction. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 102 as residues: Asp-29 to Tyr-34.

The tissue distribution in neutrophils and T-cells, in conjunction with the biological activity data, indicates that polynucleotides and polypeptides corresponding to this gene are useful for the diagnosis and treatment of a variety of immune system disorders. Expression of this gene product in immune cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses).

Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Expression of this gene product in T cells and neutrophils also strongly indicates a role for this protein in immune function and immune surveillance. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:34 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1685 of SEQ ID NO:34, b is an integer of 15 to 1699, where both a and b correspond to the positions of nucleotide residues shown in SEQ ID NO:34, and where b is greater than or equal to a +14.

### FEATURES OF PROTEIN ENCODED BY GENE NO: 25

This gene is expressed primarily in smooth muscle.

Therefore, polynucleotides and polypeptides of the invention are useful as reagents for differential identification of the tissue(s) or cell type(s) present in a biological sample and for diagnosis of diseases and conditions which include, but are not limited to, various diseases of the gastrointestinal tract including hiatal hernia and inhereted susceptability to ulceretic disorders, as well as disorders of the vascular system. Similarly, polypeptides and antibodies directed to these polypeptides are useful in providing immunological probes for differential identification of the tissue(s) or cell type(s). For a number of disorders of the above tissues or cells, particularly of the gastrointestinal and vascular systems, expression of this gene at significantly higher or lower levels may be routinely detected in certain tissues or cell types (e.g., gastrointestinal, vascular, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 103 as residues: Lys-43 to Phe-48.

The tissue distribution in smooth muscle tissues indicates that polynucleotides and polypeptides corresponding to this gene are useful for the diagnosis, prevention, and/or treatment of various metabolic disorders such as Tay-Sachs disease, phenylkenonuria, galactosemia, porphyrias, and Hurler's syndrome. Furthermore, The tissue distribution in smooth muscle tissue indicates that the protein product of this gene is useful for the diagnosis and treatment of conditions and pathologies of the cardiovascular system, such as heart disease, restenosis, atherosclerosis, stoke, angina, thrombosis, and wound healing. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:3 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1806 of SEQ ID NO:35, b is an integer of 15 to 1820, where both a and b correspond to the positions of nucleotide residues shown in SEQ ID NO:35, and where b is greater than or equal to a + 14.

### FEATURES OF PROTEIN ENCODED BY GENE NO: 26

In specific embodiments, polypeptides of the invention comprise the following amino acid sequence: NCMHGKITPFQ (SEQ ID NO:184). Polynucleotides encoding these polypeptides are also encompassed by the invention.

This gene is expressed primarily in brain cells, and to a lesser extent in fetal liver.

Therefore, polynucleotides and polypeptides of the invention are useful as reagents for differential identification of the tissue(s) or cell type(s) present in a biological sample and for diagnosis of diseases and conditions which include, but are not limited to, neurological, immune, and/or hematopoietic disorders. Similarly, polypeptides and antibodies directed to these polypeptides are useful in providing immunological probes for differential identification of the tissue(s) or cell type(s). For a number of disorders of the above tissues or cells, particularly of the nervous and immune systems, expression of this gene at significantly higher or lower levels may be routinely detected in certain tissues or cell types (e.g., neural, immune, hematopoietic, and cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain tissues indicates that polynucleotides and polypeptides corresponding to this gene are useful for the treatment or diagnosis of diseases related to the brain and it's functions, such as depression, anxiety, attention deficite disorder, Huntington's disease, Alzheimer's disease, Parkinsons Disease, Tourette Syndrome, schizophrenia, mania, dementia, paranoia, obsessive compulsive disorder, panic disorder, learning disabilities, ALS, psychoses, autism, and altered behaviors, including disorders in feeding, sleep patterns, balance, and perception. In addition, the gene or gene product may also play a role in the treatment and/or detection of developmental disorders associated with the developing embryo, or sexually-linked disorders. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:36 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 2558 of SEQ ID NO:36, b is an integer of 15 to 2572, where both a and b correspond to the positions of nucleotide residues shown in SEQ ID NO:36, and where b is greater than or equal to a + 14.

### FEATURES OF PROTEIN ENCODED BY GENE NO: 27

This gene is expressed primarily in bone marrow stromal cells.

Therefore, polynucleotides and polypeptides of the invention are useful as reagents for differential identification of the tissue(s) or cell type(s) present in a biological sample and for diagnosis of diseases and conditions which include, but are not limited to, a variety of immune system or hematpoietic disorders and conditions, particularly immunodeficiencies, such as AIDS. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in stromal cells indicates that polynucleotides and polypeptides corresponding to this gene are useful for the treatment and diagnosis of hematopoietic related disorders such as anemia, pancytopenia, leukopenia, thrombocytopenia or leukemia, since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:37 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 690 of SEQ ID NO:37, b is an integer of 15 to 704, where both a and b correspond to the positions of nucleotide residues shown in SEQ ID NO:37, and where b is greater than or equal to a + 14.

### FEATURES OF PROTEIN ENCODED BY GENE NO: 28

This gene is expressed primarily in kidney medulla.

Therefore, polynucleotides and polypeptides of the invention are useful as reagents for differential identification of the tissue(s) or cell type(s) present in a biological sample and for diagnosis of diseases and conditions which include, but are not limited to, renal failure, kidney stones, medullary cystic kidney disease and other renal or urogenital disorders. Similarly, polypeptides and antibodies directed to these polypeptides are useful in providing immunological probes for differential identification of the tissue(s) or cell type(s). For a number of disorders of the above tissues or cells, particularly of the endocrine znd renal systems, expression of this gene at significantly higher or lower levels may be routinely detected in certain tissues or cell types (e.g., renal, urogenital, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 106 as residues: Glu-30 to Ala-35.

The tissue distribution in kidney tissue indicates that polynucleotides and polypeptides corresponding to this gene are useful for the treatment and/or diagnois of renal failure, medullary cystic kidney disease, nephritus, renal tubular acidosis, proteinuria, pyuria, edema, pyelonephritis, hydronephritis, nephrotic syndrome, crush syndrome, glomerulonephritis, hematuria, renal colic and kidney stones, in addition to Wilms Tumor Disease, and congenital kidney abnormalities such as horseshoe kidney, polycystic kidney, and Falconi's syndrome. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:38 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucledtide sequence described by the general formula of a-b, where a is any integer between 1 to 423 of SEQ ID NO:38, b is an integer of 15 to 437, where both a and b correspond to the positions of nucleotide residues shown in SEQ ID NO:38, and where b is greater than or equal to a + 14.

### FEATURES OF PROTEIN ENCODED BY GENE NO: 29

The translation product of this gene shares sequence homology with human chromosome 16p13.1 BAC gene CIT987SK-388D4 who's function has not been determined (See Geribank Accession No.: gb|U95737). Polynucleotides of the invention may exclude those consisting of the full-length nucleic acid sequence described in gb|U95737.

This gene is expressed primarily in kidney medulla.

Therefore, polynucleotides and polypeptides of the invention are useful as reagents for differential identification of the tissue(s) or cell type(s) present in a biological sample and for diagnosis of diseases and conditions which include, but are not limited to, kidney disease. Similarly, polypeptides and antibodies directed to these polypeptides are useful in providing immunological probes for differential identification of the tissue(s) or cell type(s). For a number of disorders of the above tissues or cells, particularly of the renal system, expression of this gene at significantly higher or lower levels may be routinely detected in certain tissues or cell types (e.g., renal, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in kidney indicates that polynucleotides and polypeptides corresponding to this gene are useful for the treatment and diagnois of diseases of the kidney, possibly before the onset of symptoms. Furthermore, the tissue distribution in kidney indicates that this gene or gene product is useful in the treatment and/or detection of kidney diseases including renal failure, nephritus, renal tubular acidosis, proteinuria, pyuria, edema, pyelonephritis, hydronephritis, nephrotic syndrome, crush syndrome, glomerulonephritis, hematuria, renal colic and kidney stones, in addition to Wilms Tumor Disease, and congenital kidney abnormalities such as horseshoe kidney, polycystic kidney, and Falconi's syndrome. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:39 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 929 of SEQ ID NO:39, b is an integer of 15 to 943, where both a and b correspond to the positions of nucleotide residues shown in SEQ ID NO:39, and where b is greater than or equal to a + 14.

### FEATURES OF PROTEIN ENCODED BY GENE NO: 30

The translation product of this gene shares sequence homology with rat carnitine/acylcarnitine carrier protein, which is thought to be important in metabolic transport in the inner membrane of the mitochondria (See Genbank Accession No. e290677). Based on the sequence similarity, the translation product of this clone is expected to share biological activities with fatty-acid metabolism proteins. Such activities are known in the art and described elsewhere herein.

This gene is expressed primarily in t-cells, and to a lesser extent in endothelial cells.

Therefore, polynucleotides and polypeptides of the invention are useful as reagents for differential identification of the tissue(s) or cell type(s) present in a biological sample and for diagnosis of diseases and conditions which include, but are not limited to, metabolic, immune, and/or hematopoietic disorders, particularly leukemia, HIV and hemophilia. Similarly, polypeptides and antibodies directed to these polypeptides are useful in providing immunological probes for differential identification of the tissue(s) or cell type(s). For a number of disorders of the above tissues or cells, particularly of the immune and vascular systems, expression of this gene at significantly higher or lower levels may be routinely detected in certain tissues or cell types (e.g., immune, hematopoietic, vascular, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 108 as residues: Lys-23 to Asp-32, Ser-69 to Gly-77, Pro-125 to Val-130, Pro-167 to Gly-174.

The tissue distribution in T-cells and endothelial cells, and homology to carnitine/acylcarnitine carrier protein, indicates that the protein product of this gene shares activities with carnitine/acylcarnitine carrier protein, and is useful for the treatment or diagnosis of diseases that effect the transport of proteins to and from the mitochondria, and is useful for the diagnosis, prevention, and/or treatment of various metabolic disorders which include, but are not limited to, Tay-Sachs disease, phenylkenonuria, galactosemia, hyperlipidemias, porphyrias, and Hurler's syndrome. Protein may also be useful in the detection, treatment, and/or prevention of developmental or neural disorders, which occur secondary to aberrant fatty-acid metabolism. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:40 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1861 of SEQ ID NO:40, b is an integer of 15 to 1875, where both a and b correspond to the positions of nucleotide residues shown in SEQ ID NO:40, and where b is greater than or equal to a + 14.

### FEATURES OF PROTEIN ENCODED BY GENE NO: 31

This gene is expressed primarily in rhabdomyosarcoma.

Therefore, polynucleotides and polypeptides of the invention are useful as reagents for differential identification of the tissue(s) or cell type(s) present in a biological sample and for diagnosis of diseases and conditions which include, but are not limited to, muscular, or proliferative diseases and conditions, particularly rhabdomyosarcoma. Similarly, polypeptides and antibodies directed to these polypeptides are useful in providing immunological probes for differential identification of the tissue(s) or cell type(s). For a number of disorders of the above tissues or cells, particularly of the muscular system, expression of this gene at significantly higher or lower levels may be routinely detected in certain tissues or cell types (e.g., muscular, fibroid, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 109 as residues: Phe-8 to Phe-13.

The tissue distribution in rhabdomyosarcoma tissue indicates that polynucleotides and polypeptides corresponding to this gene are useful for the diagnosis and/or treatment of rhabdomyosarcoma, in addition to degenerative neuromuscular and muscular disorders and diseases, such as MS. Furthermore, the expression in rhabdomyosarcoma indicates that polynucleotides and polypeptides corresponding to this gene are useful for the detection, treatment, and/or prevention of various muscle disorders, such as muscular dystrophy, cardiomyopathy, fibroids, myomas, and rhabdomyosarcomas. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:41 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 476 of SEQ ID NO:41, b is an integer of 15 to 490, where both a and b correspond to the positions of nucleotide residues shown in SEQ ID NO:41, and where b is greater than or equal to a + 14.

### FEATURES OF PROTEIN ENCODED BY GENE NO: 32

The gene encoding the disclosed cDNA is thought to reside on chromosome 4. Accordingly, polynucleotides related to this invention are useful as a marker in linkage analysis for chromosome 4.

This gene is expressed primarily in lymphocytes.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders and conditions, such as Hodgkin's lymphoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in lymphocytes indicates that polynucleotides and polypeptides corresponding to this gene are useful for the diagnosis and/or treatment of Hodgkin's lymphoma, as well as cancers of other tissues where expression has been observed. This gene product may be involved in the regulation of cytokine production, antigen presentation, or other processes that may also suggest a usefulness in the treatment of cancer (e.g. by boosting immune responses).

Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:42 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 772 of SEQ ID NO:42, b is an integer of 15 to 786, where both a and b correspond to the positions of nucleotide residues shown in SEQ ID NO:42, and where b is greater than or equal to a + 14.

### FEATURES OF PROTEIN ENCODED BY GENE NO: 33

In specific embodiments, polypeptides of the invention comprise the following amino acid sequence: EQIPKKVQKSLQETIQSLKLTNQELLRKGSSNNQDWSCD (SEQ ID NO:185). Polynucleotides encoding these polypeptides are also encompassed by the invention. The gene encoding the disclosed cDNA is thought to reside on chromosome 2. Accordingly, polynucleotides related to this invention are useful as a marker in linkage analysis for chromosome 2.

This gene is expressed primarily in spleen, prostate, intestine, ovarian and endometrial tumors, breast cancer and placental tissue.

Therefore, polynucleotides and polypeptides of the invention are useful as reagents for differential identification of the tissue(s) or cell type(s) present in a biological sample and for diagnosis of diseases and conditions which include, but are not limited to, Crohn's disease and cancers of the female reproductive system. Similarly, polypeptides and antibodies directed to these polypeptides are useful in providing immunological probes for differential identification of the tissue(s) or cell type(s). For a number of disorders of the above tissues or cells, particularly of the digestive and female reproductive systems, expression of this gene at significantly higher or lower levels may be routinely detected in certain tissues or cell types (e.g., gastrointestinal, reproductive, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 111 as residues: Asp-35 to Ser-41, Ser-69 to Gly-74.

The tissue distribution in intestinal tissue indicates that polynucleotides and polypeptides corresponding to this gene are useful for the diagnosis and/or treatment of Crohn's disease. Furthermore, the tissue distribution in cancerous tissues of the female reproductive system, such as ovaries, endometrium, and breast tissues, indicates that the translation product of this gene is useful for the detection and/or treatment of disorders and cancers of the female reproductive system, as well as cancers of other tissues where expression has been observed. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:43 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1662 of SEQ ID NO:43, b is an integer of 15 to 1676, where both a and b correspond to the positions of nucleotide residues shown in SEQ ID NO:43, and where b is greater than or equal to a + 14.

### FEATURES OF PROTEIN ENCODED BY GENE NO: 34

In specific embodiments, polypeptides of the invention comprise the following amino acid sequence: GTSFCSHLPSQRPLHLSGSSCLV (SEQ ID NO:186). Polynucleotides encoding these polypeptides are also encompassed by the invention. The gene encoding the disclosed cDNA is thought to reside on chromosome 22. Accordingly, polynucleotides related to this invention are useful as a marker in linkage analysis for chromosome 22.

This gene is expressed primarily in brain tissue and in T cells.

Therefore, polynucleotides and polypeptides of the invention are useful as reagents for differential identification of the tissue(s) or cell type(s) present in a biological sample and for diagnosis of diseases and conditions which include, but are not limited to, neurodegenerative and immune disorders. Similarly, polypeptides and antibodies directed to these polypeptides are useful in providing immunological probes for differential identification of the tissue(s) or cell type(s). For a number of disorders of the above tissues or cells, particularly of the central nervous and immune systems, expression of this gene at significantly higher or lower levels may be routinely detected in certain tissues or cell types (e.g., brain, immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain tissue and T-cells indicates that polynucleotides and polypeptides corresponding to this gene are useful for the diagnosis and/or treatment of neural and immune system disorders. This gene product may be involved in the regulation of cytokine production, antigen presentation, or other processes that may also suggest a usefulness in the treatment of cancer (e.g., by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis.

In addition, this gene product may have commercial utility in the expansion of stem cells and committed progenitors of various blood lineages, and in the differentiation and/or proliferation of various cell types. Alternatively, polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered behaviors, including disorders in feeding, sleep patterns, balance, and perception. In addition, the gene or gene product may also play a role in the treatment and/or detection of developmental disorders associated with the developing embryo, or sexually-linked disorders. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:44 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 752 of SEQ ID NO:44, b is an integer of 15 to 766, where both a and b correspond to the positions of nucleotide residues shown in SEQ ID NO:44, and where b is greater than or equal to a + 14.

### FEATURES OF PROTEIN ENCODED BY GENE NO: 35

This gene is expressed primarily in fetal tissues including brain, and to a lesser extent in retina, hepatocellular tumors, stromal cells, T cell helper II cells, adipose tissue, placenta and hypothalamus.

Therefore, polynucleotides and polypeptides of the invention are useful as reagents for differential identification of the tissue(s) or cell type(s) present in a biological sample and for diagnosis of diseases and conditions which include, but are not limited to, tumors, particularly of the liver. Similarly, polypeptides and antibodies directed to these polypeptides are useful in providing immunological probes for differential identification of the tissue(s) or cell type(s). For a number of disorders of the above tissues or cells, particularly of the hepatic system, expression of this gene at significantly higher or lower levels may be routinely detected in certain tissues or cell types (e.g., liver, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 113 as residues: Thr-26 to Met-33.

The tissue distribution in hepatocellular tumor tissue indicates that polynucleotides and polypeptides corresponding to this gene are useful for treating and/or diagnosing tumors, particularly those of the liver, and those containing poorly differentiated cell types, as well as cancers of other tissues where expression has been observed. Protein, as well as, antibodies directed against the protein may show utility as a tumor marker and/or immunotherapy targets for the above listed tissues.

Many polynucleotide sequences, such as EST sequences, are publicly available and accessible through sequence databases: Some of these sequences are related to SEQ ID NO:45 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1007 of SEQ ID NO:45, b is an integer of 15 to 1021, where both a and b correspond to the positions of nucleotide residues shown in SEQ ID NO:45, and where b is greater than or equal to a + 14.

### FEATURES OF PROTEIN ENCODED BY GENE NO:36

This gene is expressed primarily in brain frontal cortex tissue.

Therefore, polynucleotides and polypeptides of the invention are useful as reagents for differential identification of the tissue(s) or cell type(s) present in a biological sample and for diagnosis of diseases and conditions which include, but are not limited to, neurodegenerative disorders and other disorders of the central nervous system. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g., brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 114 as residues: His-55 to His-67.

The tissue distribution in frontal cortex tissue indicates that polynucleotides and polypeptides corresponding to this gene are useful for the diagnosis and/or treatment of brain disorders. Elevated expression of this gene product within the frontal cortex of the brain indicates that it may be involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:46 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1859 of SEQ ID NO:46, b is an integer of 15 to 1873, where both a and b correspond to the positions of nucleotide residues shown in SEQ ID NO:46, and where b is greater than or equal to a + 14.

### FEATURES OF PROTEIN ENCODED BY GENE NO: 37

In specific embodiments, polypeptides of the invention comprise the following amino acid sequence: FCIQVPGFVSCWYASPDRPSCIHVTRLYLLGLSQILASYS SSCPNSILSLRNGGKILR (SEQ ID NO:187). Polynucleotides encoding these polypeptides are also encompassed by the invention. When tested against K562 leukemia cell lines, supernatants removed from cells containing this gene activated the ISRE assay. Thus, it is likely that this gene activates leukemia cells, or more generally, immune or hematopoietic cells, in addition to other cells or cell types, through the JAK-STAT signal transduction pathway. The interferon-sensitive response element is a promoter element found upstream of many genes which are involved in the JAK-STAT pathway. The JAK-STAT pathway is a large, signal transduction pathway involved in the differentiation and proliferation of cells. Therefore, activation of the JAK-STAT pathway, reflected by the binding of the ISRE element, can be used to indicate proteins involved in the proliferation and differentiation of cells.

This gene is expressed primarily in bone marrow stromal cells and endothelial cells, and to a lesser extent in osteosarcoma, synovial cells, breast, kidney, fibroblasts, adipocytes, and whole brain tissue.

Therefore, polynucleotides and polypeptides of the invention are useful as reagents for differential identification of the tissue(s) or cell type(s) present in a biological sample and for diagnosis of diseases and conditions which include, but are not limited to, diseases of the bone and joints including arthritis, osteoporosis, and tumors such as osteosarcoma, and immune disorders. Similarly, polypeptides and antibodies directed to these polypeptides are useful in providing immunological probes for differential identification of the tissue(s) or cell type(s). For a number of disorders of the above tissues or cells, particularly of the skeletal and immune systems, expression of this gene at significantly higher or lower levels may be routinely detected in certain tissues or cell types (e.g., skeletal, immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 115 as residues: Thr-36 to Leu-41.

The tissue distribution in bone marrow stromal cells indicates that polynucleotides and polypeptides corresponding to this gene are useful for treating diseases of the skeletal system including osteosarcoma, arthritis, osteoporosis and osteopetrosis. Furthermore, the tissue distribution indicates that polynucleotides and polypeptides corresponding to this gene are useful for the treatment and diagnosis of hematopoietic related disorders such as anemia, pancytopenia, leukopenia, thrombocytopenia or leukemia, since stromal cells are important in the production of cells of hematopoietic lineages. The uses include bone marrow cell ex vivo culture, bone marrow transplantation, bone marrow reconstitution, radiotherapy or chemotherapy of neoplasia.

The gene product may also be involved in lymphopoiesis, and therefore it can be used in immune disorders such as infection, inflammation, allergy, immunodeficiency, etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:47 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 607 of SEQ ID NO:47, b is an integer of 15 to 621, where both a and b correspond to the positions of nucleotide residues shown in SEQ ID NO:47, and where b is greater than or equal to a + 14.

### FEATURES OF PROTEIN ENCODED BY GENE NO: 38

In specific embodiments, polypeptides of the invention comprise the following amino acid sequence: PRVRSAARLPRTLRPSRTSAPAGPCVPRLAPLTPSRPGRA (SEQ ID NO:188). Polynucleotides encoding these polypeptides are also encompassed by the invention. The gene encoding the disclosed cDNA is thought to reside on chromosome 11. Accordingly, polynucleotides related to this invention are useful as a marker in linkage analysis for chromosome 11.

This gene is expressed primarily in rhabdomyosarcoma, placental tissue, and a Soares fetal liver/spleen cDNA library.

Therefore, polynucleotides and polypeptides of the invention are useful as reagents for differential identification of the tissue(s) or cell type(s) present in a biological sample and for diagnosis of diseases and conditions which include, but are not limited to, Rhabdomyosarcoma, vascular and placental disorders. Similarly, polypeptides and antibodies directed to these polypeptides are useful in providing immunological probes for differential identification of the tissue(s) or cell type(s). For a number of disorders of the above tissues or cells, particularly of the muscular and immune systems, as well as placenta, expression of this gene at significantly higher or lower levels may be routinely detected in certain tissues or cell types (e.g., placental, muscle, immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 116 as residues: Arg-94 to Leu-99, Glu-101 to Lys-107, Pro-117 to He-125, Arg-141 to Gly-150, Pro-166 to Pro-178.

The tissue distribution in rhabdomyosarcoma tissue indicates that polynucleotides and polypeptides corresponding to this gene are useful for the diagnosis of Rhabdomyosarcoma, as well as cancers of other tissues where expression has been observed. Furthermore, the expression in rhabdomyosarcoma indicates that polynucleotides and polypeptides corresponding to this gene are useful for the detection, treatment, and/or prevention of various muscle disorders, such as muscular dystrophy, cardiomyopathy, fibroids, and myomas. The tissue distribution indicates that polynucleotides and polypeptides corresponding to this gene are useful for the diagnosis and/or treatment of disorders of the placenta. Specific expression within the placenta indicates that this gene product may play a role in the proper establishment and maintenance of placental function.

Alternately, this gene product may be produced by the placenta and then transported to the embryo, where it may play a crucial role in the development and/or survival of the developing embryo or fetus. Expression of this gene product in a vascular-rich tissue such as the placenta also indicates that this gene product may be produced more generally in endothelial cells or within the circulation. In such instances, it may play more generalized roles in vascular function, such as in angiogenesis. It may also be produced in the vasculature and have effects on other cells within the circulation, such as hematopoietic cells. It may serve to promote the proliferation, survival, activation, and/or differentiation of hematopoietic cells, as well as other cells throughout the body. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:48 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1276 of SEQ ID NO:48, b is an integer of 15 to 1290, where both a and b correspond to the positions of nucleotide residues shown in SEQ ID NO:48, and where b is greater than or equal to a + 14.

### FEATURES OF PROTEIN ENCODED BY GENE NO: 39

This gene is expressed primarily in brain tissue from a patient suffering from manic depression.

Therefore, polynucleotides and polypeptides of the invention are useful as reagents for differential identification of the tissue(s) or cell type(s) present in a biological sample and for diagnosis of diseases and conditions which include, but are not limited to, manic depression. Similarly, polypeptides and antibodies directed to these polypeptides are useful in providing immunological probes for differential identification of the tissue(s) or cell type(s). For a number of disorders of the above tissues or cells, particularly of the immune and central nervous systems, expression of this gene at significantly higher or lower levels may be routinely detected in certain tissues or cell types (e.g., brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain tissue from a patient suffering from manic depression indicates that polynucleotides and polypeptides corresponding to this gene are useful for the diagnosis and/or treatment of manic depression. Furthermore, the tissue distribution in brain tissue indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered behaviors, including disorders in feeding, sleep patterns, balance, and perception. In addition, the gene or gene product may also play a role in the treatment and/or detection of developmental disorders associated with the developing embryo, or sexually-linked disorders. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:49 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 2112 of SEQ ID NO:49, b is an integer of 15 to 2126, where both a and b correspond to the positions of nucleotide residues shown in SEQ ID NO:49, and where b is greater than or equal to a + 14.

### FEATURES OF PROTEIN ENCODED BY GENE NO: 40

The gene encoding the disclosed cDNA is thought to reside on chromosome 6. Accordingly, polynucleotides related to this invention are useful as a marker in linkage analysis for chromosome 6.

This gene is expressed primarily in hepatocellular carcinoma.

Therefore, polynucleotides and polypeptides of the invention are useful as reagents for differential identification of the tissue(s) or cell type(s) present in a biological sample and for diagnosis of diseases and conditions which include, but are not limited to, hepatocellular carcinoma. Similarly, polypeptides and antibodies directed to these polypeptides are useful in providing immunological probes for differential identification of the tissue(s) or cell type(s). For a number of disorders of the above tissues or cells, particularly of the hepatic system, expression of this gene at significantly higher or lower levels may be routinely detected in certain tissues or cell types (e.g., liver, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 118 as residues: Ala-66 to Gly-72, Ser-108 to Trp-114.

The tissue distribution in hepatocellular carcinoma tissue indicates that polynucleotides and polypeptides corresponding to this gene are useful for the diagnosis of hepatocellular carcinoma, as well as cancers of other tissues where expression has been observed. Furthermore, the tissue distribution indicates that polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). Protein, as well as, antibodies directed against the protein may show utility as a tumor marker and immunotherapy targets for the above listed tumors and tissues.

Many polynucleotide sequences, such as EST sequences, are publicly available and accessible through sequence databases. Some of these sequences are related to SEQ ID NO:50 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1349 of SEQ ID NO:50, b is an integer of 15 to 1363, where both a and b correspond to the positions of nucleotide residues shown in SEQ ID NO:50, and where b is greater than or equal to a + 14.

### FEATURES OF PROTEIN ENCODED BY GENE NO: 41

In specific embodiments, polypeptides of the invention comprise the following amino acid sequence:
SVLWGGSKGPWSWPRPRHRERLDFLSLCAEWLRWRPLSLTQQLKHTISGSN WLPHPLPCPLGSAENNGNANILIAANGTKRKAIAAEDPSLDFRNNPTKEDLGK LQPLVASYLCSDVTSVPSKESLKLQGVFSKQTVLKSHPLLSQSYELRAELLGR QPVLEFSLENLRTMNTSGQTALPQAPVNGLAKKLTKSSTHSDHDNSTSLNGG KRALTSSALHGGEMGGSESGDLKGGMXNCTLPHRSLDVEHTILYSNNSTANK SSVNSMEQPALQGSSRLSPGTDSSSNLGGVKLEGKKSPLSSILFSALDSDTRIT ALLRRQADXESRARRLQKRLQWQAKQVERHIQHQLGGFLEKTLSKLPNLESLRP RSQLMLTRKAEAALRKAASETTTSEGLSNFLKSNSISEELERFTASGIANLRCSEQ AFDSDVTDSSSGGESDIEEEELTRADPEQRHVPL (SEQ ID NO:189), SVLWGGSKG PWSWPRPRHRERLDFLSLCAEWLRWRPLSLTQQL (SEQ ID NO:190), KHTISG SNWLPHPLPCPLGSAENNGNANILIAANGTKRKAIAAED (SEQ ID NO:191), PSLDFRNNPTKEDLGKLQPLVASYLCSDVTSVPSKESLKLQGVFS (SEQ ID NO:192), KQTVLKSHPLLSQSYELRAELLGRQPVLEFSLENLRTMNTSGQTAL (SEQ ID NO:193), PQAPVNGLAKKLTKSSTHSDHDNSTSLNGGKRALTSSAL HGGEM (SEQ ID NO:194), GGSESGDLKGGMXNCTLPHRSLDVEHTILYSN NSTANKSSVNSME (SEQ ID NO:195), QPALQGSSRLSPGTDSSSNLGGVKLE GKKSPLSSILFSALDSDTRIT (SEQ ID NO:196), ALLRRQADXESRARRLQK RLQVVQAKQVERQHIQHLGGFLEKTLSKL (SEQ ID NO:197), PNLESLRPRSQ LMLTRKAEAALRKAASETTTSEGLSNFLKSNSISEE (SEQ ID NO:198), and/or LERFTASGIANLRCSEQAFDSDVTDSSSGGESDIEEEELTRADPEQRHVPL (SEQ ID NO:199). Polynucleotides encoding these polypeptides are also encompassed by the invention.

When tested against Jurkat T-cells and U937 Myeloid cell lines, supernatants removed from cells containing this gene activated the GAS assay. Thus, it is likely that this gene activates both T-cells and myeloid cells, and to a lesser extent other immune cells, in addition to other cells or cell-types, through the JAK-STAT signal transduction pathway. The gamma activating sequence (GAS) is a promoter element found upstream of many genes which are involved in the JAK-STAT pathway. The JAK-STAT pathway is a large, signal transduction pathway involved in the differentiation and proliferation of cells. Therefore, activation of the JAK-STAT pathway, reflected by the binding of the GAS element, can be used to indicate proteins involved in the proliferation and differentiation of cells.

This gene is expressed primarily in prostate cancer and Hodgkin's lymphoma tissues.

Therefore, polynucleotides and polypeptides of the invention are useful as reagents for differential identification of the tissue(s) or cell type(s) present in a biological sample and for diagnosis of diseases and conditions which include, but are not limited to, prostate cancer and Hodgkin's lymphoma. Similarly, polypeptides and antibodies directed to these polypeptides are useful in providing immunological probes for differential identification of the tissue(s) or cell type(s). For a number of disorders of the above tissues or cells, particularly of the gastrointestinal and immune systems, expression of this gene at significantly higher or lower levels may be routinely detected in certain tissues or cell types (e.g., gastrointestinal, immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 119 as residues: Asp-51 to His-56.

The tissue distribution in prostate cancer and Hodgkin's lymphoma, in conjunction with the biological activity data, indicates that polynucleotides and polypeptides corresponding to this gene are useful for diagnosis and treatment of prostate cancer and Hodgkin's lymphoma, as well as cancers of other tissues where expression has been observed. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:51 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 2384 of SEQ ID NO:51, b is an integer of 15 to 2398, where both a and b correspond to the positions of nucleotide residues shown in SEQ ID NO:51, and where b is greater than or equal to a + 14.

### FEATURES OF PROTEIN ENCODED BY GENE NO: 42

The gene encoding the disclosed cDNA is thought to reside on chromosome 2. Accordingly, polynucleotides related to this invention are useful as a marker in linkage analysis for chromosome 2.

This gene is expressed primarily in messangial cells.

Therefore, polynucleotides and polypeptides of the invention are useful as reagents for differential identification of the tissue(s) or cell type(s) present in a biological sample and for diagnosis of diseases and conditions which include, but are not limited to, brain diseases. Similarly, polypeptides and antibodies directed to these polypeptides are useful in providing immunological probes for differential identification of the tissue(s) or cell type(s). For a number of disorders of the above tissues or cells, particularly of the central nervous system; expression of this gene at significantly higher or lower levels may be routinely detected in certain tissues or cell types (e.g., brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in messangial cells indicates that polynucleotides and polypeptides corresponding to this gene are useful for the diagnosis and/or treatment of brain diseases. Furthermore, the tissue distribution indicates that polynucleotides and polypeptides corresponding to this gene are useful for the detection/treatment of neurodegenerative disease states and behavioural disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses, autism, and altered behaviors, including disorders in feeding, sleep patterns, balance, and perception. In addition, the gene or gene product may also play a role in the treatment and/or detection of developmental disorders associated with the developing embryo, or sexually-linked disorders. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:52 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 2220 of SEQ ID NO:52, b is an integer of 15 to 2234, where both a and b correspond to the positions of nucleotide residues shown in SEQ ID NO:52, and where b is greater than or equal to a + 14.

### FEATURES OF PROTEIN ENCODED BY GENE NO: 43

This gene is expressed primarily in CD34 depleted Buffy Coat (Cord Blood) blood cells.

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 121 as residues: Gln-17 to Arg-41.

The tissue distribution in CD34 depleted Buffy Coat (Cord Blood) blood cells indicates that polynucleotides and polypeptides corresponding to this gene are useful for the diagnosis and/or treatment of immune disorders. This gene product may be involved in the regulation of cytokine production, antigen presentation, or other processes that may also suggest a usefulness in the treatment of cancer (e.g. by boosting immune responses). Since the gene is expressed in cells of lymphoid origin, the gene or protein, as well as, antibodies directed against the protein may show utility as a tumor marker and/or immunotherapy targets for the above listed tissues. Therefore it may be also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:53 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 524 of SEQ ID NO:53, b is an integer of 15 to 538, where both a and b correspond to the positions of nucleotide residues shown in SEQ ID NO:53, and where b is greater than or equal to a + 14.

### FEATURES OF PROTEIN ENCODED BY GENE NO: 44

In specific embodiments, polypeptides of the invention comprise the following amino acid sequence: AKVVSWPSQETCGIRT (SEQ ID NO:200). Polynucleotides encoding these polypeptides are also encompassed by the invention. The gene encoding the disclosed cDNA is thought to reside on chromosome 2. Accordingly, polynucleotides related to this invention are useful as a marker in linkage analysis for chromosome 2.

This gene is expressed primarily in prostate cancer and spleen, as well as in lung, uterine and colon cancers.

Therefore, polynucleotides and polypeptides of the invention are useful as reagents for differential identification of the tissue(s) or cell type(s) present in a biological sample and for diagnosis of diseases and conditions which include, but are not limited to, prostate cancer, as well as other cancers. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., prostate, lung, colon, uterus, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 122 as residues: Ile-26 to Met-32, Pro-39 to Trp-44, Ser-46 to Glu-55.

The tissue distribution in cancerous tissues of the prostate, colon, lung, and uterus indicates that polynucleotides and polypeptides corresponding to this gene are useful for the diagnosis and/or treatment of prostate cancer, as well as colon cancer, lung cancer, and uterine cancer, as well as cancers of other tissues where expression has been observed. Protein, as well as, antibodies directed against the protein may show utility as a tumor marker and immunotherapy targets for the above listed tumors and tissues.

Many polynucleotide sequences, such as EST sequences, are publicly available and accessible through sequence databases. Some of these sequences are related to SEQ ID NO:54 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1470 of SEQ ID NO:54, b is an integer of 15 to 1484, where both a and b correspond to the positions of nucleotide residues shown in SEQ ID NO:54, and where b is greater than or equal to a + 14.

### FEATURES OF PROTEIN ENCODED BY GENE NO: 45

This gene shows sequence similarity to calmodulin-related polypeptides. Thus, the protein product of this gene is expected to have'activities normally associated with the calmodulin superfamily of genes and polypeptides. Moreover, the protein product of this gene also shares homology with the conserved troponin-C protein of Drosophila melanogaster (See Genbank Accession No. gi|429074), which is involved in the regulation of normal muscle function. In specific embodiments, polypeptides of the invention comprise the following amino acid sequence:
LPSGTFLKRSFRSLPELKDAVLDQYS (SEQ ID NO:201). Polynucleotides encoding these polypeptides are also encompassed by the invention.The gene encoding the disclosed cDNA is believed to reside on chromosome 10. Accordingly, polynucleotides related to this invention are useful as a marker in linkage analysis for chromosome 10.

This gene is expressed primarily in osteoclastoma and brain tissues.

Therefore, polynucleotides and polypeptides of the invention are useful as reagents for differential identification of the tissue(s) or cell type(s) present in a biological sample and for diagnosis of diseases and conditions which include, but are not limited to, neural or skeletal disorders, particularly osteoclastoma. Similarly, polypeptides and antibodies directed to these polypeptides are useful in providing immunological probes for differential identification of the tissue(s) or cell type(s). For a number of disorders of the above tissues or cells, particularly of the immune and central nervous system, expression of this gene at significantly higher or lower levels may be routinely detected in certain tissues or cell types (e.g., neural, skeletal, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 123 as residues: Asn-23 to Ser-32, Trp-61 to Ser-68, Ala-130 to Ala-135, Thr-141 to Gly-148, Asn-176 to Gly-182, Pro-197 to Glu-205, His-211 to Glu-222, Gln-242 to Ile-248, Thr-265 to Leu-271.

The tissue distribution in osteoclastoma tissue indicates that the protein product of this gene is useful for the diagnosis and/or treatment of osteoclastoma, as well as other skeletal disorders and conditions which include, but are not limited to, disorders afflicting connective tissues (e.g. arthritis, trauma, tendonitis, chrondomalacia and inflammation). Furthermore, the homology to calmodulin and troponin C indicates that this protein is useful for treating disease of the musculo-skeletal system and cardiac diseases such as arythmia. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:55 and may have been publicly available prior to conception of the present invention. Preferably, such related polynucleotides are specifically excluded from the scope of the present invention. To list,every related sequence would be cumbersome. Accordingly, preferably excluded from the present invention are one or more polynucleotides comprising a nucleotide sequence described by the general formula of a-b, where a is any integer between 1 to 1751 of SEQ ID NO:55, b is an integer of 15 to 1765, where both a and b correspond to the positions of nucleotide residues shown in SEQ ID NO:55, and where b is greater than or equal to a + 14.

### FEATURES OF PROTEIN ENCODED BY GENE NO: 46

The translation product of this gene shares sequence homology with disulfide isomerases (see e.g., Wong JM, et al., Gene. 1994 Dec 2; 150(1): 175-179. PMID: 7959048; UI: 95047534., which is hereby incorporated by reference, herein). Furthermore, the translation product of this gene contains a thioredoxin motif beginning at residue 48 which reads as follows: MIEFYAPWCPACQNLQPEW, which was determined by sequence homology to the Prosite motif PS00194. In specific embodiments, polypeptides of the invention comprise the following amino acid sequence: GTRRAEVGAATALPVRWASGE (SEQ ID NO:202). Polynucleotides encoding these polypeptides are also encompassed by the invention.

This gene is expressed primarily in T-cell and osteoclastoma, and to a lesser extent, in bone marrow tissue.

Therefore, polynucleotides and polypeptides of the invention are useful as reagents for differential identification of the tissue(s) or cell type(s) present in a biological sample and for diagnosis of diseases and conditions which include, but are not limited to, immune, hematopoietic, or skeletal disorders and conditions. Similarly, polypeptides and antibodies directed to these polypeptides are useful in providing immunological probes for differential identification of the tissue(s) or cell type(s). For a number of disorders of the above tissues or cells, particularly of the immune system and hematopoietic tissues, expression of this gene at significantly higher or lower levels may be routinely detected in certain tissues or cell types (e.g., immune, hematopoietic, skeletal, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 124 as residues: Thr-24 to Asn-30, Tyr-104 to Asp-122, Ser-128 to Ser-134, Pro-208 to Lys-222, Lys-233 to Pro-262.

The tissue distribution in T-cells and bone marrow cells, combined with the homology to thioredoxin and disulfide isomerase proteins, indicates that the protein product of this gene is useful for the diagnosis and treatment of different immune deficiency and hemopoietic diseases, particularly those related to deficient levels of thioredoxin activity. The protein product of this gene is useful for the treatment and diagnosis of hematopoietic related disorders such as anemia, pancytopenia, leukopenia, thrombocytopenia or leukemia since stromal cells are important in the production of cells of hematopoietic lineages. The uses include bone marrow cell ex-vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types.

Moreover, the protein is useful for detection and treatment of disorders and conditions affecting the skeletal system, in particular osteoporosis, bone cancer, as well as, disorders afflicting connective tissues (e.g. arthritis, trauma, tendonitis, chrondomalacia and inflammation), autoimmune disorders such as rheumatoid arthritis, lupus, scleroderma, and dermatomyositis as well as dwarfism, spinal deformation, and specific joint abnormalities as well as chondrodysplasias (i.e. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:56 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1464 of SEQ ID NO:56, b is an integer of 15 to 1478, where both a and b correspond to the positions of nucleotide residues shown in SEQ ID NO:56, and where b is greater than or equal to a + 14.

### FEATURES OF PROTEIN ENCODED BY GENE NO: 47

The protein product of this gene was found to have homology to the human epithelial V-like antigen precursor (See Genbank Accession No. gi|3169830 (AF030455), and J. Cell Biol. 141 (4), 1061-1071 (1998) which is hereby incorporated by reference herein), which is thought to play an integral role in regulating the earliest phases of thymus organogenesis. Epithelial V-like antigen (EVA) is a new member of the immunoglobulin superfamily, which is expressed in thymus epithelium and strongly down-regulated by thymocyte developmental progression.

This gene is expressed in the thymus and in several epithelial structures early in embryogenesis. EVA is highly homologous to the myelin protein zero and, in thymus-derived epithelial cell lines, is poorly soluble in nonionic detergents, strongly suggesting an association to the cytoskeleton. Its capacity to mediate cell adhesion through a homophilic interaction and its selective regulation by T-cell maturation might imply the participation of EVA in the earliest phases of thymus organogenesis. Moreover, the translation product of this gene shares sequence homology with glycoproteins of myelin. In specific embodiments, polypeptides of the invention comprise the following amino acid sequence: VTGTGEELNSNSSLWENAVLAPPGVALAGCWSPRSAPSGLWGQG WVSL (SEQ ID NO:203), SNSSLWENAVLAPPGVALAGCWSPRSAP (SEQ ID NO:204), IPFQPMSGRFKDRVSWDGNPERYDASILLWKLQFDDNGTYTCQ VKNPPDVDGVIGXIRLSWHTVRFSEIHFLALAIGSACALNIBIVIVVVLFQ HYRKKRWAERAHKWEIKSKEEERLNQEKKVSVYLEDTD (SEQ ID NO:205), RVSWDGNPERYDASILLWKLQFDDNGTYT (SEQ ID NO:206), PDVDGVIGXIR LSVVHTVRFSEIH (SEQ ID NO:207), and/or MIIIVIVVVLFQHYRKKRWAERA HKVVE (SEQ ID NO:208). Polynucleotides encoding these polypeptides are also encompassed by the invention.

This gene is expressed primarily in healing wound tissue, and to a lesser extent, in cancerous tissues.

Therefore, polynucleotides and polypeptides of the invention are useful as reagents for differential identification of the tissue(s) or cell type(s) present in a biological sample and for diagnosis of diseases and conditions which include, but are not limited to, integumentary, immune, or proliferative conditions, such as cancers. Similarly, polypeptides and antibodies directed to these polypeptides are useful in providing immunological probes for differential identification of the tissue(s) or cell type(s). For a number of disorders of the above tissues or cells, particularly integumentary and immune tissues, expression of this gene at significantly higher or lower levels may be routinely detected in certain tissues or cell types (e.g., integumentary, immune, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 125 as residues: Met-1 to Ser-6.

The tissue distribution in healing wound and cancerous tissues, combined with the homology to the EVA and myelin PO proteins, indicates that the protein product of this gene is useful for treating wounded tissues, as well as for the diagnosis of cancers. Moreover, the expression of this gene product indicates a role in regulating the proliferation; survival; differentiation; and/or activation of hematopoietic cell lineages, including blood stem cells.

This gene product may be involved in the regulation of cytokine production, antigen presentation, or other processes that may also suggest a usefulness in the treatment of cancer (e.g., by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues.

In addition, this gene product may have commercial utility in the expansion of stem cells and committed progenitors of various blood lineages, and in the differentiation and/or proliferation of various cell types. The protein is also useful for inhibiting the progression of proliferative cells and tissues. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:57 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1131 of SEQ ID NO:57, b is an integer of 15 to 1145, where both a and b correspond to the positions of nucleotide residues shown in SEQ ID NO:57, and where b is greater than or equal to a + 14.

### FEATURES OF PROTEIN ENCODED BY GENE NO: 48

The translation product of this gene shares sequence homology with murine TALLA, cell surface associated tetraspan glycoprotein. Tetraspans are expressed in a wide variety of species and regulate cell adhesion, migration, proliferation and differentiation. They can be used in the treatment of immune disorders, cancers, blood disorders, juvenile rheumatoid arthritis, Graves disease or immunocompromised disease states, for example. The products can also be used for detection and diagnosis of these diseases and disorders. In specific embodiments, polypeptides of the invention comprise the following amino acid sequence: PARGAPR (SEQ ID NO:209). Polynucleotides encoding these polypeptides are also encompassed by the invention.

This gene is expressed primarily in pregnant uterus, pancreas, primary dendritic cells, and to a lesser extent, in colon tissues.

Therefore, polynucleotides and polypeptides of the invention are useful as reagents for differential identification of the tissue(s) or cell type(s) present in a biological sample and for diagnosis of diseases and conditions which include, but are not limited to, developmental, immune, hematopoietic, gastrointestinal, or proliferative conditions, such as cancers. Similarly, polypeptides and antibodies directed to these polypeptides are useful in providing immunological probes for differential identification of the tissue(s) or cell type(s). For a number of disorders of the above tissues or cells, particularly of the immune, gastrointestinal, and developing systems, expression of this gene at significantly higher or lower levels may be routinely detected in certain tissues or cell types (e.g., integumentary, immune, developmental, and cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 126 as residues: Met-1 to Gln-8, Glu-48 to Leu-55; Arg-130 to Asp-138, Cys-155 to Ser-172.

The tissue distribution in uterine cells and tissues, combined with the homology to members of the tetraspan family of proteins, indicates that the protein product of this gene is useful in the detection, treatment, and/or prevention of a variety of developmental conditions and diseases, particularly metabolic disorders such as Tay-Sachs disease, phenylkenonuria, galactosemia, hyperlipidemias, porphyrias, and Hurler's syndrome. Alternatively, the protein is useful for the treatment, detection, and/or prevention of immune or hematopoietic disorders, such as leukemia. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:58 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1758 of SEQ ID NO:58, b is an integer of 15 to 1772, where both a and b correspond to the positions of nucleotide residues shown in SEQ ID NO:58, and where b is greater than or equal to a + 14.

### FEATURES OF PROTEIN ENCODED BY GENE NO: 49

In specific embodiments, polypeptides of the invention comprise the following amino acid sequence: ARVYFK (SEQ ID NO:210). Polynucleotides encoding these polypeptides are also encompassed by the invention. The gene encoding the disclosed cDNA is believed to reside on chromosome 2. Accordingly, polynucleotides related to this invention are useful as a marker in linkage analysis for chromosome 2.

This gene is expressed primarily in colon cancer and lamyx carcinoma.

Therefore, polynucleotides and polypeptides of the invention are useful as reagents for differential identification of the tissue(s) or cell type(s) present in a biological sample and for diagnosis of diseases and conditions which include, but are not limited to, integumentary or gastrointestinal disorders, particularly cancers of the digestive tract, epithelial and endothelial cells and tissues. Similarly, polypeptides and antibodies directed to these polypeptides are useful in providing immunological probes for differential identification of the tissue(s) or cell type(s). For a number of disorders of the above tissues or cells, particularly of the gastrointestinal system, expression of this gene at significantly higher or lower levels may be routinely detected in certain tissues or cell types (e.g., immune, hematopoietic, gastrointestinal, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 127 as residues: His-32 to Pro-37.

The tissue distribution in colon cancer and larnyx carcinoma indicates that the protein product of this gene is useful for diagnosing and/or treating cancers, particularly those of the digestive tract. Protein is useful in correcting or ameliorating ulcers of the gastrointestinal tract, including proliferative conditions of the larynx. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:59 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1265 of SEQ ID NO:59, b is an integer of 15 to 1279, where both a and b correspond to the positions of nucleotide residues shown in SEQ ID NO:59, and where b is greater than or equal to a + 14.

### FEATURES OF PROTEIN ENCODED BY GENE NO: 50

When tested against K562 cell lines, supernatants removed from cells containing this gene activated the ISRE (interferon-sensitive responsive element) promoter element. Thus, it is likely that this gene activates leukemia cells, or more generally immune or hematopoietic cells and tissues, in addition to other cells or cell-types, through the JAK-STAT signal transduction pathway. ISRE is a promoter element found upstream in many genes which are involved in the JAK-STAT pathway. The JAK-STAT pathway is a large, signal transduction pathway involved in the differentiation and proliferation of cells. Therefore, activation of the JAK-STAT pathway, reflected by the binding of the ISRE element, can be used to indicate proteins involved in the proliferation and differentiation of cells.

In specific embodiments, polypeptides of the invention comprise the following amino acid sequence: TKLFHDK (SEQ ID NO:211). Polynucleotides encoding these polypeptides are also encompassed by the invention. The gene encoding the disclosed cDNA is believed to reside on chromosome 3. Accordingly, polynucleotides related to this invention are useful as a marker in linkage analysis for chromosome 3.

This gene is expressed primarily in tissues of the central nervous system (CNS).

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly neurodegenerative conditions. Similarly, polypeptides and antibodies directed to these polypeptides are useful in providing immunological probes for differential identification of the tissue(s) or cell type(s). For a number of disorders of the above tissues or cells, particularly of the CNS, expression of this gene at significantly higher or lower levels may be routinely detected in certain tissues or cell types (e.g., neural, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in central nervous system cells and tissues, combined with the detected ISRE biological activity data, indicates that polynucleotides and polypeptides corresponding to this gene are useful for the detection, treatment, and/or prevention of neurodegenerative disease states, behavioral disorders, or inflammatory conditions which include, but are not limited to Alzheimeris Disease, Parkinsonis Disease, Huntingtonis Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, depression, panic disorder, learning disabilities, ALS, psychoses, autism, and altered behaviors, including disorders in feeding, sleep patterns, balance, and perception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Protein is useful in modulating the immune response, particularly for degenerative neural conditions, or autoimmune disorders. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:60 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1525 of SEQ ID NO:60, b is an integer of 15 to 1539, where both a and b correspond to the positions of nucleotide residues shown in SEQ ID NO:60, and where b is greater than or equal to a + 14.

### FEATURES OF PROTEIN ENCODED BY GENE NO: 51

The translation product of this gene shares sequence homology with IAP, and MIHC, which are intracellular inhibitors of apoptosis and are thought to be important in modulating the response of cells to apoptotic signals, thereby altering cell survival. The translation product of this gene also shares homology with the zinc finger, C3HC4 type protein (See Genbank Accession No. gnl|PID|e1297770), which could implicate this protein as serving a role in modulating gene expression, perhaps in the context of inhibiting apoptosis. In specific embodiments, polypeptides of the invention comprise the following amino acid sequence: PHIHPCWKEGDTVGFLLDLNEKQMIFFLNGN QLPPEKQVFSSTVSGFFAAASFMSYQQCEFNFGAKPFKYPPSMKFSTFNDYAF LTAEEKIILPR:HRRLALLKQVSIRENCCSLCCDEVADTQLKPCGHSDLCMDCAL QLETCPLCRKEIVSRIRQISHIS (SEQ ID NO:212), NEKQMIFFLNGNQLPPEKQ VFSSTVSGFFAA (SEQ ID NO:213), SYQQCEFNFGAKPFKYPPSMKFSTFND (SEQ ID NO:214), EEKIILPRHRRLALLKQVSIRENCCSLCC (SEQ ID NO:215), TQLKPCGHSDLCMDCALQLETCPLCRKEIV (SEQ ID NO:216), ALEKFAQT (SEQ ID NO:217), GFCAQW (SEQ ID NO:218), DVSEYLKI (SEQ ID NO:219), GLEARCD (SEQ ID NO:220), FESVRCTF (SEQ ID NO:221), GVWYYE (SEQ ID NO:222), TSGVMQIG (SEQ ID NO:223), FLNHEGYGIGDD (SEQ ID NO:224), and/or AYDGCRQ (SEQ ID NO:225). Polynucleotides encoding these polypeptides are also encompassed by the invention. The gene encoding the disclosed cDNA is believed to reside on chromosome 16. Accordingly, polynucleotides related to this invention are useful as a marker in linkage analysis for chromosome 16.

This gene is expressed primarily in serum treated smooth muscle, and to a lesser extent, in fetal liver, T-cells, endothelial cells, and various immune system related cells.

Therefore, polynucleotides and polypeptides of the invention are useful as reagents for differential identification of the tissue(s) or cell type(s) present in a biological sample and for diagnosis of diseases and conditions which include, but are not limited to, vascular, immune, or hematopoietic disorders and diseases, particularly conditions characterized by altered survival and migration of immune system cells, including tumors of the blood. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., vascular, immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 129 as residues: Asp-48 to Glu-64, Ala-71 to Val-100, Asp-116 to Tyr-122, Asp-191 to Thr-201, Ala-253 to Lys-259, Ser-276 to Arg-286, Asp-393 to Cys-398, Gly-421 to Gln-426.

The tissue distribution in vascular and immune cells, combined with the homology to inhibitors of apoptosis, indicates that the protein product of this gene is useful for diagnosing and/or treating disorders of the immune system resulting from hyperactivation or hyperproliferation of specific immune cells or their progenitors. Moreover, the protein in useful in treating and preventing disorders related to aberrant cellular proliferation and migration of immune cells, in addition to immune chemotaxis. Protein is also useful in inhibiting apoptosis of immune or hematopoietic cells, particularly for degenerative conditions. In addition, the protein is useful in the detection, treatment, and/or prevention of vascular conditions, which include, but are not limited to, microvascular disease, vascular leak syndrome, aneurysm, stroke, atherosclerosis, arteriosclerosis, or embolism. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:61 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1923 of SEQ ID NO:61, b is an integer of 15 to 1937, where both a and b correspond to the positions of nucleotide residues shown in SEQ ID NO:61, and where b is greater than or equal to a + 14.

### FEATURES OF PROTEIN ENCODED BY GENE NO: 52

In specific embodiments, polypeptides of the invention comprise the following amino acid sequence: HASADGGRTRGWTPT (SEQ ID NO:226). Polynucleotides encoding these polypeptides are also encompassed by the invention.

This gene is expressed primarily in merkel cell and teratocarcinoma, and to a lesser extent, in spleen metastic melanoma and eosinophils.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, particularly metastic tumors. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 130 as residues: Met-1 to Ala-7, Pro-28 to Glu-34, Phe-86 to Val-108, Glu-110 to Gln-118, His-131 to Pro-147, Leu-159 to Gln-166, Lys-172 to Thr-178, Arg-203 to Asp-211, Pro-222 to Glu-245, Thr-262 to Thr-271, Gly-278 to Thr-285, Cys-315 to His-322.

The tissue distribution in teratocarcinoma and spleen metastic melanoma cells indicates that the protein product of this gene is useful for the diagonosis and treatment of various tumors. Moreover, the expression within cellular sources marked by proliferating cells indicates this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:62 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1438 of SEQ ID NO:62, b is an integer of 15 to 1452, where both a and b correspond to the positions of nucleotide residues shown in SEQ ID NO:62, and where b is greater than or equal to a + 14.

### FEATURES OF PROTEIN ENCODED BY GENE NO: 53

In specific embodiments, polypeptides of the invention comprise the following amino acid sequence: AFDEGNKMELRKNTILIIYYISR (SEQ ID NO:227). Polynucleotides encoding these polypeptides are also encompassed by the invention.

This gene is expressed primarily in bone marrow stromal cells.

Therefore, polynucleotides and polypeptides of the invention are useful as reagents for differential identification of the tissue(s) or cell type(s) present in a biological sample and for diagnosis of diseases and conditions which include, but are not limited to, immune or hemopoietic disorders and diseases. Similarly, polypeptides and antibodies directed to these polypeptides are useful in providing immunological probes for differential identification of the tissue(s) or cell type(s). For a number of disorders of the above tissues or cells, particularly of the bone marrow, expression of this gene at significantly higher or lower levels may be routinely detected in certain tissues or cell types (e.g., immune, hemopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in bone marrow stromal cells indicates that the protein product of this gene is useful for the treatment or dignosis of hemopoietic diseases. Moreover, polynucleotides and polypeptides corresponding to this gene are useful for the treatment and diagnosis of hematopoietic related disorders such as anemia, pancytopenia, leukopenia, thrombocytopenia or leukemia since stromal cells are important in the production of cells of hematopoietic lineages. The uses include bone marrow cell ex- vivo culture, bone marrow transplantation, bone marrow reconstitution, radiotherapy or chemotherapy of neoplasia. The gene product may also be involved in lymphopoiesis, and therefore can be used in immune disorders such as infection, inflammation, allergy, immunodeficiency, etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:63 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 957 of SEQ ID NO:63, b is an integer of 15 to 971, where both a and b correspond to the positions of nucleotide residues shown in SEQ ID NO:63, and where b is greater than or equal to a + 14.

### FEATURES OF PROTEIN ENCODED BY GENE NO: 54

When tested against K562 cell lines, supernatants removed from cells containing this gene activated the ISRE (interferon-sensitive responsive element) promoter element. Thus, it is likely that this gene activates leukemia cells, or more generally, immune or hematopoietic cells, in addition to other cells or cell-types, through the JAK-STAT signal transduction pathway. ISRE is a promoter element found upstream in many genes which are involved in the JAK-STAT pathway. The JAK-STAT pathway is a large, signal transduction pathway involved in the differentiation and proliferation of cells. Therefore, activation of the JAK-STAT pathway, reflected by the binding of the ISRE element, can be used to indicate proteins involved in the proliferation and differentiation of cells. In specific embodiments, polypeptides of the invention comprise the following amino acid sequence: GTRWKLFQQRFLYRGNREFQNKKLS (SEQ ID NO:228). Polynucleotides encoding these polypeptides are also encompassed, by the invention. The gene encoding the disclosed cDNA is believed to reside on chromosome 8. Accordingly, polynucleotides related to this invention are useful as a marker in linkage analysis for chromosome 8.

This gene is expressed in fetal heart, fetal brain, and breast tissues.

Therefore, polynucleotides and polypeptides of the invention are useful as reagents for differential identification of the tissue(s) or cell type(s) present in a biological sample and for diagnosis of diseases and conditions which include, but are not limited to, developmental, vascular, neural, or reproductive disorders, particularly cancers of the breast and brain, and neurodegenerative conditions such as Alzheimer's disease and Parkinson's disease. Similarly, polypeptides and antibodies directed to these polypeptides are useful in providing immunological probes for differential identification of the tissue(s) or cell type(s). For a number of disorders of the above tissues or cells, particularly of the central nervous system, immune system, and reproductive system, expression of this gene at significantly higher or lower levels may be routinely detected in certain tissues or cell types (e.g., developmental, vascular, neural, reproductive, and cancerous and wounded tissues) or bodily fluids (e.g., lymph, serum, plasma, urine, amniotic fluid, breast milk, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fetal heart and brain tissues, combined with the detected ISRE biological activity data, indicates that the protein product of this gene is useful for the diagnosis and/or treatment of disorders (particularly tumors) affecting the brain, central nervous system and breast. Moreover, the expression within fetal tissue and other cellular sources marked by proliferating cells indicates this protein may play a role in the regulation of cellular division, and may show utility in the diagnosis and treatment of cancer and other proliferative disorders.

Similarly, developmental tissues rely on decisions involving cell differentiation and/or apoptosis in pattern formation. Thus this protein may also be involved in apoptosis or tissue differentiation and could again be useful in cancer therapy. In addition, polynucleotides and polypeptides corresponding to this gene are useful for the detection, treatment, and/or prevention of neurodegenerative disease states, behavioral disorders, or inflammatory conditions. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:64 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1709 of SEQ ID NO:64, b is an integer of 15 to 1723, where both a and b correspond to the positions of nucleotide residues shown in SEQ ID NO:64, and where b is greater than or equal to a + 14.

### FEATURES OF PROTEIN ENCODED BY GENE NO: 55

The translation product of this gene shares sequence homology with a DHHC-domain-containing cysteine-rich protein, which is thought to be involved in gene regulation, particularly during development. In specific embodiments, polypeptides of the invention comprise the following amino acid sequence: GTSAIPVFAA (SEQ ID NO:229), LDFILSSWLSTRQPMKDIKGSWTGKNRVQNPYSHGNIVKNCCE VLCGPLPPSVLDRRGILPLEESGSRPPSTQETSSSLLPQSPAPTEHLNSNEMPEDS ST PEEMPPPEPPEPPQEAAEAEK (SEQ ID NO:229), KGSWTGKNRVQNPYSHG NIVKNCCEVL (SEQ ID NO:231), DRRGILPLEESGSRPPSTQETSSSL (SEQ ID NO:232), and/or PEDSSTPEEMPPPEPPE (SEQ ID NO:233). Polynucleotides encoding these polypeptides are also encompassed by the invention. The gene encoding the disclosed cDNA is believed to reside on the X chromosome. Accordingly, polynucleotides related to this invention are useful as a marker in linkage analysis for the X chromosome.

This gene is expressed in the brain and prostate tissues.

Therefore, polynucleotides and polypeptides of the invention are useful as reagents for differential identification of the tissue(s) or cell type(s) present in a biological sample and for diagnosis of diseases and conditions which include, but are not limited to, neural or reproductive disorders and disease, in particular cancers of the brain and prostate. Similarly, polypeptides and antibodies directed to these polypeptides are useful in providing immunological probes for differential identification of the tissue(s) or cell type(s). For a number of disorders of the above tissues or cells, particularly of the central nervous system, immune system, and the reproductive system, expression of this gene at significantly higher or lower levels may be routinely detected in certain tissues or cell types (e.g., neural, reproductive, and cancerous and wounded tissues) or bodily fluids (e.g., lymph, serum, plasma, urine, seminal fluid, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 133 as residues: Pro-44 to Lys-54, Cys-88 to His-95, Val-103 to Tyr-108, Leu-146 to Pro-157, Pro-176 to Gln-184.

The tissue distribution in brain tissue indicates that the protein product of this gene is useful for the detection, treatment, and/or prevention of neurodegenerative disease states, behavioral disorders, or inflammatory conditions which include, but are not limited to Alzheimeris Disease, Parkinsonis Disease, Huntingtonis Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, depression, panic disorder, learning disabilities, ALS, psychoses, autism, and altered behaviors, including disorders in feeding, sleep patterns, balance, and perception.

In addition, elevated expression of this gene product in regions of the brain indicates it plays a role in normal neural function. Potentially, this gene product is involved in synapse formation, neurotransmission, learning, cognition, homeostasis, or neuronal differentiation or survival. Protein is also useful for the treatment, detection, and/or prevention of reproductive conditions, particularly prostate cancer. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:65 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1941 of SEQ ID NO:65, b is an integer of 15 to 1955, where both a and b correspond to the positions of nucleotide residues shown in SEQ ID NO:65, and where b is greater than or equal to a + 14.

### FEATURES OF PROTEIN ENCODED BY GENE NO: 56

When tested against U937 cell lines, supernatants removed from cells containing this gene activated the GAS (gamma activating sequence) promoter element. Thus, it is likely that this gene activates myeloid cells, or more generally immune or hematopoietic cells, in addition to other cells or cell types, through the JAK-STAT signal transduction pathway: GAS is a promoter element found upstream of many genes which are involved in the JAK-STAT pathway. The JAK-STAT pathway is a large, signal transduction pathway involved in the differentiation and proliferation of cells. Therefore, activation of the JAK-STAT pathway, reflected by the binding of the GAS element, can be used to indicate proteins involved in the proliferation and differentiation of cells. In specific embodiments, polypeptides of the invention comprise the following amino acid sequence: YLLQENNL (SEQ ID NO:234). Polynucleotides encoding these polypeptides are also encompassed by the invention.

This gene is expressed primarily in metastatic melanoma tissue, and to a lesser extent, in the brain.

Therefore, polynucleotides and polypeptides of the invention are useful as reagents for differential identification of the tissue(s) or cell type(s) present in a biological sample and for diagnosis of diseases and conditions which include, but are not limited to, integumentary or neural disorders and conditions, particularly metastatic melanoma. Similarly, polypeptides and antibodies directed to these polypeptides are useful in providing immunological probes for differential identification of the tissue(s) or cell type(s). For a number of disorders of the above tissues or cells, particularly cancers of the integumentary system, expression of this gene at significantly higher or lower levels may be routinely detected in certain tissues or cell types (e.g., integumentary, neural, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 134 as residues: Lys-29 to Asp-36, GIn-40 to His-50.

The tissue distribution in metastatic melanoma tissues, combined with the GAS biological activity data, indicates that polynucleotides and polypeptides corresponding to this gene are useful for the treatment, diagnosis, and/or prevention of various skin disorders including congenital disorders (i.e. nevi, moles, freckles, Mongolian spots, hemangiomas, port-wine syndrome), integumentary tumors (i.e. keratoses, Bowenis disease, basal cell carcinoma, squamous cell carcinoma, malignant melanoma, Pagetis disease, mycosis fungoides, and Kaposiis sarcoma), injuries and inflammation of the skin (i.e.wounds, rashes, prickly heat disorder, psoriasis, dermatitis), atherosclerosis, uticaria, eczema, photosensitivity, autoimmune disorders (i.e. lupus erythematosus, vitiligo, dermatomyositis, morphea, scleroderma, pemphigoid, and pemphigus), keloids, striae, erythema, petechiae, purpura, and xanthelasma. In addition, such disorders may predispose increased susceptibility to viral and bacterial infections of the skin (i.e. cold sores, warts, chickenpox, molluscum contagiosum, herpes zoster, boils, cellulitis, erysipelas, impetigo, tinea, althletes foot, and ringworm).

Moreover, the protein product of this gene may also be useful for the treatment or diagnosis of various connective tissue disorders such as arthritis, trauma, tendonitis, chrondomalacia and inflammation, autoimmune disorders such as rheumatoid arthritis, lupus, scleroderma, and dermatomyositis as well as dwarfism, spinal deformation, and specific joint abnormalities as well as chondrodysplasias (i.e. spondyloepiphyseal dysplasia congenita, familial osteoarthritis, Atelosteogenesis type II, metaphyseal chondrodysplasia type Schmid). Moreover, polynucleotides and polypeptides corresponding to this gene are useful for the detection, treatment, and/or prevention of neurodegenerative disease states, behavioral disorders, or inflammatory conditions. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:66 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1178 of SEQ ID NO:66, b is an integer of 15 to 1192, where both a and b correspond to the positions of nucleotide residues shown in SEQ ID NO:66, and where b is greater than or equal to a + 14.

### FEATURES OF PROTEIN ENCODED BY GENE NO: 57

The translation product of this gene shares sequence homology with a proteinase fragment from rattlesnake venom, which is thought to be important in altering the function of extracellular proteins. In specific embodiments, polypeptides of the invention comprise the following amino acid sequence: VRLLGLCIAQGH (SEQ ID NO:235), MRVGRRPKAQRVQGQNGNHSSDSEGSFSLLCLQLFSKFAVVSILLLL LLLFNTSKKKLMTFSLDSLLSPISIPTALLFGSPPPPPSHRGYGVGSAPLKEKQ MKELVPPRRECTVQGQPWQGPSLPGPAELGHRPGTRLGVECDGEWCPRSCFWELL GPPYLKCSQP SPIPPLDGTQTSAERGRGXALK (SEQ ID NO:236), PKAQRV QGQNGNHSSDSEGS FSLLCLQLFSKFAVV (SEQ ID NO:237), LDSLLSPISIPTA LLFGSPPPP (SEQ ID NO:238), ELVPPRRECTVQGQPWQGPSLPGP (SEQ ID NO:239), and/or RLGVECDGEWCPRSCFWELLGPPYL (SEQ ID NO:240). Polynucleotides encoding these polypeptides are also encompassed by the invention.The gene encoding the disclosed cDNA is believed to reside on chromosome 11. Accordingly, polynucleotides related to this invention are useful as a marker in linkage analysis for chromosome 11.

This gene is expressed primarily in retina and synovial sarcoma tissues, and to a lesser extent in activated monocytes, cerebellum, and colon tissues.

Therefore, polynucleotides and polypeptides of the invention are useful as reagents for differential identification of the tissue(s) or cell type(s) present in a biological sample and for diagnosis of diseases and conditions which include, but are not limited to, skeletal disorders, particularly degeneration of the joints. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues or cell types (e.g., skeletal, visual, immune, hematopoietic, neural, gastrointestinal, and cancerous and wounded tissues) or bodily fluids (e.g., lymph, serum, plasma, urine, vitreous humar, aqueous humoor, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in synovium, combined with the homology to snake venom proteinases, indicates that the protein product of this gene is useful for diagnosing and/or treating conditions involving altered secretion and processing of proteins and proteoglycans in the retina and joints. Moreover, the protein is also useful for the treatment, detection, and/or prevention of immune or hematopoietic disorders involving aberrations in cellular proliferation or migration; neural disorders, particularly neurodegenerative conditions, or conditions related to aberrant neurotransmitter function. Moreover, the expression of this gene product in synovium would suggest a role in the detection and treatment of disorders and conditions affecting the skeletal system, in particular osteoporosis, bone cancer, as well as, disorders afflicting connective tissues (e.g. arthritis, trauma, tendonitis, chrondomalacia and inflammation), autoimmune disorders such as rheumatoid arthritis, lupus, scleroderma, and dermatomyositis as well as dwarfism, spinal deformation, and specific joint abnormalities as well as chondrodysplasias (i.e. spondyloepiphyseal dysplasia congenita, familial osteoarthritis; Atelosteogenesis type II, metaphyseal chondrodysplasia type Schmid). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:67 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1529 of SEQ ID NO:67, b is an integer of 15 to 1543, where both a and b correspond to the positions of nucleotide residues shown in SEQ ID NO:67, and where b is greater than or equal to a + 14.

### FEATURES OF PROTEIN ENCODED BY GENE NO: 58

The protein product of this sequence shows homology to kidney injury molecule (gi|2665892), and to the hepatitis A virus receptor from African green monkeys (PID|d1022406 hepatitis A virus receptor), which are thought to play important roles in the restoration of the morphological integrity and function to postischemic kidney. KIM, or an agonist, can be used to treat renal disease and to promote the growth of new tissue or the survival of damaged tissue, generally in conditions where the binding of specific ligands to KIM stimulates cell growth, maintains cellular differentiation, or reduces apoptosis, such as in cases of renal failure, nephritis, kidney transplants, toxic or hypoxic injury, for example. A monoclonal antibody specific for KIM can be used to treat renal disease, for example, where binding of KIM to ligand results in neoplasia, loss of cellular function, susceptibility to apoptosis or promotion of inflammation. The delivery of imaging agents to KIM expressing cells in vivo or in vitro will enable the measurement of KIM concentrations by immunoassay, for example. By this method, damage or regeneration of renal cells can be determined by measuring KIM, in particular to diagnose or monitor the progress of diseases or therapy. Based on the homology of the protein product of this gene, it is expected to share certain biological activities with Kidney Injury Molecule (KIM) and HAV receptor (See J Biol Chem 1998 Feb 13;273(7):4135-42, which is hereby incorporated by reference, herein).

This gene is expressed primarily in the liver and immune system tissues.

Therefore, polynucleotides and polypeptides of the invention are useful as reagents for differential identification of the tissue(s) or cell type(s) present in a biological sample and for diagnosis of diseases and conditions which include, but are not limited to, renal or hepatic disorders or disease, particularly kidney injuries and Hepatitis A. Similarly, polypeptides and antibodies directed to these polypeptides are useful in providing immunological probes for differential identification of the tissue(s) or cell type(s). For a number of disorders of the above tissues or cells, particularly of the immune, renal and hepatic systems, expression of this gene at significantly higher or lower levels may be routinely detected in certain tissues or cell types (e.g., renal, hepatic, immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 136 as residues: Ser-44 to Ser-51, Cys-53 to Cys-64, Val-76 to Lys-83, Pro-102 to Gly-108, Arg-133 to Thr-162, Thr-204 to Ala-209, Asp-235 to Glu-241, Lys-270 to Ala-282, Ala-286 to Gly-297, Ser-346 to Arg-351, Gly-368 to Gly-374.

The tissue distribution in liver, combined with the homology to the hepatitis A receptor, indicates that the protein product of this gene is useful for the diagnosis and/or treatment of liver disorders and cancers (e.g. hepatoblastoma, jaundice, hepatitis, liver metabolic diseases and conditions that are attributable to the differentiation of hepatocyte progenitor cells). In addition the expression in fetus suggests a useful role for the protein product in developmental abnormalities, fetal deficiencies, pre-natal disorders and various would-healing models and/or tissue trauma.

Moreover, the homology to the KIM molecule indicates that the protein product of this gene is useful in the treatment and/or detection of kidney diseases including renal failure, nephritus, renal tubular acidosis, proteinuria, pyuria, edema; pyelonephritis, hydronephritis, nephrotic syndrome, crush syndrome, glomerulonephritis, hematuria, renal colic and kidney stones, in addition to Wilmis Tumor Disease, and congenital kidney abnormalities such as horseshoe kidney, polycystic kidney, and Falconi's syndrome. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:68 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1268 of SEQ ID NO:68, b is an integer of 15 to 1282, where both a and b correspond to the positions of nucleotide residues shown in SEQ ID NO:68, and where b is greater than or equal to a + 14.

### FEATURES OF PROTEIN ENCODED BY GENE NO: 59

In specific embodiments, polypeptides of the invention comprise the following amino acid sequence: WHISEPNGQ (SEQ ID NO:241). Polynucleotides encoding these polypeptides are also encompassed by the invention.

This gene is expressed primarily in fetal bone and cord blood tissues.

Therefore, polynucleotides and polypeptides of the invention are useful as reagents for differential identification of the tissue(s) or cell type(s) present in a biological sample and for diagnosis of diseases and conditions which include, but are not limited to, skeletal, developmental, or hematopoietic disorders, particularly cancers of the hematopoietic tissues. Similarly, polypeptides and antibodies directed to these polypeptides are useful in providing immunological probes for differential identification of the tissue(s) or cell type(s). For a number of disorders of the above tissues or cells, particularly of the hematopoietic system, expression of this gene at significantly higher or lower levels may be routinely detected in certain tissues or cell types (e.g., skeletal, developmental, hematopoietic, and cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fetal bone and cord blood tissues indicates that the protein product of this gene is useful for diagnosing cancers of the hematopoietic system. Moreover, polynucleotides and polypeptides corresponding to this gene are useful for the treatment and diagnosis of hematopoietic related disorders such as anemia, pancytopenia, leukopenia, thrombocytopenia or leukemia since stromal cells are important in the production of cells of hematopoietic lineages. The uses include bone marrow cell ex- vivo culture, bone marrow transplantation, bone marrow reconstitution, radiotherapy or chemotherapy of neoplasia.

The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein is useful in the amelioration of prevention of proliferative conditions of the skeletal tissues, particularly osteoclastoma and osteoblastoma. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:69 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1426 of SEQ ID NO:69, b is an integer of 15 to 1440, where both a and b correspond to the positions of nucleotide residues shown in SEQ ID NO:69, and where b is greater than or equal to a + 14.

### FEATURES OF PROTEIN ENCODED BY GENE NO: 60

The translation product of this gene was found to have homology to the conserved human activated p21cdc42Hs kinase (See Genbank Accession No. gi|307305), which is thought to sustain the GTP-bound active form of G-proteins and other receptor types, and may serve to modulate signal transduction pathways. In specific embodiments, polypeptides of the invention comprise the following amino acid sequence: RPSRLRRRLKAPFSAWKTRLAGAKGGLSVGDFRKVL (SEQ ID NO:242), WPSGLGRTSSLRGSEAQSWCSSAGHGPPPALGSPASCGGCFSPTRA SAPAAGG (SEQ ID NO:243), SLRGSEAQSWCSSAGHGPPPALGSPASCG (SEQ ID NO:244), KPHLGPRGSIEPSQASSRNPGLVTEQSCLQGPSGHRAWAGHHLS EGQRLRAGAAQQVTALHQLWVLPHHVVAAFPPPGPQLQQLVGELSTAYSKH VLR HAEH (SEQ ID NO:245), SRNPGLVTEQSCLQGPSGHRAWAGHHLSEG (SEQ ID NO:246), and/or TALHQLWVLPHHVVAAFPPPGPQLQQLVGELST (SEQ ID NO:247). Polynucleotides encoding these polypeptides are also encompassed by the invention.

This gene is expressed primarily in 2 week old early stage human, placenta, and human normal breast tissues.

Therefore, polynucleotides and polypeptides of the invention are useful as reagents for differential identification of the tissue(s) or cell type(s) present in a biological sample and for diagnosis of diseases and conditions which include, but are not limited to, developmental, or reproductive disorders and conditions, particularly breast cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., developmental, reproductive, and cancerous and wounded tissues) or bodily fluids (e.g., lymph, serum, plasma, urine, amniotic fluid, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 138 as residues: Pro-129 to Tyr-136.

The tissue distribution 2 week old early stage human, placenta, and human normal breast tissues indicates that the protein product of this gene is useful for the detection, treatment, and/or prevention of developmental disorders, particularly congenital defects which include, but are not limited to, nevi, moles, freckles, Mongolian spots, hemangiomas, port-wine syndrome, Tay-Sachs disease, phenylkenonuria, galactosemia, hyperlipidemias, porphyrias, and Hurler's syndrome. The expression in breast indicates the protein is useful in the treatment, amelioration and/or detection of breast cancer. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:70 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1054 of SEQ ID NO: 70, b is an integer of 15 to 1068, where both a and b correspond to the positions of nucleotide residues shown in SEQ ID NO:70, and where b is greater than or equal to a + 14.

### FEATURES OF PROTEIN ENCODED BY GENE NO: 61

The translation product of this gene shares sequence homology with Schwanoma associated protein, which is thought to be important in the neural signal pathway, and development thereof. In specific embodiments, polypeptides of the invention comprise the following amino acid sequence:
AEGLQSAAGIRIDTKAGPPEMLKPLWKAAVAPTWPCS (SEQ ID NO:248), GPAVCGWNQDRHQGRTPRDAEASLESSSGPHMAMLHAAPPPVGQRGWHVA GPGSAGCAVAGLRGSYLPPVASAPSSHLGPGAAQGRAQVLGAWLPAQLGSP WKQRARQQMJSCQLVLVESIPQDLPS AAGSPSAQPLGQAMILQLLDTAQESVH VA SYYWSLTGPDIGVNDSSSQLGEALLQKLQQLLGRNISLAVATSSPTLARTSTDL QVLAARGAHVRQVPMGRLTMGVLHSKFWVVDGRHIYMGSANMDWRSLTQV KELGAVIYNCSHLGQDLEKTFQTYWVLGVPKAVLPKTWPQNFSSHFNRFQPF HGLFDGVPTTAYFSASPPALCPQGRTRDLEALLAVMGSAQEFIYASVMEYFPT TRFSHPPRYWPVLDNALRAAAFGKGVRVRLLVGCGLNTDPTMFPYLRSLQAL SNPAANVSVDVKVFIVPVGNHSNIPFSRVNHSKFMVTEKAAYIGTSNWSEDY FSSTAGVGLVVTQSPGAQPAGATVQEQLRQLFERDWSSRYAVGLDGQAPGQDC VWQG (SEQ ID NO:249), QGRTPRDAEASLESSSGPHMAMLH (SEQ ID NO:250), GSAGCAVAGLRGSYLPPVASAPS (SEQ ID NO:251), AQGRAQVLGAWLPAQL GSPWKQRARQQRD (SEQ ID NO:252), PSAAGSPSAQPLGQAWLQLLD (SEQ ID NO:253), VASYYWSLTGPDIGVNDSSSQLGEAL (SEQ ID NO:254), SLAVATSS PTLARTSTDLQVLAARG (SEQ ID NO:255), PQNFSSHFNRFQPFHGLFDGV PTTAY (SEQ ID NO:256), PQGRTRDLEALLAVMGSAQEFIYASVM (SEQ ID NO:257), SHPPRYWPVLDNALRAAAFGKGVR (SEQ ID NO:258), TDPTMFP YLRSLQALSNPAANVSVDVKVF (SEQ ID NO:259), DVKVFIVPVGNHSNIPFS RVNHSKFMVTEKA (SEQ ID NO:260), and/or QLRQLFERDWSSRYAVGLDGQ APG (SEQ ID NO:261). Polynucleotides encoding these polypeptides are also encompassed by the invention.

This gene is expressed primarily in lymph nodes.

Therefore, polynucleotides and polypeptides of the invention are useful as reagents for differential identification of the tissue(s) or cell type(s) present in a biological sample and for diagnosis of diseases and conditions which include, but are not limited to, immune, hematopoietic, or neural disorders, particularly inflammatory and neurodegenerative conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic, neural, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 139 as residues: Met-1 to Gly-12, Pro-38 to Trp-43, Val-46 to Trp-55, Gly-67 to Thr-76, Ala-85 to His-91, Thr-122 to Gly-128, Gly-132 to Glu-141, Pro-168 to Cys-174, Asp-185 to Gly-191.

The tissue distribution in lymph nodes indicates that the protein product of this gene is useful for the diagnosis and/or treatment of immune disorder. Moreover, the secreted protein can also be used to determine biological activity, to raise antibodies, as tissue markers, to isolate cognate ligands or receptors, to identify agents that modulate their interactions, and as nutritional supplements. It may also have a very wide range of biological activities. Typical of these are cytokine, cell proliferation/differentiation modulating activity or induction of other cytokines; immunostimulating/immunosuppressant activities (e.g. for treating human immunodeficiency virus infection, cancer, autoimmune diseases and allergy); regulation of hematopoiesis (e.g. for treating anemia or as adjunct to chemotherapy); stimulation or growth of bone, cartilage, tendons, ligaments and/or nerves (e.g. for treating wounds, stimulation of follicle stimulating hormone (for control of fertility); chemotactic and chemokinetic activities (e.g. for treating infections, tumors); hemostatic or thrombolytic activity (e.g. for treating hemophilia, cardiac infarction etc.); anti-inflammatory activity (e.g. for treating septic shock, Crohn's disease); as antimicrobials; for treating psoriasis or other hyperproliferative diseases; for regulation of metabolism, and behavior. Also contemplated is the use of the corresponding nucleic acid in gene therapy procedures.

In addition, the homology to the Schwanoma associated protein indicates that the protein is useful in the treatment, detection, and/or prevention of demyelinating disorders, in addition to disorders in fatty acid metabolism. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:71 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1934 of SEQ ID NO:71, b is an integer of 15 to 1948, where both a and b correspond to the positions of nucleotide residues shown in SEQ ID NO:71, and where b is greater than or equal to a + 14.

### FEATURES OF PROTEIN ENCODED BY GENE NO: 62

In specific embodiments, polypeptides of the invention comprise the following amino acid sequence: KQPRQLFNSL (SEQ ID NO:262). Polynucleotides encoding these polypeptides are also encompassed by the invention. The gene encoding the disclosed cDNA is believed to reside on chromosome 7. Accordingly, polynucleotides related to this invention are useful as a marker in linkage analysis for chromosome 7.

This gene is expressed primarily in merckel cells.

Therefore, polynucleotides and polypeptides of the invention are useful as reagents for differential identification of the tissue(s) or cell type(s) present in a biological sample and for diagnosis of diseases and conditions which include, but are not limited to, integumentary disorders and disease. Similarly, polypeptides and antibodies directed to these polypeptides are useful in providing immunological probes for differential identification of the tissue(s) or cell type(s). For a number of disorders of the above tissues or cells, particularly of the integumentary system, expression of this gene at significantly higher or lower levels may be routinely detected in certain tissues or cell types (e.g., integumentary, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in merkel cells indicates that the protein product of this gene is useful for the diagnosis and/or treatment of skin disorders. Moreover, polynucleotides and polypeptides corresponding to this gene are useful for the treatment, diagnosis, and/or prevention of various skin disorders including congenital disorders (i.e. nevi, moles, freckles, Mongolian spots, hemangiomas, port-wine syndrome), integumentary tumors (i.e. keratoses, Bowenis disease, basal cell carcinoma, squamous cell carcinoma, malignant melanoma, Pagetis disease, mycosis fungoides, and Kaposiis sarcoma), injuries and inflammation of the skin (i.e.wounds, rashes, prickly heat disorder, psoriasis, dermatitis), atherosclerosis, uticaria, eczema, photosensitivity, autoimmune disorders (i.e. lupus erythematosus, vitiligo, dermatomyositis, morphea, scleroderma, pemphigoid, and pemphigus), keloids, striae, erythema, petechiae, purpura, and xanthelasma.

In addition, such disorders may predispose increased susceptibility to viral and bacterial infections of the skin (i.e. cold sores, warts, chickenpox, molluscum contagiosum, herpes zoster, boils, cellulitis, erysipelas, impetigo, tinea, althletes foot, and ringworm). Moreover, the protein product of this gene may also be useful for the treatment or diagnosis of various connective tissue disorders such as arthritis, trauma, tendonitis, chrondomalacia and inflammation, autoimmune disorders such as rheumatoid arthritis, lupus, scleroderma, and dermatomyositis as well as dwarfism, spinal deformation, and specific joint abnormalities as well as chondrodysplasias (i.e. spondyloepiphyseal dysplasia congenita, familial osteoarthritis, Atelosteogenesis type II, metaphyseal chondrodysplasia type Schmid). Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:72 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1823 of SEQ ID NO:72, b is an integer of 15 to 1837, where both a and b correspond to the positions of nucleotide residues shown in SEQ ID NO:72, and where b is greater than or equal to a + 14.

### FEATURES OF PROTEIN ENCODED BY GENE NO: 63

In specific embodiments, polypeptides of the invention comprise the following amino acid sequence: TQSTGLESSCSEAPGLPLTFLVAATQRALEWTQG (SEQ ID NO:263). Polynucleotides encoding these polypeptides are also encompassed by the invention.

This gene is expressed primarily in hippocampus.

Therefore, polynucleotides and polypeptides of the invention are useful as reagents for differential identification of the tissue(s) or cell type(s) present in a biological sample and for diagnosis of diseases and conditions which include, but are not limited to, neural disorders, particularly learning, memory, and mood/behavior disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g., neural, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 141 as residues: Gly-43 to Gly-48.

The tissue distribution in hippocampus indicates that the protein product of this gene is useful for the diagnosis and/or treatment of memory loss and learning disorders. Moreover, polynucleotides and polypeptides corresponding to this gene are useful for the detection, treatment, and/or prevention of neurodegenerative disease states, behavioral disorders, or inflammatory conditions which include, but are not limited to Alzheimeris Disease, Parkinsonis Disease, Huntingtonis Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, depression, panic disorder, learning disabilities, ALS, psychoses, autism, and altered behaviors, including disorders in feeding, sleep patterns, balance, and perception. In addition, elevated expression of this gene product in regions of the brain indicates that it plays a role in normal neural function. Potentially, this gene product is involved in synapse formation, neurotransmission, learning, cognition, homeostasis, or neuronal differentiation or survival. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:73 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1147 of SEQ ID NO:73, b is an integer of 15 to 1161, where both a and b correspond to the positions of nucleotide residues shown in SEQ ID NO:73, and where b is greater than or equal to a + 14.

### FEATURES OF PROTEIN ENCODED BY GENE NO: 64

The translation product of this gene was found to have homology with h-caldesmon from Gallus gallus (See Genbank Accession No. gi|211896), which is thought to be important in cytoskeletal regulation and targeting. In specific embodiments, polypeptides of the invention comprise the following amino acid sequence:
DTKNCGQELANLEKWKEQNRAKPVHLVPRRLGGSQSETEVRQKQQLQLMQ SKYKQKLKREESVRIKKEAEEAELQKMKAIQREKSNKLEEKKRLQENLRREA FREHQQYKTAEFLSKLNTESPDRSACQSAVCGPQSSTWARSWAYRDSLKAE ENRKLQKMKDEQHQKSELLELKRQQQEQERAKIHQTEHRRVNNAFLDRLQ GKSQPGGLEQSGGCWNMNSGNSWGI (SEQ ID NO:264), GQELANLEKWKE QNRAKPVHL (SEQ ID NO:265), RRLGGSQSETEVRQKQQLQLMQSKYK (SEQ ID NO:266), EEAELQKMKAIQREKSNKLEE (SEQ ID NO:267), HQQYKTAEF LSKLNTESPDRSA (SEQ ID NO:268), LLELKRQQQEQERAKIHQTEHRR (SEQ ID NO:269), and/or LDRLQGKSQPGGLEQSGGCWNM (SEQ ID NO:270). Polynucleotides encoding these polypeptides are also encompassed by the invention. The gene encoding the disclosed cDNA is believed to reside on chromosome 13. Accordingly, polynucleotides related to this invention are useful as a marker in linkage analysis for chromosome 13.

This gene is expressed primarily in human adult small intestine and ovarian tumor tissues, and to a lesser extent in T cells, lymphoma tissue and dendritic cells.

Therefore, polynucleotides and polypeptides of the invention are useful as reagents for differential identification of the tissue(s) or cell type(s) present in a biological sample and for diagnosis of diseases and conditions which include, but are not limited to, gastrointestinal, immune, or reproductive disorders, and in particular proliferative conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., gastrointestinal, immune, reproductive, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 142 as residues: Asn-22 to Ile-29, Ala-33 to Arg-51.

The tissue distribution in small intestine, in addition to immune cells and tissues, indicates that the protein product of this gene is useful for the treatment and/or diagnosis of the certain types of tumors, particularly those of the digestive tract. Moreover, the expression of this gene product indicates a role in regulating the proliferation; survival; differentiation; and/or activation of hematopoietic cell lineages, including blood stem cells. This gene product may be involved in the regulation of cytokine production, antigen presentation, or other processes that may also suggest a usefulness in the treatment of cancer (e.g. by boosting immune responses).

Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues.

In addition, this gene product may have commercial utility in the expansion of stem cells and committed progenitors of various blood lineages, and in the differentiation and/or proliferation of various cell types. The protein is also useful in the treatment, detection, and/or prevention of reproductive disorders, which include, but are not limited to polycistic ovary, ovarian cancer, infertility, etc. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:74 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 1436 of SEQ ID NO:74, b is an integer of 15 to 1450, where both a and b correspond to the positions of nucleotide residues shown in SEQ ID NO:74, and where b is greater than or equal to a + 14.

### FEATURES OF PROTEIN ENCODED BY GENE NO: 65

In specific embodiments, polypeptides of the invention comprise the following amino acid sequence: LFSGECLQRLWVR (SEQ ID NO:271). Polynucleotides encoding these polypeptides are also encompassed by the invention.

This gene is expressed primarily in activated neutrophils and dendritic cells.

Therefore, polynucleotides and polypeptides of the invention are useful as reagents for differential identification of the tissue(s) or cell type(s) present in a biological sample and for diagnosis of diseases and conditions which include, but are not limited to, immune or hematopoietic disorders, and in particular inflammatory diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., immune, hematopoietic cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 143 as residues: Met-1 to Trp-8.

The tissue distribution in neutrophils and dendritic cells indicates that the protein product of this gene is useful for the diagnosis and/or treatment of immune disorders, particularly in the immune response. Moreover, the expression of this gene product indicates a role in the regulation of the proliferation; survival; differentiation; and/or activation of hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immunodeficiency diseases such as AIDS, leukemia, rheumatoid arthritis, granulomatous disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's disease, scleroderma and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:75 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 543 of SEQ ID NO:75, b is an integer of 15 to 557, where both a and b correspond to the positions of nucleotide residues shown in SEQ ID NO:75, and where b is greater than or equal to a + 14.

### FEATURES OF PROTEIN ENCODED BY GENE NO: 66

In specific embodiments, polypeptides of the invention comprise the following amino acid sequence:
RHELVPLVPGLVNSEVHNEDGRNGDVSQFPYVEFTGRDSVTCPTCQGTGRIPR GQENQLVALIPYSDQRLRPRRTKLYV (SEQ ID NO:272), PGLVNSEVHNEDGR NGDVSQFPY (SEQ ID NO:273), and/or TCPTCQGTGRIPRGQENQLVALIPYS (SEQ ID NO:274). Polynucleotides encoding these polypeptides are also encompassed by the invention.

This gene is expressed primarily in endothelial cells and fibroblasts.

Therefore, polynucleotides and polypeptides of the invention are useful as reagents for differential identification of the tissue(s) or cell type(s) present in a biological sample and for diagnosis of diseases and conditions which include, but are not limited to, vascular disorders, including cancers derived from endothelial and fibroblast cells. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., vascular, endothelial, immune, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 144 as residues: Thr-55 to Tyr-60, Glu-143 to Tyr-152, Asp-154 to Gln-165.

The tissue distribution in endothelial and fibroblast cells indicates that the protein product of this gene is useful in the detection, treatment, and/or prevention of vascular conditions, which include, but are not limited to, microvascular disease, vascular leak syndrome, aneurysm, stroke, atherosclerosis, arteriosclerosis, or embolism. Protein is also useful for the treatment, detection, and/or prevention of autoimmune disorders and conditions. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:76 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 2469 of SEQ ID NO:76, b is an integer of 15 to 2483, where both a and b correspond to the positions of nucleotide residues shown in SEQ ID NO:76, and where b is greater than or equal to a + 14.

### FEATURES OF PROTEIN ENCODED BY GENE NO: 67

In specific embodiments, polypeptides of the invention comprise the following amino acid sequence: ALSTETRTPD (SEQ ID NO:275). Polynucleotides encoding these polypeptides are also encompassed by the invention.

This gene is expressed primarily in colon cancer, hepatocellular tumor, hepatoma, and uterine cancer tissues, and to a lesser extent in normal liver tissue.

Therefore, polynucleotides and polypeptides of the invention are useful as reagents for differential identification of the tissue(s) or cell type(s) present in a biological sample and for diagnosis of diseases and conditions which include, but are not limited to, certain cancers. Similarly, polypeptides and antibodies directed to these polypeptides are useful in providing immunological probes for differential identification of the tissue(s) or cell type(s). For a number of disorders of the above tissues or cells, particularly of the metabolic and tumor systems, expression of this gene at significantly higher or lower levels may be routinely detected in certain tissues or cell types (e.g., cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred epitopes include those comprising a sequence shown in SEQ ID NO. 145 as residues: Trp-35 to Trp-45, Pro-52 to Asp-57, Thr-73 to Thr-80, Pro-96 to Leu-103, Pro-106 to Leu-119.

The tissue distribution in cancerous tissues of the colon, liver, and uterus indicates that the protein product of this gene is useful for the diagnosis and/or treatment of certain cancers, including colon cancer, hepatocellular tumor, hepatoma, and uterine cancer. Expression within embryonic tissue and other cellular sources marked by proliferating cells indicates this protein may play a role in the regulation of cellular division, and may show utility in the diagnosis and treatment of cancer and other proliferative disorders. Similarly, developmental tissues rely on decisions involving cell differentiation and/or apoptosis in pattern formation. Thus, this protein may also be involved in apoptosis or tissue differentiation and could again be useful in cancer therapy. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:77 and may have been publicly available prior to conception of the present invention. Preferably, such related polynucleotides are specifically excluded from the scope of the present invention. To list every related sequence would be cumbersome. Accordingly, preferably excluded from the present invention are one or more polynucleotides comprising a nucleotide sequence described by the general formula of a-b, where a is any integer between 1 to 653 of SEQ ID NO:77, b is an integer of 15 to 667, where both a and b correspond to the positions of nucleotide residues shown in SEQ ID NO:77, and where b is greater than or equal to a + 14.

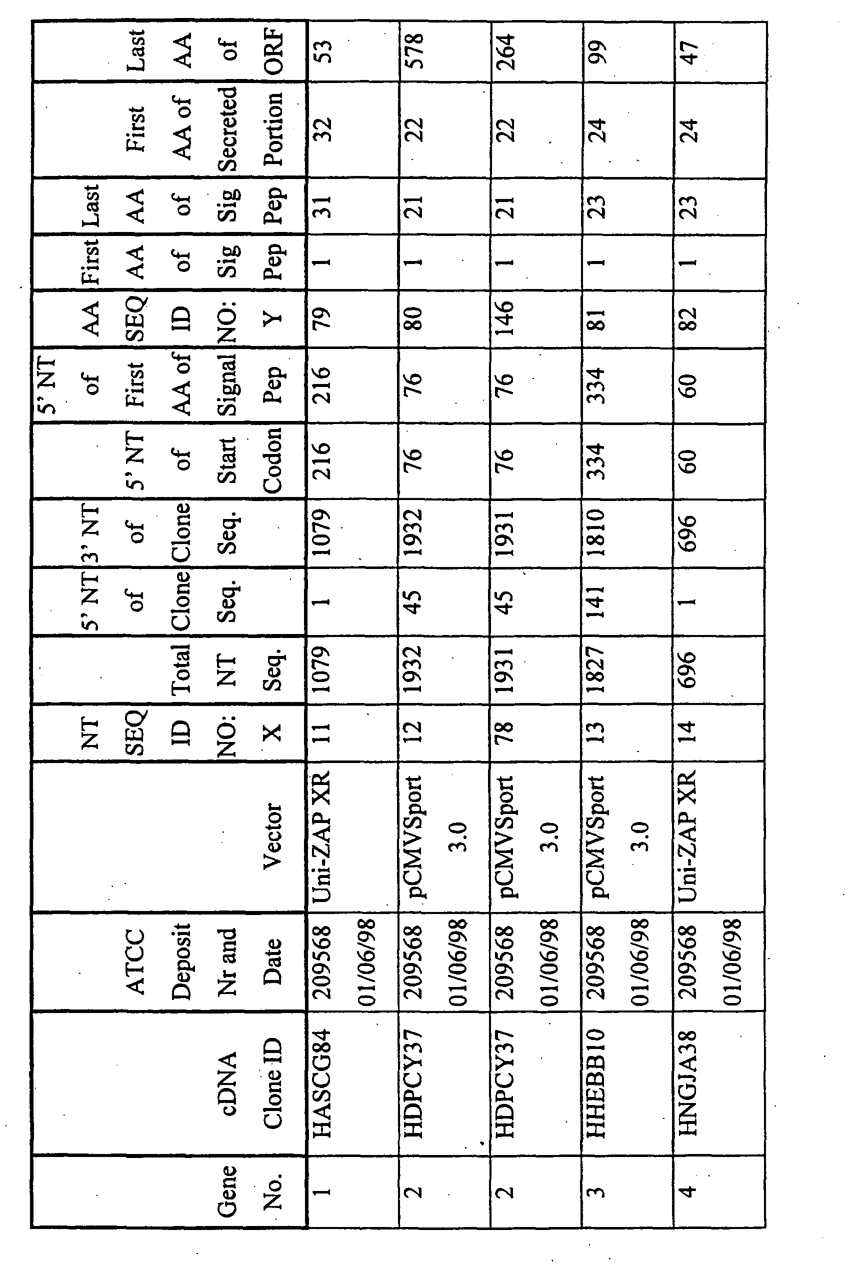

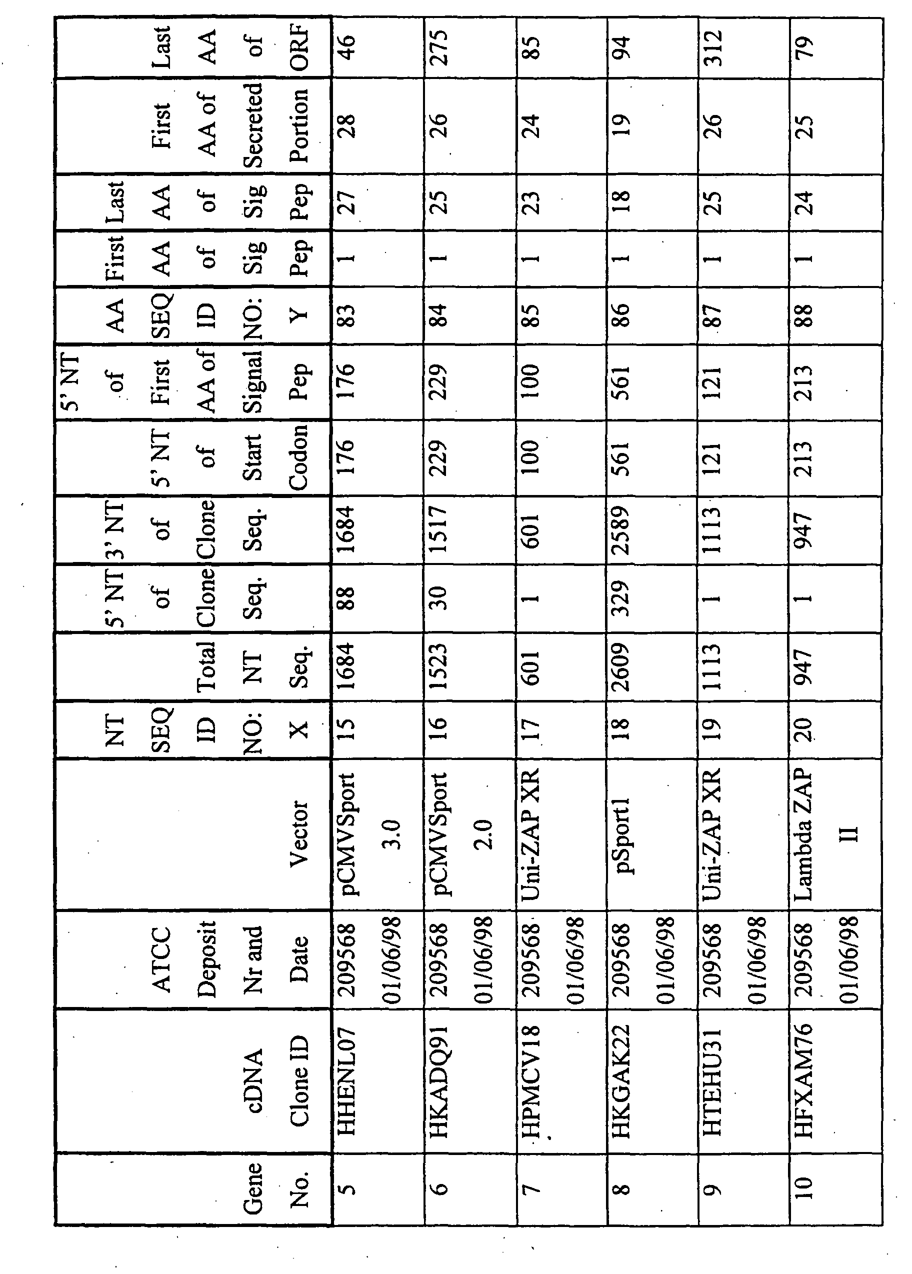

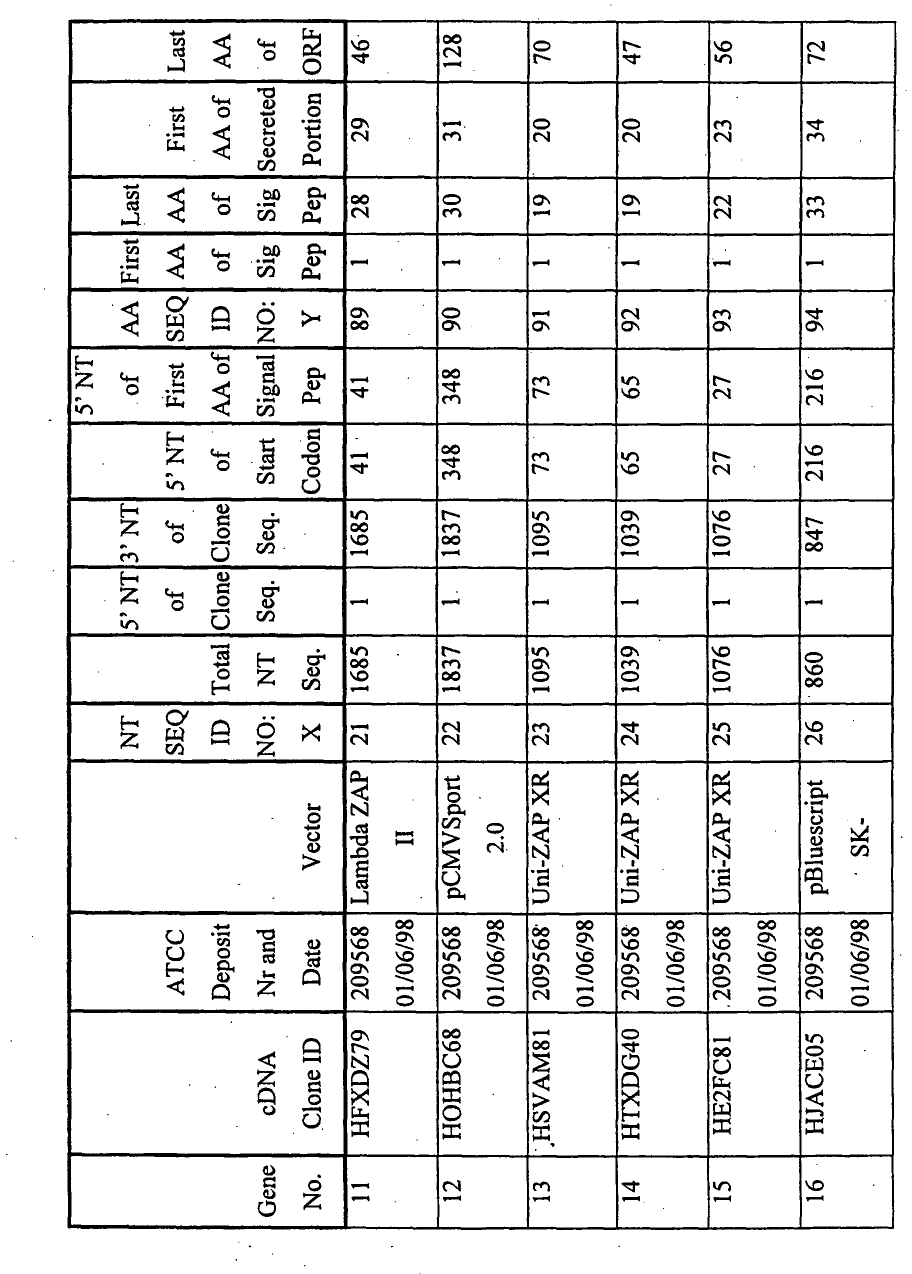

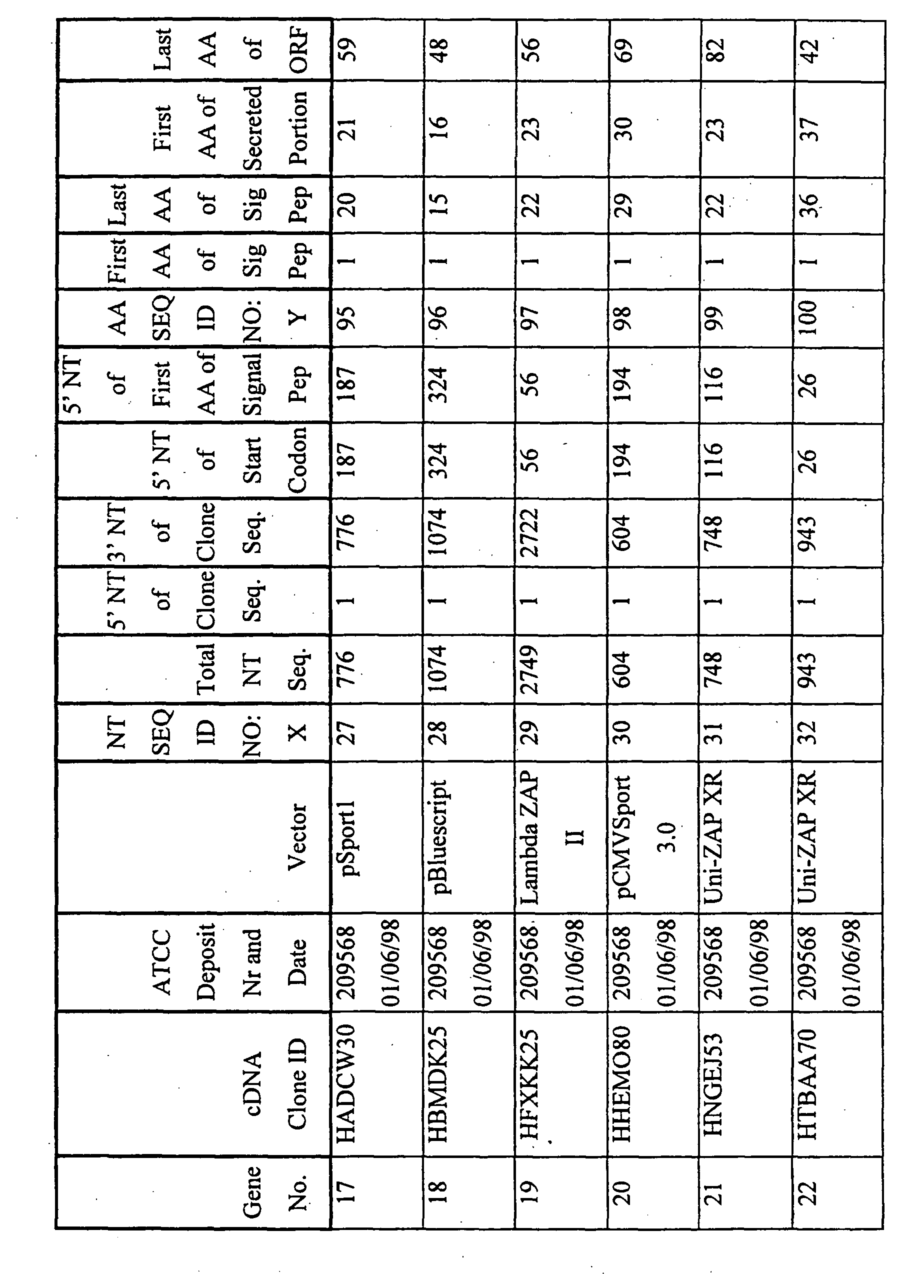

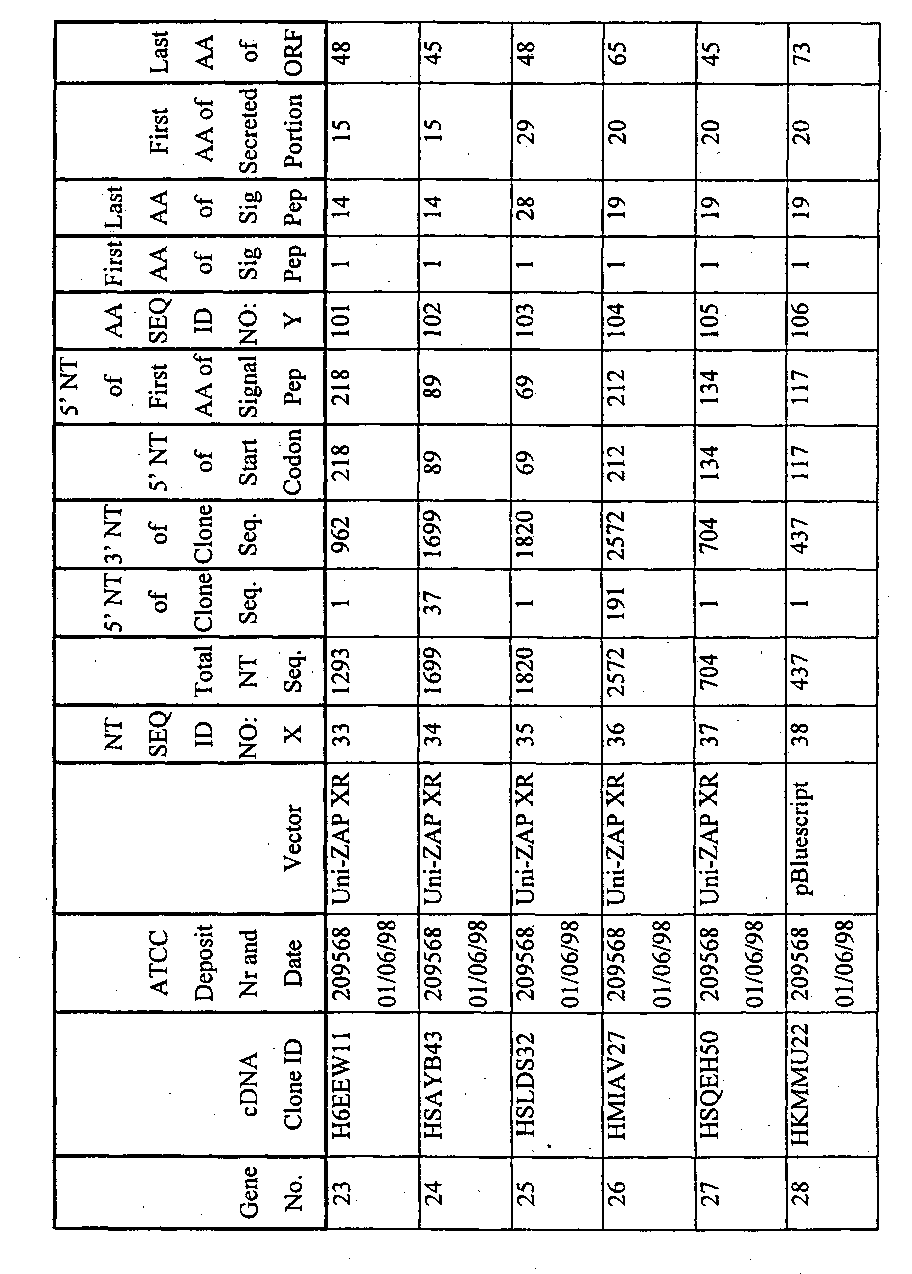

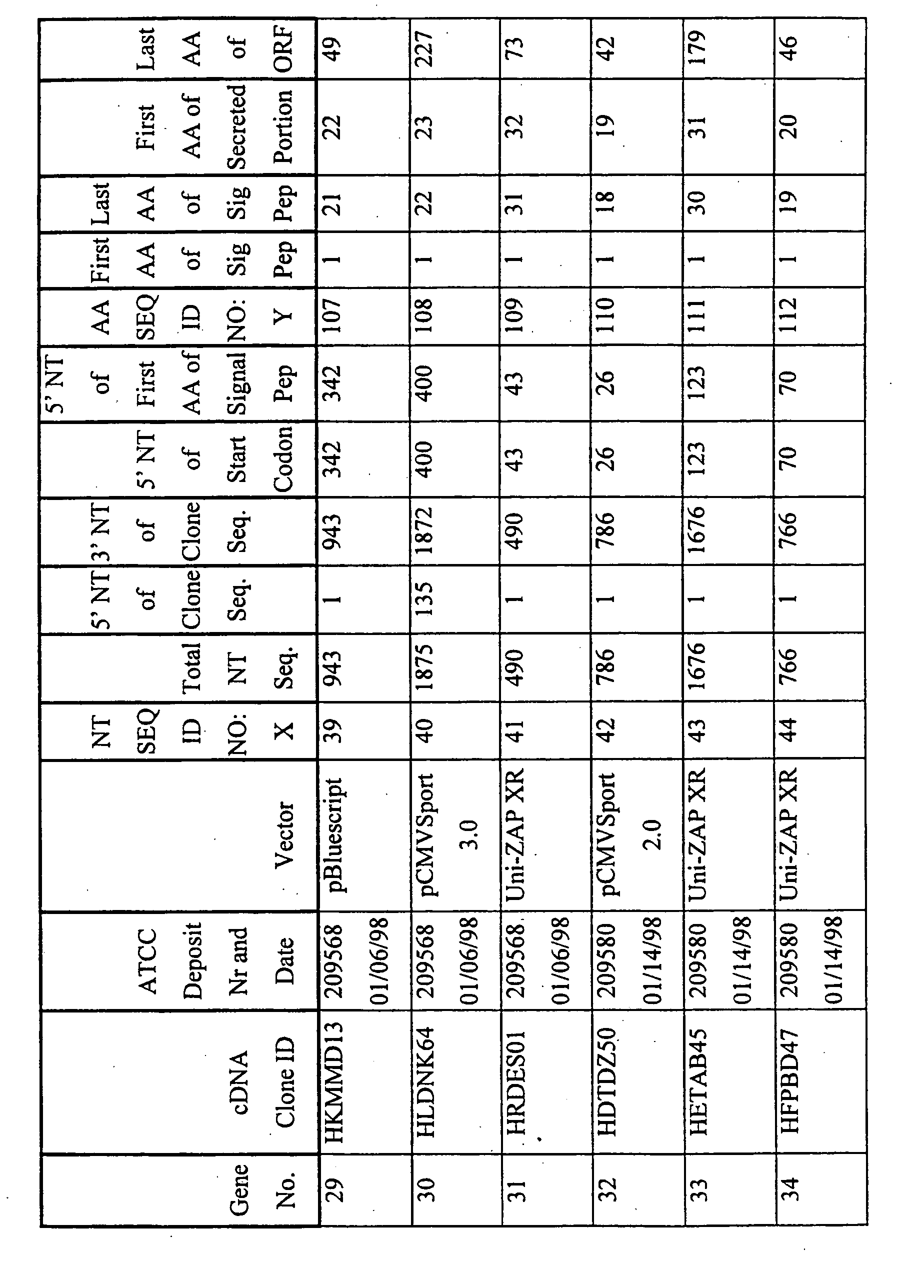

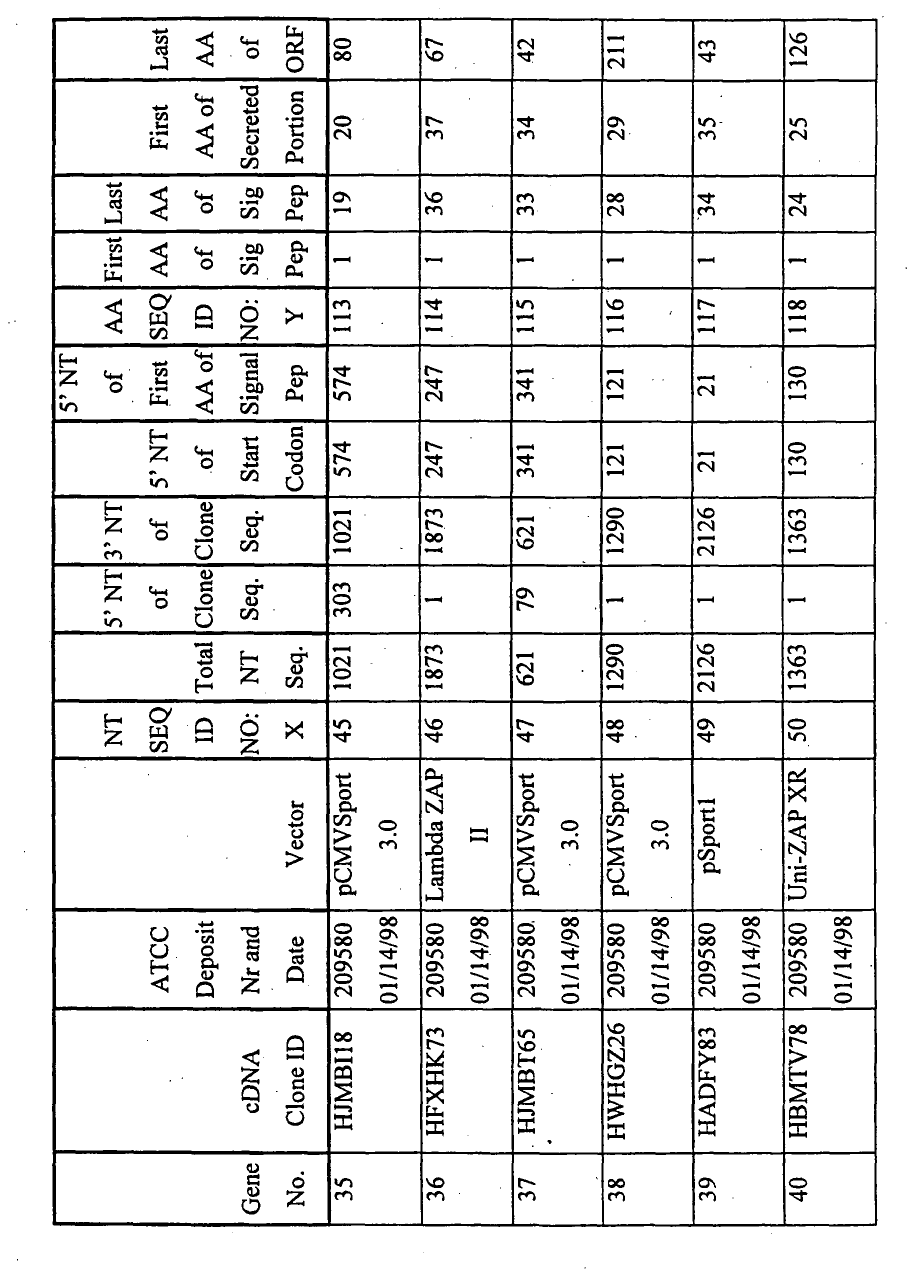

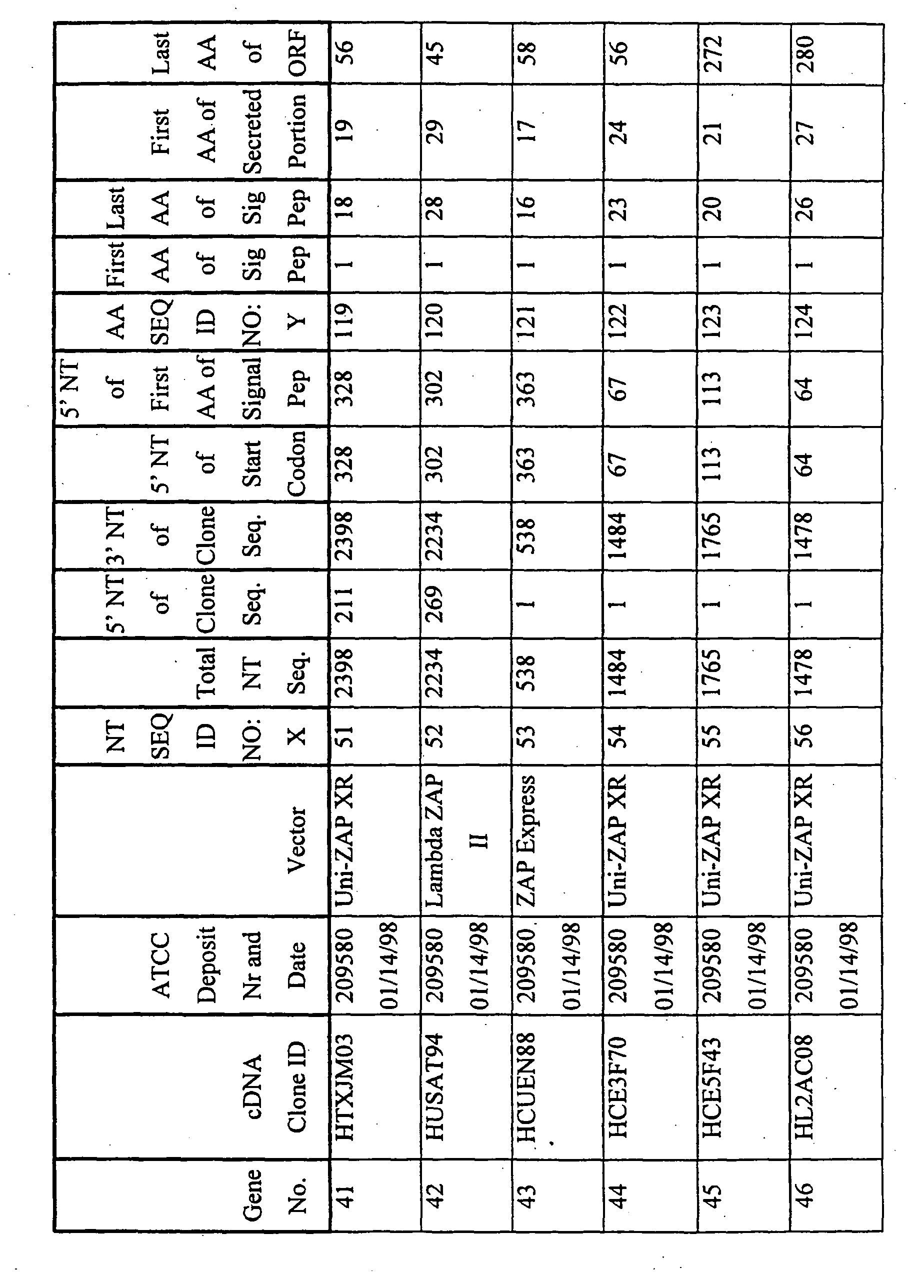

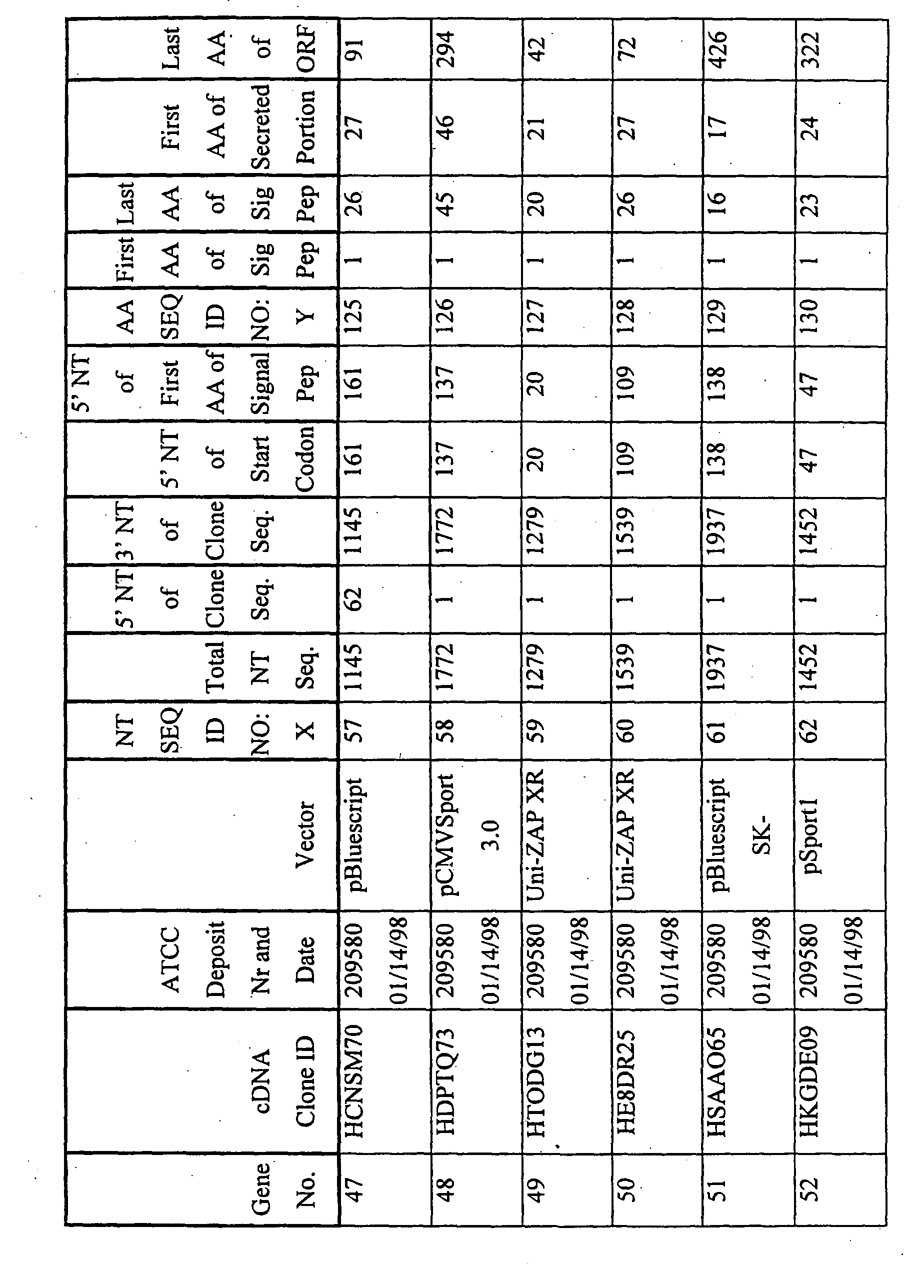

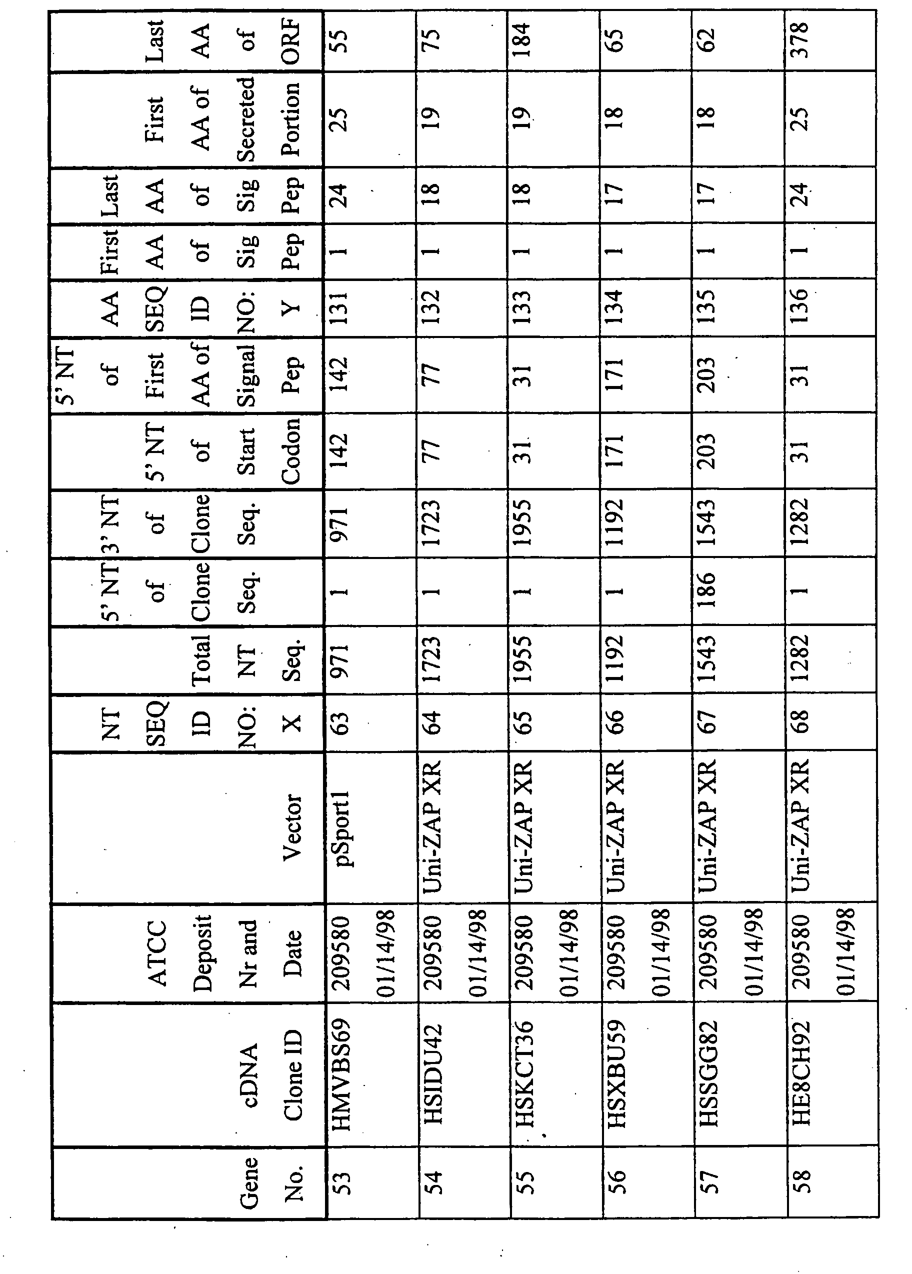

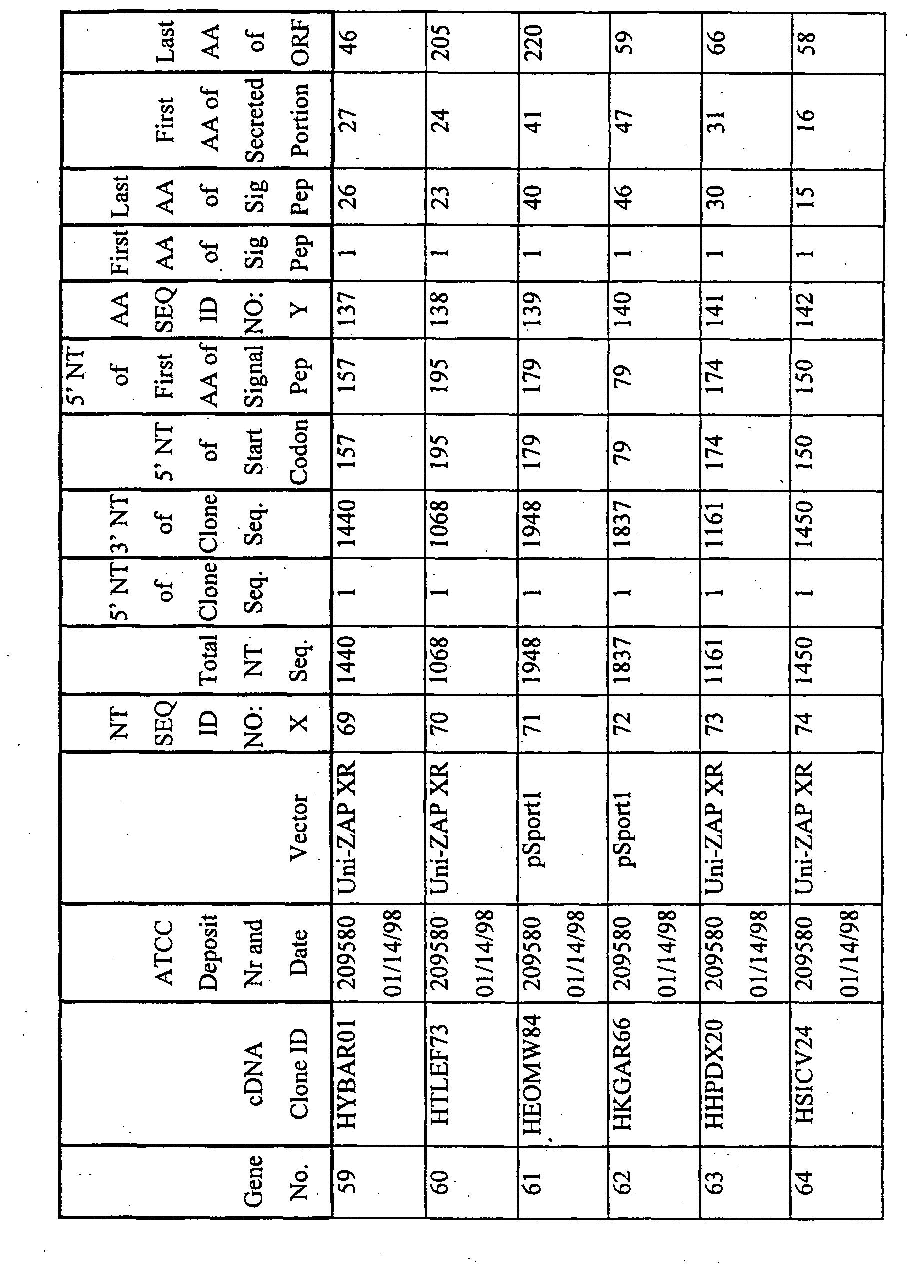

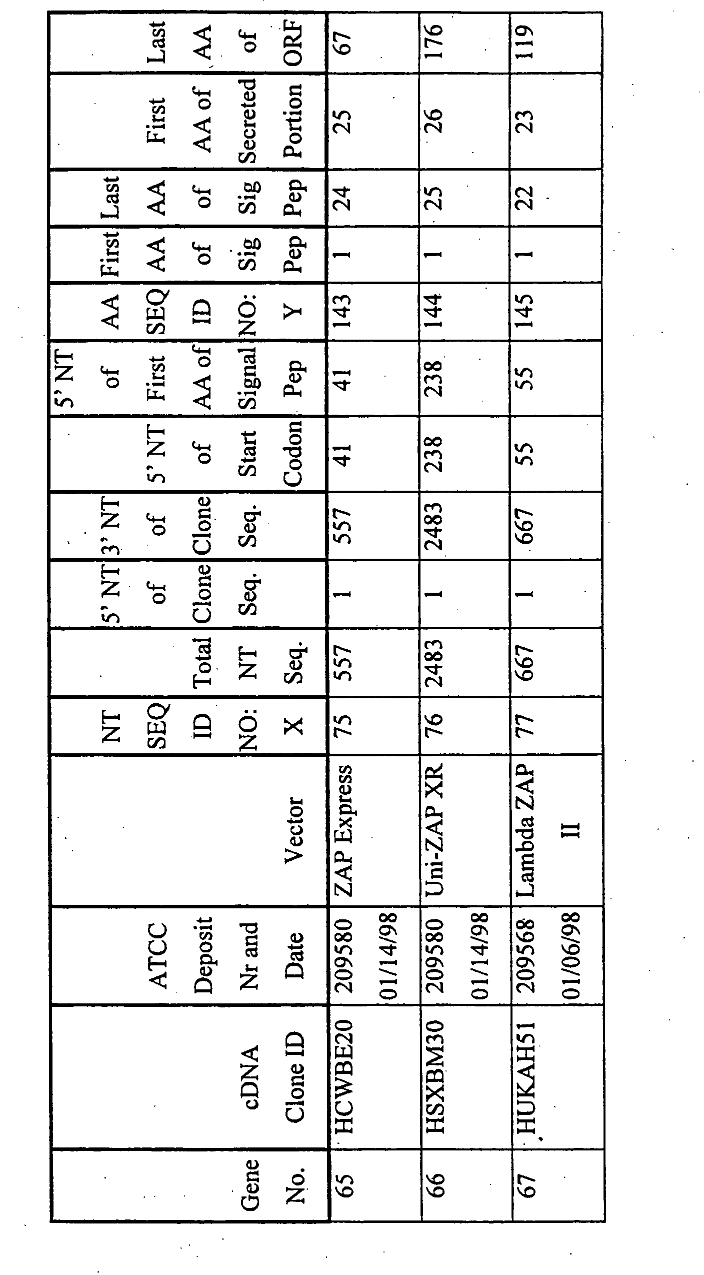

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly, the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification , such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988). Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje; Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown inTable 1, the ORF (open reading frame), or any fragement specified as described herein.

As a practical matter, whether any particular nucleic acid molecule or polypeptide is at least 90%, 95%, 96%, 97%, 98% or 99% identical to a nucleotide sequence of the presence invention can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignement of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequnce are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequences shown in Table 1 or to the amino acid sequence encoded by deposited DNA clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is becuase the FASTDB program does not account for N-and C-terminal truncations of the subject sequence when calculating global percent identity. For subject sequences truncated at the N- and C-termini, relative to the the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C-termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example; guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

A further embodiment of the invention relates to a polypeptide which comprises the amino acid sequence of the present invention having an amino acid sequence which contains at least one amino acid substitution, but not more than 50 amino acid substitutions, even more preferably, not more than 40 amino acid substitutions, still more preferably, not more than 30 amino acid substitutions, and still even more preferably, not more than 20 amino acid substitutions. Of course, in order of ever-increasing preference, it is highly preferable for a polypeptide to have an amino acid sequence which comprises the amino acid sequence of the present invention, which contains at least one, but not more than 10, 9, 8, 7, 6, 5, 4, 3, 2 or 1 amino acid substitutions. In specific embodiments, the number of additions, substitutions, and/or deletions in the amino acid sequence of the present invention or fragments thereof (e.g., the mature form and/or other fragments described herein), is 1-5, 5-10, 5-25, 5-50, 10-50 or 50-150, conservative amino acid substitutions are preferable.

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998- 4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995)).

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).) Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

In addition to encompassing host cells containing the vector constructs discussed herein, the invention also encompasses primary, secondary, and immortalized host cells of vertebrate origin, particularly mammalian origin, that have been engineered to delete or replace endogenous genetic material (e.g., coding sequence), and/or to include genetic material (e.g., heterologous polynucleotide sequences) that is operably associated with the polynucleotides of the invention, and which activates, alters, and/or amplifies endogenous polynucleotides. For example, techniques known in the art may be used to operably associate heterologous control regions (e.g., promoter and/or enhancer) and endogenous polynucleotide sequences via homologous recombination (see, e.g., U.S. Patent No. 5,641,670, issued June 24, 1997; International Publication No. WO 96/29411, published September 26, 1996; International Publication No. WO 94/12650, published August 4, 1994; Koller et al., Proc. Natl. Acad. Sci. USA 86:8932-8935 (1989); and Zijlstra et al., Nature 342:435-438 (1989), the disclosures of each of which are incorporated by reference in their entireties).

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360 (1991) ) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell. Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to:
hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Bimaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox, hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Cbrynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, burns, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vasculature (including vascular and lymphatics), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically; diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E. coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention, (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID. NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library Plasmid | Corresponding Deposited |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR® 2.1 | pCR®2.1 |

Vectors Lambda Zap (U.S. Patent Nos. 5,128,256 and 5,286,636), Uni-Zap XR (U.S. Patent Nos. 5,128,256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the fl origin of replication ("ori"), such that in one orientation, single stranded rescue initiated from the fl ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e.; within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01% (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA): This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra).* Proteins with a 6 x His tag bind to the Ni-NTA resin with high affinity and can be purified in a simple one-step procedure (for details see: The QIAexpressionist (1995) QIAGEN, Inc., *supra).*

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4° C or frozen at -80° C.

In addition to the above expression vector, the present invention further includes an expression vector comprising phage operator and promoter elements operatively linked to a polynucleotide of the present invention, called pHE4a. (ATCC Accession Number 209645, deposited on February 25, 1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC 19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCI in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM 1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39(1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.).

The fragment and the dephosphorylated plasmid are ligated together with T4 DNA ligase. *E. coli* HB101 1 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4°C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra).* The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, ifpC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

Human IgG Fc region:
GGGATCCGGAGCCCAAATCTTCTGACAAAACTCACACATGCCCACCGTGC
CCAGCACCTGAATTCGAGGGTGCACCGTCAGTCTTCCTCTTCCCCCCAAAA
CCCAAGGACACCCTCATGATCTCCCGGACTCCTGAGGTCACATGCGTGGT
GGTGGACGTAAGCCACGAAGACCCTGAGGTCAAGTTCAACTGGTACGTGG
ACGGCGTGGAGGTGCATAATGCCAAGACAAAGCCGCGGGAGGAGCAGTA
CAACAGCACGTACCGTGTGGTCAGCGTCCTCACCGTCCTGCACCAGGACT
GGCTGAATGGCAAGGAGTACAAGTGCAAGGTCTCCAACAAAGCCCTCCCA
ACCCCCATCGAGAAAACCATCTCCAAAGCCAAAGGGCAGCCCCGAGAAC

CACAGGTGTACACCCTGCCCCCATCCCGGGATGAGCTGACCAAGAACCAG
GTCAGCCTGACCTGCCTGGTCAAAGGCTTCTATCCAAGCGACATCGCCGT
GGAGTGGGAGAGCAATGGGCAGCCGGAGAACAACTACAAGACCACGCCT
CCCGTGCTGGACTCCGACGGCTCCTTCTTCCTCTACAGCAAGCTCACCGTG
GACAAGAGCAGGTGGCAGCAGGGGAACGTCTTCTCATGCTCCGTGATGCA
TGAGGCTCTGCACAACCACTACACGCAGAAGAGCCTCTCCCTGTCTCCGG
GTAAATGAGTGCGACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP20), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567;.Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media (116.6 mg/L of CaCl2 (anhyd); 0.00130 mg/L CuSO₄-5H₂O; 0.050 mg/L of Fe(NO₃)₃-9H₂O; 0.417 mg/L of FeSO₄-7H₂O; 311.80 mg/L of Kcl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂O; 71.02 mg/L of Na₂HPO4; .4320 mg/L of ZnSO₄-7H₂O; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L-Alanine; 147.50 mg/ml of L-Arginine-HCL; 7.50 mg/ml of L-Asparagine-H₂0; 6.65 mg/ml of L-Aspartic Acid; 29.56 mg/ml of L-Cystine-2HCL-H₂0; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂0; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0. mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂0; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L ofNa Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1%BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proximal region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | JAKs | | | | STATS GAS(elements) or ISRE | |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE |
| IFN-g | | + | + | - | 1 | GAS (IRFI>Lys6>IFP) |
| Il-10 | + | ? | ? | - | 1,3 | |
| | | | | | | |

| gp130 family | | | | | | |
|---|---|---|---|---|---|---|
| IL-6 (Pleiotrophic) | + | + | + | ? | 1,3 | GAS (IRFI>Lys6>IFP) |
| Il- 11(Pleiotrophic) | ? | + | ? | ? | 1,3 | |
| OnM(Pleiotrophic) | ? | + | + | ? | 1,3 | |
| LIF(Pleiotrophic) | ? | + | + | ? | 1,3 | |
| CNTF(Pleiotrophic) | -/+ | + | + | ? | 1,3 | |
| G-CSF(Pleiotrophic) | ? | + | ? | ? | 1,3 | |
| IL-12(Pleiotrophic) | + | - | + | + | 1,3 | |
| | | | | | | |

| g-C family | | | | | | |
|---|---|---|---|---|---|---|
| IL-2 (lymphocytes) | - | + | - | + | 1,3,5 | GAS |
| IL-4 (lymph/myeloid) | - | + | - | + | 6 | GAS (IRFl = IFP >>Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |
| | | | | | | |

| gp140 family | | | | | | |
|---|---|---|---|---|---|---|
| IL-3 (myeloid) | - | - | + | - | 5 | GAS (IRFl>IFP>>Ly6) |
| IL-5 (myeloid) | - | - | + | - | 5 | GAS |
| GM-CSF (myeloid) | - | - | + | - | 5 | GAS |
| | | | | | | |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRFl=IFP>>Ly6) |
| | | | | | | |

| Receptor Tyrosine Kinases | | | | | | |
|---|---|---|---|---|---|---|
| EGF | ? | + | + | - | 1,3 | GAS (IRFl) |
| PDGF | ? | + | + | - | 1,3 | |
| CSF-1 | ? | + | + | - | 1,3 | GAS (not IRFl) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRFl promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCC
GAAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAA
TGATTTCCCCGAAATATCTGCCATCTCAATTAGTCAGCAACCATAGTCCCG
CCCCTAACTCCGCCCATCCCGCCCCTAACTCCGCCCAGTTCCGCCCATTCT
CCGCCCCATGGCTGACTAATTTTTTTTATTTATGCAGAGGCCGAGGCCGCC
TCGGCCTCTGAGCTATTCCAGAAGTAGTGAGGAGGCTTTTTTGGAGGCCTA
GGCTTTTGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SalI and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

The above protocol may be used in the generation of both transient, as well as, stable transfected cells, which would be apparent to those of skill in the art.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC 12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC 12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC 12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-κB regulates the expression of genes involved in immune cell activation, control of apoptosis (NF- κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I- κB is phosphorylated and degraded, causing NF- κB to shuttle to the nucleus, thereby activating transcription of target genes. Target genes activated by NF- κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-kB would be useful in treating diseases. For example, inhibitors of NF-κB could be used to treat those diseases related to the acute or chronic activation of NF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGGACTTTCCGGGACTTTCC
ATCTGCCATCTCAATTAGTCAGCAACCATAGTCCCGCCCCTAACTCCGCCC

ATCCCGCCCCTAACTCCGCCCAGTTCCGCCCATTCTCCGCCCCATGGCTGA
CTAATTTTTTTTATTTATGCAGAGGCCGAGGCCGCCTCGGCCTCTGAGCTA
TTCCAGAAGTAGTGAGGAGGCTTTTTTGGAGGCCTAGGCTTTTGCAAAAA
GCTT:3' (SEQ ID NO:10)

Next, replace the SV40 minimal promoter element present in the pSEAP2-promoter plasmid (Clontech) with this NF-κB/SV40 fragment using XhoI and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes SalI and NotI, and inserted into a vector containing neomycin resistance. Particularly, the NF-κB/SV40/SEAP cassette was inserted into pGFP-1 (Clontech), replacing the GFP gene, after restricting pGFP-1 with SalI and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-4 (Molecular Probes, Inc.; catalog no. F-14202), used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-4 is made in 10% pluronic acid DMSO. To load the cells with fluo-4, 50 ul of 12 ug/ml fluo-4 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml fluo-4 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-4. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCI, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 xg.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂+ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl₂, 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1
and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place
of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The secreted polypeptide is also suitably administered by sustained-release systems. Suitable examples of sustained-release compositions include semipermeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

### Example 27: Method of Treatment Using Gene Therapy - In Vivo

Another aspect of the present invention is using *in vivo* gene therapy methods to treat disorders, diseases and conditions. The gene therapy method relates to the introduction of naked nucleic acid (DNA, RNA, and antisense DNA or RNA) sequences into an animal to increase or decrease the expression of the polypeptide. The polynucleotide of the present invention may be operatively linked to a promoter or any other genetic elements necessary for the expression of the polypeptide by the target tissue. Such gene therapy and delivery techniques and methods are known in the art, see, for example, W090/11092, W098/11779; U.S. Patent NO. 5693622, 5705151, 5580859; Tabata H. et al. (1997) Cardiovasc. Res. 35(3):470-479, Chao J et al. (1997) Pharmacol. Res. 35(6):517-522, Wolff J.A. (1997) Neuromuscul. Disord. 7(5):314-318, Schwartz B. et al. (1996) Gene Ther. 3(5):405-.411, Tsurumi Y. et al. (1996) Circulation 94(12):3281-3290 (incorporated herein by reference).

The polynucleotide constructs may be delivered by any method that delivers injectable materials to the cells of an animal, such as, injection into the interstitial space of tissues (heart, muscle, skin, lung, liver, intestine and the like). The polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, the polynucleotides of the present invention may also be delivered in liposome formulations (such as those taught in Felgner P.L. et al. (1995) Ann. NY Acad. Sci. 772:126-139 and Abdallah B. et al. (1995) Biol. Cell 85(1):1-7) which can be prepared by methods well known to those skilled in the art.

The polynucleotide vector constructs used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. *In vivo* muscle cells are particularly competent in their ability to take up and express polynucleotides.

For the naked polynucleotide injection, an effective dosage amount of DNA or RNA will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked polynucleotide constructs can be delivered to arteries during angioplasty by the catheter used in the procedure.

The dose response effects of injected polynucleotide in *muscle in vivo* is determined as follows. Suitable template DNA for production of mRNA coding for polypeptide of the present invention is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitbneal injection with 0.3 ml of 2.5% Avertin. A 1.5 cm incision is made on the anterior thigh, and the quadriceps muscle is directly visualized. The template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e.g., 7 days) muscle extracts are prepared by excising the entire quadriceps. Every fifth 15 um cross-section of the individual quadriceps muscles is histochemically stained for protein expression. A time course for protein expression may be done in a similar fashion except that quadriceps from different mice are harvested at different times. Persistence of DNA in muscle following injection may be determined by Southern blot analysis after preparing total cellular DNA and HIRT supernatants from injected and control mice. The results of the above experimentation in mice can be use to extrapolate proper dosages and other treatment parameters in humans and other animals using naked DNA.

### Example 28: Transgenic Animals.

The polypeptides of the invention can also be expressed in transgenic animals. Animals of any species, including, but not limited to, mice, rats, rabbits, hamsters, guinea pigs, pigs, micro-pigs, goats, sheep, cows and non-human primates, *e.g.*, baboons, monkeys, and chimpanzees may be used to generate transgenic animals. In a specific embodiment, techniques described herein or otherwise known in the art, are used to express polypeptides of the invention in humans, as part of a gene therapy protocol.

Any technique known in the art may be used to introduce the transgene (i.e., polynucleotides of the invention) into animals to produce the founder lines of transgenic animals. Such techniques include, but are not limited to, pronuclear microinjection (Paterson et al., Appl. Microbiol. Biotechnol. 40:691-698 (1994); Carver et al., Biotechnology (NY) 11:1263-1270 (1993); Wright et al., Biotechnology (NY) 9:830-834 (1991); and Hoppe et al., U.S. Pat. No. 4,873,191 (1989)); retrovirus mediated gene transfer into germ lines (Van der Putten et al., Proc. Natl. Acad. Sci., USA 82:6148-6152 (1985)), blastocysts or embryos; gene targeting in embryonic stem cells (Thompson et al., Cell 56:313-321 (1989)); electroporation of cells or embryos (Lo, 1983, Mol Cell. Biol. 3:1803-1814 (1983)); introduction of the polynucleotides of the invention using a gene gun (see, e.g., Ulmer et al., Science 259:1745 (1993); introducing nucleic acid constructs into embryonic pleuripotent stem cells and transferring the stem cells back into the blastocyst; and sperm-mediated gene transfer (Lavitrano et al., Cell 57:717-723 (1989); etc. For a review of such techniques, see Gordon, "Transgenic Animals," Intl. Rev. Cytol. 115:171-229 (1989), which is incorporated by reference herein in its entirety.

Any technique known in the art may be used to produce transgenic clones containing polynucleotides of the invention, for example, nuclear transfer into enucleated oocytes of nuclei from cultured embryonic, fetal, or adult cells induced to quiescence (Campell et al., Nature 380:64-66 (1996); Wilmut et al., Nature 385:810-813 (1997)).

The present invention provides for transgenic animals that carry the transgene in all their cells, as well as animals which carry the transgene in some, but not all their cells, *i.e.,* mosaic animals or chimeric. The transgene may be integrated as a single transgene or as multiple copies such as in concatamers, *e.g.,* head-to-head tandems or head-to-tail tandems. The transgene may also be selectively introduced into and activated in a particular cell type by following, for example, the teaching of Lasko et al. (Lasko et al., Proc. Natl. Acad. Sci. USA 89:6232-6236 (1992)). The regulatory sequences required for such a cell-type specific activation will depend upon the particular cell type of interest, and will be apparent to those of skill in the art. When it is desired that the polynucleotide transgene be integrated into the chromosomal site of the endogenous gene, gene targeting is preferred. Briefly, when such a technique is to be utilized, vectors containing some nucleotide sequences homologous to the endogenous gene are designed for the purpose of integrating, via homologous recombination with chromosomal sequences, into and disrupting the function of the nucleotide sequence of the endogenous gene. The transgene may also be selectively introduced into a particular cell type, thus inactivating the endogenous gene in only that cell type, by following, for example, the teaching of Gu et al. (Gu et al., Science 265:103-106 (1994)). The regulatory sequences required for such a cell-type specific inactivation will depend upon the particular cell type of interest, and will be apparent to those of skill in the art.

Once transgenic animals have been generated, the expression of the recombinant gene may be assayed utilizing standard techniques. Initial screening may be accomplished by Southern blot analysis or PCR techniques to analyze animal tissues to verify that integration of the transgene has taken place. The level of mRNA expression of the transgene in the tissues of the transgenic animals may also be assessed using techniques which include, but are not limited to, Northern blot analysis of tissue samples obtained from the animal, *in situ* hybridization analysis, and reverse transcriptase-PCR (rt-PCR). Samples of transgenic gene-expressing tissue may also be evaluated immunocytochemically or immunohistochemically using antibodies specific for the transgene product.

Once the founder animals are produced, they may be bred, inbred, outbred, or crossbred to produce colonies of the particular animal. Examples of such breeding strategies include, but are not limited to: outbreeding of founder animals with more than one integration site in order to establish separate lines; inbreeding of separate lines in order to produce compound transgenics that express the transgene at higher levels because of the effects of additive expression of each transgene; crossing of heterozygous transgenic animals to produce animals homozygous for a given integration site in order to both augment expression and eliminate the need for screening of animals by DNA analysis; crossing of separate homozygous lines to produce compound heterozygous or homozygous lines; and breeding to place the transgene on a distinct background that is appropriate for an experimental model of interest.

Transgenic animals of the invention have uses which include, but are not limited to, animal model systems useful in elaborating the biological function of polypeptides of the present invention, studying conditions and/or disorders associated with aberrant expression, and in screening for compounds effective in ameliorating such conditions and/or disorders.

### Example 29: Knock-Out Animals.

Endogenous gene expression can also be reduced by inactivating or "knocking out" the gene and/or its promoter using targeted homologous recombination. *(E.g.,* see Smithies et al., Nature 317:230-234 (1985); Thomas & Capecchi, Cell 51:503-512 (1987); Thompson et al., Cell 5:313-321 (1989); each of which is incorporated by reference herein in its entirety). For example, a mutant, non-functional polynucleotide of the invention (or a completely unrelated DNA sequence) flanked by DNA homologous to the endogenous polynucleotide sequence (either the coding regions or regulatory regions of the gene) can be used, with or without a selectable marker and/or a negative selectable marker, to transfect cells that express polypeptides of the invention *in vivo.* In another embodiment, techniques known in the art are used to generate knockouts in cells that contain, but do not express the gene of interest. Insertion of the DNA construct, via targeted homologous recombination, results in inactivation of the targeted gene. Such approaches are particularly suited in research and agricultural fields where modifications to embryonic stem cells can be used to generate animal offspring with an inactive targeted gene (*e.g.*, see Thomas & Capecchi 1987 and Thompson 1989, *supra).* However this approach can be routinely adapted for use in humans provided the recombinant DNA constructs are directly administered or targeted to the required site *in vivo* using appropriate viral vectors that will be apparent to those of skill in the art.

In further embodiments of the invention, cells that are genetically engineered to express the polypeptides of the invention, or alternatively, that are genetically engineered not to express the polypeptides of the invention (e.g., knockouts) are administered to a patient *in vivo.* Such cells may be obtained from the patient (i.e., animal, including human) or an MHC compatible donor and can include, but are not limited to fibroblasts, bone marrow cells, blood cells (eg., lymphocytes), adipocytes, muscle cells, endothelial cells etc. The cells are genetically engineered *in vitro* using recombinant DNA techniques to introduce the coding sequence of polypeptides of the invention into the cells, or alternatively, to disrupt the coding sequence and/or endogenous regulatory sequence associated with the polypeptides of the invention, e.g., by transduction (using viral vectors, and preferably vectors that integrate the transgene into the cell genome) or transfection procedures, including, but not limited to, the use of plasmids, cosmids, YACs, naked DNA, electroporation, liposomes, etc. The coding sequence of the polypeptides of the invention can be placed under the control of a strong constitutive or inducible promoter or promoter/enhancer to achieve expression, and preferably secretion, of the polypeptides of the invention. The engineered cells which express and preferably secrete the polypeptides of the invention can be introduced into the patient systemically, e.g., in the circulation, or intraperitoneally.

Alternatively, the cells can be incorporated into a matrix and implanted in the body, e.g., genetically engineered fibroblasts can be implanted as part of a skin graft; genetically engineered endothelial cells can be implanted as part of a lymphatic or vascular graft. (See, for example, Anderson et al. U.S. Patent No. 5,399,349; and Mulligan & Wilson, U.S. Patent No. 5,460,959 each of which is incorporated by reference herein in its entirety).

When the cells to be administered are non-autologous or non-MHC compatible cells, they can be administered using well known techniques which prevent the development of a host immune response against the introduced cells. For example, the cells may be introduced in an encapsulated form which, while allowing for an exchange of components with the immediate extracellular environment, does not allow the introduced cells to be recognized by the host immune system.

Transgenic and "knock-out" animals of the invention have uses which include, but are not limited to, animal model systems useful in elaborating the biological function of polypeptides of the present invention, studying conditions and/or disorders associated with aberrant expression, and in screening for compounds effective in ameliorating such conditions and/or disorders.

It will be clear that the invention may be practiced otherwise than as particularly described in the foregoing description and examples. Numerous modifications and variations of the present invention are possible in light of the above teachings and, therefore, are within the scope of the'appended claims.

The entire disclosure of each document cited (including patents, patent applications, journal articles, abstracts, laboratory manuals, books, or other disclosures) in the Background of the Invention, Detailed Description, and Examples is hereby incorporated herein by reference. Further, the hard copy of the sequence listing submitted herewith and the corresponding computer readable form are both incorporated herein by reference in their entireties.

INDICATIONS RELATING TO A DEPOSITED MICROORGANISM

(PCT Rule 13bis)

A. The indications made below relate to the microorganism referred to in the description
on page 131 , line N/A

B. IDENTIFICATION OF DEPOSIT | Further deposits are identified on an additional sheet [ ]

Name of depositary institution American Type Culture Collection

Address of depositary institution (including postal code and country)
10801 University Boulevard
Manassas, Virginia 20110-2209
United States of America

Date of deposit | Accession Number
January 6, 1998 | 209568

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
[ ] This sheet was received with the international application | [ ] This sheet was received by the International Bureau on:
Authorized officer | Authorized officer

Form PCT/RO/134 (July 1992)

INDICATIONS RELATING TO A DEPOSITED MICROORGANISM

(PCT Rule 13bis)

A. The indications made below relate to the microorganism referred to in the description
on page 13b , line N/A .

B. IDENTIFICATION OF DEPOSIT | Further deposits are identified on an additional sheet ☐

Name of depositary institution American Type Culture Collection

Address of depositary institution (including postal code and country)
10801 University Boulevard
Manassas, Virginia 20110-2209
United States of America

Date of deposit | Accession Number
January 14, 1998 | 209580

C. ADDITIONAL INDICATIONS (leave blank if not applicable) | This information is continued on an additional sheet ☐

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession Number of Deposit")

For receiving Office use only | For International Bureau use only
☐ This sheet was received with the international application | ☐ This sheet was received by the International Bureau on:
Authorized officer | Authorized officer

Form PCT/RO/134 (July 1992)

## Claims

1. A nucleic acid molecule comprising a polynucleotide having a nucleotide sequence selected from the group consisting of:
(a) a polynucleotide having the entire nucleotide sequence of SEQ ID NO:19;
(b) a polynucleotide having the entire cDNA sequence included in ATCC Deposit No. 209568, which is hybridizable to SEQ ID NO:19;
(c) a polynucleotide fragment of SEQ ID NO:19;
(d) a polynucleotide fragment of the cDNA sequence included in ATCC Deposit No. 209568, which is hybridizable to SEQ ID NO:19;
(e) a polynucleotide encoding a polypeptide fragment of SEQ ID NO:87;
(f) a polynucleotide encoding a polypeptide fragment encoded by the cDNA sequence included in ATCC Deposit No. 209568, which is hybridizable to SEQ ID NO:19;
(g) a polynucleotide encoding a polypeptide fragment of SEQ ID NO:87 or a polypeptide fragment encoded by the cDNA sequence included in ATCC Deposit No. 209568, having biological activity;
(h) a polynucleotide encoding a polypeptide fragment of SEQ ID NO:87 or a polypeptide fragment encoded by the cDNA sequence included in ATCC Deposit No. 209568, which is secreted;
(i) a polynucleotide encoding a full length polypeptide of SEQ ID NO:87 or being encoded by the cDNA sequence included in ATCC Deposit No. 209568;
(j) a polynucleotide encoding a polypeptide domain of SEQ ID NO:87;
(k) a polynucleotide encoding a polypeptide domain encoded by the cDNA sequence included in ATCC Deposit No. 209568, which is hybridizable to SEQ ID NO:19;
(l) a polynucleotide encoding a polypeptide epitope of SEQ ID NO: 87;
(m) a polynucleotide encoding a polypeptide epitope encoded by the cDNA sequence included in ATCC Deposit No. 209568, which is hybridizable to SEQ ID NO:19;
(n) a polynucleotide which is at least 90% identical to a polynucleotide as defined in any one of (a) to (m);
(o) a polynucleotide encoding a polypeptide having an amino acid sequence at least 95% identical to the amino acid sequence of a polypeptide encoded by a polynucleotide of any one of (a) to (n);
(p) the polynucleotide of any one of (a) to (o), wherein the polynucleotide fragment comprises a nucleotide sequence encoding a secreted protein; and
(q) a polynucleotide capable of hybridizing under stringent conditions to any one of the polynucleotides specified in (a) to (p), wherein said polynucleotide does not hybridize under stringent conditions to a nucleic acid molecule having a nucleotide sequence of only A residues or of only T residues;
or the complementary strand of polynucleotide (a) to (q).

2. The nucleic acid molecule of claim 1, wherein the nucleotide sequence comprises sequential nucleotide deletions from either the 5' terminus or the 3' terminus.

3. The nucleic acid molecule of claim 1 or 2 which is DNA or RNA.

4. A gene corresponding to the cDNA sequence of SEQ ID NO:19 or encoding the polypeptide having the amino acid sequence of SEQ ID NO:87.

5. A vector comprising the nucleic acid molecule of any one of claims 1 to 3 or the gene of claim 4.

6. A method of making a recombinant host cell comprising introducing the nucleic acid molecule of any one of claims 1 to 3, the gene of claim 4 or the vector of claim 5.

7. A recombinant host cell containing the nucleic acid molecule of claims 1 to 3, the gene of claim 4 or the vector of claim 5 or produced by the method of claim 6.

8. The recombinant host cell of claim 7 which expresses a polypeptide encoded by the nucleic acid molecule of any one of claims 1 to 3 or the gene of claim 4.

9. A method of making a polypeptide encoded by the nucleic acid molecule of any one of claims 1 to 3 or the gene of claim 4 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3, the gene of claim 4 or obtainable by the method of claim 9.

11. An antibody that binds specifically to the polypeptide of claim 10.

12. An antagonist of the polypeptide of claim 10 comprising a molecule closely related to the natural ligand of said polypeptide or a polypeptide related to the natural receptor of the polypeptide of claim 10 or a fragment thereof.

13. An agonist of the polypeptide of claim 10 comprising a molecule closely related to the natural ligand of said polypeptide or a polypeptide related to the natural receptor of the polypeptide of claim 10 or a fragment thereof.

14. A method for identifying antagonists or agonists of the polypeptide of claim 10 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

15. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

16. An in vitro method of diagnosing a pathological condition or a susceptibility to a pathological condition in a subject comprising:
(a) determining the presence or amount of expression of the polypeptide of claim 10 in a biological sample; and
(b) diagnosing a pathological condition or a susceptibility to a pathological condition based on the presence or amount of expression of the polypeptide.

17. A method for identifying a binding partner to the polypeptide of claim 10 comprising:
(a) contacting the polypeptide of claim 10 with a binding partner; and
(b) determining whether the binding partner effects an activity of the polypeptide.

18. A method of identifying an activity in a biological assay, wherein the method comprises:
(a) expressing the nucleic acid molecule of any one of claims 1 to 3 or the gene of claim 4 in a cell;
(b) isolating the supernatant;
(c) detecting an activity in a biological assay; and
(d) identifying the protein in the supernatant having the activity.

19. The product produced by the method of any one of claims 14, 17 and 18.

20. A pharmaceutical composition comprising the nucleic acid molecule of any one of claims 1 to 3, the gene of claim 4, the polypeptide of claim 10, a DNA encoding and capable of expressing said polypeptide in vivo, the antibody of claim 11, the antagonist of claim 12, the agonist of claim 13 or the product of claim 19 and optionally a pharmaceutically acceptable carrier.

21. Use of the nucleic acid molecule of any one of claims 1 to 3, the polypeptide of claim 10, the gene of claim 4, a DNA encoding and capable of expressing said polypeptide in vivo the antibody of claim 11, antagonist of claim 12, the agonist of claim 13 or the product of claim 19 for the preparation of a pharmaceutical composition for preventing, treating, or ameliorating a medical condition.

22. A diagnostic composition comprising the nucleic acid molecule of any one of claims 1 to 3, the gene of claim 4 or the antibody of claim 11.

23. A method for the production of a pharmaceutical composition comprising the steps of the method of any one of claim 14, 17 or 18 and the further step of formulating the compound identified in a pharmaceutically acceptable form.


<110> Human Genome Sciences, Inc.et al.

<120> 67 Human secreted proteins

<130> PZ023PCT

<140> Unassigned
<141> 1999-01-27

<150> 60/073,164
<151> 1998-01-30

<150> 60/073,165
<151> 1998-01-30

<150> 60/073,159
<151> 1998-01-30

<150> 60/073,160
<151> 1998-01-30

<150> 60/073,170
<151> 1998-01-30

<150> 60/073,161
<151> 1998-01-30

<150> 60/073,162
<151> 1998-01-30

<150> 60/073,167
<151> 1998-01-30

<160> 275

<170> PatentIn Ver. 2.0


<210> 1
<211> 733
<212> DNA
<213> Homo sapiens

<400> 1
gggatccgga gcccaaatct tctgacaaaa ctcacacatg cccaccgtgc ccagcacctg 60
aattcgaggg tgcaccgtca gtcttcctct tccccccaaa acccaaggac accctcatga 120
tctcccggac tcctgaggtc acatgcgtgg tggtggacgt aagccacgaa gaccctgagg 180
tcaagttcaa ctggtacgtg gacggcgtgg aggtgcataa tgccaagaca aagccgcggg 240
aggagcagta caacagcacg taccgtgtgg tcagcgtcct caccgtcctg caccaggact 300
ggctgaatgg caaggagtac aagtgcaagg tctccaacaa agccctccca acccccatcg 360
agaaaaccat ctccaaagcc aaagggcagc cccgagaacc acaggtgtac accctgcccc 420
catcccggga tgagctgacc aagaaccagg tcagcctgac ctgcctggtc aaaggcttct 480
atccaagcga catcgccgtg gagtgggaga gcaatgggca gccggagaac aactacaaga 540
ccacgcctcc cgtgctggac tccgacggct ccttcttcct ctacagcaag ctcaccgtgg 600
acaagagcag gtggcagcag gggaacgtct tctcatgctc cgtgatgcat gaggctctgc 660
acaaccacta cacgcagaag agcctctccc tgtctccggg taaatgagtg cgacggccgc 720
gactctagag gat 733

<210> 2
<211> 5
<212> PRT
<213> Homo sapiens

<220>
<221> Site
<222> (3)
<223> Xaa equals any of the twenty naturally ocurring L-amino acids

<400> 2
Trp Ser Xaa Trp Ser
 1               5

<210> 3
<211> 86
<212> DNA
<213> Homo sapiens

<400> 3
gcgcctcgag atttccccga aatctagatt tccccgaaat gatttccccg aaatgatttc      60
cccgaaatat ctgccatctc aattag                                           86

<210> 4
<211> 27
<212> DNA
<213> Homo sapiens

<400> 4
gcggcaagct ttttgcaaag cctaggc                                          27

<210> 5
<211> 271
<212> DNA
<213> Homo sapiens

<400> 5
ctcgagattt ccccgaaatc tagatttccc cgaaatgatt tccccgaaat gatttccccg      60
aaatatctgc catctcaatt agtcagcaac catagtcccg cccctaactc cgcccatccc     120
gcccctaact ccgcccagtt ccgcccattc tccgccccat ggctgactaa ttttttttat     180
ttatgcagag gccgaggccg cctcggcctc tgagctattc cagaagtagt gaggaggctt     240
ttttggaggc ctaggctttt gcaaaaagct t                                    271

<210> 6
<211> 32
<212> DNA
<213> Homo sapiens

<400> 6
gcgctcgagg gatgacagcg atagaacccc gg                                    32

<210> 7

<211> 31
<212> DNA
<213> Homo sapiens

<400> 7
gcgaagcttc gcgactcccc ggatccgcct c                                    31


<210> 8
<211> 12
<212> DNA
<213> Homo sapiens

<400> 8
ggggactttc cc                                                         12


<210> 9
<211> 73
<212> DNA
<213> Homo sapiens

<400> 9
gcggcctcga ggggactttc ccggggactt tccggggact ttccgggact ttccatcctg      60
ccatctcaat tag                                                        73


<210> 10
<211> 256
<212> DNA
<213> Homo sapiens

<400> 10
ctcgagggga ctttcccggg gactttccgg ggactttccg ggactttcca tctgccatct      60
caattagtca gcaaccatag tcccgcccct aactccgccc atcccgcccc taactccgcc     120
cagttccgcc cattctccgc cccatggctg actaattttt tttatttatg cagaggccga     180
ggccgcctcg gcctctgagc tattccagaa gtagtgagga ggcttttttg gaggcctagg     240
cttttgcaaa aagctt                                                     256


<210> 11
<211> 1079
<212> DNA
<213> Homo sapiens

<400> 11
ggcacgagcc aatttgccaa ggttctaaag gcttatgagg tcctgaagga gccaggcctt      60
gtgatggagt aggtgacaca ggcctggttg tcctgtcagc'agaagggaaa gcaggggctg     120
ggctgagagg aggacacgga gggctctgct gaggttcctt cctgggttcc accaacaggg     180
acagggagtc acttgccttc cagttctgtg ctgggatggc gggacagcac ttggcttgct     240
tggccagctg cgtcatgagt ttgatttggt tttttttttt ttgcagctgc ttcatatgct     300
ctgctccagc ccctccccaa cagctggtag cttatggttt cttcaagagg aaagtagact     360
ttatgctgta catttgagct gtagagctaa gattcgctta ctggtgagct gtgaaacctt     420
gttgcttttt cccagagtct gatggcagtg actgtgatca agggaatctt caccgccaca     480
agtgcaggca gcaggtgtgg ttcaggtccc cccccacccc actgtgctcc tttgaagcca     540
acgtgcctcc ctcgcctcca tactggaggg acgacgcagg ggagaacaga gaagtgcttg     600
gccctaggat tgaggcactt gtttcctagc ccgctgggtt agggctggtg caagcgaggc     660
aatgttgagg atgctttaag cactaccagc cgaatccggg aactctgtta acagttgtcc     720

aaccagcaga atgaggctaa ctgtataaag catgggaccc aggatgagga taaggaaagg 780
acagcggctt tccctgggca gtacaatggc ttgaaggcaa aaagggataa agtgacagcc 840
gactgtgact ctggtgagga ggggtgagca gggaggttga ttctctgatg ttaactaagt 900
ggcaaagtct caaccgtgct cagccctccc cctcccaggg aagagaaaca aagattcaaa 960
gtaagcatga tactagtggg tttaccagtg tttcttccaa ggagacatat attttttaat 1020
aaacgatagt tgcaatgaaa aaaaaaaaa aaaaaaaaa aaaaaaaaa aaaaaaaa 1079

<210> 12
<211> 1932
<212> DNA
<213> Homo sapiens

<400> 12
   cccgcagcag ctcccaggat gaactggttg cagtggctgc tgctgctgcg ggggcgctga 60
   gaggacacga gctctatgcc tttccggctg ctcatcccgc tcggcctcct gtgcgcgctg 120
   ctgcctcagc accatggtgc gccaggtccc gacggctccg cgccagatcc cgcccactac 180
   agggagcgag tcaaggccat gttctaccac gcctacgaca gctacctgga gaatgccttt 240
   cccttcgatg agctgcgacc tctcacctgt gacgggcacg acacctgggg cagtttttct 300
   ctgactctaa ttgatgcact ggacaccttg ctgattttgg ggaatgtctc agaattccaa 360
   agagtggttg aagtgctcca ggacagcgtg gactttgata ttgatgtgaa cgcctctgtg 420
   tttgaaacaa acattcgagt ggtaggagga ctcctgtctg ctcatctgct ctccaagaag 480
   gctggggtgg aagtagaggc tggatggccc tgttccgggc ctctcctgag aatggctgag 540
   gaggcggccc gaaaactcct cccagccttt cagaccccca ctggcatgcc atatggaaca 600
   gtgaacttac ttcatggcgt gaacccagga gagacccctg tcacctgtac ggcagggatt 660
   gggaccttca ttgttgaatt tgccaccctg agcagcctca ctggtgaccc ggtgttcgaa 720
   gatgtggcca gagtggcttt gatgcgcctc tgggagagcc ggtcagatat cgggctggtc 780
   ggcaaccaca ttgatgtgct cactggcaag tgggtggccc aggacgcagg catcggggct 840
   ggcgtggact cctactttga gtacttggtg aaaggagcca tcctgcttca ggataagaag 900
   ctcatggcca tgttcctaga gtataacaaa gccatycgga actacacccg cttcgatgac 960
   tggtacctgt gggtwcagat gtacaagggg actgtgtcca tgccagtctt ccagtccytr 1020
   gaggcctact ggcctggtct kcagagcctc rttggrgaca ttgacaatgc catgaggacc 1080
   ttcctcaact actacactrt atggaagcag tttggggggc tcccrgaatt ctacaacatt 1140
   cctcagggat acacagtgga gaagcgagag ggctacccwc ttcggccaga actyattgar 1200
   agcgcaatgt acctctaccg tgccacgggg gaycccaccc tcytagaact cggaagagat 1260
   gctgtggaat ccattgaaaa aatcagcaag gtggagtgyg gatttgcaac aatcaaagat 1320
   ctgcgagacc acaagctgga caaccgcatg gagtckttct tcctggccga gacygtgaaa 1380
   tacctctacc tyctgttyga cccrrccaac ttcatccaca acaayggstc caccttcgac 1440
   gcggtgatca ccccctatgg ggagtgcatc ctgggggctg gggggtacat cttcaacaca 1500
   gaagctcacc ccatcgaccc tgccgccctg cactgctgcc agaggctgaa ggaagagcag 1560
   tgggaggtgg aggacttgat gagggaattc tactctctca aacggagcag gtcgaaattt 1620
   cagaaaaaca ctgttagttc ggggccatgg gaacctccag caaggccagg aacactcttc 1680
   tcaccagaaa accatgacca ggcaagggag aggaagcctg ccaaacagaa ggtcccactt 1740
   ctcagctgcc ccagtcagcc cttcacctcc aagttggcat tactgggaca ggttttccta 1800
   gactcctcat aaccactgga taattttttt atttttattt ttttgaggct aaactataat 1860
   aaattgcttt tggctatcaa aaaaaaaaaa aaaaaaaaaa aaaaaaaaaa aaaaaaaaaa 1920
   aagggcggcc gc 1932

<210> 13
<211> 1827
<212> DNA
<213> Homo sapiens

<400> 13
caaactgcac gacatcgacg gcgtacctca cctcatcctc atcgcctccc gagacatcga 60
ggctggggag gagctcctgt atgactatgg ggaccgcagc aaggcttcca ttgaagccca 120
cccgtggctg aagcattaac cggtgggccc cgtgcctccc cgccccactt tcccttcttc 180

aaaggacaaa gtgccctcaa agggaattga attttttttt tacacactta atcttagcgg 240
attacttcag atgtttttaa aaagtatatt aagatgcctt ttcactgtag tatttaaata 300
tctgttacag gtttccaagg tggacttgaa cagatggcct tatattacca aaacttttat 360
attctagttg tttttgtact ttttttgcat acaagccgaa cgtttgtgct tcccgtgcat 420
gcagtcaaag actcagcaca ggttttagag gaaatagtca aacatgaact aggaagccag 480
gtgagtctcc tttctccagt ggaagagccg ggaccttccc cctgcacccc cgacatccag 540
ggacggggtg tgaggaagac gctgcctccc aatggcctgg acgggatgtt tccaagctct 600
tgttccccta acgtctcaac aggcgctcac tgaagtgtat gaatattttt taaaaaggtt 660
tttgcagtaa gctagtcttc ccctctgctt tctcgaaagc ttactgagcc ctgggcccca 720
agcacgggcc gggcatagat ttcctcttcc acaagtgccg cttttctggg caccttgaag 780
catcagggcg tgaaatcaaa ctagatgtgg gcagggagag kgttgcttac ctgcctgctg 840
gggcagggtt tcctgaaact gggttaattc tttatagaaa tgtgaacact gaatttattt 900
taaaaaataa taataaaaat ttaaaaaaat taaaaataaa aaaaaccaca gaaaacaact 960
ttacatgtat ataggtcttg aagtgagtga agtggctgct tttttttttt tttttttttt 1020
gctttttttt gctttttgta gaagagattg agaatggtac tctaatcaaa aataaagttt 1080
tgtagtggga ccagaaatta cttacctgac atccaccccc attccccctc atcctgctgg 1140
ggttgaaagt tccagacctg ctgtcgaggc cttgtgtttg tcagacaccc agtgtcctcc 1200
tgcaaggacg caactgtgag ctgaggtgtg agcctaggag cccaggaccc ctgaccccgg 1260
ccgctgctgc cagcctcaga aaggcaccca ggtgtgcagg ggagcacaca gggcccggca 1320
gcccccagga atcaaggata gggctaaggt tttcacctta actgtgaagg caggaggaat 1380
aggtgactgc ttcctcccgc ccttcacaga actgattctc acacactgtc ccttcagtcc 1440
agggggccgg ggctcaggag ccatgacctg gtgtctcctg cccaccctgg tcccaggtaa 1500
atgtgaatgg agacaggtat gagaggctgt cctcgtcttt gattcccccc caaccccacc 1560
tcgggcctca cgacggtgct acctaagaaa gtcttccctc ccaccccccg ctagcctggt 1620
cagtggtcag caaattggaa gaggatccga tgggagtgta aatgtgagac acaatgtctt 1680
gattatacct gtttgtggtt tagctttgta tttaaacaag gaaataaact tgaaaattat 1740
ttgtcatcat aaaaatgaaa caaattaaaa tatttattgc caggcaaaaa aaaaaaaaaa 1800
aaaaaaaaaa aaaaaaaaaa aaaaaaa 1827

<210> 14
<211> 696
<212> DNA
<213> Homo sapiens

<400> 14
ggcacgaggt ggaggagaaa tttaacagtc ctctcatgca gacggagggt gacattcaaa 60
tgggagaatt tacttctgtg gtttgctact gtttcattct ttcccttatc attggtagtg 120
ttgttaggtg gcagggttgt ggggcagagt ggggtttcgc cctgggggag catatgtggc 180
agagggcaca ggaagatctg taagcaagag ggcatagcaa attaaatgac cacactgtca 240
ggaaggttga caggccaaag aaagatcagc tcctccaaat ctgctgaact aactctcccc 300
tcgtagcccc agacacgttt tctcaatttg agcacaatat ccattactat ttcccgtact 360
gggtttcaat taaagagagt gagagtagaa agttcactgg tgtttggggg ttcatttatt 420
tccaagcagg atgcaaatga aagggagccg tgggcacaga gttgtcatgt gtgtttttcc 480
tccctcttct ttccatttcc ttcttgcaac cttccctcca cttcttgcca gccacccagc 540
acacccgtgt tcccaaagca aatgttttca wgtcttgaaa atccagttag ggtgaggaga 600
gaaggaaggt gataacatca tacctactga tgccccctag agatgaagct gtcctggggg 660
cacttaaggc ttgagggaag gatttacctt ctcgag 696

<210> 15
<211> 1684
<212> DNA
<213> Homo sapiens

<220>
<221> SITE
<222> (736)

<223> n equals a,t,g, or c

<400> 15
gtatccgcga cgagctatcc gggaaagggc cgaatgcgat caaacctaat ccgcgagact 60
tgctaaggtt ctgtgctaca aattgatgtt tagataaact tcagtgaaat gactcttcag 120
gaattggtgc ataaggctgc ctcctgytat atggacagag tagctgtatg ttttgatgaa 180
tgcaacaacc agcttccagt ttactacacc tacaagactg tggttaatgc tgcttctgaa 240
ttatcaaatt ttctgctgtt acactgtgac tttcaaggaa ttcgggaaat tggtctctac 300
tgccaacctg ggatagactt accctcttgg attttaggaa ttctccaagt cccggctgct 360
tatgtaccta tcgagccaga ttcaccaccg tcattatcaa ctcattttat gaaaaaatgt 420
aatctaaagt atatccttgt tgaaaaaaaa caaattaata aatttaaatc ttttcatgaa 480
acattattga actatgatac atttacagtg gaacataatg acctagtgct cttcagactt 540
cactggaaaa atactgaggt gaacttgatg ctaaatgatg gaaaagagaa atatgaaaaa 600
gaaaaaataa aaagcataag ttctgagcat gtcaatgaag aaaaagcaga agaacacatg 660
gatctgaggs taaagcattg cttagcctat gttctacata catcagggac tacagggata 720
ccgaagattg tcagantgcc tcataagtgt atagtaccaa atatccagca ttttcgggta 780
ctttttgaca tcacacaaga agatgttttg tttctgkytt cacctytgac cttcgatcct 840
tctgttgtgg aaatatttct tgctctatca agtggtgcct ctctgcttat tgtaccaact 900
tctgtcaagt tgctcccatc aaaattagcc agcgttctct tttcccatca tagagtgact 960
gttttgcagg caacaccaac attgcttaga agatttggat ctcagcttat caagtcaact 1020
gttttgtcag ccactacttc tcttcgagta ttagcccttg gtggtgaagc gtttccatca 1080
ttgacagttc tcagaagctg gagaggagaa ggcaataaaa cacaaatatt taatgtttat 1140
ggtatcacag aggtatcaag ttgggcgacc attwatagga ttccagagaa gactcttaac 1200
tctactctca aatgtgaatt gcctgwacaa ctgggatttc cacttcttgg aacagtagtt 1260
gaagtcagag atactaatgg cttcacaatt caggaaggca gtggccaagt atttttaggt 1320
tgttttatat ttgttgattg ggaatttttt tttcaagaaa aatgatctga tgtgttaatt 1380
ttattccttt cgtctttttc ttttgtctat ctcatgcttt tcagtgataa tttttattct 1440
cattcatata gtcatgaaat accaaatgtt acaataatta tttcagataa taatgtctaa 1500
cacattaata aaagtaattt agagactgta acttggacct tcatatttat atttatagcc 1560
aaaattatat ttaatcagta gtctaagaat ttttttaatt ccataaattt taagaaataa 1620
atttcatttt atctctgctt aaaaaaaaaa aaaaaaaaaa aaaaaaaaaa aaaagggcgg 1680
ccgc 1684

<210> 16
<211> 1523
<212> DNA
<213> Homo sapiens

<400> 16
cagacattgt tagctactga gtggcacatc ttcagtacgc atggattcgt gggggactca 60
ggcagaggta aaagtgtgaa acttttcagc attacctaag aagcaaaggc tcaattttgg 120
ctgcttcatt cttatctctt ctgccacagt tctaacgtgc ctgatctact gagaccaagg 180
atgaccaatg actcagaagg gaaaatggga tttaaacacc caaagatcat ggggaatttc 240
agaggtcatg ccctccctgg aaccttcttt tttattattg gtctttggtg gtgtacaaag 300
agtattctga agtatatctg caaaaagcaa aagcgaacct gctatcttgg ttccaaaaca 360
ttattctatc gattggaaat tttggaggga attacaatag ttggcatggc tttaactggc 420
atggctgggg agcagtttat tcctggaggg ccccatctga tgttatatga ctataaacaa 480
ggtcactgga atcaactcct gggctggcat catttcacca tgtatttctt ctttgggctg 540
ttgggtgtgg cagatatctt atgtttcacc atcagttcac ttcctgtgtc cttaaccaag 600
ttaatgttgt caaatgcctt atttgtggag gcctttatct tctacaacca cactcatggc 660
cgggaaatgc tggacatctt tgtgcaccag ctgctggttt tggtcgtctt tctgacaggc 720
ctcgttgcct tcctagagtt ccttgttcgg aacaatgtac ttctggagct attgcggtca 780
agtctcattc tgcttcaggg gagctggttc tttcagattg gatttgtcct gtatcccccc 840
agtggaggtc ctgcatggga tctgatggat catgaaaata ttttgtttct caccatatgc 900
ttttgttggc attatgcagt aaccattgtc atcgttggaa tgaattatgc tttcattacc 960
tggttggtta aatctagact taagaggctc tgctcctcag aagttggact tctgaaaaat 1020
gctgaacgag aacaagaatc agaagaagaa atgtgacttt gatgagcttc cagtttttct 1080

agataaacct tttctttttt acattgttct tggttttgtt tctcgatctt ttgtttggag 1140
aacagctggc taaggatgac tctaagtgta ctgtttgcat ttccaatttg gttaaagtat 1200
ttgaatttaa atattttctt tttagctttg aaaatatttt gggtgatact ttcattttgc 1260
acatcatgca catcatggta ttcaggggct agagtgattt ttttccagat tatctaaagt 1320
tggatgccca cactatgaaa gaaatatttg ttttatttgc cttatagata tgctcaaggt 1380
tactgggctt gctactattt gtaactcctt gaccatggaa ttatacttgt ttatcttgtt 1440
gctgcaatga gaaataaatg aatgtatgta ttttggtgca ramaaaaaaa aaaaaaaaaa 1500
aaaaaaaaaa aaagggcggc cgc 1523

<210> 17
<211> 601
<212> DNA
<213> Homo sapiens

<400> 17
ggaattcggc acgagtgcac atgtgagcat gtcacttccc tgcttaaatt tctccagtgg 60
attcccaggg acttcaggat caagtcctag ttgttcagca tggcatccaa gactctttat 120
gatctggccc ttgcttacct ctcagcctta gctctcccaa ctcttgcaca gtcactgctc 180
ttcagccata gtggatcact caccattccc agatgtacca ggctctcgca cacctctgca 240
cctttgcacg tgctgtttgc tgtgcgtgga atgcccttca ctgtcaccac cctgctcatc 300
cactctacta atgcctcttc attcttttat actcagcttt ctttaaagtt cttctaagct 360
gagttaggtg tctgtccttt atgatcccgc agtattccat gaatacgtat attctcacat 420
ttattgtact gtattataat tgttgaaaac ttgtctgtcc catttagaat gtgagctcct 480
tgagagcaga acggtgtctt cattatctct gtatccccaa ggctttgcac agtgccttgc 540
tcatagtagg ttttcaataa atgattatta aataaataaa aaaaaaaaaa aaaaactcga 600
g 601

<210> 18
<211> 2609
<212> DNA
<213> Homo sapiens

<400> 18
ggcacaggga gggtttgtgt gtatggagtg tgtcggttgt gtgagggtgt gtgtgtgagg 60
gttatgtgca tgcaaagatg tgtttagggg tgtgtgtaag aagctatgtt gagagtgtgc 120
atgtgagggt gtgtgtgtgt gtatggatgg atgcatagat gcatagatgt ttggttggta 180
ggatagatac atagatggat gggtggtttc atgcataaat ggatggatgg atggatgggt 240
ggatgcatga gtgggtggat ggttggcatg cgtgcaagaa tggatgcagg gtggatggat 300
ggatgcakga atagatgcag ggtggttgga tgatgtgtgt rtgtgtgtgt gtgtgtgtgt 360
gtgtgtgtat gtgtgtaaag tgctaagaac tgtgcattga catccaaaca tttcttgtac 420
aaaatttccc tagcaaagca aacctgcttt gacttaattt atttgttaaa tgttgcactt 480
tgtttatgta tgttttgttt ttggtgggga ataaggagag agaggacgac aaattctatt 540
gaagtattta ttttgtgaag atggcaattt tgcatttgtt taaatttttt tcattcttta 600
attttgttat cagtgccagc ccaatatacc tgctctacca ttatttgcgg tctgataaaa 660
gggtccttgt ggggcaggtt ttgcaaagct tatcaggtaa taacatatgc cacataacct 720
tgttgatatg tttgcttctg atttgggaag ctaaacattg gtgtttgaga ggattgccaa 780
ttattaattg tcattaccac tactctccat tactttttgt ttggaaattg aacaaaggtc 840
agtaatggtt tttggctctt gttaatatcc atcataaaat agattgtttt agattctttc 900
cagggtgatt tttccctggg taccccgttt ctacttctaa agaattgctt ggcactttca 960
tgtttcaaag ggaaacattc gcttgtagtt ccattttact tgatctctac aagggactga 1020
caacatttgc tttactttta ttcacagaga aagttggctt tgatgtctct taaagataat 1080
tctgctagtt gctgatcagc cagtcagttc acctagcttc aatctttata ggacttctaa 1140
tctaattttc ctatagtgtg actaaaaggg aggcaaatta ttggaacgga ttattcaaat 1200
ggatccttaa atattgctat gtataataag ccagttatta tatcaggacc atgttctctg 1260
taggccactt tctaaaaaag ccacatatgt gcaattttca ggtttttaga ctattgctcc 1320
ctgtacttta aatgtaaaaa ccacacttct gaacaactaa gctcatgaat atgattttgg 1380

ttatatgcag cttttgacta gcatgtattg tgtctttttc tcctctatga ataattttat 1440
atttcatgct acttcttgaa agtttactct ttgatgctct aagagaacag ccagatggtt 1500
tatatgaata atctttatct gcaggatggt ggattggtaa attaggagaa tgttgtttga 1560
gatatcaaga tttatgtctg ggaactaaaa tatataatgc caaatgtgtt tttgtcaatt 1620
actagagaat tctgtgcaaa catatcatct cttcaaatgc tgcacacttt gcttttgtta 1680
aacagcaggt agtagacaga acaataacag tttcgcgtta agacttttaa aggaaataga 1740
atcgtgatta agaaatcaga atttatagat atattgggat aaatgaagaa ataaaaatgt 1800
ttgtctagaa tgtagcatct agtgactttt taaagcccta acgtttacat aaagaagctc 1860
tagttcttat agaaataaca aagcaaataa aagttcttaa caatcccctc tttcgaagtg 1920
cattttttta aagcagggca ggagacattt ggactctagc tatatgacat actgggaaag 1980
gcagagggtg gagggaagat ttcacttcat tgtctagccc agaatcttga gcaagctaaa 2040
gaaaccatca taatctaaaa ttgcttcatt taacactaac aatttagact ttttaaacca 2100
agcattgaat aatggctgga taactgccga agtaagcgcc gctccatgaa gtctgcttac 2160
ttatttaaaa attgtgtatc agttttaaat actgttcatt gtgtgcagat ataaggggaa 2220
tagggcattc tgtagaatta tacatgtcta gtttgtaaag tgtgtcctgt gtactgcaga 2280
tgtgtgttct ctgggcttta tgtatctgta cagtagcttt cacattaaaa aaattgtgga 2340
caaacttgtc cggggggttt gaggggagaa tggtggttta tatcaataac gatgctgtac 2400
tatagtccat gtaacaaaag atctggaagt caccctcctc tggcccacgg aaaattttgg 2460
taatcttcta ggttctaaaa tgaagatgta tgggtactct ggcagactgc atgttgtata 2520
atttgaaaaa tactaaaagt ggaaaataaa attgaattaa actttraaaa aaaaaaaaaa 2580
agggcgcccg ctcgcgatct agaactagt 2609

<210> 19
<211> 1113
<212> DNA
<213> Homo sapiens

<400> 19
ggcacgagcg gggacggggc taagatgata tctgggcacc tcctacaaga accgactggg 60
tctccagtag tctctgagga gccgctcgac cttctcccga ccctggatct gaggcaggag 120
atgcctcccc cgcgggtgtt caagagcttt ctgagcctgc tcttccaggg gctgagcgtg 180
ttgttatccc tggcaggaga cgtgctggtc agcatgtaca gggaggtctg ttccatccgc 240
ttcctgttca cggctgtgtc gctgctgagc ctctttctgt cagcattctg gctggggctt 300
ctgtacctgg tctctccttt ggagaatgaa cctaaggaga tgctgactct aagtgagtac 360
cacgagcgcg tgcgctccca ggggcagcag ctgcagcagc tccaggccga gctggataaa 420
ctccacaagg aggtgtccac tgttcgggca gccaacagcg agagagtggc caagctcgtg 480
ttccagaggc tgaatgagga ttttgtgcgg aagcccgact atgctttgag ctctgtggga 540
gcctccatcg acctgcagaa gacatcccac gattacgcag acaggaacac tgcctacttc 600
tggaatcgct tcagcttctg gaactacgca cggccgccca cggttatcct ggagccccac 660
gtgttccctg ggaattgctg ggcttttgaa ggcgaccaag gccaggtggt gatccaactg 720
ccgggccgag tgcagctgag cgacatcact ctgcagcatc caccgcccag cgtggagcac 780
accggaggag ccaacagcgc cccccgcgat ttcgcggtct ttggcctcca ggtttatgat 840
gaaactgaag tttccttggg gaaattcacc ttcgatgttg agaaatcgga gattcagact 900
ttccacctgc agaatgaccc cccagctgcc tttcccaagg tgaagatcca gattctaagc 960
aactggggcc acccccgttt cacgtgcttg tatcgagtcc gtgcccacgg tgtgcgaacc 1020
tcagaggggg cagagggcag tgcacagggg ccccattaaa catgctgatt tttggagtaa 1080
aaaaaaaaaa aaaaaaaaaa aaaaaaaaaa aaa 1113

<210> 20
<211> 947
<212> DNA
<213> Homo sapiens

<220>
<221> SITE
<222> (547)

<223> n equals a,t,g, or c

<220>
<221> SITE
<222> (555)
<223> n equals a,t,g, or c

<400> 20
tgaagacaag ggtggcatat atttactttg caataagtac accatattgg gtccttttga 60
gattgtcatt tgggtgtgta gcatttaaga tttaacagct ttctattata gagatcctac 120
agctttatat tagaagatta ttctgaagtc ataacatttt tttaaaaaag taatttcaga 180
aaaaaaaaag aatgttactg ggataatgag gaatgatgtc tagctgcctg gtggtggtca 240
tcactctgcg tgcttatttt agttggttgc aggccattag aagtcaagtt gtctggtcac 300
gaatgaaacg tttacagtct gcttcaaggc aatcaggact atccattccc aggagtgaaa 360
tgtctgcatt gcatagactg caagattgga gtgataaatc acacatactt tttttatttt 420
ttttgccaag agtttgtagg ttcccattat aaagccaggc acttgattta gaatgtgtaa 480
ggcaatcctt tgggaatgct ttgggatyca gcataactct ttgaatgaac tggagctttg 540
tgaattncct ttttntcctc agatcataag gtagaaaaaa attcctttta acaaaatagc 600
attcttatcc acccaccttc tgatccaggg gagtacactg ggtattgacc tcaggaaaga 660
gaacaaggga gtgagggtac aggaaatgtt aggagtgtga gcttgaagac aaagacgacc 720
caactggcaa agacagcagt tgtcaatcag agcagatgaa tcatcacatc agcaaatatt 780
cattatatat ctgctcaata ataagaaaag cttctaccaa aggccaatgc tccagacctc 840
tccccgaacc tccagattca cttacccacc tgcctacccc agcaatgtac agagcatcgc 900
ctcgtgccga attcgatatc aagcttatcg ataccgtcga cctcgag 947

<210> 21
<211> 1685
<212> DNA
<213> Homo sapiens

<220>
<221> SITE
<222> (396)
<223> n equals a,t,g, or c

<400> 21
gcaaagatca cggttatggc aaggttagtt tctggtgggg atgctcttcc ttacttgcag 60
aagcccacat tcttgctgtg tcatcacatg gtttttcctc tgtgcttgtg cacttgtctc 120
ttcttcttat caggacaaca atcctattgg tttcaggcct gagccttata accctattta 180
atgttaataa cctttgtaaa agccctatct catatcacat tgggggttag agtttcaacc 240
tatgcatttt ggggacacaa tgtagtctat atcaccttgc cttatccttt gccacttaga 300
tcatcacatg gtcgatgcct tttcattact caggtgttat tctaatatca ttccttggag 360
agttctccct caactattgc ttaatcacag tgtatngtaa ctctacagga catgtctgac 420
cctgttcact catcactaaa attactatat acaaccagaa ttgtgcttga cacatataat 480
gaagcattga gaaaacattt gttgaataaa tgttttcttc taatactggt ttatgggcat 540
aactatttct gaatgtgtcc tttctcaaag gtagacacct gagctttatg atccatggtg 600
ttatcctaaa aaacagaaca caatattatt atattaagta taccactgaa tatagcaatt 660
ggtgtcttga ggagttacaa catgtcattm tttawatagg ttatcatatt ttttccagta 720
atcaccccag ctatattaaa atgaaacttc tccccttttt ctctctaggt agcatcttcc 780
ttgactcttt cttagacaga tgctataact tttcagctac ttgagttatt agtttatttc 840
attatttatt gattttaaaa tgccaatctc aaattatact caaaggtttt tctacatttc 900
ccatctgtga tgacagctct tatagcttta arartactag gttgtgggtg ggcttcaaga 960
catctctttt cactcccact tctagatgcc agctccatct gtgatatgac aagagcgggt 1020
aaatatcttc ttacttgact caatcagatt gcagtcttct tttccttggt tgttgcttct 1080
caggctgaca cttactctag atgtcctctg catggttggg ctcctaattc ctgtaattct 1140
gaatggtctc cakgtactty cttttagaat cacctaagag gtgttccact tcttgggtca 1200
ctgaaagagg ctggtcaaga ttcaaatcca cttatttaat cactttattc ttggttaaaa 1260

tccaacaaag actgatccta gcataccttt tctttgtttt ctgcctgaat gagtattagc 1320
aggccagctt gagcacagca gcattattta catccatcat gcccaagagt agttcatatc 1380
cttgcttcat caaataggag gacaagttaa ttaccagaat tccttatctt agcacctcca 1440
tctctctgtt ggtcattgct ttcatgccgg ggcagcaata aagtatctgt ggatccaatg 1500
cctcactaac tcttttttgt ttctgagatg gagtctcatt ctgttgccca ggctggagtg 1560
cagtggcgcg atcttggctc actgaaagct ccacctcctg ttttcaagca attctcctgc 1620
ctcaacctcc tgggtagcct cgtgccgaat tcgatatcaa gcttatcgat accgtcgacc 1680
tcgta 1685

<210> 22
<211> 1837
<212> DNA
<213> Homo sapiens

<220>
<221> SITE
<222> (48)
<223> n equals a,t,g, or c

<220>
<221> SITE
<222> (987)
<223> n equals a,t,g, or c

<220>
<221> SITE
<222> (1037)
<223> n equals a,t,g, or c

<220>
<221> SITE
<222> (1312)
<223> n equals a,t,g, or c

<400> 22
cagcagagcc cagcgcggtg ctatcggaca gagcctggcg agcgcaangg acgcggggag 60
ccagcggggc tgagcgcggc cagggtctga acccagattt cccagactag ctaccactcc 120
gcttgcccac gccccgggag ctcgcggcgc ctggcggtca gcgaccagac gtccggggcc 180
gctgcgctcc tggcccgcga ggcgtgacac tgtctcggct acagacccag agagaaaagc 240
ttcattctgg aggggaagga gttttgagtg ccaaggatga aattccaccc atcactcggt 300
ctctgagctg caggacacag gcaggacaac gggagcacac tgccaggatg ggagctgctg 360
ggaggcagga cttcctcttc aaggccatgc tgaccatcag ctggctcact ctgacctgct 420
tccctggggc cacatccaca gtggctgctg ggtgccctga ccagagccct gagttgcaac 480
cctggaaccc tggccatgac caagaccacc atgtgcatat cggccagggc aagacactgc 540
tgctcacctc ttctgccacg gtctattcca tccacatctc agagggaggc aagctggtca 600
ttaaagacca cgacgagccg attgttttgc gaacccggca catcctgatt gacaacggag 660
gararctgca tgctggggag tgccctctgc cctttccagg gcaatttcac catcattttg 720
tatggaaggg ctgatgaagg tattcagccg gatccttact atggtctgaa gtacattggg 780
gttggtaaag gaggcgctct tgarttgcat ggamagaaaa aactctcctg gacatttctg 840
aacaagamcc ttcacccagg tggcatggca gaaggaggct atttttttga aggagctggg 900
ggccaccgtg gagttattgt tcatgtcatc gaccccaaat caggcacagt catccattct 960
gaccggtttg acacctatag atccaanaaa gagagtgaac gtctggtcca gtatttgaac 1020
gcggtgcccg atggcangat cctttctgtt gcagtgawtg atsaaggttc tcgaaatctg 1080
gatgacatgg ccaggaaggc gatgaccaaa ttgggaagca aacacttcct gcaccttgga 1140
tttagacacc cttggagttt tctaactgtg aaaggaaatc catcatcttc agtggaagac 1200
catattgaat atcatggaca tcgaggctct gctgctgccc gggtattcaa attgttccag 1260
acagagcatg gcgaatatty caatgtttct ttgtccagtg artgggttca anacgtggak 1320

tggacggakt ggttcgatca tgataaagtw tctcagacta aaggtgggga gaaaatttca 1380
gacctctgga aagctcaccc aggaaaaata tgcaatcgtc ccattgatat acaggccact 1440
acaatggatg gagttaacct cagcaccgag gttgtctaca aaaaagscca ggattatagg 1500
tttgcttgct acgaccgggg cagagcctgc cggagctacc gtgtacggtt cctctgtggg 1560
aagcctgtga ggcccaaact cacagtcacc attgacacca atgtgaacag caccattctg 1620
aacttggagg ataatgtaca gtcatggaaa cctggagata ccctggtcat tgccagtact 1680
gattactcca tgtaccaggc agaagagttc caggtgcttc cctgcagatc ctgcgccccc 1740
aaccaggtca aagtggcagg gaaaccaatg tacctgcaca tcgggggtcg acgcggccgc 1800
gaatcccggg tcgacgagct cactagtcgg cggccgc 1837

<210> 23
<211> 1095
<212> DNA
<213> Homo sapiens

<220>
<221> SITE
<222> (720)
<223> n equals a,t,g, or c

<400> 23
ggcacgagga atgggtgggt tttttttaag cagttattac ctcagcattt tgacatcaga 60
tatgcaaact taatggcgtt ttgttttttt atattctatt tgtattcttt ccccagtatt 120
tcccatgggg atctccacaa gtttggagtt ttttcctggt gcacacacgt gaggagattt 180
aaggtactat atgcaagtgt tttactaaaa agcactgaaa ttcttctggc aatacaagaa 240
ccattttcag gatcttggag ttacttcctt cttaatcttt cttaaagcat tcactgatgt 300
ttttgttttt tcaaaatgaa acaaaaatat cacattgaga agctagtcta tgttctgtca 360
ctaacattta aactttgcag actctaacaa aaagcacaag aggtcacgta ctattataca 420
aatttagcgg tactggattt acctctgaca ttaacacact caggcagaga ccaggagtga 480
tcagcaggtc ttcagaacca aaaaaccttt ctgttcacat ttcatctgat ttttaaactg 540
aggcaggctt tgattcttct gaaggatgcc aagaatcaaa ctaagggagg actcactgtt 600
aaagatgtgt tctgatgtct tatattaaga ccaratgtga catgatgtga ttatcttcca 660
gtactttgct tttaggtacc atttcatgac attttaggaa tgagtattgg aaaatataan 720
gaattagaaa agcagcactt tttttttaat ggaaaagtct tcggtccagt gttacacctt 780
atagtgtaat tcagtcccta agcacagaat gaatgtctgg cctgcatatg gtagttacag 840
tgtaacctct ggctgcagac cacacaggac aaccctaaca gcctagtctt gtatggtgta 900
aatatcaaga gtacagcttc aatttcattt gctttatctt agcaacaatg ccaactcagg 960
agagcagacg gccgatttca gtgaagtctg gtagtcaaca gatgttattt cagtctcagt 1020
gcatctcctc tggctttctt tgactgaagg tgtttatagg aaggaagtta aaaaaaaaaa 1080
aaaaaaaaac tcgag 1095

<210> 24
<211> 1039
<212> DNA
<213> Homo sapiens

<400> 24
ggcacgaggt tgttctgaga attaaatgag ttactacact taaggagttt agagcactgt 60
tggcatgcag tgggcagtca aatgctggct attccagctg tgcatggatt ccagcttggc 120
cagtcttgga tgggctgaga aaagggagct gcttttccct aaaagaccat cccaactgtg 180
ctctaccaca ctttgctctc ctggctaaga ctcagagaca gatgtatgta tgcccctgag 240
caatctcttt cccttctctg gatctcgatt ccttgcttgt ataatgacct ggtagtgtag 300
gaccaatgtt gctgggtgcg gtggctcatg cctgtaatcc tagcactttg gaacgccaag 360
cacgagaatc tcttgattcc aggtgttcaa gaccagcctg ggcaacatag caagacccca 420
tctctaaaaa aaaaaggcag gcgtgatggt gcacacctgt agtcccagct actcaagatg 480
ctgacgttgg gaggatcgct tgagcctggg agcttgagcc atgatcacac cactgtactc 540

cagcctgggt gacagagagg gactctgtct caaaaaatga cccactagga ccagtgtcac 600
tttcttttcc ctctaactgc ttaaagctgt gatgctcagt aggatagcca ctagccccat 660
atggctattt caatttaaat aaattaaaat tttaatgcta tttcaattta aataaattaa 720
aattttaatg ctattttaat ttaaataaat taaaattaag taaaatgaaa ttttcagttc 780
attagtcaca ttagctatat ttcaactgct cagtggccat aggtggctag tggctcccat 840
agcaagtggt acagatgcca ggacatttcc atcattgcag aaagttctat taaacaggct 900
ggcatggtgg ctcatgtctg taaccccagc actttgagag gctgaggggg caggatcgct 960
tgaagctagg agttcaagac cagcctgggc aacaaagtga gacccccatc tctacaaaaa 1020
aaaaaaaaaa aaactcgag 1039

<210> 25
<211> 1076
<212> DNA
<213> Homo sapiens

<220>
<221> SITE
<222> (910)
<223> n equals a,t,g, or c

<220>
<221> SITE
<222> (912)
<223> n equals a,t,g, or c

<220>
<221> SITE
<222> (958)
<223> n equals a,t,g, or c

<220>
<221> SITE
<222> (1038)
<223> n equals a,t,g, or c

<400> 25
aattcggcac aggaaaataa tttacaatga actggtgttt gtgcataata tctctcacca 60
ccctcctctc catcccagta cacattgttg gtgaggaaaa agacatgctt aagtgcacat 120
tctgtctcct aaacactctt aagaaatgtg ttgtatggaa gagattatat cataatggtg 180
gagcaaataa cctgtaattt tgttctagtg ttaactgcct ccattttagg ggttgagttt 240
ctactccttt tccatgatct cttctcttgc tgtttaaaaa atgatttcac agagtaaagg 300
tcagagtgcg ttaaaatgct tttgtatgaa gacctagcaa atacaagacc tgcttggctg 360
attgcttatg gttggaagtg actcatctaa gcacaggagt gtgaggttta tggcttagaa 420
cgtaagatac cagcctctgt agtggccaaa taagccggcc tttttgtttg ttattacaga 480
tgggttttga tgtcaaggtc aactgagttt tgagttgtcc ataagatgga cagaacatct 540
gcatataaca ccaactgaat gaacccccag tttgtctagg gctttgataa aaaatttggc 600
cctctagacc gggcgtggtg gctcacacct ataatcccag cactttggga ggccgaggtg 660
ggaggattgc ttaaggtcag gaatgcaaga ccaacttggt cttgtagtca gtgtagtgag 720
accccatctc taccaaaaaa aaaaaaaaaa aactcgaggg ggggcccggt acccaattcg 780
ccctatagtg agtcgtatta caattcactg gccgtcgttt tacaacgtcg tgactgggaa 840
aaccctggcg ttacccaact taatcgcctt gcagcacatc cccctttcgc cagctggcgt 900
aatagcgaan angcccgcac cgatcgccct tcccaacagt tgcgcagcct gaatggcnaa 960
tggcaaattg taagcgttaa tattttgtta aaattcgcgt taaatttttg ttaaatcagc 1020
tcatttttta accaatangc cgaaatcggc aaaatccctt ataaatcaaa agaata 1076

<210> 26

<211> 860
<212> DNA
<213> Homo sapiens

<220>
<221> SITE
<222> (15)
<223> n equals a,t,g, or c

<220>
<221> SITE
<222> (27)
<223> n equals a,t,g, or c

<400> 26
   acaaaagctg gagcnccacc gcggtgncga ccgctctaga actagtggat cccccgggct 60
   gcaggaattc ggcacgagga caaaggcttg ggaaatgagg ggaggtggag gcagggcagg 120
   ggaagcgaag agtcagcctt ggagagagca ccctggggcc tccgtgtcgg ggtacaccca 180
   gcactttgcg acctgcggcc cagcaggcgc ggaggatggc ggggaggaag ccagcagccc 240
   ctgtgtttac tgtcgtcaga aaggtcttgt gttttggttt tggggttttt gttttgtttg 300
   tgttttgttt ggcttgtttg ttttttaagg ggaaaaaagt ttgtaattat ttcatccaaa 360
   tctcccgtta tatatctgtg aataataaga gattttataa tagcaagaaa atgatgtata 420
   ttttagtttg ttgacaaata agtcatcatg atcacgaagg acactgagaa aaaataattt 480
   agaaccctgg tttttgtgaa wttttttgtt ttgtgtttct ttgttttgag atttgtgttt 540
   ggtttggttt ttgcactgca ctaaggcagg agggttggag ggctgggtgc agcctgggag 600
   tccgatggtt ttcagcagga gacggggtgt cccctgcagg gggctaaact gcaggggcct 660
   gagattagct gtgaacatgt gggagcccga tgcatgtggg tcagggatct gggggccccc 720
   ccagctggcg ggaaccccaa atggacacaa actgtacatt tgccaatggg tttttttcag 780
   accatggttt ttacttgcaa ataaacctga gttcttttct gcaaaaaaaa aaaaaaaaaa 840
   actgcggtcc gcaagggaat 860

<210> 27
<211> 776
<212> DNA
<213> Homo sapiens

<220>
<221> SITE
<222> (2)
<223> n equals a,t,g, or c

<220>
<221> SITE
<222> (13)
<223> n equals a,t,g, or c

<220>
<221> SITE
<222> (61)
<223> n equals a,t,g, or c

<220>
<221> SITE
<222> (79)
<223> n equals a,t,g, or c

<220>

<221> SITE
<222> (101)
<223> n equals a,t,g, or c

<400> 27
tnttggcccc atngatttta ccgcccaaag cttcttaatt acggactcca cttattaggg 60
naaaagcttg ttacgcctng caaggtaccc ggttccggaa nttcccgggt tcgaccccac 120
ggcgttcgag ggctcctttc tcttgcctgg aggggaaaac agaagattct ggcttgagct 180
tccctcatgc tgccctattt taagtggctc ctccacctgg tgaggctgtc ctttgtctct 240
ctggcttctc catgggacag cacagctggc cttggcctga agctccctaa catctatggg 300
atgacatcta tgggatggga tccctcacct ggggccaggg gaggggttgg cacagagaag 360
cgatgagatg ggtctccaag gccaggtctc ctttcatcct gagcaaaggg ctcagggcta 420
tgaaatgatc caagacatga aacaaatatt aaatataaaa atagagtcca aaggccaggc 480
gcggtggctc atgcctgtaa tcccagcact ttgggaggcc gaggtgggtg gatcacgagg 540
tcaggagatc gagaccatcc tggctaacat ggtgaaaccc cgtctttact aaaaatacaa 600
aaaattagcc aggtgtggtg gtgggcgcct gtggtccctg ctactcggga ggctgaggca 660
ggagaatggc atgaagctgg gaggtggagt ttgaggtgag ccgagatcac gccactgcac 720
tccagcctga gtgacagagc aactccatct caaaaaaaaa aaaaaagggc ggccgc 776

<210> 28
<211> 1074
<212> DNA
<213> Homo sapiens

<220>
<221> SITE
<222> (1063)
<223> n equals a,t,g, or c

<220>
<221> SITE
<222> (1067)
<223> n equals a,t,g, or c

<400> 28
ggcacgagcc aaattcagta gtaacagtaa attactaagg tgttttctct cttcattaca 60
gatacgtaat tcacctctgg gacctcaacc acgaagggac gtgggaagga aaggggacgt 120
atgtctatta cacagacttt gtcatggagc tcactctcct gtccctggac ctcatgcacc 180
atattcacat gttggtaagt ttcctcagaa ggagctctaa cagagggcaa gcctttcaga 240
atcaggaaca gtaatggttt cttcattaaa aaatgaaact ttagaaataa gatgtggatg 300
gactacttaa agactaaaaa tgaatgtggc tgcaaaccct ccctcttttt gccactgggt 360
gtaaggcagt gccatggaac tgctttggct ggtgcctaac tcaggaggtg tttgctgtcc 420
tgggagactt agttaactct gctgaccaag tcaatagatt attcttttag catgaaatta 480
aggagctgcc tttccccata gtttctatgg ctttaaatat ttagcaggta ctttgtaggt 540
ggtaatggga attcctgcag tgttagctac ttcacagatt tatacatttt ccatctttgt 600
aattaaaaaa agtctttaca cttaattcct acattcctac taccatcatt gtttacattt 660
tactttggta tgttagacgt tacggtgtcg tagatctgcy tcattggktg gcccttcagt 720
gatctaataa tggtgagaat taaaatagtt ggtgggcaat ttawttaaat tataagccta 780
gcaagtagca ttttaaaawt attgggctag acgtggcmca tttctaagtc tactttttga 840
aagaaacttt gaaaacatac tttttaaaga aagtatgtaa ttcttttttt taaaaaagag 900
cctcggctgg acgcggtggc tcatgcctgt aatcccagct actggggagg ctgaggcaga 960
gaattgcttg aacctgggaa atggaggttg cagtgagctg agatcgcgcc actgtactct 1020
atcctgggcg acagggtgac actccgtccc aaaaaaaaaa aanaaanact cgag 1074

<210> 29
<211> 2749

<212> DNA
<213> Homo sapiens

<400> 29
gccgctcagt gccctggaca ggagatgctg tgttaaactg ttaatggata tctatatgag 60
aagctcattt ttgtatgcta tccctgcagt tttttttttt ctaacaggcc catgtttgag 120
aataaacaag tctgtgatgt cagagacaaa ggtgtattct tcagtctgca ggtgtgtggc 180
acctcccttc tcccctgcag ccccccacat ccagagccgt tcctgagagt gacatcatgc 240
atcaagaaaa cataaccttg gtcctcaggt gaacccttgg aacattctgt gaccgcctga 300
tgtccattct gagccacctt ggcacacatg cttacaggsa gcactgctaa gggttcaggt 360
gccccatggc tgacagcccg agttgcttct gtggaccatc atgccgctcg gcacgtcctg 420
agacagaagt tgctgcagga aggagcttct ggagaggtcc tgtggcatgt gtgggggtgt 480
gtgtgtgtat gtttccttct tgaacagaca ttccaacttt agatgtgttt atagaactga 540
cctttttact aacaaaatac aatgatatat gttggaaact acttaatatg cttttcctgc 600
acaccttagc aataactgta ggggtctctg ctagagttgt ttgtatgtac agcaattttg 660
aacaaattgt tttaaatgta atataagaga attagtttaa ggaagtaaag agaatcattt 720
gcttgtgtta cattttcagt gaggattcag tttaagagtc attcttagga cttccatttc 780
ctaatattta ttcatgggta atgmagaaat ggtttgcatt ttgtggccag tcctaattta 840
ttttccagct gagccctaac ttccggctcc cacctacctc cacggacttc ctaacagaga 900
cttatgaata ccaggatgtg tttttgttaa gtcaggttca attcgttgcc cctgtcagtt 960
ttatagagtg tgagggtcac tccattaaag atctctcctg ggtggatcct acttggatgt 1020
tcaggtgatt ttgaaaactg ctaacatttt taaaaggcta gaacatcctt tgacttcttg 1080
aaaatctgca tgtctggctt gggttttatt accacatgcc tgagttcttc aagaatggaa 1140
ggctcaagta ttctcatctt ccatttgcca aacttccttc ctgatttgag tcacgtgttc 1200
cacttggaaa gaaagggaac agagagcctc ctccatggac agtgtatgaa tttcattggg 1260
aatcttgctc tctcccgcct ctatgccttt ctctcttttt aaccttactt tacataatat 1320
tatagatggg ccaagaaaag aaaagatgac ataacatttt gatgaatttc acctattcca 1380
ttcttcacgt ttcagaattg gtcgactttg ttagaagata attgaagtag ccttgggtca 1440
aaagcaacct tttcaattgt gatcatacct aaaacatata aaaaccctgc cgtagattaa 1500
aagcaattat aaaatcataa aattgaatgt ttgcagaatc ctggagcagt agatttcttt 1560
gtctttggcc tgcggactag aaagagggca gcagtagtat gctggagctt ccctgggata 1620
ccagccacat ggtttctttt cattagatct gatttttgtt tcccactgta gatctgattt 1680
tgtagttgaa aacatttcac caccatcaaa cactatttct gaatattgtg cctttttata 1740
cctagcctag atgaaaaccg atgccattct tattcagaaa atccccccat cctacatgac 1800
tgttatctag acataaagca aagtgcattt aattcaaaat ttggttcaca atataagtat 1860
tttgtaaaag ccagctgaac cagcatttta tcaggtggaa atctctgcaa gccaaattgc 1920
tgatactcct tcatgcagat caacttggtg tcccagtcag aatagaacag cataattacc 1980
tggagttagg gggagtattt ctgcactatt acttgtcagg gagagaagaa acttagaatt 2040
gtccctcaaa ggagtgtcaa gaagtatgaa taaatgtcct ttcaccagct cacaggccag 2100
aaatggagga cccaagtcaa ctaggtgaaa ctactagcag acccagcttt cccataataa 2160
cctaatctgc aaattgttct attaaagtct cattgttttc aggatgcaat gaaagtggat 2220
ttcaaaaggc tttggaaaaa taagtggaac atgactgatc ttgaaaaaaa aagcaaaagc 2280
ttaaatattt gatacaagtt tacttagcta caacatactt tacattgttg cctttagtta 2340
tctcacaggc actgacattt tatatttaga aaatactttt aatctttcta atcttttttt 2400
gtaaatatta gtgtccattc tgtatgactc gctaacctac tttgcaaggc tttgggcaac 2460
attttagctc attaacttca agatgatgtg tcatctgtat aggtcaaaga atgggacttc 2520
tgaactgagg aatttgctgt tgacagccaa agtatagtgt acaagattga tgtaacttga 2580
tatgtatttt tgttgaagtt ttttgtaaaa aaaaattatt tacaatgtta tttgaatgat 2640
ttttttaaat gctgtgaatc tatatttgtt gttttrtata ttaaaattca tttgccaaaa 2700
aaaaaaaaaa aaaaaaaaaa aaaaaaaaaa aactcgagac tagttctct 2749

<210> 30
<211> 604
<212> DNA
<213> Homo sapiens

<400> 30

gcaattttaa tatagtcaaa catttattag aagcagaaaa gtcattgtar agcacttgaa 60
ttatatttaa aagtttagcg gtctaaacta gcaatctaag atgattgtga aataaaggca 120
tttaaaatcc tcctacttgc ttatcggaac taccatacca gtcaggataa gctaagccam 180
gctgcgctaa caaatggcct gcagatcttg gtgctttaca ctactagcaa atgtttcttt 240
tacgcttctg ctgcctgtcc actgggggtc agcagaggcc gtcttctctg tcagcatcac 300
tctaggatgc cggccaccca gcagcctctc tgtgccactc agcagaggga gaagagacct 360
ggggagccac gtgctggctc ttgttgcttc tctttggaag tgacaccgtc actttcacat 420
atgtttcatc agccagagaa agtcagctat ggctggctca atagagccag taagtctaat 480
cctcctgaag cagaagctct gcagagagag gagccaaata tactgaacat aatacagtag 540
acaagagaat gtgtgtgact ctgaaaccat taagggagta aaaaaaaaaa aaaagggcgg 600
ccgc 604

<210> 31
<211> 748
<212> DNA
<213> Homo sapiens

<400> 31
ggtgagctgt gatcgtgcca ctgcactcca gcttgggtga cagagcaaga ccccggaccc 60
tgtctcaaaa aaaaaattcc ccagttctca gggtgtggta gaggccgagt cagtcatggc 120
tgagacaagg ggactgtgct ctgtgtgctt ctgtgccctg tgtttatatg gttcatacgc 180
tgcctgtcca ccatgttttt cccgagagcc tcggcagcgc aggcatcatg ggaatgactg 240
ggtcaggtgg aaattcagag gccctgccct ggtgggcaga gaagcctggc ttacctccca 300
agcacagcat gtgtgtggat cacttctgtg cactgtctcc tcatctccaa aatgggagtc 360
ataactgaac tcacctcatc aagttgttat gagatgatgt agattcagcg aagtagcaag 420
agtaggagtt tgggctttga taacagagag aagtgagttt ccatctagat tctccccctg 480
tgtcactttt ggcagttggc ttcacctctg tgggcctctg ttatgtcatc tgtaaaatgg 540
gattaaccct aaaagccacc ctcacagggt cattgtgagg attgcacaag gtgatgcaag 600
tggcacaggg tctggcccag gagagggggc tggaagagag cgagctgcca ttgtattttg 660
gttgctgtgg atctaaggag aagagatgtt taggagtctt tccctggcat ggttcctcct 720
gccttcaccc atcactcttt tcctcgag 748

<210> 32
<211> 943
<212> DNA
<213> Homo sapiens

<400> 32
cctaaatgca aacattttca tttaaatgtc aagcccatgt ttgtttttat cattaacaga 60
aaatatattc atgtcattct taattgcagg ttttggcttg ttcattataa tgttcataaa 120
cacctttgat tcaactgtta gaaatgtggg ctaaacacaa atttctataa tatttttgta 180
gttaaaaatt agaaggacta ctaacctcca gttatatcat ggattgtctg gcaacgtttt 240
ttaaaagatt tagaaactgg tactttcccc caggtaacga ttttctgttc aggcaacttc 300
agtttaaaat taatactttt atttgactct taaagggaaa ctgaaaggct atgaagctga 360
atttttttaa tgaaatattt ttaacagtta gcagggtaaa taacatctga cagctaatga 420
gatatttttt ccatacaaga taaaaagatt taaccaaaaa atttcatatt tgaaatggaa 480
gtcccaaaac ctaggtccaa gttcaatagc ttagccacat aatacggttg tgcgagcaga 540
gaatctacct ttccacttct aagcctgttt ttccccccat aaaaatgggg ataatacttt 600
acaaggttgt tgtgaggctt agatgagata gagatttatt ccataagata atcaagtgct 660
acattaatgt tatagttaga ttaatccaag aactagtcac cctactttat tagagaagag 720
aaaagctaat gatttgattt gcagaatatt taaggtttgg atttctatgc agtttttcta 780
aataaccatc acttacaaat atgtaaccaa acgtaattgt tagtatattt aatgtaaact 840
tgttttaaca actcttctca acattttgtc caggttattc actgtaacca aataaatctc 900
atgagtcttt agttgattta aaataaaaaa aaaaaaaaaa aaa 943

<210> 33
<211> 1293
<212> DNA
<213> Homo sapiens

<220>
<221> SITE
<222> (184)
<223> n equals a,t,g, or c

<220>
<221> SITE
<222> (208)
<223> n equals a,t,g, or c

<400> 33
gccgccgggg gacgcggacc caaacgccgc tcaccgcttg cggcgccggg catggggagt 60
gtggtgtgag cccgcacccg gggaggacgc aggagctgcg gagacgggcg cgaggaggag 120
gagaggagtc gtggattgga aggacccgag ggagggaggg tggggaagcg agggaaaagt 180
gaanctggga ggagaaggcg gcggaagntg gagattgatg cttctgtttt ttgttgccgc 240
tgctgccctc gcgctgggag ccgagccgga gggaaggcgg tggagagatg attgcagagt 300
tggtgagcag cgctctgggg ctcgccttgt atctcaacac cctgagtgcg gatttctgct 360
atgatgacag ccgtgctatc aagactaatc aggaccttct cccagaaact ccatggacgc 420
acattttcta caatgatttt tggggactcc ttctaaccca cagtggcagc cacaagtcct 480
accggccact ctgcactctt tcttttcgcc tgaaccatgc cattggaggg ttgaatccct 540
ggagctacca tcttgtcaat gtcctgttgc atgcagcagt cactggtctc ttcacaagct 600
tctccaagat cctccttggt gatggatact ggacattcat ggctggcttg atgtttgctt 660
ctcaccccat tcacacggag gcagtggcag gaatcgtggg acgagccgat gtcggggcca 720
gtctcttctt tctcctctcc ttgctctgct acattaaaca ctgttctaca agaggctact 780
cagccagaac ctggggctgg ttcctggggt caggactgtg cgcaggatgc agcatgttgt 840
ggaaggaaca aggagtgact gttctcgcag tttcagcagt ttatgatgtc tttgtctttc 900
acaggctgaa aataaaacag atattaccta ccatttacaa aaggaagaac ttgtcgcttt 960
tcctaagcat tagtttgtta attttctggg gttcctccct tttgggtgcc cggttatact 1020
ggatgggaaa caaaccacca agcttttcca actcggacaa ccccgctgct gattcggaca 1080
gcctcctcac ccgcactctc accttcttct acttgccaac caagaacctc tggctgttgc 1140
tawgtccaga taccctcagt tttgaatggt caatggatgc tgtgcctctg ctcaaaacag 1200
tttgtgactg gagaaaccta cacactgtgg gccttctawa atgggactcc ttctccttgg 1260
cctaactaag ggtttgaara agcccgaggc gtt 1293

<210> 34
<211> 1699
<212> DNA
<213> Homo sapiens

<220>
<221> SITE
<222> (9)
<223> n equals a,t,g, or c

<220>
<221> SITE
<222> (1692)
<223> n equals a,t,g, or c

<400> 34
   ggcatcttnt atttagcaca atgtttttaa ggtttattca tgttgtagca aggtacgcaa 60
   ttgtttttca tttaaagaaa aagtctcaat gctattacaa ttttccatat tctttgcacc 120
   tgtggtctgt ctccctaaat atagcccctt tatgaaggag gaatgcaaag ctgatccaac 180
   tagagactac aaattccttt atatttatat agaaaggggc acatagtaat gaattggaag 240
   ccatatccaa gctagaatca tctagattta gtgagattga ctagtgcaac ccaatttttt 300
   gcactcatcc cctgtccatc aggtacctgg aaatgattry aawgattttg aactaggtta 360
   ctggtataat catactgctg ttgagattag caggcaaatt accaagttag ttttttattg 420
   gagggggaga ggtcaatgtg tgagggtgca tagtggagac tggggaccag gctgacaaag 480
   atgaattgtt ttaggtagtg atgactttga ggtaatggga taagtgagtg aaaatgactg 540
   gttggcgttg gagatgggat ggagatggag cttggagaaa aagaatagca ctagtaaatg 600
   gatttagcta gacaaaggag atttacccta ttccatttag cacagtgagg agaggctaga 660
   cagctaggat gcaataaaaa aaattttaat gagaaatgtg tgtggtagat taattttatt 720
   aatctcaagt tatagattaa aaaatttaag taccacataa atgccatttg cctttgctaa 780
   tgttacattt ttatgaagaa ggagccttgc ataaagaatg atataatgga cttttgggac 840
   ttgagggaga agcttgggag ggggggtaaa ggataaaaga catattgggt gctgtgtgta 900
   cactgcttgg gtgacaagtg gactaaaatc tcagaaatca ccactaaaga acttatctac 960
   ataaccaaaa atcacctgta ccccagaaac tattgaaata aaaaaaaaga aggggacttg 1020
   gacagatagc cgtattcttt gccaaattat agttacattc tgctcatggg ggattaggag 1080
   gttcaatgga agaaaggccc cactcagctt tctcccctct taaaatgttg ccttgtaaat 1140
   tagggaattt tgcataaagc tctgaccttt acttccaagg cctttactga gaatgggttt 1200
   ggatacttgg agatagatcc tgactcccta tccctcctag atctttattt atcctatttg 1260
   gaacccaggg aaatggcctt aaagctgatg aaccacaggg tgtccaagtc atggagctat 1320
   tgaggttctc cccaagtatc ttttaaattg ctgcatttgg gatgggcgca gtggcttaca 1380
   cctgaaatcc cagcactttg ggaggctaag ttgggaggat tgcttgggtc tgggagttta 1440
   aggccagcct gggctagatg gtgagcctct gtctctattt aagaaaatta gaaattagcc 1500
   aggcatggtg acacaccagc tacttataat gctgaggcag gaggatcact tgagcccagg 1560
   agtttgcggc agacagtgag ctatgattgt gccactgtac tccagcctgg gtgacagagc 1620
   aagaccctgt ctcttattta aaaaaaaaaa aaaaaaaaaa actcgagggg gggcccgtac 1680
   ccaatcgcct tncatgatg 1699

<210> 35
<211> 1820
<212> DNA
<213> Homo sapiens

<400> 35
ggcacgagaa ggaatgagag ataaagaaag agacaggtga catctaaggg aaatgaagag 60
tgcttagcat gtgtggaata ttttccatat tatgtataaa aatatttttt ctaatcctcc 120
agttattctt ttatttccct ctgtataact gcatcttcaa tacaagtatc agtatattaa 180
atagggtatt ggtaaagaaa cggtcaacat tctaaagaga tacagtctga cctttacttt 240
tctctagttt cagtccagaa agaacttcat atttagagct aaggccactg aggaaagagc 300
catagcttaa gtctctctgt agacagggat ccattttaaa gagctactta gagaaataat 360
tttccacagt tccaaacgat aggctcaaac actagagctg ctagtaaaaa gaagaccaga 420
tgcttcacag aattatcatt ttttcaactg gaataaaaca ccaggcttgt ttgtagatgt 480
cttaggcaac actcagagca gatctccctt actgtcaggg gatatggaac ttcaaaggcc 540
acatggcaag ccaggtaaca taaatgtgtg aaaaagtaaa gataactaaa aaatttagaa 600
aaataaatcc agtatttgta aagtgaataa cttcatttct aattgtttaa tttttaaaat 660
tctgattttt atatattgag tttaagcaag gcattcttac acgaggaagt gaagtaaatt 720
ttagttcaga cataaaattt cacttattag gaatatgtaa catgctaaaa cttttttttt 780
tttaaagagt actgagtcac aacatgtttt agagcatcca agtaccatat aatccaacta 840
ccatggtaag gccagaaatc ttctaaccta ccagagccta gatgagacac cgaattaaca 900
ttaaaatttc agtaactgac tgtccctcat gtccatggcc taccatccct tctgaccctg 960
gcttccaggg gacctatgtc ttttaatact cactgtcaca ttgggcaaag ttgcttctaa 1020
tccttatttc ccatgtgcac aagtcttttt gtattccagc ttcctgataa cactgcttac 1080
tgtggaatat tcatttgaca tctgtctctt ttcatttctt ttaactacca tgcccttgat 1140
atatcttttg cacccgctga acttcatttc tgtatcacct gacctctgga tgccaaaacg 1200
tttattctgc tttgtctgtt gtagaatttt agataaagct attaatggca atattttttt 1260
gctaaacgtt tttgtttttt actgtcacta gggcaataaa atttatactc aaccatataa 1320
taacattttt taactactaa aggagtagtt tttattttaa agtcttagca atttctatta 1380

caacttttct tagacttaac acttatgata aatgactaac atagtaacag aatctttatg 1440
aaatatgacc ttttctgaaa atacatactt ttacatttct actttattga gacctattag 1500
atgtaagtgc tggtagaata taagataaaa gaggctgaga attaccatac aagggtatta 1560
caactgtaaa acaatttatc tttgtttcat tgttctgtca ataattgtta ccaaagagat 1620
aaaaataaaa gcagaatgta tatcatccca tctgaaaaac actaattatt gacatgtgca 1680
tctgtacaat aaacttaaaa tgattattaa ataatcaaat atatctacta cattgtttat 1740
attattgaat aaagtatatt ttccaaatgt aaaaaaaaaa aaaaaaaaaa aaaaaaaaaa 1800
aaaaaaaaaa aaaaaaaaaa 1820

<210> 36
<211> 2572
<212> DNA
<213> Homo sapiens

<220>
<221> SITE
<222> (13)
<223> n equals a,t,g, or c

<400> 36
   attcggcaca ggntagggtg ggggcagttt agttcccaat ggatatttct ggtttttgca 60
   gaaaaagtag gaaagggaag tgggatggtt tacctctttg tcaggaaagt taggtaacta 120
   ttagtaaaaa acaattatac actttaaaat cctgcaatta ttttacagaa agcactaaaa 180
   ctgcatgcat gggaagatca ctccatttca gatgtatttg ttacacagta tcttgtttat 240
   gctgtgctta gtaggcatgg ttgaattcaa taaaagcaca cgtgaatgca ttttatttaa 300
   gacactatgg ctaataccac tgtttacata taaactggcg tatctatgtg agaaactcaa 360
   gtttgtgaaa ttctgtgcat ctttgctaat tgctgtgttt gatcattgac atttctgaca 420
   tgccacatgg gcctgcgggg ctgtcatccc ctggggctga caactggtac tcggcccgtc 480
   cttgtaatcc agcagtattt tttcatacat ttgaaacatt tagaggaaaa ttcagtaatt 540
   gaataatgtt tgtaaatatt ctgatcgaaa atgaaaaaat tccccttaat gaaacctgaa 600
   ctctgcttct gattagctta tatgacttaa agcttcactt cagttccctt gaaaccatta 660
   catcttttat aaaatgaaag cactaagcaa tccctaaggt ttttctcaac atgttgggaa 720
   gccaatttta ttttatagca taatgtgttt attcttactt gatcatatct ttttttttca 780
   raaacacaga aaaagaaagt gcttggtcac ctcctcccat agaaattcgg ctgatttccc 840
   ccttggctag ccccagctga cggagtcaag agcaaaccaa gaaaaactac agaagtgaca 900
   ggaacaggtc ttggaaggaa cagaaagaaa ctgtcttcct atccaaagca aattttacgc 960
   agaaaaatgc tgtaatttct tgggaagatt ttaatgtaca cctatttgta aagtcatcag 1020
   aatagtgtgg attattaaat atctagtttg gaagaaaata atttatataa attattgtaa 1080
   atttttatgt aaacagaagg tcttcaataa gtaaagtaac tccatatgga gtgattgttt 1140
   cagtccaggc aatttttcta ttttatatta agacttcata catttatata tgtaaatatg 1200
   gcttattaat ggaatgttaa ataaaatgta tacttcacag tcgtttgtgt cttggatttt 1260
   tgaaagggag gggatatctg tttaaatagt tttatatgct cattggtctc attttctcta 1320
   taattaaaat actagaccag tcttaaaatg gggatgattg aagtattgat atttcttttt 1380
   acagttacta ttttataatt tatgcacttt gattctgtga ttcagatttc taatcagaaa 1440
   atgtattttt ttgtttttgg ctgttactat gttaaaattg aattatgggc atgtcatttt 1500
   gccatctttg tagtttcaca aattttgtgt aatctacctc aaatgaataa tccaagtatt 1560
   ggttaactat aatgttggca tctcttattc ggcaagctta aaggctcttt aaagtcttaa 1620
   ttagtcaaag actaatccag gttagattga ccggttcact gctcacttgc aaccttatca 1680
   aagggtttga caaagggaaa tgtaaaataa atctgtttat ggatattgag tgcatcttgt 1740
   atgtgcctaa tattgatagg atgagatgtc tgaacaaatt tttataatat tgctgtgaag 1800
   gagcttgcta ttgaaccaca gaaatccsty aatattcagg ttttaaaact ggcaaattct 1860
   cacaggacct caggcacaga ttattgaggt tgggagagag tgagtagatg tagaaaagga 1920
   gaaaaacaac acacgccctg ttctctacag tacaactgtg tgcaattaag caatggtact 1980
   tgatgtaggc tctaacactc atcaataaat aagtgttgta aaataattta taacaggtaa 2040
   tcgatagtgt gtaatgaatg gactattaat aattgattat ctagaaacga actgctttcg 2100
   tgggctttta atattttaat gtgaagcata tgcagtgtgc tttctgcatt tattttycta 2160
   ccaaataata cagataatga gaaattggtg aaaatgccta cgcaaagtgt tgacagtgtg 2220
   aaagcagtgc gagtgcggcc ttttagtcag gttagtgatg gatgttacgc tgccttgttg 2280
   aaaatttcac tgactttgat tttattactt ttttaatgat agttatcaaa cttgtattta 2340
   agctgcttgt catttatgga atattgaact tatttaaatg aacttgttaa atgaataaag 2400
   agctaaacat aattcagtaa acaattcctt tgcgcaagta gcacaataaa catggatgca 2460
   acgtatgtca agttaatact tttttaaacc aacgcaattt ggtgaatata gatgtgtggt 2520
   acctgttttt aaaaaaaaaa aaaaaaaaaa aaaaaaaaaa aaaaactcgt ag 2572

<210> 37
<211> 704
<212> DNA
<213> Homo sapiens

<400> 37
ggcagaggaa aggctgtcag ggtgaaaata ctcttcttgc ccttcggctg agataattct 60
gaagcatatt ttacttagtt ttctagagtt cttcttggta attaatgcaa tcaagctcca 120
gtctcctgct gtgatgactg ccttcataac atacccttta ttatttatct gtcttccctc 180
cgtatctcac ttcctacctg ttcctacttg tctatttccc tgtgagggac tgaactgtga 240
gcccctcaga ttcaacgtac gaagccccta aatttatttg ttcgagtctg aagccaaagt 300
acctaagaat gtggctttat ttggagatac agctttaaag aggtgatgaa attaaaatga 360
gatcatgaag gtacactcta atccactatg actggtgtcc ttataagaag agattaggac 420
acaacacaca cagagggaat cccatgggca gacacaggga gaacacagac atctgcaagc 480
caagggcagg agcctcagaa gaaaccaaac ctgctgacac cttgatctca gatttcagcc 540
tccagaaatg tgagaaaaat aaatttctgt tgtttaagcc acctagcctg tgatactttg 600
ttacggcagc ccaagctaat taattcactc ccaattaaac tgttcgccct tgaaaaaaaa 660
aaaaaaaaaa aaaaaaaaaa aaaaaaaaaa aaaaaaaaaa aaaa 704

<210> 38
<211> 437
<212> DNA
<213> Homo sapiens

<400> 38
ggcacgagct gaattctaca catctctcta gtccctctga agccccacct ctggagcgct 60
gcctctgatc accccagccc acagtgatct gagttcacag agcacatcct gtttgaatgc 120
cccatttgaa tcacagccta ttcctctttt tgagtgttgg ttgtgcctta agtgcacaga 180
tggcttttca ccagctggac ctcgagcagc ctgaggatgc caccctgcct tctgagccat 240
tcttccatca cactgtagtg ccacagcgct catttagtag gattttggta aacatgggtc 300
aactaagtga gacactggca gagcaaggtt atatttagtg ctagaaagga cctacaacat 360
ggtgacttcc tcctagtcta gagaatgtag gccctgacgc tttgatattc ccaataagca 420
aaaaaaaaaa aaaaaaa 437

<210> 39
<211> 943
<212> DNA
<213> Homo sapiens

<400> 39
gtattttcaa gggtctgtcc tgttatagca cataacggaa cttcattcct tttttaaaag 60
atataattca tgtaccaggt gattcacccc tttaaagtct caaattcagt ggtttttagt 120
atatttccag aattgtgcag ttatcactag gagcaatttt agaatgtttt catcacccgg 180
aaagaaactc tatatccata cgcagcctct ccccatttct ccccaacccc cagccctagg 240
caaccactca tctgctttcc gtgtctgtag gattgcttgt tctggaaatg ttgtatacat 300
ggaatcatgc actgtgaact cttgtgtgtc acagaaggat catgtttcca tggtgcgtct 360
gtgtcatagc atgtatcagt gcagtaaccc cccttatcca aggttttact ttctgcagtt 420
tcagttaccc acagtacagt acagtaagat attttgagag agagaccaca ctcacattac 480

ttttattgta atatatcgtt ataattgttc tatttgatta ttgttgttaa tctcttactg 540
tgccttattt agaagttaga ctttgtcata agtatgtatg tataggagaa aagatagtat 600
atataaggtt tggtgctatc cacagtttcg gacatcccct gggggtcttg gaatgtawcc 660
tgtggataag cgggaccact gtacttcatt ccttttattt gtcaaataat attycatkgk 720
gtggctawgc catawtttgc cyattcattc gtcagttggt agacatttga ggtgtttcca 780
twttttggct tttgtgaaga atcctaggcc gggcacagtg gctcatactc ctgggacctt 840
gggaggccaa gacggacgga tcacttgagc tcaggaattt aagaccagcc tgggcaacat 900
agtgagactc tgtctctaca aaaaaaaaaa aaaaaaactc gag 943

<210> 40
<211> 1875
<212> DNA
<213> Homo sapiens

<220>
<221> SITE
<222> (38)
<223> n equals a,t,g, or c

<400> 40
aagcagccct cgtcggaagc cctaccgtgc caactggncc ctcctcccga cctgctcccg 60
gctcgtgccc cgtcccaccc aaaagtgggt aaaggttgcc ggcgccggca ctgcagctgg 120
ggctgagaag ccaggacggc ccgagaactg acagacggag tgacagacgg actgaccatg 180
gccgaccagc caaaacccat cagcccgctc aagaacctgc tggccggcgg ctttggcggc 240
gtgtgcctgg tgttcgtcgg tcaccctctg gacacggtca aggtccgact gcagacacag 300
ccaccgagtt tgcctggaca acctcccatg tactctggga cctttgactg tttccggaag 360
actcttttta gagagggcat cacggggcta tatcggggaa tggctgcccc tatcatcggg 420
gtcactccca tgtttgccgt gtgcttcttt gggtttggtt tggggaagaa actacaacag 480
aaacacccag aagatgtgct cagctatccc cagctttttg cagctgggat gttatctggc 540
gtattcacca caggaatcat gactcctgga gaacggatca agtgcttatt acagattcag 600
gcttcttcag gagaaagcaa gtacactggt accttggact gtgcaaagaa gctgtaccag 660
gagtttggga tccgaggcat ctacaaaggg actgtgctta cccttatgcg agatgtccca 720
gctagtggaa tgtatttcat gacatatgaa tggctgaaaa atatcttcac tccggaggga 780
aagagggtca gtgagctcag tgcccctcgg atcttggtgg ctgggggcat tgcagggatc 840
ttcaactggg ctgtggcaat ccccccagat gtgctcaagt ctcgattcca gactgcacct 900
cctgggaaat atcctaatgg tttcagagat gtgctgaggg agctgatccg ggatgaagga 960
gtcacatcct tgtacaaagg gttcaatgca gtgatgatcc gagccttccc agccaatgcg 1020
gcctgtttcc ttggctttga agttgccatg aagttcctta attgggccac ccccaacttg 1080
tgaggctgaa ggctgctcaa gttcacttct ggatgctgga agctgtcgtt gaggagaagg 1140
agtagtaagc agaactaagc agtcttggag ggcaagggga ggggaatggt gagatccgag 1200
ccctgtgcat ggacttggtg agactgttgc cttaatgaca tcctgcaccg tgtataactt 1260
agtgtgtcat tttgaaactt gaattcattc ttatcaattt aagggatctt aaaaggattt 1320
ggaaatggaa caagtagctt ccagaccaga tactacctgt ggcaagaatg ctgcctacca 1380
gttaactgct ggtcctacca cagtcaaagt attcctyakt aaagagwgaa tctcaggttc 1440
tcactggagg cactgtgcat attttcaacc agatcaccag gagctgagat cttcttcagt 1500
ccctagccag gaatacccat ttgatttcca gggtgccatc taatcctggg ctgtacatgt 1560
ggatatggac ttgaggccca cctctgtgtc caagtggatt gagcatatat gcctaggagg 1620
agatagactg ttaatcgttg gattttgatt tttttttttt atgcctgcaa ataatcaaaa 1680
gtaaaactgg agtagcctaa ttttctggga gcaggtggag aactttccct cctacacagt 1740
gaggacagtc ccagtctgct gggataagtg agaaagccca gggtgtagga aggccctttt 1800
tacatactct tttctcatga gagctcacta ttttaacaat aaacaataaa cgttgtttct 1860
aattttaaaa aaaaa 1875

<210> 41
<211> 490
<212> DNA

<213> Homo sapiens

<400> 41
aattcggcac gagaaaagct tagagaagga aatagtaagt agatgaccag ggctactact 60
gagttcccct cccctaaatt tagcacgttg cttgtcctgg tattatcttt actgagagct 120
cacatactta ttccaaagga gcctcttcag tctagctgct tactgaaaac actatattgg 180
gcctgttcat gtaatagtga tttcattcgt tgcattctta gggaagtttc cggtaaaata 240
tggagattta gtaaaacctt ataattatat ttggggtcaa aactagtttg gaatatttta 300
atagtgtaac ttaaaattaa caaaggaaag tttccccccg cctcctccac ccagtgtttg 360
tgctttacca taacattatt aagactggta aagtgtaatg acatatcaaa ttgcaaagtc 420
tagcaaatac tgtagcaaac cctaaaacac tccccaccgc ccccccaaaa aaaaaaaaaa 480
aaaactcgag 490

<210> 42
<211> 786
<212> DNA
<213> Homo sapiens

<220>
<221> SITE
<222> (770)
<223> n equals a,t,g, or c

<400> 42
gatatgtttt aattatctga tttagatgat ctacttttta tgcctggctt actgtaagtt 60
ttttattctg atacacagtt caaacatcat tgcaacaaag aagtgcctgt atttagatca 120
aaggcaagac tttctatgtg tttgttttgc ataataatat gaatataatt taagtctatc 180
aatagtcaaa acataaacaa aagctaatta actggcactg ttgtcacctg agactaagtg 240
gatgttgttg gctgacatac aggctcagcc agcagagaaa gaattctgaa ttccccttgc 300
tgaactgaac tattctgtta catatggttg acaaatctgt gtgttatttc ttttctacct 360
accatattta aatttatgag tatcaaccga ggacatagtc aaaccttcga tgatgaacat 420
tcctgatttt ttgcctgatt attctctgtt gagctctact tgtggtcatt caagatttta 480
tgatgttgaa aggaaaagtg aatatgacct ttaaaaattg tattttgggt gatgatagtc 540
tcaccactat aaaactgtca attattgcct aatgttaaag atatccatca ttgtgattaa 600
ttaaacctat aatgagtatt cttaatggag aattcttaat ggatggatta tcccctgatc 660
ttttcyttaa aatttctctg cacacacagg acttctcatt ttccaataaa tgggtgtact 720
ctgccccaat ttctaaaaaa aaaaaaaaaa aaaaaaaaaa aaaaaaaaan aaaaaagggc 780
ggccgc 786

<210> 43
<211> 1676
<212> DNA
<213> Homo sapiens

<220>
<221> SITE
<222> (798)
<223> n equals a,t,g, or c

<220>
<221> SITE
<222> (927)
<223> n equals a,t,g, or c

<220>
<221> SITE

<222> (944)
<223> n equals a,t,g, or c

<220>
<221> SITE
<222> (974)
<223> n equals a,t,g, or c

<220>
<221> SITE
<222> (1035)
<223> n equals a,t,g, or c

<220>
<221> SITE
<222> (1058)
<223> n equals a,t,g, or c

<400> 43
acgagcagat tcccaagaag gtacagaagt ctttgcaaga aaccattcag tccctcaagc 60
ttaccaacca ggagctgctg aggaagggta gcagtaacaa ccaggatgtc gtctcctgtg 120
acatggcctg caagggcctg ttgcagcagg ttcagggtcc tcggctgccc tggacgcggc 180
tcctcctgtt gctgctggtc ttcgctgtag gcttcctgtg ccatgacctc cggtcacaca 240
gctccttcca ggcctccctt actggccggt tgcttcgatc atctggcttc ttacctgcta 300
gccaacaagc gtgtgccaag ctctactcct acagtctgca aggctacagc tggctggggg 360
agacactgcc gctctggggc tcccacctgc tcaccgtggt gcggcccagc ttgcagctgg 420
cctgggctca caccaatgcc acagtcagct tcctttctgc ccactgtgcc tctcaccttg 480
cgtggtttgg tgacagtctc accagtctct ctcagaggct acagatccag ctccccgatt 540
ccgtgaatca gctactccgc tatctgagag agctgcccct gcttttccac cagaatgtgc 600
tgctgccact gtggcacctc ttgcttgagg ccctggcctg ggcccaggga gcactgccat 660
gaggcatgca gaggtgaggt gacctgggac tgcatgaaga cacagctcag tgaggctgtc 720
cactggacct ggctttgcct acaggacatt acagtggctt tcttggactg ggcacttgcc 780
ctgatatccc agcagtangc cctgccttcc tggccactga tttctgcatg ggtagaccat 840
ccaagactgc agcgggtaga aggtggcagt tcttcatggg agtcttttta acttggtgcc 900
tgagttctct cctaagcaag tggccanttg cctccacctc agtncttcca tctttgggtg 960
ggggacaggg gccnagcaag catctcagcc tcctacccac aattccactg aacacttttc 1020
tggccctact gcacntggcc cccagcctcc atccttgngc tggtagcctc tcacaactcc 1080
gtccttgccc tttgccttcc acttccttcc atctcatttc taaaccccaa acagctcatc 1140
tctaaaaaga tagaactccc agcaggtggc ttctgtgttc ttctgacaaa tgattcctgc 1200
ttctccagac tttagcagct cctgatccca ttcttggtca cagctctagc cacagcagaa 1260
ggaaaggggc ttgcagaaga atatagcacc gaattgggaa acagcagcct cacctccacc 1320
tgaagcctgg gtgtggctgt cagtggacat ggggagctgg atggaaatgc ctctcacttc 1380
aaaatgccca gcctgcccca aatgcctcta agcccctccc tgtcccctcc cttgtagtcc 1440
tacttcttcc aactttccat tccccatcat gctgggggtc ttggtcacaa ggctcagctt 1500
ctctccactg tccatccctc ctatcatctg tagagcagag cacaggcagt tgtgtgcctt 1560
gggcccaggg aaccctccat caacctgaga caggactcag tatatggttc ttgggtatgc 1620
cctaccaggt ggaataaagg acacagattt gatttctaaa aaaaaaaaaa aaaaaa 1676

<210> 44
<211> 766
<212> DNA
<213> Homo sapiens

<400> 44
ggcacgagct tttgctctca tttgccttca cagaggccac tccacctgtc cggatccagc 60
tgtctggtca tggtttggtt tatttatttt gtccttcagg ggctgttttg ccctaagaat 120
gaggggcctt cccctggtct gcagttccca actttatccc ttgctggcca tgcgagccca 180

gccctggtgc ctcatgggat gggggggtag gggtccccag gatcttctgg aggaaggtgg 240
gcatggatgg atgggctgta tctgtgtttt ccctctggga gtctcatggg tccagcatca 300
ggcctgaggt cagcaacagg gaaagagggt gggcacgggg agggcttggc cccgcctatc 360
tagaggcttg cctcgggccc ctccttgggg aaggtttgcg tgcagagctg caagggagag 420
ggttccagaa gcattgcctt ttgcctcgtc taataggatc cttaggacac tgtgggcttt 480
aggaatgact atagatgctc acacgtgttt aaagtgacat ttggagatgc tctcagtcct 540
gtggcatctg gcacgaagtc tccaagaagc cactttgcct cttctccctt caagcacaag 600
ctttactgca aaagggccag tcgcgtttct atttctctcg atcccaggct tctgcggacc 660
gacgatacgt ttaaatgttg ttctagtaaa tattcttgaa tgtattaaaa tggctgaaac 720
aacaaaaaaa aaaaaaaaaa aaaaaaaaaa aaaaaaaaaa aaaaaa 766

<210> 45
<211> 1021
<212> DNA
<213> Homo sapiens

<400> 45
gtaattcctt aaacatacca tctgtcacag ttaatctaga tttgtaaata ggtagtaatt 60
tatagaattt ttaaagcgta aaatccggta atattaaaag ataggtaaac ctaggcctgg 120
aaagctgtta tttggctaaa attgcacagg aggccatgaa cagaggcaag tgccccagag 180
actccacttt cattcctaac tgttctcaaa ttaatgctca tgattgagta ttctcagtgc 240
aactcgtaga gtttgataag taaaagttac atgcccctgt tttcctagca tgatattcac 300
tgttatcaaa gacaagaggc agaccattca ttcattctca aaacactgaa tgccattctg 360
tgcctagtgc tatacaaggc atgggagatt cagtgtgaat aagtctttgc tctccaccta 420
acaagggaca gttttaatta tagattgtct tcctattaag tatgagtttt agtaggcatt 480
aaaaatcgta attagtttga taatatgaga cccaacccta acttgccaga agagtaatca 540
gttcatgaac cattgatatt tcctgtatat ttcatgaatg tgacttcagt cattctagtg 600
ttaatactgt ggaatgtcat tggtgtagca acgtgggttc accaaaacac ctttttatac 660
aaaagacaga tgygtgaatt aaagagatta aaggatagag tattctgttt ctttgttttg 720
atttggcttt taggtattaa aataaggccc agatcactaa aaattagtaa cagagggaga 780
cctctaatag atttaaagtc agttaattct ctctgaaatt tgatgttttc ttctataaag 840
aataactcta aaataggcat cttcccagga ctttccattc tcaggaaaag acctagttac 900
gtataaaaaa taacttctac tgctttatgt agtcatatag gtctgcctaa aataagaatt 960
tgtatttaat aaataccaaa attttcaaat ggtaaaaaaa aaaaaaaaaa aaaggggggg 1020
c 1021

<210> 46
<211> 1873
<212> DNA
<213> Homo sapiens

<400> 46
ggcacgagct caggctcccg tcggacttca cttggccaca tccttcacta ctctccttcc 60
ttatgcttta tttaacacat ttccacgaga catgtgttcc catgaccttc ttccatgtcc 120
acctccacag ttttgctcag gttctcgttc cctctcccag gcctctctcc actctatact 180
ttcaggaatt ctacccatgc aaagcccatc tcagcttcca cctcactcct gacttgacac 240
ctcctcatgc agcctgcctg cctggcgcct tgtctagatg ctctcacctc gttctgcctt 300
ggattactaa aacttacttt ctgtcttgct ttctttcctt ctggagttct tgagggggag 360
tgcagcttct ttacaatgtc tagatccctg tcccatccac gcacactgca cagatacact 420
acagagcgcc cagctcacag cagacactaa atggtgaaag aatgcaagag ggtcctgtgt 480
ctccctaagt ccaaaaggag acataagaat attacaggcc gatatttgta acccattaag 540
aaaaaaggtg aaatagtgtc aatacctaag caaaatacca tgagaatata aatcaaagtg 600
tgaacaggag taatattaag acagaaaggc aatggttctc ttctggaacc attagcattt 660
aaatacagaa aagaaaatgc accattttaa cagctgcaga agataataac agacacaatt 720
atttttccct aactagatgc catgccccat gtacagtagt tcctaatcat cccctcatct 780
tagtctcata acaaccctat tattgtctct atgttacgta ggaggaaact gaggtaccga 840

gcagttaatt aacctttttcc atcatgcaac cagcaaggca gagctaggat ttgtatccca 900
gtagcacctt ttccagattc aagctcaact cctaaattct cctgcgtctt cactgtattg 960
tttttacaac acatttgcag gttgtgggct aagtcaccgg ctactgagag ataaagaagt 1020
aacactccta tgaattttac atttctggct gggcaccgca gctcacacct gtaatcccag 1080
cactttagga agctgaggca ggagaattgt gtgagcccag aagtttgaga ccagcctggg 1140
caatatagcc agaccccatc tcaaaaacaa ttgtgcattt ctaatactca ctgagcccct 1200
gctatcccct ggctcagtgt acattgctct atatctccta gcaaacccag gagctatgta 1260
tgaactgaaa ccctggttaa atagcttggt caaagtcaca cagctcaggt gggggaggct 1320
gggtttaaag gcaggctgct gatgctatga tccatacttg aggctactgc tggccacagg 1380
ctccatctga ggccctgtag gggtgagag gagaaacccg gccccagaga cagggtctga 1440
accctctgct gccagccagt agagaaaaca gtccctcacc cacaacgtgg ggataacact 1500
gcctaccaca ccaggcagtg gaaagaatta aattaattta aataaaggag acagtgcaga 1560
gtacctgaca cgcaataagc actcaatgag agctattatt agaggtaact ctccctgctt 1620
tcagtctaat gccatgtttc ttatcactta aggtgatcac cttgttgctc tttaaaatat 1680
tatgtatggt tttctctaag atacatgtaa gtgtaaaatg cagaagaaaa gcatgcgggg 1740
acgggggggg ggaagaaatt cccttttctt tattgatcag cctttccccc aaaatacttt 1800
ctcaaggaat tattaaatac tcaacatggc gcctcgtgcc gaattcgata tcaagcttat 1860
cgataccgtc gac 1873

<210> 47
<211> 621
<212> DNA
<213> Homo sapiens

<220>
<221> SITE
<222> (488)
<223> n equals a,t,g, or c

<220>
<221> SITE
<222> (536)
<223> n equals a,t,g, or c

<220>
<221> SITE
<222> (539)
<223> n equals a,t,g, or c

<220>
<221> SITE
<222> (548)
<223> n equals a,t,g, or c

<400> 47
acagagtctc gctctgttgt ccagcctggg caacagagaa aacaaaaagg aaaacaaatg 60
atgaaggtct gcagaaactg aaacccagac atgtgtctgc cccctctatg tgggcatggt 120
tttgccagtg cttctaagtg caggagaaca tgtcacctga ggctagtttt gcattcaggt 180
ccctggcttc gtttcttgtt ggtatgcctc cccagatcgt ccttcctgta tccatgtgac 240
cagactgtat ttgttgggac tgtcgcagat cttggcttct tacagttctt cctgtccaaa 300
ctccatcctg tccctcagga acggggggaa aattctccga atgtttttgg ttttttggct 360
gcttggaatt tacttctgcc acctgctggt catcactgtc ctcactaagt ggattctggc 420
tcccccgtac ctcatggctc aaactaccac tcctcagtcg ctatattaaa gcttatattt 480
tgctgganta ctgctaaata caaaagaaag tccaatatgt ttccattctg tagggnaana 540
gggatgcngg cttaaaattc tgagcaaggg tttttggca gtgcagtgtt ggcactatgg 600
aaaacccttg gtcccccgga a 621

<210> 48
<211> 1290
<212> DNA
<213> Homo sapiens

<400> 48
   ccacgcgtcc ggtcagcggc tcggctcccg cgcacgctcc ggccgtcgcg cacctcggca 60
   cctgcaggtc cgtgcgtccc gcggctggcg cccctgactc cgtcccggcc agggagggcc 120
   atgatttccc tcccggggcc cctggtgacc aacttgctgc ggtttttgtt cctggggctg 180
   agtgccctcg atgtcatccg tgggtcttta agcctcacca acctttcgtc ttccatggct 240
   ggagtctatg tctgcaaggc ccacaatgag gtgggcactg cccaatgtaa tgtgacgctg 300
   gaagtgagca cagggcctgg agctgcagtg gttgctggag ctgttgtggg taccctggtt 360
   ggactggggt tgctggctgg gctggtcctc ttgtaccacc gccggggcaa ggccctggag 420
   gagccagcca atgatatcaa ggaggatgcc attgctcccc ggaccctgcc ctggcccaag 480
   agctcagaca caatctccaa gaatgggacc ctttcctctg tcacctccgc acgagccctc 540
   cggccacccc atggccctcc caggcctggt gcattgaccc ccacgcccag tctctccagc 600
   caggccctgc cctcaccaag actgcccacg acagatgggg cccaccctca accaatatcc 660
   cccatccctg gtggggtttc ttcctctggc ttgagccgca tgggtgctgt gcctgtgatg 720
   gtgcctgccc agagtcaagc tggctctctg gtatgatgac cccaccactc attggctaaa 780
   ggatttgggg tctctccttc ctataagggt cacctctagc acagaggcct gagtcatggg 840
   aaagagtcac actcctgacc cttagtactc tgcccccacc tctctttact gtgggaaaac 900
   catctcagta agacctaagt gtccaggaga cagaaggaga agaggaagtg gatctggaat 960
   tgggaggagc ctccacccac ccctgactcc tccttatgaa gccagctgct gaaattagct 1020
   actcaccaag agtgaggggc agagacttcc agtcactgag tctcccaggc ccccttgatc 1080
   tgtaccccac ccctatctaa caccaccctt ggctcccact ccagctccct gtattgatat 1140
   aacctgtcag gctggcttgg ttaggtttta ctggggcaga ggatagggaa tctcttatta 1200
   aaactaacat gaaatatgtg ttgttttcat ttgcaaattt aaataaagat acataatgtt 1260
   tgtatgaaaa aaaaaaaaaa aaaaaaaaaa 1290

<210> 49
<211> 2126
<212> DNA
<213> Homo sapiens

<400> 49
cgtccgcgga cgcgtggggg atgaaattgc cctggaacat tgtgaatata ctaaaagcaa 60
gtgcattgta tgctttaaaa tggttgttat taattttata ttatgtgatt tttaccttaa 120
aaaaagagaa aatagcctta ctctatacat aataaactca agatatgtta caaatttaca 180
tgtgaaatcc gaaatactat aatatttaag gaatagctaa gtagaataac actgaaattt 240
aacataatga aacatttcct taaaaaagag aaaagcacag taattaaaaa ggaaaataat 300
attttttctc tccattaagc atgccattaa ctgagtaaaa gaatcaagct gcaattatgt 360
aaactacgtt ttctaaaacc ataaagaaaa gaagaaataa aaaggtattt gggaaaaaaa 420
tccaaaggta cagtcaacta cacaaaaaaa gcttagtctc attaatcatt atgaaaatgc 480
aaatggtaac tgaaagaaga taaaactaca attcaaagag aaagcctaaa atttcaaccc 540
cccaaaaagt ctgggttttg gagatctggg atggaatagg gttcctaacc tgacaacaat 600
gaaagaacca aactaacctc aaagtcatga ctttattttt atagcaacga gttgccaaga 660
actgagtcaa aatgtgaggg aaaacaagca cctgcaagga gaaagaggac agatgcactt 720
acatagggac agatgcaaat agacccacta tgacaagtaa agctggaata atcaataaat 780
tcctaaagac aaagtggggc tggtcagatt gggagacggc tgacagctgc agaagttggg 840
aaagatccat catcttgaaa actttttctc cacaaaccca ctgtgatctc tcaagcaatt 900
ggtaaggaat ccaagagagt ctgtatatga cacagatcag ggagagcaga acacttggga 960
ggtgaccagg tcttgggggc cgagccctta tgaatcggat tagtgccttt ataaaagaag 1020
ctcaatggag ttcttgtgtg ccttccacta tgtgaggaca tagaaagaag gcaccatcta 1080
tgaaccatga aatgggctct catcaacact gaatttgtga gcatcttgac ctgagatctt 1140
acagcctcaa gaagtatgaa aaaagaaata tctgttgttt tttagtcacc cagtttatgt 1200
tattttgtta taagagtcca aatagaccaa gatattccac ttaatatgta ggggaaggca 1260

acaaaaactg ccacacttag aatactcctg atgctgggag tatgaaaaca ggaaaaacaa 1320
aaacaaaact gctcttgaag gtgaaggagg aatatcactg agctcaccaa cacagccagg 1380
aaaagaacag aagtgtgaga aggctacatt cctgagaccc tgagaaaaag taacctgcat 1440
aagacagaga tgaaattacc tactctagtt atgattgaaa tcccaaaaag aaaacaggga 1500
aaaataatgg agcaaaagaa atatttttca aaataactgc caaaaatatt ctaaaagaag 1560
tgacagaaaa tcaaacttca gatataggaa actcagagaa tgtcgaatag aacaaaaaga 1620
aataagaatt ccatcttgaa aaatctttga aaaatcttta aaaaaatcag tctaaatttt 1680
atatcttgct ccaatatatg agatataaat aggttatcat caagatatgg agaaagccat 1740
attcatggaa acactaaaat aaggctgtgg aaggactaca ttgatattag acacaacaga 1800
gttcggaaca agaaatagta tcagagatga gagacaatag ataatagaat aatcaattct 1860
caagaagatg taaacatcct actaattagg gtatgcagct aacaacagag cctccaaata 1920
cgtgaggtaa aacacgaaag aaatcaaagg tgaactagaa aaatccaaaa ttatatttgc 1980
agacttcaac acttttgtct tagtaatgga aagactaggc acaaactcag taatcatgtg 2040
gaagataaga acaacagtat caccaacaag acatccaatc ttcaatggca gatactcttt 2100
cctttcaagt gaaaaaaaaa aaaaaa 2126

<210> 50
<211> 1363
<212> DNA
<213> Homo sapiens

<400> 50
ggcacgagtg gcataggggc ctcaggtatg agggctggaa gctctgggca ggtgggctgt 60
gtggcatctc cctcttcact agccctgcca cttgtccctg agccaggtgc tacctgatgg 120
ttgagctgta tggggacctc tgccctgtgg cctttcctcc cactgttatt tctccttggt 180
ttcctgtttt ccagctgtgg gttcccagag gcgtcatttg gaccctgggt agtagttagg 240
gctgagctct ggggttgtgt ggttggagcg gcgtgtgtct tagggctgta ctggcaagtg 300
ggccaaagca gtctaaacac cctggctagg agccagaaac cggggctccg tgtccaaccc 360
gggaagcctg ggaagctcct ccccgtcacc ttccagatgc tgccgcctcc atgtgggggg 420
tgttgctccc cgctgggtct ttgcccgagt tctgggggaa gccggatgtg gaggaggacc 480
tgggtgggtg ccagagcact tcatccttaa gctcacctca cctaaatgtt cccaccccca 540
cagccaccac cggcacaggc aggaccatgc ttcaacttgc caagagtgtt tccagggact 600
ggtccctctg gttcaacgag tttggtggtt ctcagcacca actgcttatt ggaatcatct 660
gagtagattt cagaaaagaa actgtcaatg cctggcccca gcccctgaga gtctgctgtt 720
attggtctcc agtggaacct gggccccagc atttttcaaa gctccccagg taatttgaat 780
gtgcagtcag agttgaaagc agctgccata tccagtttgg gtctccctgc ctctcccatg 840
tccctgggtt gccccagaaa ttttttctca ttcactgata attttaatga tcaatacaga 900
gtttgcaaaa gtgaagacag acatgtcaga ccaaacactg gattcagtgt tctgttccat 960
gagactgttc catgagttca tagttattaa aaccagaact taagcgggaa actatagcaa 1020
atgatagaaa ctgaattttc tcctcagttt ttaattttta aaaactttta aggctgggtg 1080
cagtggctca tgcgtgtaat cccagcactt tgggaggctg aggtggccag atcatgaggt 1140
caggagttga aaaccagcct ggccaacatg gagaaacccc gtctctacta aaaattatct 1200
gggtgcggtg gtgggtgccc ataatcccag ctactaagga gactgaggca ggagaatcgc 1260
ttgaacccgg gaggcagagg ttgcagtggg ccaagatcgt gccactgcac tccagcctgg 1320
gcgacagaga gagactccgt ttcaaaaaaa aaaaaaaaaa aaa 1363

<210> 51
<211> 2398
<212> DNA
<213> Homo sapiens

<220>
<221> SITE
<222> (1874)
<223> n equals a,t,g, or c

<400> 51
attgcttagt ttgatgtgtc ttgctttaaa tccatttatt tcaacaagct taaagagatt 60
tttttttaat ggagatgatt taattttaac aatctgtgat tttctctgaa tcgaacttgt 120
gttttggcac ctttcaatct gtggtaacaa atgacaagaa gggtgcaatt cttccttccc 180
ttgtgcaggg attttgcctc cccctttctc ccagatgaaa gatatttggg tctctagaat 240
aactgtggta cagttagctc cagagtgttt tctttctgga ggcagtttag acaacagcct 300
caagtagtgc ttttgttaaa aatatacatg tttttaaaag tgcttgtatt tctaatattc 360
ttttctcctt tctcttctag tctgttctct ggggaggcag taaggggccg tggagctggc 420
ctcggcctcg gcatcgggag aggctggact tcctgtctct ctgtgctgaa tggctgcgat 480
ggcgcccgct ctcactgacg cagcagctga agcacaccat atccggttca aactggctcc 540
cccatcctct accttgtccc ctgggcagtg ccgaaaataa cggcaacgcc aacatcctta 600
ttgctgccaa cggaaccaaa agaaaagcca ttgctgcaga ggatcccagc ctagatttcc 660
gaaataatcc taccaaggaa gacttgggaa agctgcaacc actggtggca tcttatctct 720
gctctgatgt aacatctgtt ccctcaaagg agtctttgaa gttgcaaggg gtcttcagca 780
agcagacagt ccttaaatct catcctctct tatctcagtc ctatgaactc cgagctgagc 840
tgttggggag acagccagtt ttggagtttt cyttagaaaa tcttagaacc atgaatacga 900
gtggtcagac agctctgcca caagcacctg taaatgggtt ggctaagaaa ttgactaaaa 960
gttcaacaca ttctgatcat gacaattcca cttccctcaa tgggggaaaa cgggctctca 1020
cttcatctgc tcttcatggg ggtgaaatgg gaggatctga atctggggac ttgaaggggg 1080
gtatgmccaa ttgcactctt ccacatagaa gccttgatgt agaacacaca attttgtata 1140
gcaataatag cactgcaaac aaatcytctg tcaattccat ggaacagccg gcacttcaag 1200
gaagcagtag attatcacct ggtacagact ccagctctaa cttggggggt gtcaaattgg 1260
agggtaaaaa gtctcccctg tcttccattc ttttcagtgc tttagattct gacacaagga 1320
taacagcttt actgcggcga caggctgaca ytgagagccg tgcccgcaga ttacaaaagc 1380
gcttacaggt tgtgcaagcc aagcaggttg agaggcatat acaacatcag ctgggtggat 1440
ttttggagaa gactttgagc aaactgccaa acttggaatc sttgagacca cggagccagt 1500
tgatgctgac tcgaaaggct gaagctgcct tgagaaaagc tgccagtgag accaccactt 1560
cagagggact tagcaacttt ctgaaaagca attcaatttc agaagaattg gagagattta 1620
cagctagtgg catagccaac ttgaggtgca gtgaacaggc atttgattca gatgtcactg 1680
acagtagttc aggaggggag tctgatattg aagaggaaga actgaccaga gctgatcccg 1740
agcagcgtca tgtacccctg tgagtagacc tcatgcatga tagcattctt gagaaatgtt 1800
ggcacaagga agaatgaatg aatcgccatt atggagagaa tgtgttsttt gtacataggt 1860
gtytagttcy gttngttttt tccctgatgt tgggtagatg agtgcatata catgctagtg 1920
aagaagggga agatactttg ctgtagggtt gtattgttgt agtctaaatg gtggtaattt 1980
ccttttgaag tctaagaaaa ataactagga gacatcttat gtgtaaaatt gtactagtac 2040
ctctttaaga gtgaatttag atttcttttg aaactatata taggacatga taagttaatg 2100
gcctgattgt tgagattttg ttgtttccag taagcaggga caaatgctga gttgacctag 2160
ttacctttgt aggaaattac agttgctttt gattgaactt tcagcagaga gcacacccag 2220
tcttcaattt taacacttga gattttctta cattttaagg actgacaatt agaaaatgct 2280
tcagaatatt taatacatcg cctccaagca cagtctagtt tcacaacctg actctcttcc 2340
tattaaaaaa aaaaaaaaaa aactcgrggg ggggcccgta cccaatcgcc cctcatga 2398

<210> 52
<211> 2234
<212> DNA
<213> Homo sapiens

<220>
<221> SITE
<222> (5)
<223> n equals a,t,g, or c

<220>
<221> SITE
<222> (136)
<223> n equals a,t,g, or c

<400> 52
ggctncaaag tggtccctgt cggaaagtaa tttaatcaac tggagaactc ccggagtcca 60
gcccccaact cccccacccc ccatcccagt gggaatgcca ccaacagccc atctcaacaa 120
tttcccaaag taacantctc caggtggaag acctgtgaag tatccccacc cagaaacctt 180
ggatactgag tctcctaatc ttatcaattc tgatggtttc ttttttcccc agcttttgag 240
ccaacaactc tgattaacta ttcctatagc atttactata tttgtttagt gaacaaacaa 300
tatgtggtca attaaattga cttgtagact gaggggattt tggttttggt tttgggtttt 360
gtttttttgc ggtggggggg ctggtatttg gaagaattta gctctttatg ttacagaaat 420
cttttttgca aggacttaga aatgataatg cttaagattg ttcttgcccm atgtgggaag 480
agaatctaag gtttttatat gtcttgcaac ctcatcaaag gaaaattact ggcatcattt 540
ycataatttg aaaaaaaaag ccaaattaat atatttcttt tttgattcac tttttaagtg 600
atcattttta aaactttact tttgacccac tgaatttatt tagatagaag gaaaagagat 660
gatgggaggg aagtttagat aaaggatgga agttggtttt atttaaacaa tagcccygtg 720
atttccyaat gagaagtgac tagaaattga agaaaccaaa taaggrggrt awtggkcaat 780
ttagcyttag tttctcttac tctctcaagc ctgccctgtt taactccaaa gttcatggct 840
cataatttga gaaacactgt tttaaacaca ggagaaaaaa atgtccattt taaatcatag 900
ctattgaatt ctacaattac aaagaaacaa acaaacaaaa tttgaccaac ccaggcggtt 960
aaatttaaac tcttcaggaa aaatttaagc tgttaamatt attctttttc taaatttcta 1020
aagtggaggg acagaatttt tcagatttaa aagggcctcc taggtgccca gaaaattagt 1080
ggaaagaacc acgtctagac gcatctttga tgtgtcagag ttccaaggat aaaaagaaac 1140
ttttaaagtc ttctatactc agccaggtta tcaatcaaat atgagggcaa aataatattt 1200
tcagacagat tttaggcagt ttatcttcca tatatccttt tctttaaggg tatttgtaga 1260
tacactccag aaaaacaaga gtgaaatatg aaggaagttg tggggtccag caaacagtgc 1320
ttccaaatca gacccctgat agaggtggaa aactttgcaa tgcaacaact gcgtagctgg 1380
cttagaggac agcctacaga tggwwcagaa agatgagsat gggattgagg gatcagggat 1440
tgaggtctcc aagaataaaa agggacttca tggaaaaagt aggcttgtgg ataattaatc 1500
acaggggcaa ataatgcagt taaaataaca acatgacaat caggtggagg aatgtataat 1560
aaacccaaat gtggctgggt agagtggctc acacctgtaa tcccagcact ttgggaggcc 1620
aagccgggca gattacctga ggtcaggagt tcgagaccag cttggccaac atggcgaaac 1680
cccgtctcta ctaaaaatac aaaaattagc caggcttggg ggcgcacgcy tgtagtccca 1740
gctcctcagg agctgaggta ggagaatcac ttgaacccag gaggcaaagg gtgcagggag 1800
ttgagcccaa gatcgcgcca ttgcacccta gcctgggcaa cagagcgaga ttctgtttca 1860
aaaaaccccc aagtgtatta taaggcaata attcctatac gaagcaaact aaaatgcagc 1920
aatattaagg tataaaaaca aagaggaata attccattga accttgattc tggaaacttt 1980
gatccaccca gcagtcatga tgttagactc attgaaaaga atgtatttct aatgcatgat 2040
gcaatcggtc tatagatgtg tcatggaaac ttggttgcaa cttcaagaca aaataaaaag 2100
taaacattta catgaaaaat ggtggatatg gaaggtggag aagagaggag ataacagctt 2160
tatctttcaa aatagagaat tgagagatgg taccaaaagc tgatgaagta aaaaaaaaaa 2220
aaaaaaactc gtag 2234

<210> 53
<211> 538
<212> DNA
<213> Homo sapiens

<220>
<221> SITE
<222> (502)
<223> n equals a,t,g, or c

<400> 53
ggcacgagct ccaccaccag cagcgggtaa ccccaggcct tgccgaacgt cacggcaaag 60
ggcttgaggg ccaggcgctt ggcagcgctg ggctccactt ggatcatgcc tttgacgtag 120
gcacgcaagg cagccttgtt tttcttcatc cagatagacg cgcgcttgcg ctcttcgtgg 180
gcgtgttcgt gattgttctc atccacggct ttttcgtgca gcagcaagaa gggctgctca 240
cgggccagca gacgttcgaa ggtcaggaag gcgtcttccg gcgcaccttc gctaggcgcg 300
tcgaaaaaga ttttcaccac cgggaaagtt gaactgtcga gtcgcatggc aaagctcctt 360

tgatgagatt gattctcatc atagggcgcc tggcgctgga cagcattgca cagaatagcc 420
agaatgtttc gcaatccagc caaggcagtt atcaccatgg ttcatcaccg cctcgaccag 480
tacgacccct gccgggtccg cnacgccgcc gcgatccctc gctcgattgt tgcagtgg 538

<210> 54
<211> 1484
<212> DNA
<213> Homo sapiens

<400> 54
cggcacgagg gacaataagc taaggtagta tcttggccat cccaggaaac ttgtggcatt 60
aggacgatga aggccatgct tcagtgtttt cgtttctatt tcatgagact ttttgtcttc 120
ctgcttacaa gtgggaagat gattgacagt gactctacta tgcagggctg ttggtaccaa 180
cctgagccct ataggtggca gtccctggag aagtggtcac agaagatgga gctctgatcc 240
cctgcttacc tcttcacaac acttgtgtgc aaagatagtt ttagatttgg tttagaagct 300
atcctccaga acaggctccc atacttagaa tgtttctagt taaggtaata aattaggcaa 360
cccaagtgtg actccactca agtgtccttt tctgtaggca ggaagggccc acaacatggc 420
ttaaaatgta gtccatggtt ctggcccaca gtacagtgtg tatctatacc aggtcacctg 480
tgttcaatct ggggagcctt cctggccagt ctgagtggca gccagaaggg agctcatagt 540
gtctaggaat ctcaggcaaa gtaggtcagg gtactgtggg caggggggat gtgtgtgata 600
ggagagggta ccctaaaccc cataccttcc ctccctgacc tgaaaagctg atctcaacag 660
ggattcacac agaattaggc tgtgtttttg cattaactgg taggtgactt tctcaaaatt 720
cttaaattca gaaagtattt agtaaacttg aggaaggtat gaaatctgga ggaggcatcc 780
aggacccagg ggtttgatag ctttacaggt aggatcatac cacaccaaaa gagcagtgga 840
caataagact atatgagcta tatgaagctt ttaggaatca tttaggacag acagagccct 900
aaacaaccca ttcatgactt aagttgttgg ctcagtgtat gctggggaca aagaaaaact 960
aacaagccga cctgccttta tgataaattc tagtgtgctt acaagggatg acttcctgag 1020
gtgtgatctg tccaccttga agaactccac aactgaagaa ggggagctgt gagaacgtgg 1080
attgttctac aacttgcaca gggtaacaga ggaagtggct gaggcctaga gtcacgtttt 1140
ccagttccct tcgcaaacta tatttcttgg aacgcgaaag gaagctttac ctatttcata 1200
gaagacctgg aatccataac ctcagaaggc aatattattg atagaaaatg tggaaggatc 1260
aggaagttct tagattcttg gatgacagat gcatgttgat gccctatgga gatgtccttg 1320
tgttttgagg tcactgaggt aggaagacct gtctactctt ggtttcacca ctagaacagt 1380
cttgggctgg atgggttata gagctgagcg gctgtgatgg ttctgttttt acattaacaa 1440
aaacaattaa aaacaccaaa aacaacaaaa aaaaaaaaaa aaaa 1484

<210> 55
<211> 1765
<212> DNA
<213> Homo sapiens

<400> 55
ggcacgagat ttctgggagt cctgcagagt ctagttgcca agtggaacat tcttaaaaag 60
atcgttcaga agtttaccag aattaaaaga tgctgtcttg gaccagtatt caatgtgggg 120
aaataaattt ggagtattgc tttttctgta ttctgtatta ctgacaaagg gcattgaaaa 180
cataaaaaac gaaattgaag atgcaagtga acccttgata gatcctgtat atggacatgg 240
cagccaaagt ttaattaatc tcctgctgac gggacatgct gtttctaatg tatgggatgg 300
tgatagagag tgctcaggaa tgaaacttct tggtatacat gaacaagcag cagtaggatt 360
tttaacacta atggaagctt taagatactg taaggttggt tcttacttga aatctccaaa 420
attccctatt tggattgttg gcagtgagac tcacctcacc gtattttttg ccaaggatat 480
ggctttagtt gcccctgaag ctccttcaga acaagccaga agagtttttc aaacctacga 540
cccagaagat aatggattca tacccgattc acttctggaa gatgtgatga aagcattgga 600
ccttgtttca gatcctgaat atataaatct catgaagaat aaattagatc cagaaggatt 660
aggaatcata ttattgggcc catttcttca agaatttttt cctgatcagg gctccagtgg 720
tccagaatct tttactgtct accactacaa tggattgaag cagtcaaatt ataatgaaaa 780
ggtcatgtac gtagaaggga ctgcagttgt gatgggtttt gaagatccca tgctacagac 840

agatgacact cctattaaac gctgtctgca aaccaaatgg ccatacattg agttactctg 900
gaccacagat cgctctcctt cactaaatta atttgtctaa gtatttataa ggaagatctt 960
aataacagat gttgaaagaa ggagtcaaga ctggcaattg gctggattaa gctaaacact 1020
ggtatcactg attaactgta aataacaatt aaaaacacat tttcagtgtt tatgatatgt 1080
ttaaattatt tgtcctaaag ctttatgtta aagattatcc tattttaccc cttcgtgtga 1140
aatttactag caaaattaag ctttcatcaa agttcatcac ttttgcattc agatacttgg 1200
tcatttactt accaaattac aaacgcaata ctacagcatt tgtatattaa gtatcacagt 1260
tactattgat aaactacttt tgggttttat ttcattgagg cacttttttt attgtttgaa 1320
tgattccggc ttgtaatata tcagcctcta caatgaaatg cagaagagtt catttttcta 1380
agatctgttt ttcattagaa atattgacaa ataacacatt gtcaacctgg atcctttgac 1440
aatttactta actctggcat gttcacaaaa agtagaaact ctaagagacc attaccattt 1500
attcacagat gtatagggga tgtattctaa aaactgacag aaaagagaat ctgatagtca 1560
acactgttaa cttttactgt gtaattgcca aatacacttt tccaaatttg tcccaacagc 1620
cctgtaagcc agctttcttc tatatttata aacacgataa atgcatgaga agatctgtta 1680
ttacattagt atattacgtt atttattatg atcctagttg atggcctaaa taaacacctt 1740
tttctttaaa aaaaaaaaaa aaaaa 1765

<210> 56
<211> 1478
<212> DNA
<213> Homo sapiens

<400> 56
   ggcacgagga gggcggaagt gggagctgcg accgcgctcc ctgtgaggtg ggcaagcggc 60
   gaaatggcgc cctccgggag tcttgcagtt cccctggcag tcctggtgct gttgctttgg 120
   ggtgctccct ggacgcacgg gcggcggagc aacgttcgcg tcatcacgga cgagaactgg 180
   agagaactgc tggaaggaga ctggatgata gaattttatg ccccgtggtg ccctgcttgt 240
   caaaatcttc aaccggaatg ggaaagtttt gctgaatggg gagaagatct tgaggttaat 300
   attgcgaaag tagatgtcac agagcagcca ggactgagtg gacggtttat cataactgct 360
   cttcctacta tttatcattg taaagatggt gaatttaggc gctatcaggg tccaaggact 420
   aagaaggact tcataaactt tataagtgat aaagagtgga agagtattga gcccgtttca 480
   tcatggtttg gtccaggttc tgttctgatg agtagtatgt cagcactctt tcagctatct 540
   atgtggatca ggacttgcca taactacttt attgaagacc ttggattgcc agtgtgggga 600
   tcatatactg tttttgcttt agcaactctg ttttccggac tgttattagg actctgtatg 660
   atatttgtgg cagattgcct ttgtccttca aaaaggcgca gaccacagcc gtacccatac 720
   ccttcaaaaa aattattatc agaatctgca caacctttga aaaaagtgga ggaggaacaa 780
   gaggcggatg aagaagatgt ttcagaagaa gaagctgaaa gtaaagaagg aacaaacaaa 840
   gactttccac agaatgccat aagacaacgc tctctgggtc catcattggc cacagataaa 900
   tcctagttaa attttatagt tatcttaata ttatgatttt gataaaaaca gaagattgat 960
   cattttgttt ggtttgaagt gaactgtgac ttttttgaat attgcagggt tcagtctaga 1020
   ttgtcattaa attgaagagt ctacattcag aacataaaag cactaggtat acaagtttga 1080
   aatatgattt aagcacagta tgatggttta aatagttctc taatttttga aaaatcgtgc 1140
   caagcaataa gatttatgta tatttgttta ataataacct atttcaagtc tgagttttga 1200
   aaatttacat ttcccaagta ttgcattatt gaggtattta agaagattat tttagagaaa 1260
   aatatttctc atttgatata atttttctct gtttcactgt gtgaaaaaaa gaagatattt 1320
   cccataaatg ggaagtttgc ccattgtctc aagaaatgtg tatttcagtg acaatttcgt 1380
   ggtcttttta gaggtatatt ccaaaatttc cttgtatttt taggttatgc aactaataaa 1440
   aactacctta cattaattaa aaaaaaaaaa aaaaaaaa 1478

<210> 57
<211> 1145
<212> DNA
<213> Homo sapiens

<220>
<221> SITE

<222> (9)
<223> n equals a,t,g, or c

<220>
<221> SITE
<222> (410)
<223> n equals a,t,g, or c

<400> 57
caggcagang ggctgagtca caggcacagg tgaggaactc aactcaaact cctctctctg 60
ggaaaacgcg gtgcttgctc ctcccggagt ggccttggca gggtgttgga gccctcggtc 120
tgccccgtcc ggtctctggg gccaaggctg ggtttccctc atgtatggca agagctctac 180
tcgtgcggtg cttcttctcc ttggcataca gctcacagct ctttggccta tagcagctgt 240
ggaaatttat acctcccggg tgctggaggc tgttaatggg acagatgctc ggttaaaatg 300
cactttctcc agctttgccc ctgtgggtga tgctctaaca gtgacctgga attttcgtcc 360
tctagacggg ggacctgagc agtttgtatt ctactaccac atagatcccn ttccaaccca 420
tgagtgggcg gtttaaggac cgggtgtctt gggatgggaa tcctgagcgg tacgatgcct 480
ccatccttct ctggaaactg cagttcgacg acaatgggac atacacctgc caggtgaaga 540
acccacctga tgttgatggg gtgatagggg asatccggct cagcgtcgtg cacactgtac 600
gcttctctga gatccacttc ctggctctgg ccattggctc tgcctgtgca ctgatgatca 660
taatagtaat tgtagtggtc ctcttccagc attaccggaa aaagcgatgg gccgaaagag 720
ctcataaagt ggtggagata aaatcaaaag aagaggaaag gctcaaccaa gagaaaaagg 780
tctctgttta tttagaagac acagactaac aattttagat ggtaaggttc acaaataggt 840
tgatttcttt cttcagcttt ctgacatgtc cagcccatct ctaatgagga ctcccagatc 900
atcactttat ggctgttarg tgtttcccat atgaaattag aggagctggg tcagggagac 960
aaaagtcttc tattagtctt atggatagct cctccttgag tgtattttgt gcaaaagatt 1020
aagaagctgg actctactgc cattaaagct gagagaatcc taaggttatt tgtggcttcg 1080
gggttatatt tattactact actactaata aatattcaac aagtaaataa atctttttta 1140
aatca 1145

<210> 58
<211> 1772
<212> DNA
<213> Homo sapiens

<220>
<221> SITE
<222> (1480)
<223> n equals a,t,g, or c

<400> 58
tcgacccacg cgtccgggag agaacgccgg tggcggggct ggtagcccgg cagccgcagt 60
ggggccacga gcgctggctg agggaccgag ccggagagcc ccggagcccc cgtaacccgc 120
gcggggagcg cccaggatgc cgcgcgggga ctcggagcag gtgcgctact gcgcgcgctt 180
ctcctacctc tggctcaagt tttcacttat catctattcc accgtgttct ggctgattgg 240
ggccctggtc ctgtctgtgg gcatctatgc agaggttgag cggcagaaat ataaaaccct 300
tgaaagtgcc ttcctggctc cagccatcat cctcatcctc ctgggcgtcg tcatgttcat 360
ggtctccttc attggtgtgc tggcgtccct ccgtgacaac ctgtaccttc tccaagcatt 420
catgtacatc cttgggatct gcctcatcat ggagctcatt ggtggcgtgg tggccttgac 480
cttccggaac cagaccattg acttcctgaa cgacaacatt cgaagaggaa ttgagaacta 540
ctatgatgat ctggacttca aaaacatcat ggactttgtt cagaaaaagt tcaagtgctg 600
tggcggggag gactaccgag attggagcaa gaatcagtac cacgactgca gtgcccctgg 660
acccctggcc tgtggggtgc cctacacctg ctgcatcwgg aacacracag aagttgtcaa 720
caccatgtgt ggctacaaaa ctatcgacaa ggagcgtttc agtgtgcakg atgtcatcta 780
cgtgcggggc tgcaccaacg ccgtgatcat ctggttcatg gacaactaca ccatcatggc 840
gggcatcctc ctgggcatcc tgcttcccca gttcctgggg gtgctgctga cgctgctgta 900
catcacccgg gtggaggaca tcatcatgga gcactctgtc actgatgggc tcctggggcc 960

cggtgccaag cccagcgtgg aggcggcagg cacgggatgc tgcttgtgct accccaatta 1020
gggcccagcc tgccatggca gctccaacaa ggaccgtctg ggatagcacc tctcagtcaa 1080
catcgtgggg ctggacaggg ctgcggccct ctgcccacac tcagtactga ccaaagccag 1140
ggctgtgtgt gcctgtgtgt aggtcccacg gcctctgcct ccccagggag cagagcctgg 1200
gcctccccta agaggctttc cccgaggcag ctctggaatc tgtgcccacc tggggcctgg 1260
ggaacaaggc cctcctttct ccaggcctgg gctacrgggg agggagagcc tgaggctctg 1320
ctcagggccc atttcatctc tggcagtgcc ttggcggtgg tattcaaggc agttttgtag 1380
cacctgtaat tggggagagg gagtgtgccc ctcggggcag gagggaaggg catctgggga 1440
agggcaggag ggaagagctg tccatgcagc cacgcccatn gccaggttgg cctcttctca 1500
gcctcccagg tgccttgagc cctcttgcaa gggcggctgc ttccttgagc ctagtttttt 1560
tacgtgattt ttgtaacatt catttttttg tacagataac aggagtttct gactaatcaa 1620
agctggtatt tccccgcatg tcttattctt gcccttcccc caaccagttt gttaatcaaa 1680
caataaaaac atgttttktt ttkttttttt aaaaaaaaaa aaaaaaaaaa aaaaaaaaaa 1740
aaaaaaaaaa aaaaaaaaaa aagggcggcc gc 1772

<210> 59
<211> 1279
<212> DNA
<213> Homo sapiens

<400> 59
ggcacgagtt tattttaaaa tgtacaataa attattgttg actgtagtaa ccctgttttg 60
ctatcaaata gtagatttta tttattctaa ctatattttt atatccatta accatccccc 120
acatcccccc aatattttag ttttttgagg aactccagtg catcattaat acccactttt 180
cctccctcct cctctctcac cactccccaa gccatttcta attcgtctcc aagccttgtg 240
taattgttta ttaatattta tttatttggc tgggtgcggt ggcttacacc tgtagtccca 300
gcactttggg aagccgaggc ggctgggtcg cctgaggtca ggagttcaag accagcctgg 360
ccaacatggc aaaaccccgt ctctgctaaa aatacaaaaa ttagctgggc gtggtgatgc 420
acacctgtaa tcccaaccac ctgcgaggct gaagcaggag aatcgcttga acccaggaag 480
tggaggaggt tatatatata tgagacatat atacacacac acacacacac aaatataaaa 540
tatgtgttga tatatatata taaacatata tatatgttta tttgtcccct ctttcccatt 600
ctcattgctg ctgtccctat taagaccttt atcatcattt ctttggccta attagaatag 660
cctctggtct tctagttttc attcttatcc attgctagtt accttttatt ttgtcactaa 720
tgtgatcatt caaaattgct agtttggaga taatatattc ctgtttcaaa accctcccct 780
tgaggtgtac ccaacagctc attgagaacg ggccacgatg acaatggcgg ttttgtggaa 840
tagaaaaggg ggaaaggtgg ggaaaagatt gagaaatcgg atggttgctg tgtctgtgta 900
gaaagaagta gacatgggag acttttcatt ttgttctgta ctaagaaaaa ttcttctgcc 960
ttgggatcct gttgatctat gaccttaccc ccaaccctgt gctctctgaa acatgtgctg 1020
tgtccactca gggttaaatg gattaagggc ggtgcaagat gtgctttgtt aaacagatgc 1080
ttgaaggcag catgctcgtt aagagtcatc accactccct aatctcaagt acccagggac 1140
acaaacactc tgcctaggaa aaccagagac ctttgttcac ttgtttgtct gttgaccttc 1200
cctccactgt tgtcctgtga ccctgccaag tcccctctgc gagaaacacc caagaatgat 1260
caaaaaaaaa aaaaaaaaa 1279

<210> 60
<211> 1539
<212> DNA
<213> Homo sapiens

<400> 60
gaattcggca cgagtatcac tgcatatttt tacccttatt tttgctcctt acagcaagat 60
tagtaggtta taaaaattta aatttaaaca aaattatttc atgacaaaat gggaaacttc 120
acatcatact tatttttgtt tgccttttca ggcatcatat tagcttttat aaaaaatggt 180
cttgctgctg aaattgtact tattttatca gaggctgggt gcagtcaaga caaaagtaaa 240
atggtttacc tgagcccagg ggagggaaaa ttgattaaga tatcatattt ttgtttggtt 300
tggttttgct ttttcctctt actttaattg aaatactctg aattcccctc aggaaacaga 360

gagcatgaga gcactttctt taaaaggacc aaaaataaat tcctaataga ttttgtccta 420
agagagtgtt tttttttcta gcatcatttt ctttacatgc cactcatgtc ataaggcatg 480
gacaggctat ctttcagtgg ccattactat gtttcgtaca catgctttat tttacttggg 540
ctctgagaaa tgtgtggctt tccttcagca ttttatttgt gcttctcttt ttaatggaga 600
ttgaaaaggg agaataatgt gaatatcacg gcttatatta ttaaatgttg attgatggct 660
tgtaatgtac tgcacacaat atatgttaac tctgcagaat gacagaccct gggagaagta 720
atgccccagt tgtcccccac tcctaatgcc aggcagagaa ggacagcctt tatagactta 780
atctgctttt tgtcccattt gacaaggtac caggaggaaa ttttttaagg gatcaactgt 840
atcacagtgc ccactctgga cctaagtcta gtgtatccat acaattggtg cagagaaata 900
aggtgtaaat ggtgctttgt tcctgctggt tccaagctca gaaaccaaga ctagctttgt 960
aggagagaat gagagcctgc aagcctctct ttggattggc tgaggagtgg tgggagcagg 1020
gggttgatag aaaacatcca gacacacata taagcaagtg gccgtgctac ctttttagag 1080
aataaagaaa cagacttttg agtttatatg caatgccttc attaggtacc accggcactt 1140
acaaaatgtg cggactgaat cccagagaac actggcagat gtatacagta tatggattgt 1200
atcgcttccc caatgtttgt aaattcacag tatttggaaa actgccttca ttttccagtg 1260
tgggaaaaac tcttgctacc tgtattactt gatctcagac ccatacctga tggttcagtc 1320
tgtccttaag ttaaaagaat tttgcttttc taatgttata ctatttacct gtcagtgtat 1380
tactgcaact tgaatcactc ttttactgtt gttggatata aacttatcct gtaccaatgt 1440
atttattaac acttgtattt tattattgag catatcaata aaaatattaa aaaataacag 1500
attgtttttt accaacaaaa aaaaaaaaaa aaaactcga 1539

<210> 61
<211> 1937
<212> DNA
<213> Homo sapiens

<400> 61
ggcacgagct gtagttgata atgttgggaa taagctctgc aactttcttt ggcattcagt 60
tgttaaaaac aaataggatg caaattcctc aactccaggt tatgaaaaca gtacttggaa 120
aactgaaaac tacctaaatg atcgtctttg gttgggccgt gttcttagcg agcagaagcc 180
ttggccaggg tctgttgttg actctcgaag agcacatagc ccacttccta gggactggag 240
gtgccgctac taccatgggt aattcctgta tctgccgaga tgacagtgga acagatgaca 300
gtgttgacac ccaacagcaa caggccgaga acagtgcagt acccactgct gacacaagga 360
gccaaccacg ggaccctgtt cggccaccaa ggaggggccg aggacctcat gagccaagga 420
gaaagaaaca aaatgtggat gggctagtgt tggacacact ggcagtaata cggactcttg 480
tagataatga tcaggaaccc tattcaatga taacattaca cgaaatggca gaaacagatg 540
aaggatggtt ggatgttgtc cagtctttaa ttagagttat tccactggaa gatccactgg 600
gaccagctgt tataacattg ttactagatg aatgtccatt gcccactaaa gatgcactcc 660
agaaattgac tgaaattctc aatttaaatg gagaagtagc ttgccaggac tcaagccatc 720
ctgccaaaca caggaacaca tctgcagtcc taggctgctt ggccgagaaa ctagcaggtc 780
ctgcaagtat aggtttactt agcccaggaa tactggaata cttgctacag tgtctgaagt 840
tacagtccca ccccacagtc atgctttttg cacttatcgc actggaaaag tttgcacaga 900
caagtgaaaa taaattgact atttctgaat ccagtattag tgaccggctt gtcacattgg 960
agtcctgggc taatgatcct gattatctga aacgtcaagt tggtttctgt gcccagtgga 1020
gcttagacaa tctcttttta aaagaaggta gacagctgac ctatgagaaa gtgaacttga 1080
gtagcattag ggccatgctg aatagcaatg atgtcagcga gtacctgaag atctcacctc 1140
atggcttaga ggctcgctgt gatgcctcct cttttgaaag tgtgcgttgc accttttgtg 1200
tggatgccgg ggtatggtac tatgaagtaa cagtggtcac ttctggcgtc atgcagattg 1260
gctgggtcac tcgagacagc aaattcctca atcatgaagg ctacggaatt ggggatgatg 1320
aatactcctg tgcgtatgat ggctgccggc agctgatttg gtacaatgcc agaagtagcc 1380
tcacatacac ccatgctgga aagaaggaga tacagtagga tttctgttag acttgaatga 1440
aaagcaaatg atcttctttt taaatggcaa ccagctgcct cctgaaaagc aagtcttttc 1500
atctactgta tctggatttt ttgctgcagc tagtttcatg tcatatcaac aatgtgagtt 1560
caattttgga gcaaaaccat tcaaataccc accatctatg aaatttagca cttttaatga 1620
ctacgccttc ctaacagctg aagaaaaaat cattttgcca aggcacaggc gtcttgctct 1680
gttgaagcaa gtcagtatcc gagaaaactg ctgttccctt tgttgtgatg aggtagcaga 1740
cacacaattg aagccatgtg gacacagtga cctgtgcatg gattgtgcct tgcagctgga 1800

gacctgccca ttgtgtcgta aagaaatagt atctagaatc agacagattt ctcatatttc 1860
atgacacatg tgaagaggca tcgtggactt ttttctactc aattccagcc aatgttgaaa 1920
aaaaaaaaaa aaaaaaa 1937

<210> 62
<211> 1452
<212> DNA
<213> Homo sapiens

<400> 62
ccacgcgtcc gcggacggtg gacggacgcg tgggtggacg cccaccatgc cgccccgagg 60
gccagcctct gagctgctgc tgctgcggct gctcctgctg ggggcggcca ccgctgctcc 120
cttggcaccg agaccctcca aggaggagct gacccgctgt ctggcagagg tggtcacaga 180
ggtgctgacc gtgggccagg tccagagagg accctgcact gctcttctcc acaaggagtt 240
gtgcgggaca gagccccacg gctgtgcgtc caccgaggag aaaggcctgc tgcttgggga 300
tttcaagaag caggaggctg ggaagatgag gtccagccag gaggtgaggg atgaggaaga 360
ggaggaggta gcagagagga cccacaagtc tgaggtccag gaacaagcca tccgcatgca 420
agggcatcgc cagctccacc aggaggagga cgaggaggag gagaaggagg agaggaagag 480
ggggcccatg gagacctttg aggacctgtg gcagcggcat ctagagaatg gaggggacct 540
ccagaagcgg gtggcagaga aggccagtga caaagagacg gcccagttcc aggcagagga 600
gaagggggtg cgggtgctgg gcggggaccg cagcctgtgg cagggggccg agagaggcgg 660
aggagagagg cgcgaggact tgccccacca ccaccaccac caccaccagc cagaggctga 720
gcccaggcag gagaaggagg aggcttcgga gagggaggtg agtaggggga tgaaggagga 780
acaccaacac agtttggagg cagggttgat gatggtcagt ggagtcacaa ctcacagcca 840
ccggtgttgg ccctgcacca ccagatccat cactagtgga tcacagtggc caagactgac 900
accacgactg gctaacaact tccgtgcaag gcctttacct tatacttcca cactactgta 960
tggactacag caaccaagat ggcaccattg cacagaagca agccaccatc actagcaagt 1020
tggccactgt gaaaagtggc tgctgtgcct acttcactag gtgacagaca gacaccattg 1080
ctgggtcatg gaaaacaaga tgtcaccatg attggtggca ccaaaagtgc cgtaacaggg 1140
tgggcatggt ggctcacacc tataatccta gggagggtta atcctttcag aggccaaggt 1200
gggagaatcc cttgaggcca ggagtttgag accagcgtgg gcaacatagt gaaaccgtga 1260
ctctacaaat aatttaaaaa attagccagc aatggtggcg cacgcctgtg gtcccagctc 1320
tcaggaggct gaggtggtgg gattgcttga acccgggagt ttgaggctgc attgagtcat 1380
gattgtgcca cagcagtccc gcctgggcca cagagcaaaa ccatcttaaa aaaaaaaaaa 1440
aaaaaaaaaa aa 1452

<210> 63
<211> 971
<212> DNA
<213> Homo sapiens

<400> 63
gataaaatct tggtgtgtca gtgggtgaga cagtgccata tcccactcgg tatcatggcc 60
ctagaaacat gagcttttga tgaaggcaat aaaatggagc ttagaaaaaa cactattttg 120
ataatatact atattagcag aatgttgttt ttgagatcca tcttatggct ctcttcatta 180
ttcttttgtc attttgtacc tacatcccat tcattgggat tccaaaatat aacttctgtg 240
tataatgcca ctctgcaaca aacagtgttc cagcatgatt ctaagacagt tactacatgc 300
tttacgtgaa acatgatcca aaatatcaat caccctcaag tcctttgtat ttagaatatt 360
ctgactatat attcatgaaa gcayttcaac ttagagacat cttcattcaa aaggtgagta 420
tccttccata tctgtctggt gtacacaatg atttacgtgc tatgctcgaa caaagataaa 480
caaaattcat taagaagctt ccatttcaat agcacakgtt taatttgaat actgagttag 540
tacttgttct gtgsctagta ttaaaagcaa agtaataaag gctttgtttc atgatctttg 600
gtacatctta ccactctcgc cagcaaaatt ttaaaatatt aataaatatt tgtaacattt 660
tgtttctttt gtcccttttt taaaaaatgt tttcttgtct gccttcccca gattttgcta 720
tctgaggcca ttttctcaga aggggttgtg ggaggaaca ggtagtgagt atttagatta 780
gactcccctc tgtagagcag agccccatga cttctatagg ccctagacac ttttgccttg 840

gtgggttcct ttctccatag aaaaagtaaa acctttattt catgtctgca ttggtataaa 900
gattaatacc attattattg ktatcctcat tttttccttc tgattgaaaa aaaaaaaaaa 960
agggcggccg c 971

<210> 64
<211> 1723
<212> DNA
<213> Homo sapiens

<400> 64
cggcacgagg tggaaactgt ttcagcaaag gttcttgtat agagggaata gggaatttca 60
aaataaaaaa ttaagtatgt tctgtgtttt cattttaact ttttttatgg tgtttaattt 120
gtggttggct gcaactgtgt atcatgtata tggaacttgt aaaaaagttc tcgacattca 180
gatcttaaga gatgaaatca cttttaccta taaaaaccac ttttattgcg gtttgactgc 240
attgagctct aggatattaa atgatatcac taatattttg catgtaattt gctcatttga 300
gtgagggcac tttttttgta catatgatgg ggccaatgca caatactttt atcacaatca 360
actttttctt tgtatcccta tttcaatgag cagtcagtct caagaggtta ctgcacttca 420
gttctaacta gacatttgta ctaaggtatt tcagttatgt aaactcagcc tgggcacttt 480
ctgataactg taaaatgttt tataagatca tgattattga agatacattt tggaaaattt 540
taaatgttcg tgagcagctt aactactttt gtatctagcc ttttttaagt atcttgttac 600
atttactttt ttaaatgaag aaattacaga agaaatgtca agtaatattg aagaaacaat 660
agtttttatt tatgtagttg tacattttta aactaagggc aatacactga catggttatg 720
tgcataaaaa ttttgactta aagaactgga agtttatata cacctggact ataagaaacg 780
gaagaaaatc agtccacatt ttacagttag cagaatccta aatggcactg gcctggccac 840
cttttcattt tacaaatggg gaagtgaatg tgacccctta cttggcatag gaagttaact 900
tacacctaat aactgacagg tttttgtttg atgacctatt aattatgtag cctaggatta 960
atatcccaaa attactctgg tttaagtagc tttattcagt ggcataataa cactgttttc 1020
ttccttaagt cttcaatgaa gtgacttaaa acagtcactt tacatattaa aaatgaggag 1080
agcaattctc tggaatctct cctttcagtt cctttgtagg atttctggcc ttgaggatag 1140
tcttcatgtt caaaggcact atgcttttat tatataactt ccttcagaag actgaaccac 1200
atgatattct cagccctgtt aacactaaaa atatttaaaa ctgaatgata gtagtgactc 1260
attgtattac ttaaaactta tataacacgc tgtattagat gtgtgtaaat tagccaaagg 1320
ttattttaca aagtgagaca ttggttttta tgtctaaatg ctatttctga ataaatgaaa 1380
tagtaattag atcaagagct gattagcatc aatgtgtttg aaagatataa aatttataca 1440
tcaccttaac ctctgtatgc acatgatggg attgataaaa tattaaatga gaacaaacta 1500
gatatgatta ggacatttga aaccctaatt gtgaatttat ttttaatagt tactgaaatg 1560
aaaatattta aaataatgca caatgtctta agtcttccta aatcaagatt ttggttaaaa 1620
aatacttcta ataatagtaa aagatttttt ttttaagtaa atcataaaac ggttctaaat 1680
gtaaaataaa gacatgtaaa ataaaaaaaa aaaaaaaaaa aaa 1723

<210> 65
<211> 1955
<212> DNA
<213> Homo sapiens

<400> 65
   ggcacgagtg ccatccctgt atttgctgcc atgctcttcc ttttctccat ggctacactg 60
   ttgaggacca gcttcagtga ccctggagtg attcctcggg cgctaccaga tgaagcagct 120
   ttcatagaaa tggagataga agctaccaat ggtgcggtgc cccagggcca gcgaccaccg 180
   cctcgtatca agaatttcca gataaacaac cagattgtga aactgaaata ctgttacaca 240
   tgcaagatct tccggcctcc ccgggcctcc cattgcagca tctgtgacaa ctgtgtggag 300
   cgcttcgacc atcactgccc ctgggtgggg aattgtgttg gaaagaggaa ctaccgctac 360
   ttctacctct tcatcctttc tctctccctc ctcacaatct atgtcttcgc cttcaacatc 420
   gtctatgtgg ccctcaaatc tttgaaaatt ggcttcttgg agacattgaa aggaaactcc 480
   tggaactgtt ctagaagtcc tcatttgctt ctttacactc tggtccgtcg tgggactgac 540
   tggatttcat actttcctcg tggctctcaa ccagacaacc aatgaagaca atcaaaggat 600
   catggacagg gaagaatcgc gtccagaatc cctacagcca tggcaatatt gtgaagaact 660
   gctgtgaagt gctgtgtggc cccttgcccc ccagtgtgct ggatcgaagg ggtattttgc 720
   cactggagga aagtggaagt cgacctccca gtactcaaga gaccagtagc agcctcttgc 780
   cacagagccc agcccccaca gaacacctga actcaaatga gatgccggag gacagcagca 840
   ctcccgaaga gatgccacct ccagagcccc cagagccacc acaggaggca gctgaagctg 900
   agaagtagcc tatctatgga agagactttt gtttgtgttt aattagggct atgagagatt 960
   tcaggtgaga agttaaacct gagacagaga gcaagtaagc tgtccctttt aactgttttt 1020
   ctttggtctt tagtcaccca gttgcacact ggcattttct tgctgcaagc ttttttaaat 1080
   ttctgaactc aaggcagtgg cagaagatgt cagtcacctc tgataactgg aaaaatgggt 1140
   ctcttgggcc ctggcactgg ttctccatgg cctcagccac agggtcccct tggaccccct 1200
   ctcttccctc cagatcccag ccctcctgct tggggtcact ggtctcattc tggggctaaa 1260
   agttttcgag actggctcaa atcctcccaa gctgctgcac gtgctgagtc cagaggcagt 1320
   cacagagacc tctggccagg ggatcctaac tgggttcttg ggtcttcagg gactgaagag 1380
   gagggagagt ggggtcagaa gattctcctg gccaccaagt gccagcattg cccacaaatc 1440
   cttttaggaa tgggacaggt accttccact agttgtattt attagtgtag cttctccttt 1500
   gtctcccatc cactctgaca ccttaagccc cactcttttc ccattagata tatgtaagta 1560
   gttgtagtag agataataat tgacatttct cgtagactac ccagaaactt ttttaatacc 1620
   tgtgccattc tcaataagaa tttatgagat gccagcggca tagcccttca cactctctgt 1680
   ctcatctctc ctcctttctc attagcccct tttaatttgt ttttcctttt gactcctgct 1740
   cccattagga gcaggaatgg cagtaataaa agtctgcact ttggtcattt cttttcctca 1800
   gaggaagcct gagtgctcac ttaaacacta tcccctcaga ctccctgtgt gaggcctgca 1860
   gaggccctga atgcacaaat gggaaaccaa ggcacagaga ggctctcctc tcctctcctc 1920
   tcccccgatg taccctcaaa aaaaaaaaaa aaaaa 1955

<210> 66
<211> 1192
<212> DNA
<213> Homo sapiens

<400> 66
ggcacgagca cattttagtg tacattttta gaatatattt aaaacaataa gatagtctga 60
attggatggt tgagtaacct ttaaactcat ctggtaaacc tctaatgtat agtagaaata 120
atttgaaagc ttttaatgta taatagtact tacttcagga aaataatttg atgtttcatt 180
gttggtctct ttttctatat tatttcagcc taagtctatc ttcataccac aggaaatgca 240
ttctactgag gatgaaaatc aaggaacaat caagagatgt cccatgtcag ggagcccagc 300
aaagccatcc caagttccac ctagaccacc acctcccaga ttacccccac acaaacctgt 360
tgccttaggt aatggtggag ggtgacagca aatatgttac caggttttca tactatgggg 420
agaaaaaaaa ctttctttta agagattatt tgaaattctt ttggtggagg acagaaggaa 480
agcagtggct atggagatgt tttctgcttt ttgcctacta gcttaaagtg tttttatgac 540
aggattccct atgacacagt ctgagatatt ttgtcctcat ttctcatttc atatttagcc 600
ttctctcttc tagagactgg ttccccattc atttagctac ggtgtggaaa caatgcaaat 660
taaactatga acaaacatgg aaaatgtgtt ttgcgtctag gttacttctg ttttagaaga 720
gagtaccttg tcctaactcc ttatttcatt taatcatttc taaaaaaata attggtatta 780
tttgctaggt atttgcctcc aaattaatac tagaaggtgc tattttaaca ctgtaaagac 840
tcctctgtgt ttatccagaa gaagcaattt taaaaaagag caactaggct gggcatggtg 900
gctcacacct gtaatcccag cactttggga ggccgaggca ggtggatcac ctgaggtcaa 960
gagtttgaga ccagcctgac caacatggtg aaactccgtc tctactaaaa aaaaaaatac 1020
aaaattagct gggcgtggta gcgcatgcct gtaatcctac ttgggagact aaggcaggag 1080
aatcgcttgc ttgaacctgg gaggcggagt ttgcactgag ccaagatcac gccattgcac 1140
tctagcctgg gtgataagag caaaactcct tctcaaaaaa aaaaaaaaaa aa 1192

<210> 67
<211> 1543
<212> DNA
<213> Homo sapiens

<220>
<221> SITE
<222> (76)
<223> n equals a,t,g, or c

<400> 67
cttgactgtg ttttattatt tcatggcttg tatgagtgtg actgggtgtg tttctttagg 60
gttctgattg ccagtnattt tcatcaataa gtcttgcaaa gaatgggatt gtcattcttc 120
acttcagcac agttctagtc ctgcttctct ggagtagggt tgttgagtaa ggttgcttgg 180
gttgtgcatt gcacaagggc acatggctgt gaggtgtatc ctggcggggg gctgtctacc 240
tgcagtgagg ggcacctttt ctgttttgct caaaggcatg tataagccaa tgggtgacct 300
tatttcctgt gtcttcaggt gtgtggcagg gggcctgggg tggggaggtg gggcgagcga 360
gcagtgtgtg gaaagccttg ttgtcacctg aagcacgcca ggtccagatt gaccaatggt 420
tttctcactt cagggccmac ccacgccccc tttctgctga ggtttgggtg ccatctagtg 480
gtgggatggg acttggttga ctacatttaa ggtaaggtgg acccagcaac tcccagaaac 540
aactccgggg acaccactcc ccatcacact ccacaccgag cctggtgccc ggtctgtgcc 600
cgagctcagc gggaccagga agggatgggc cctgccaggg ttgcccctgc actgtgcatt 660
ctcgcctggg aggcacaagt tctttcatct gcttttcctt cagaggtgct gagcccacgc 720
catagcccct gtgggatggt gggggagggg gcgacccgaa caacagtgca gtcggtatcg 780
agattgggga gaggagcgag tccaaggaga aggtcatgag tttcttttta ctcgtgttga 840
ataataacaa taacaataac aatatggaaa ccaccgcaaa cttggagaaa agttgtaagc 900
acagtaaaga gaagcttcct tctgagtcac ttgagtggtt gccgttctgg ccctgcaccc 960
tctgtgcttt gggacggcgt ccaacccgca ttcatgtcag gagtgagtcg cacgtggctt 1020
tgtggtcatg gcgacttaat ctgcctggac ggtggctccg tctccctggg cttagacgac 1080
cttggcactt ctggagataa gcccatggct cccaggttgt gttcatgtga cgtttccttg 1140
tggtaggttc tgggtctgcg ttttgtctag gagtgtcaca ggatggacac tgcctcctgg 1200
caggggctgc ccaatgcagt tagcctcctg ctggtgttct ctcttgttgc ttggtgaagg 1260
tggccctggt cagcttctcc actgcccagt gaacgacccc tttgtaatga atgagtgggg 1320
aggtagtgtg aagcgatgcc aatatcccat ccctgtcaaa ctgcctttac tttttccttc 1380
cttccttgct cccacctgtg tggatcctgg tcccttcttg tattcagggc tgtggtctgt 1440
tatgacattt actctcaggc tcaggtcctg cttgtttggc ccgtgggagc cccttcttct 1500
gccttttgtg ttkttttggt atgtacctac attatttaac tgg 1543

<210> 68
<211> 1282
<212> DNA
<213> Homo sapiens

<400> 68
   ggcacgagct gggtccggtc aaccgtcaaa atgtccaaag aacctctcat tctctggctg 60
   atgattgagt tttggtggct ttacctgaca ccagtcactt cagagactgt tgtgacggag 120
   gttttgggtc accgggtgac tttgccctgt ctgtactcat cctggtctca caacagcaac 180
   agcatgtgct gggggaaaga ccagtgcccc tactccggtt gcaaggaggc gctcatccgc 240
   actgatggaa tgagggtgac ctcaagaaag tcagcaaaat atagacttca ggggactatc 300
   ccgagaggtg atgtctcctt gaccatctta aaccccagtg aaagtgacag cggtgtgtac 360
   tgctgccgca tagaagtgcc tggctggttc aacgatgtaa agataaacgt gcgcctgaat 420
   ctacagagag cctcaacaac cacgcacaga acagcaacca ccaccacacg cagaacaaca 480
   acaacaagcc ccaccaccac ccgacaaatg acaacaaccc cagctgcact tccaacaaca 540
   gtcgtgacca cacccgatct cacaaccgga acaccactcc agatgacaac cattgccgtc 600
   ttcacaacag caaacacgtg cctttcacta accccaagca cccttccgga ggaagccaca 660
   ggtcttctga ctcccgagcc ttctaaggaa gggcccatcc tcactgcaga atcagaaact 720
   gtcctcccca gtgattcctg gagtagtgct gagtctactt ctgctgacac tgtcctgctg 780
   acatccaaag agtccaaagt ttgggatctc ccatcaacat cccacgtgtc aatgtggaaa 840
   acgagtgatt ctgtgtcttc tcctcagcct ggagcatctg atacagcagt tcctgagcag 900
   aacaaaacaa caaaaacagg acagatggat ggaataccca tgtcaatgaa gaatgaaatg 960
   cccatctccc aactactgat gatcatcgcc ccctccttgg gatttgtgct cttcgcattg 1020
   tttgtggcgt ttctcctgag agggaaactc atggaaacct attgttcgca gaaacacaca 1080
   aggctagact acattggaga tagtaaaaat gtcctcaatg acgtgcagca tggaagggaa 1140
   gacgaagacg gcctttttac cctctaacaa cgcagtagca tgttagattg aggatggggg 1200
   catgacactc cagtgtcaaa ataagtctta gtagatttcc ttgtttcata aaaaagactc 1260
   acttaaaaaa aaaaaaaaaa aa 1282

<210> 69
<211> 1440
<212> DNA
<213> Homo sapiens

<220>
<221> SITE
<222> (323)
<223> n equals a,t,g, or c

<220>
<221> SITE
<222> (337)
<223> n equals a,t,g, or c

<400> 69
gcttccacac agtatgacag acctctagac tagaagtaca tgatgaaaat agttggtaat 60
taagataaaa ttgatttaat ttactttagt cctgaacatt gaatacttgt caggatgcca 120
ttgcaataat ggcatatatc ggagccaaat ggtcaaatga tacacagagc caggagccta 180
gcagccttgt ccagtttgat gctctatacc aagcttgtcc aaccagtggc ctgcatatca 240
catgtggccc aggacggctt tgaatatggc ccaacacaaa ttcataaact ttcttaaaac 300
aatatgagct tatgaaattt tyntcatgat atttttnctt ttttcttttt tttttttttt 360
taactcatya gctatcatta gtgttaatgt attttatgtg tggcccaaga cagttcttcc 420
aatgtggccc aggaaagcca aaagattgga cacccctgct ttataccctt tacactgtcc 480
tcggtagaga aaaaaaaaat gcttcaaaga atcgctaatt ttaaagaaga gtagatgata 540
aaagttacca aaacaaaccg aaaaatttat tgtatttggg attttagaaa atccaactat 600
taggaaccag aatttagtct gctacagtag gaaaacaatg tgaatattca catcatcaag 660
ttgatgttac ataaccttag aaagctactg ctgaatcttt tatatcaatg gattctattt 720
ttaaatactt ttcataataa tcattatttt atgacatgac tataatatta aatctgttag 780
gactagaaga atttttacct ttttcaagga aattgttagt agttcagcaa acagtttcta 840
ctctgtgaca taagcccagg aaagtgaagt ctcttgaaaa ctttttttct ctaaccttca 900
ttcttgatgg caagcaacta tgtgcttaga acgatggttt tcaactttgg ttgcacctta 960
actctgaaac ttaaaaaaaa gataccccct gagattctga tttaattggt gtggagtata 1020
atctgggcct tgataggggt cagagctctt caggtgattc taatgtgcat ccgtgattga 1080
gaattgctag ttaagaagct gtttaatgtc cttaaagaag aaactaattt ttctttctcg 1140
gagttgtatt catcttcaac agatattacw tagtcataag agaaaaatat aaaatcagga 1200
aaagcgtata tagagttatg aaagaggggt tatgaattat aaacagtttt atgattaagt 1260
ccaatcgttt aattgttatt gaaagatagt cttatatttt taagtcctat tttgctattt 1320
aacccttgtt tatacttttg ttcagtgctt tgctctcctg gtgtcacctt cataataata 1380
attcaacttt gatcaataaa ataaacaatc ttctggaaaa aaaaaaaaaa aaaactcgta 1440

<210> 70
<211> 1068
<212> DNA
<213> Homo sapiens

<400> 70
gcaggcatga gccaccgcac ccggccacaa gtgtctgaac atttataata tgagaattat 60
ccctgatttt ccaaggacgg aactgaaggc cctcccgact aagaaggaga cttaaagcgc 120
ctttttcagc gtggaagaca agactcgcgg gcgctaaagg aggcctgagt gtgggcgact 180
tccggaaggt gctgatgaag acaggcctgg tgctggtggt gctgggccat gtgagcttca 240

tcacagctgc cctgttccat ggcacagtgc tgcgctacgt gggcacccct caagatgcgg 300
tggctctgca gtactgcgtg gtcaacatcc tctctgtcac ttccgccatc gtggtcatca 360
cttcaggcat cgcagccatc gtgttgtcac gctacctccc tagcaccccc ctgcgctgga 420
cagtgtttag ctcgagcgtg gcctgtgctc tcctttctct gacctgtgcc ctcggcctct 480
tggcctccat cgccatgacc tttgccaccc agggcaaggc actgctggct gcctgcactt 540
ttgggagctc tgaactactg gccctcgcac ctgactgtcc cttcgacccc acacgcattt 600
atagctccag cctgtgcctc tggggcatcg ccctagtgct ctgcgtggcg gagaacgtgt 660
ttgctgtacg ctgtgctcag ctcacccacc agctgctgga gctgaggccc tggtggggga 720
aaagcagcca ccacatgatg cgggagaacc cagagctggt ggagggccgt gacctgctga 780
gctgcaccag ctctgagcct ctgaccctct gagagatgat gtcctgccca ggcccgatgg 840
ccactaggac cctgcaagca actctgctct gtgaccaggc caggattcct ggagctggcc 900
tgagagggct caatggaccc tcggggaccc aagtggggct ttcaaccctc tcccccacca 960
cccagcccac tgcactgaaa tgagacttta ttctgaaatt attaaaaaga acagagatgc 1020
tcaaaaaaaa aaaaaaaaaa aaaaaaaaaa aaaaaaaaaa aaaaaaaa 1068

<210> 71
<211> 1948
<212> DNA
<213> Homo sapiens

<400> 71
cgcgtccgga gctgcagaga agaggaggtt ggtgtggagc acaggcagca ccgagcctgc 60
cccgtgagct gagggcctgc agtctgcggc tggaatcagg atagacacca aggcaggacc 120
cccagagatg ctgaagcctc tttggaaagc agcagtggcc cccacatggc catgctccat 180
gccgccccgc cgcccgtggg acagagaggc tggcacgttg caggtcctgg gagcgctggc 240
tgtgctgtgg ctgggctccg tggctcttat ctgcctcctg tggcaagtgc cccgtcctcc 300
cacctggggc caggtgcagc ccaaggacgt gcccaggtcc tgggagcatg gcttccagcc 360
cagcttggga gcccctggaa gcagagggcc aggcagcaga gggactcctg ccagcttgtc 420
cttgtggaaa gcatccccca ggacctgcca tctgcagccg gcagcccctc tgcccagcct 480
ctgggccagg cctggctgca gctgctggac actgcccagg agagcgtcca cgtggcttca 540
tactactggt ccctcacagg gcctgacatc ggggtcaacg actcgtcttc ccagctggga 600
gaggctcttc tgcagaagct gcagcagctg ctgggcagga acatttccct ggctgtggcc 660
accagcagcc cgacactggc caggacatcc accgacctgc aggttctggc tgcccgaggt 720
gcccatgtac gacaggtgcc catggggcgg ctcaccatgg gtgttttgca ctccaaattc 780
tgggttgtgg atggacggca catatacatg ggcagtgcca acatggactg gcggtctctg 840
acgcaggtga aggagcttgg cgctgtcatc tataactgca gccacctggg ccaagacctg 900
gagaagacct tccagaccta ctgggtactg ggggtgccca aggctgtcct ccccaaaacc 960
tggcctcaga acttctcatc tcacttcaac cgtttccagc ccttccacgg cctctttgat 1020
ggggtgccca ccactgccta cttctcagcg tcgccaccag cactctgtcc ccagggccgc 1080
acccgggacc tggaggcgct gctggcggtg atggggagcg cccaggagtt catctatgcc 1140
tccgtgatgg agtatttccc caccacgcgc ttcagccacc ccccgaggta ctggccggtg 1200
ctggacaacg cgctgcgggc ggcagccttc ggcaagggcg tgcgcgtgcg cctgctggtc 1260
ggctgcggac tcaacacgga ccccaccatg ttcccctacc tgcggtccct gcaggcgctc 1320
agcaaccccg cggccaacgt ctctgtggac gtgaaagtct tcatcgtgcc ggtggggaac 1380
cattccaaca tcccattcag cagggtgaac cacagcaagt tcatggtcac ggagaaggca 1440
gcctacatag gcacctccaa ctggtcggag gattacttca gcagcacggc gggggtgggc 1500
ttggtggtca cccagagccc tggcgcgcag cccgcggggg ccacggtgca ggagcagctg 1560
cggcagctct ttgagcggga ctggagttcg cgctacgccg tcggcctgga cggacaggct 1620
ccgggccagg actgcgtttg gcagggctga ggggggcctc tttttctctc ggcgaccccg 1680
ccccgcacgc gccctcccct ctgaccccgg cctgggcttc agccgcttcc tcccgcaagc 1740
agcccgggtc cgcactgcgc caggagccgc ctgcgaccgc ccgggcgtcg caaaccgccc 1800
gcctgctctc tgatttccga gtccagcccc ccctgagccc cacctcctcc agggagccct 1860
ccaggaagcc ccttccctga ctcctggccc acaggccagg cctaaaaaaa actcgtggct 1920
tcaaaaaaaa aaaaaaaaaa aaaaaaaa 1948

<210> 72

<211> 1837
<212> DNA
<213> Homo sapiens

<400> 72
ccgggtcgac ccacgcgtcc gcccacgcgt ccgcagaatc aagagtaaaa gcaacccaga 60
caactcttta atagtctgat gctactgtgc atattaatat ttaaagtcca cttgttatta 120
ttttgcagat ccttttctgc attccttaat ctgaaagaga gatttttatt cttaatactt 180
gtatggattt ttgtggcttt ttatgggtgt aaatattctc ctctctcgtt tgacagtttc 240
aaaagcctag gttcataagc tctccatgaa taaatatgtt cttagtcatg tgatgtaaaa 300
agatcgctta caaagcttgt gaaacctgag ccttcctttt gaacctttta ctacccatga 360
gctcaggaac catacatgca aaattttatt cttgcgtcat gacttcagct tatgagggaa 420
atgagctatg aatttaaatg actcttctac tctataccaa gtttctatga aaataaaatt 480
gtattttttc ctttttccta aaaggaaagt ttcatctgac tagtgtttct gccggtattt 540
gttcccattg ttaaaagatt tgtttcttaa gattagcatt aaaatagaca tcctgttttt 600
gaaggcatct ttttttgttt atactgtaat cccaaaaatg tccaactggc tgaatggcca 660
agaaactccc ttgtaatttc ctaatagagc taaagttaac aagtcacctt aaagtctact 720
aattccaatt aagttcacct tggagaaatt ttcattagtc tagtcctttg gcacttaccc 780
aatacaccct taattaaagt tcttatgcat gggaccagtt gtatctatta taaagattat 840
cataattcta agttttctct cccaccccca tttttttttc agggtgtgtt tccatataaa 900
gatcgaaaaa gtccattttc ttttcatgta tcttcaagat ggaagatctt ttccttccct 960
tccttcctcc cttcttccct ccctcactcc ctccttccct ccctcactcc ctgcctccct 1020
cccttccttc ctttcttctt ccttccttcc ttttcagttt tatactactc agaagtttga 1080
ggaggagaga gaatacatta aaatgtattc agccccagtt caggcactat atagtgctag 1140
ctatgtgtta cttatttgga ttctcatgtg aacctggtga gatggactgg atcccacttt 1200
acaaacgagg aacgagaagc ttagataagt taaacctttt ccaaattttc acatctttaa 1260
atgatagagt caagttttga actaagatct gacttcagag ttcttgctca ctagattgcc 1320
tttcaggtag tatttggagg cctctgcacc tctcctacca ggatacttcc cccatcgcat 1380
tgtgtagctt ttctccattt catttctata gcactttgac atctagcaaa tgttattttc 1440
tcatcttcct cctcttccta cctcttgctg cttgtataaa tatcttgttc aggctgaact 1500
gagagaagta gtgtattcag aaaacttact atctcttttc ggctgggtgt ggtccctcac 1560
acctgtaatc ccagcacttt gggaggccta ggtgggcgga tcacttgagg tcaggagttc 1620
ggggccagcc tggccaacgg gatgaaactt tgtctctact aaaagtgcaa aaattaggtg 1680
gatgtggtgg ctgcacctgt tgtcccagct actcaggaag ctgaggtggg gagactcact 1740
tgaacctggg aggcggaggt tgcagtgggc cgggattgcg ccactgtact ccagcctggg 1800
tgagggagca agactctgtc tcaaaaaaaa aaaaaaa 1837

<210> 73
<211> 1161
<212> DNA
<213> Homo sapiens

<400> 73
   ggggaaacgg agctctgggt gtgatatttc ctctgcattt tcctgtcggg gtggtgaaat 60
   aactggtttg aacccagtcc actggactcg aaagctcatg ctcagaagcc ccagggctcc 120
   ctctaacttt cttggttgct gcaactcaga gagcgctgga atggacccag ggcatgctcc 180
   tcatctcagc ggttcaggtt ttcattcttc tatctccatc cttctattta attctgtact 240
   tactaagacc tgggggtaca gggaggggct tggagcctat ttgcccagct gctgaatggg 300
   gaggttggag agatggatac ttatggctcc agtaccagga gccaactgtt tcccttgaca 360
   actggggaaa ctgaggccca cagagccaag gccacttgcc cgtggttacc taaagatgtt 420
   aacgagaaat ccgggtctgg aactcagatc cctttgtatc ctgtttcggt gttggtgtag 480
   tttgttgctt tccctaagat gagcccagat agggaaactg aagtgcctgg gstcctggtt 540
   gggtcttctg cggggagaga atggcgattc aactcccgtg tactgttgaa cttgacacaa 600
   acacgctcac atcccaggct gcatacgtgt tttgctttag aaatgacatg aagccttttg 660
   actattttta agagaaaggc aatggctgtg atatttcccc tgcacctccc tctcggggcc 720
   acttggttaa atgtcaggaa agggagagta tttcctggtc aggaacattc agagcttgct 780
   gggagctgaa gttttgtttt ccattaagta ggtattcggg gagtctattt ccctctgcct 840
   cctctgtttc cctggaarct tgcgcttgac agttgcaggg aggaggggtt tgagaatgag 900
   cagccgagat gcccacgtat cgcgtgcccg ctctaggagt ggcggggtgg ctatttttag 960
   ccatcctgat tcagtagagg catttcagcg tttgttcaat atttaattat ccatctgaaa 1020
   ttggcccatg tggccttcag tttggaagca gctctctgtg ctgtgatttc ccagttgcat 1080
   aaataaggaa gtcaagggaa tctcaatagc cctccaaata ataataacga aaaaaaaaaa 1140
   aaaaaaactc gacggcacgt a 1161

<210> 74
<211> 1450
<212> DNA
<213> Homo sapiens

<400> 74
gggcacgagt caagattgtg aggtccaaga gaacagatca gggtcttaag aagattatct 60
ttcatagtgc ctatttgatg gtaatgatca taaatacagt ataatagaag gaaaaatatc 120
tggtggctta tatgcattgg tagtttctca tggtaataag catttttttt tctcttcctt 180
ttagcacaag tgcatacacc ttgatagcac caaatataaa ccggagaaat gagatacaaa 240
gaattgcgga caggagctgg ccaacctgga gaagtggaag gagcagaaca gagctaaacc 300
ggttcacctg gtgcccagac ggctaggtgg aagccagtca gaaactgaag tcagacagaa 360
acaacaactc cagctgatgc aatctaaata caagcaaaag ctaaaaagag aagaatctgt 420
aagaatcaag aaggaagctg aagaagctga actccaaaaa atgaaggcaa ttcagagaga 480
gaagagcaat aaactggagg agaaaaaaag acttcaagaa aaccttagaa gagaagcatt 540
tagagagcat cagcaataca aaaccgctga gttcttgagc aaactgaaca cagaatcgcc 600
agacagaagt gcctgtcaaa gtgctgtttg tggcccacaa tcctcaacat gggccagaag 660
ctgggcttac agagattctc taaaggcaga agaaaacaga aaattgcaaa agatgaagga 720
tgaacaacat caaaagagtg aattactgga actgaaacgg cagcagcaag agcaagaaag 780
agccaaaatc caccagactg aacacaggag ggtaaataat gcttttctgg accgactcca 840
aggcaaaagt caaccaggtg gcctcgagca atctggaggc tgttggaata tgaatagcgg 900
taacagctgg ggtatatgag aaaatattga ctcctatctg gccttcatca actgacctcg 960
aaaagcctca tgagatgctt tttcttaatg tgattttgtt cagcctcact gtttttacct 1020
taatttcaac tgcccacaca cttgaccgtg cagtcaggag tgactggctt ctccttgtcc 1080
tcatttatgc atgtttggag gagctgattc ctgaactcat atttaaactc tactgccagg 1140
gaaatgctac attatttttc taattggaag tataattaga gtgatgttgg tagggtagaa 1200
aaagagggag tcacttgatg ctttcaggtt aatcagagct atgggtgcta caggcttgtc 1260
tttctaagtg acatattctt atctaattct cagatcaggt tttgaaagct ttgggggtct 1320
ttttagattt taatccctac tttctttatg gtacaaatat gtacaaaaga aaaaggtctt 1380
atattctttt acacaaattt ataaataaat tttgaactcc ttctgtataa aaaaaaaaaa 1440
aaaaaaaaaa 1450

<210> 75
<211> 557
<212> DNA
<213> Homo sapiens

<220>
<221> SITE
<222> (136)
<223> n equals a,t,g, or c

<400> 75
   gcttttttcg ggggaatgtt tacagaggct gtgggtcaga atgaagcaac accagaagct 60
   atggagactg gggtttctgc tgtgtttcaa cttggttttt tgtgttctcg ggagaagaca 120
   cccttggccg tgggcngtga gacctttgat gtgtgtttac gctgaccgcg agttgttggg 180
   atggcttctg cggtgggtgg ttctcttggt attctcggtt ttgaagctta tttttagact 240
   ctgaactctc cttcttggca ggagttgaat ccccctgggg gttttcaagt tgttcttgga 300
   ctgctggttt ttgaaataga agcccctttg gtggggtccc ccataaaccc aggcgctggt 360
   gcccaccttg tgatgtgaag gctcctgtaa cacgacctca ctttcctggc cccgcactac 420
   tcacctgccc cacgggacac aggtacatgg cttctgggtg tctgtccccg ctgtacccag 480
   atctgccccc ttgcccttgt ccccagatcc tccactcgct cctaggaacc gtacccctcc 540
   caaaacaaaa aaaaaaa 557

<210> 76
<211> 2483
<212> DNA
<213> Homo sapiens

<400> 76
   cggcacgagc tcgtgccgct cgtgccggga ctggttaata gtgaagtcca taatgaagat 60
   ggaagaaatg gagatgtctc tcagtttcca tatgtggaat ttacaggaag agatagtgtc 120
   acctgcccta cttgtcaggg aacaggaaga attcctaggg ggcaagaaaa ccaactggtg 180
   gcattgattc catatagtga tcagagatta aggccaagaa gaacaaagct gtatgtgatg 240
   gcttctgtgt ttgtctgtct actcctttct ggattggctg tgtttttcct tttccctcgc 300
   tctatcgacg tgaaatacat tggtgtaaaa tcagcctatg tcagttatga tgttcagaag 360
   cgtacaattt atttaaatat cacaaacaca ctaaatataa caaacaataa ctattactct 420
   gtcgaagttg aaaacatcac tgcccaagtt caattttcaa aaacagttat tggaaaggca 480
   cgcttaaaca acataagcat tattggtcca cttgatatga aacaaattga ttacacagta 540
   cctaccgtta tagcagagga aatgagttat atgtatgatt tctgtactct gatatccatc 600
   aaagtgcata acatagtact catgatgcaa gttactgtga caacaacata ctttggccac 660
   tctgaacaga tatcccagga gaggtatcag tatgtcgact gtggaagaaa cacaacttat 720
   cagttggggc agtctgaata tttaaatgta cttcagccac aacagtaaaa actggaagag 780
   atggatttaa agaagaaata tctattgata tttcctatac tctcaatgaa gaggtatttc 840
   ctaataggag accttaaatt gaacaaacct aaagtttaca cttctaagag tacagttaaa 900
   agtatgtgga cctgcagttc ttgtaactct ccactctgtg ttaatgatat atttgtacta 960
   ggatctttta cttgaatcta aatttactgg ttgatttcct tctccagcct atcccctaca 1020
   gggaaaagct gatacttccc ctatagtaca ataaataatt atttaaaagt catagctcca 1080
   gtcactactg aaaacataat tttggtgata aacataattt gagaaactta atttctgaat 1140
   gtttttatag aaaattactg aaaatctatt actcatggaa gacttttaaa gagtaacctt 1200
   ttttcctgtt ttataaattc ccattgttat atggtagtat ttcagctaca caatatttta 1260
   gcttttagct agacatttat aggttttcat ttgttgaaat ggtaatcatc tgcatgtttt 1320
   tgtcacttat ttcaggttag tgattgccta acacttataa gccaaaataa tctttgcaaa 1380
   attccatacc taaaattttg aaagccccta atgttttcac acatctttct gtattagtta 1440
   tagttttgtg aaatctttgt gtgatcttca aacattatca tttaatgtac aatactgtaa 1500
   ataaactgtg catggctttt atacagcttt agtaaatgtc aaataaagtg gtacagactc 1560
   attacaacaa gtttctcata aaaatacaat aaataggaaa atgaaattca gaaacccata 1620
   gactgggaat aggttccagt tacagcttgg atctggcata aaataaattt gaaataaaat 1680
   attttgatgc tccatttttt tatgttgctt ttcatactaa agaatggtgt agacttgttt 1740
   gcaactgtag gtacccagtt atcaatttta tcaatgttta gagagaaatt atttttttgg 1800
   tagaaatgtc aagaaatcct taattgaatg tcattaaatg atggtggcca aaataaaacc 1860
   tatttagaaa tttaatcact ttgcacatca cttggaatat gatgcctcta gtagttactt 1920
   ttttatagtt ttctactttt ggttttattt aaaattgttt tcaaatatag attattgact 1980
   tattcaactt tgctgtttta tattttcagt atcatttttc atttgttttt ttttttttgt 2040
   cttttcactt accaagttct agggacattt aaaatatgta ctaagtgtag gagtggttat 2100
   gataccaaaa aatgtagctg ggttgagatt aatttcgttc tgttttctca tgacagaaat 2160
   caggtttccc tttccccacc cctaagtgcc taacttaggt ctgaaacagc ctgtttatta 2220
   gtctgactct ctcaaccata aaacataagc tttatttaat tctgccttta aacacactca 2280
   ggtttcccct taattttcat attattttct gcaggttttc ttgagtatct tcaattcgtt 2340
   gaatgtggtt tttggttttt ttttgtttta acactagtct tcccttaatt cattgctaac 2400
   tcaagccatc cttactatta aacccaaatc agtcctttaa gttcattatg gcctttctag 2460
   tatttaaaaa aaaaaaaaaa aaa 2483

<210> 77
<211> 667

<212> DNA
<213> Homo sapiens

<400> 77
ggcacgagca ctgcagctcc ctgagcactc tctacagaga cgcggacccc agacatgagg 60
aggctcctcc tggtcaccag cctggtggtt gtgctgctgt gggaggcagg tgcagtccca 120
gcacccaagg tccctatcaa gatgcaagtc aaacactggc cctcagagca ggacccagag 180
aaggcctggg gcgcccgtgt ggtggagcct ccggagaagg acgaccagct ggtggtgctg 240
ttccctgtcc agaagccgaa actcttgacc accgaggaga agccacgagg caccaaggcc 300
tggatggaga ccgaggacac cctgggccgt gtcctgagtc ccgagcccga ccatgacagc 360
ctgtaccacc ctccgcctga agaggaccag ggcgaggaga ggccccggtt gtaggtgatg 420
ccaaatcacc aggtgctcct gggaccggag gaagaccaag acacatctac cacccccagt 480
aggggctcca ggggccatca atgcccccgc cctgtcccaa ggcccaggct gttgggactg 540
ggaccctccc taccctgccc cagctagaca aataaacccc agcaggccgg aaaaaaaaaa 600
aaaaaaaaaa aaaaaaaaaa aaaaaaaaaa aaaaaaaaaa aaaaaaaaaa aaaaaaaaaa 660
aaaaaaa 667

<210> 78
<211> 1931
<212> DNA
<213> Homo sapiens

<220>
<221> SITE
<222> (1212)
<223> n equals a,t,g, or c

<400> 78
   cccgcagcag ctcccaggat gaactggttg cagtggctgc tgctgctgcg ggggcgctga 60
   gaggacacga gctctatgcc tttccggctg ctcatcccgc tcggcctcct gtgcgcgctg 120
   ctgcctcagc accatggtgc gccaggtccc gacggctccg cgccagatcc cgcccactac 180
   agggagcgag tcaaggccat gttctaccac gcctacgaca gctacctgga gaatgccttt 240
   cccttcgatg agctgcgacc tctcacctgt gacgggcacg acacctgggg cagtttttct 300
   ctgactctaa ttgatgcact ggacaccttg ctgattttgg ggaatgtctc agaattccaa 360
   agagtggttg aagtgctcca ggacagcgtg gactttgata ttgatgtgaa cgcctctgtg 420
   tttgaaacaa acattcgagt ggtaggagga ctcctgtctg ctcatctgct ctccaagaag 480
   gctggggtgg aagtagaggc tggatggccc tgttccgggc ctctcctgag aatggctgag 540
   gaggcggccc gaaaactcct cccagccttt cagaccccca ctggcatgcc atatggaaca 600
   gtgaacttac ttcatggcgt gaacccagga gagacccctg tcacctgtac ggcagggatt 660
   gggaccttca ttgttgaatt tgccaccctg agcagcctca ctggtgaccc ggtgttcgaa 720
   gatgtggcca gagtggcttt gatgcgcctc tgggagagcc ggtcagatat cgggctggtc 780
   ggcaaccaca ttgatgtgct cactggcaag gggtggccca ggacgcaggc atcggggctg 840
   gcgtggactc ctactttgag tacttggtga aaggagccat cctgcttyag gataagaagc 900
   tcatggccat gttcctagag tataacaaag ccatccggaa ctacacccgc ttcgatgact 960
   ggtacctgtg ggttcagatg tacaagggga ctgtgtccat gccagtcttc cagtccttgg 1020
   aggcctactg gcctggtctt cagagcctca ttggagacat tgacaatgcc atgaggacct 1080
   tcctcaacta ctacactgta tggaagcagt ttggggggct cccggaattc tacaacattc 1140
   ctcagggata cacagtggag aagcgagagg gctacccact tcggccagaa cttattgaaa 1200
   gcgcaatgta cntctaccgt gccacggggg atcccaccct cctagaactc ggaagagatg 1260
   ctgtggaatc cattgaaaaa atcagcaagg tggagtgcgg atttgcaaca atcaaagatc 1320
   tgcgagacca caagctggac aaccgcatgg agtcgttctt cctggccgag actgtgaaat 1380
   acctctacct cctgtttgac ccaaccaact tcatccacaa caatgggtcc accttcgacg 1440
   cggtgatcac cccctatggg gagtgcatcc tgggggctgg ggggtacatc ttcaacacag 1500
   aagctcaccc catcgaccct gccgccctgc actgctgcca gaggctgaag gaagagcagt 1560
   gggaggtgga ggacttgatg agggaattct actctctcaa acggagcagg tcgaaatttc 1620
   agaaaaacac tgttagttcg gggccatggg aacctccagc aaggccagga acactcttct 1680
   caccagaaaa ccatgaccag gcaagggaga ggaagcctgc caaacagaag gtcccacttc 1740
   tcagctgccc cagtcagccc ttcacctcca agttggcatt actgggacag gttttcctag 1800
   actcctcata accactggat aattttttta tttttatttt tttgaggcta aactataata 1860
   aattgctttt ggctatcaaa aaaaaaaaaa aaaaaaaaaa aaaaaaaaaa aaaaaaaaaa 1920
   agggcggccg c 1931

<210> 79
<211> 54
<212> PRT
<213> Homo sapiens

<220>
<221> SITE
<222> (54)
<223> Xaa equals stop translation

<400> 79
Met Ala Gly Gln His Leu Ala Cys Leu Ala Ser Cys Val Met Ser Leu
1               5                   10                  15

Ile Trp Phe Phe Phe Phe Cys Ser Cys Phe Ile Cys Ser Ala Pro Ala
            20                  25                  30

Pro Pro Gln Gln Leu Val Ala Tyr Gly Phe Phe Lys Arg Lys Val Asp
        35                  40                  45

Phe Met Leu Tyr Ile Xaa
    50

<210> 80
<211> 578
<212> PRT
<213> Homo sapiens

<220>
<221> SITE
<222> (326)
<223> Xaa equals any of the naturally occurring L-amino acids

<220>
<221> SITE
<222> (342)
<223> Xaa equals any of the naturally occurring L-amino acids

<220>
<221> SITE
<222> (444)
<223> Xaa equals any of the naturally occurring L-amino acids

<400> 80
Met Pro Phe Arg Leu Leu Ile Pro Leu Gly Leu Leu Cys Ala Leu Leu
1               5                   10                  15

Pro Gln His His Gly Ala Pro Gly Pro Asp Gly Ser Ala Pro Asp Pro
            20                  25                  30

Ala His Tyr Arg Glu Arg Val Lys Ala Met Phe Tyr His Ala Tyr Asp

35                  40                  45

Ser Tyr Leu Glu Asn Ala Phe Pro Phe Asp Glu Leu Arg Pro Leu Thr
    50                  55                  60

Cys Asp Gly His Asp Thr Trp Gly Ser Phe Ser Leu Thr Leu Ile Asp
65                  70                  75                  80

Ala Leu Asp Thr Leu Leu Ile Leu Gly Asn Val Ser Glu Phe Gln Arg
                85                  90                  95

Val Val Glu Val Leu Gln Asp Ser Val Asp Phe Asp Ile Asp Val Asn
            100                 105                 110

Ala Ser Val Phe Glu Thr Asn Ile Arg Val Val Gly Gly Leu Leu Ser
        115                 120                 125

Ala His Leu Leu Ser Lys Lys Ala Gly Val Glu Val Glu Ala Gly Trp
    130                 135                 140

Pro Cys Ser Gly Pro Leu Leu Arg Met Ala Glu Glu Ala Ala Arg Lys
145                 150                 155                 160

Leu Leu Pro Ala Phe Gln Thr Pro Thr Gly Met Pro Tyr Gly Thr Val
                165                 170                 175

Asn Leu Leu His Gly Val Asn Pro Gly Glu Thr Pro Val Thr Cys Thr
            180                 185                 190

Ala Gly Ile Gly Thr Phe Ile Val Glu Phe Ala Thr Leu Ser Ser Leu
        195                 200                 205

Thr Gly Asp Pro Val Phe Glu Asp Val Ala Arg Val Ala Leu Met Arg
    210                 215                 220

Leu Trp Glu Ser Arg Ser Asp Ile Gly Leu Val Gly Asn His Ile Asp
225                 230                 235                 240

Val Leu Thr Gly Lys Trp Val Ala Gln Asp Ala Gly Ile Gly Ala Gly
                245                 250                 255

Val Asp Ser Tyr Phe Glu Tyr Leu Val Lys Gly Ala Ile Leu Leu Gln
            260                 265                 270

Asp Lys Lys Leu Met Ala Met Phe Leu Glu Tyr Asn Lys Ala Ile Arg
        275                 280                 285

Asn Tyr Thr Arg Phe Asp Asp Trp Tyr Leu Trp Val Gln Met Tyr Lys
    290                 295                 300

Gly Thr Val Ser Met Pro Val Phe Gln Ser Leu Glu Ala Tyr Trp Pro
305                 310                 315                 320

Gly Leu Gln Ser Leu Xaa Gly Asp Ile Asp Asn Ala Met Arg Thr Phe
                325                 330                 335

Leu Asn Tyr Tyr Thr Xaa Trp Lys Gln Phe Gly Gly Leu Pro Glu Phe
            340                 345                 350

Tyr Asn Ile Pro Gln Gly Tyr Thr Val Glu Lys Arg Glu Gly Tyr Pro
        355                 360                 365

Leu Arg Pro Glu Leu Ile Glu Ser Ala Met Tyr Leu Tyr Arg Ala Thr
    370                 375                 380

Gly Asp Pro Thr Leu Leu Glu Leu Gly Arg Asp Ala Val Glu Ser Ile
385                 390                 395                 400

Glu Lys Ile Ser Lys Val Glu Cys Gly Phe Ala Thr Ile Lys Asp Leu
                405                 410                 415

Arg Asp His Lys Leu Asp Asn Arg Met Glu Ser Phe Phe Leu Ala Glu
            420                 425                 430

Thr Val Lys Tyr Leu Tyr Leu Leu Phe Asp Pro Xaa Asn Phe Ile His
        435                 440                 445

Asn Asn Gly Ser Thr Phe Asp Ala Val Ile Thr Pro Tyr Gly Glu Cys
    450                 455                 460

Ile Leu Gly Ala Gly Gly Tyr Ile Phe Asn Thr Glu Ala His Pro Ile
465                 470                 475                 480

Asp Pro Ala Ala Leu His Cys Cys Gln Arg Leu Lys Glu Glu Gln Trp
                485                 490                 495

Glu Val Glu Asp Leu Met Arg Glu Phe Tyr Ser Leu Lys Arg Ser Arg
            500                 505                 510

Ser Lys Phe Gln Lys Asn Thr Val Ser Ser Gly Pro Trp Glu Pro Pro
        515                 520                 525

Ala Arg Pro Gly Thr Leu Phe Ser Pro Glu Asn His Asp Gln Ala Arg
    530                 535                 540

Glu Arg Lys Pro Ala Lys Gln Lys Val Pro Leu Leu Ser Cys Pro Ser
545                 550                 555                 560

Gln Pro Phe Thr Ser Lys Leu Ala Leu Leu Gly Gln Val Phe Leu Asp
                565                 570                 575

Ser Ser


<210> 81
<211> 100
<212> PRT
<213> Homo sapiens

<220>
<221> SITE
<222> (100)
<223> Xaa equals stop translation

<400> 81

Met Ala Leu Tyr Tyr Gln Asn Phe Tyr Ile Leu Val Val Phe Val Leu
1               5                   10                  15

Phe Leu His Thr Ser Arg Thr Phe Val Leu Pro Val His Ala Val Lys
            20                  25                  30

Asp Ser Ala Gln Val Leu Glu Glu Ile Val Lys His Glu Leu Gly Ser
        35                  40                  45

Gln Val Ser Leu Leu Ser Pro Val Glu Glu Pro Gly Pro Ser Pro Cys
    50                  55                  60

Thr Pro Asp Ile Gln Gly Arg Gly Val Arg Lys Thr Leu Pro Pro Asn
65                  70                  75                  80

Gly Leu Asp Gly Met Phe Pro Ser Ser Cys Ser Pro Asn Val Ser Thr
                85                  90                  95

Gly Ala His Xaa
            100


<210> 82
<211> 48
<212> PRT
<213> Homo sapiens

<220>
<221> SITE
<222> (48)
<223> Xaa equals stop translation

<400> 82
Met Gly Glu Phe Thr Ser Val Val Cys Tyr Cys Phe Ile Leu Ser Leu
1               5                   10                  15

Ile Ile Gly Ser Val Val Arg Trp Gln Gly Cys Gly Ala Glu Trp Gly
            20                  25                  30

Phe Ala Leu Gly Glu His Met Trp Gln Arg Ala Gln Glu Asp Leu Xaa
        35                  40                  45


<210> 83
<211> 47
<212> PRT
<213> Homo sapiens

<220>
<221> SITE
<222> (47)
<223> Xaa equals stop translation

<400> 83
Met Asn Ala Thr Thr Ser Phe Gln Phe Thr Thr Pro Thr Arg Leu Trp

1                   5                   10                  15

Leu Met Leu Leu Leu Asn Tyr Gln Ile Phe Cys Cys Tyr Thr Val Thr
                20                  25                  30

Phe Lys Glu Phe Gly Lys Leu Val Ser Thr Ala Asn Leu Gly Xaa
            35                  40                  45


<210> 84
<211> 276
<212> PRT
<213> Homo sapiens

<220>
<221> SITE
<222> (276)
<223> Xaa equals stop translation

<400> 84
Met Gly Asn Phe Arg Gly His Ala Leu Pro Gly Thr Phe Phe Phe Ile
  1               5                  10                  15

Ile Gly Leu Trp Trp Cys Thr Lys Ser Ile Leu Lys Tyr Ile Cys Lys
                20                  25                  30

Lys Gln Lys Arg Thr Cys Tyr Leu Gly Ser Lys Thr Leu Phe Tyr Arg
            35                  40                  45

Leu Glu Ile Leu Glu Gly Ile Thr Ile Val Gly Met Ala Leu Thr Gly
     50                  55                  60

Met Ala Gly Glu Gln Phe Ile Pro Gly Gly Pro His Leu Met Leu Tyr
 65                  70                  75                  80

Asp Tyr Lys Gln Gly His Trp Asn Gln Leu Leu Gly Trp His His Phe
                 85                  90                  95

Thr Met Tyr Phe Phe Phe Gly Leu Leu Gly Val Ala Asp Ile Leu Cys
            100                 105                 110

Phe Thr Ile Ser Ser Leu Pro Val Ser Leu Thr Lys Leu Met Leu Ser
        115                 120                 125

Asn Ala Leu Phe Val Glu Ala Phe Ile Phe Tyr Asn His Thr His Gly
    130                 135                 140

Arg Glu Met Leu Asp Ile Phe Val His Gln Leu Leu Val Leu Val Val
145                 150                 155                 160

Phe Leu Thr Gly Leu Val Ala Phe Leu Glu Phe Leu Val Arg Asn Asn
                165                 170                 175

Val Leu Leu Glu Leu Leu Arg Ser Ser Leu Ile Leu Leu Gln Gly Ser
            180                 185                 190

Trp Phe Phe Gln Ile Gly Phe Val Leu Tyr Pro Pro Ser Gly Gly Pro
        195                 200                 205

Ala Trp Asp Leu Met Asp His Glu Asn Ile Leu Phe Leu Thr Ile Cys
    210                 215                 220

Phe Cys Trp His Tyr Ala Val Thr Ile Val Ile Val Gly Met Asn Tyr
225                 230                 235                 240

Ala Phe Ile Thr Trp Leu Val Lys Ser Arg Leu Lys Arg Leu Cys Ser
                245                 250                 255

Ser Glu Val Gly Leu Leu Lys Asn Ala Glu Arg Glu Gln Glu Ser Glu
            260                 265                 270

Glu Glu Met Xaa
        275


<210> 85
<211> 86
<212> PRT
<213> Homo sapiens

<220>
<221> SITE
<222> (86)
<223> Xaa equals stop translation

<400> 85
Met Ala Ser Lys Thr Leu Tyr Asp Leu Ala Leu Ala Tyr Leu Ser Ala
  1               5                  10                  15

Leu Ala Leu Pro Thr Leu Ala Gln Ser Leu Leu Phe Ser His Ser Gly
             20                  25                  30

Ser Leu Thr Ile Pro Arg Cys Thr Arg Leu Ser His Thr Ser Ala Pro
         35                  40                  45

Leu His Val Leu Phe Ala Val Arg Gly Met Pro Phe Thr Val Thr Thr
     50                  55                  60

Leu Leu Ile His Ser Thr Asn Ala Ser Ser Phe Phe Tyr Thr Gln Leu
 65                  70                  75                  80

Ser Leu Lys Phe Phe Xaa
                 85


<210> 86
<211> 95
<212> PRT
<213> Homo sapiens

<220>
<221> SITE
<222> (95)
<223> Xaa equals stop translation

<400> 86

Met Ala Ile Leu His Leu Phe Lys Phe Phe Ser Phe Phe Asn Phe Val
1               5                   10                  15

Ile Ser Ala Ser Pro Ile Tyr Leu Leu Tyr His Tyr Leu Arg Ser Asp
            20                  25                  30

Lys Arg Val Leu Val Gly Gln Val Leu Gln Ser Leu Ser Gly Asn Asn
        35                  40                  45

Ile Cys His Ile Thr Leu Leu Ile Cys Leu Leu Leu Ile Trp Glu Ala
    50                  55                  60

Lys His Trp Cys Leu Arg Gly Leu Pro Ile Ile Asn Cys His Tyr His
65                  70                  75                  80

Tyr Ser Pro Leu Leu Phe Val Trp Lys Leu Asn Lys Gly Gln Xaa
                85                  90                  95

<210> 87
<211> 313
<212> PRT
<213> Homo sapiens

<220>
<221> SITE
<222> (313)
<223> Xaa equals stop translation

<400> 87
Met Pro Pro Pro Arg Val Phe Lys Ser Phe Leu Ser Leu Leu Phe Gln
1               5                   10                  15

Gly Leu Ser Val Leu Leu Ser Leu Ala Gly Asp Val Leu Val Ser Met
            20                  25                  30

Tyr Arg Glu Val Cys Ser Ile Arg Phe Leu Phe Thr Ala Val Ser Leu
        35                  40                  45

Leu Ser Leu Phe Leu Ser Ala Phe Trp Leu Gly Leu Leu Tyr Leu Val
    50                  55                  60

Ser Pro Leu Glu Asn Glu Pro Lys Glu Met Leu Thr Leu Ser Glu Tyr
65                  70                  75                  80

His Glu Arg Val Arg Ser Gln Gly Gln Gln Leu Gln Gln Leu Gln Ala
                85                  90                  95

Glu Leu Asp Lys Leu His Lys Glu Val Ser Thr Val Arg Ala Ala Asn
            100                 105                 110

Ser Glu Arg Val Ala Lys Leu Val Phe Gln Arg Leu Asn Glu Asp Phe
        115                 120                 125

Val Arg Lys Pro Asp Tyr Ala Leu Ser Ser Val Gly Ala Ser Ile Asp
    130                 135                 140

Leu Gln Lys Thr Ser His Asp Tyr Ala Asp Arg Asn Thr Ala Tyr Phe

145                 150                 155                 160

Trp Asn Arg Phe Ser Phe Trp Asn Tyr Ala Arg Pro Pro Thr Val Ile
                165                 170                 175

Leu Glu Pro His Val Phe Pro Gly Asn Cys Trp Ala Phe Glu Gly Asp
            180                 185                 190

Gln Gly Gln Val Val Ile Gln Leu Pro Gly Arg Val Gln Leu Ser Asp
        195                 200                 205

Ile Thr Leu Gln His Pro Pro Pro Ser Val Glu His Thr Gly Gly Ala
    210                 215                 220

Asn Ser Ala Pro Arg Asp Phe Ala Val Phe Gly Leu Gln Val Tyr Asp
225                 230                 235                 240

Glu Thr Glu Val Ser Leu Gly Lys Phe Thr Phe Asp Val Glu Lys Ser
                245                 250                 255

Glu Ile Gln Thr Phe His Leu Gln Asn Asp Pro Pro Ala Ala Phe Pro
            260                 265                 270

Lys Val Lys Ile Gln Ile Leu Ser Asn Trp Gly His Pro Arg Phe Thr
        275                 280                 285

Cys Leu Tyr Arg Val Arg Ala His Gly Val Arg Thr Ser Glu Gly Ala
    290                 295                 300

Glu Gly Ser Ala Gln Gly Pro His Xaa
305                 310


<210> 88
<211> 80
<212> PRT
<213> Homo sapiens

<220>
<221> SITE
<222> (80)
<223> Xaa equals stop translation

<400> 88
Met Met Ser Ser Cys Leu Val Val Val Ile Thr Leu Arg Ala Tyr Phe
  1               5                  10                  15

Ser Trp Leu Gln Ala Ile Arg Ser Gln Val Val Trp Ser Arg Met Lys
             20                  25                  30

Arg Leu Gln Ser Ala Ser Arg Gln Ser Gly Leu Ser Ile Pro Arg Ser
         35                  40                  45

Glu Met Ser Ala Leu His Arg Leu Gln Asp Trp Ser Asp Lys Ser His
     50                  55                  60

Ile Leu Phe Phe Ile Phe Leu Pro Arg Val Cys Arg Phe Pro Leu Xaa
 65                  70                  75                  80

<210> 89
<211> 47
<212> PRT
<213> Homo sapiens

<220>
<221> SITE
<222> (47)
<223> Xaa equals stop translation

<400> 89
Met Leu Phe Leu Thr Cys Arg Ser Pro His Ser Cys Cys Val Ile Thr
  1               5                  10                  15

Trp Phe Phe Leu Cys Ala Cys Ala Leu Val Ser Ser Ser Tyr Gln Asp
             20                  25                  30

Asn Asn Pro Ile Gly Phe Arg Pro Glu Pro Tyr Asn Pro Ile Xaa
         35                  40                  45


<210> 90
<211> 129
<212> PRT
<213> Homo sapiens

<220>
<221> SITE
<222> (106)
<223> Xaa equals any of the naturally occurring L-amino acids

<220>
<221> SITE
<222> (129)
<223> Xaa equals stop translation

<400> 90
Met Gly Ala Ala Gly Arg Gln Asp Phe Leu Phe Lys Ala Met Leu Thr
  1               5                  10                  15

Ile Ser Trp Leu Thr Leu Thr Cys Phe Pro Gly Ala Thr Ser Thr Val
             20                  25                  30

Ala Ala Gly Cys Pro Asp Gln Ser Pro Glu Leu Gln Pro Trp Asn Pro
         35                  40                  45

Gly His Asp Gln Asp His His Val His Ile Gly Gln Gly Lys Thr Leu
     50                  55                  60

Leu Leu Thr Ser Ser Ala Thr Val Tyr Ser Ile His Ile Ser Glu Gly
 65                  70                  75                  80

Gly Lys Leu Val Ile Lys Asp His Asp Glu Pro Ile Val Leu Arg Thr

85                  90                  95

Arg His Ile Leu Ile Asp Asn Gly Gly Xaa Leu His Ala Gly Glu Cys
            100                 105                 110

Pro Leu Pro Phe Pro Gly Gln Phe His His His Phe Val Trp Lys Gly
        115                 120                 125

Xaa

<210> 91
<211> 71
<212> PRT
<213> Homo sapiens

<220>
<221> SITE
<222> (71)
<223> Xaa equals stop translation

<400> 91
Met Ala Phe Cys Phe Phe Ile Phe Tyr Leu Tyr Ser Phe Pro Ser Ile
  1               5                  10                  15

Ser His Gly Asp Leu His Lys Phe Gly Val Phe Ser Trp Cys Thr His
             20                  25                  30

Val Arg Arg Phe Lys Val Leu Tyr Ala Ser Val Leu Leu Lys Ser Thr
         35                  40                  45

Glu Ile Leu Leu Ala Ile Gln Glu Pro Phe Ser Gly Ser Trp Ser Tyr
     50                  55                  60

Phe Leu Leu Asn Leu Ser Xaa
 65                  70

<210> 92
<211> 48
<212> PRT
<213> Homo sapiens

<220>
<221> SITE
<222> (48)
<223> Xaa equals stop translation

<400> 92
Met Gln Trp Ala Val Lys Cys Trp Leu Phe Gln Leu Cys Met Asp Ser
  1               5                  10                  15

Ser Leu Ala Ser Leu Gly Trp Ala Glu Lys Arg Glu Leu Leu Phe Pro
             20                  25                  30

Lys Arg Pro Ser Gln Leu Cys Ser Thr Thr Leu Cys Ser Pro Gly Xaa
         35                  40                  45

<210> 93
<211> 57
<212> PRT
<213> Homo sapiens

<220>
<221> SITE
<222> (57)
<223> Xaa equals stop translation

<400> 93
Met Asn Trp Cys Leu Cys Ile Ile Ser Leu Thr Thr Leu Leu Ser Ile
 1               5                  10                  15

Pro Val His Ile Val Gly Glu Glu Lys Asp Met Leu Lys Cys Thr Phe
            20                  25                  30

Cys Leu Leu Asn Thr Leu Lys Lys Cys Val Val Trp Lys Arg Leu Tyr
        35                  40                  45

His Asn Gly Gly Ala Asn Asn Leu Xaa
    50                  55


<210> 94
<211> 73
<212> PRT
<213> Homo sapiens

<220>
<221> SITE
<222> (73)
<223> Xaa equals stop translation

<400> 94
Met Ala Gly Arg Lys Pro Ala Ala Pro Val Phe Thr Val Val Arg Lys
 1               5                  10                  15

Val Leu Cys Phe Gly Phe Gly Val Phe Val Leu Phe Val Phe Cys Leu
            20                  25                  30

Ala Cys Leu Phe Phe Lys Gly Lys Lys Val Cys Asn Tyr Phe Ile Gln
        35                  40                  45

Ile Ser Arg Tyr Ile Ser Val Asn Asn Lys Arg Phe Tyr Asn Ser Lys
    50                  55                  60

Lys Met Met Tyr Ile Leu Val Cys Xaa
65                  70


<210> 95
<211> 60

<212> PRT
<213> Homo sapiens

<220>
<221> SITE
<222> (60)
<223> Xaa equals stop translation

<400> 95
Met Leu Pro Tyr Phe Lys Trp Leu Leu His Leu Val Arg Leu Ser Phe
1               5                   10                  15

Val Ser Leu Ala Ser Pro Trp Asp Ser Thr Ala Gly Leu Gly Leu Lys
            20                  25                  30

Leu Pro Asn Ile Tyr Gly Met Thr Ser Met Gly Trp Asp Pro Ser Pro
        35                  40                  45

Gly Ala Arg Gly Gly Val Gly Thr Glu Lys Arg Xaa
    50                  55                  60


<210> 96
<211> 49
<212> PRT
<213> Homo sapiens

<220>
<221> SITE
<222> (49)
<223> Xaa equals stop translation

<400> 96
Met Trp Leu Gln Thr Leu Pro Leu Phe Ala Thr Gly Cys Lys Ala Val
1               5                   10                  15

Pro Trp Asn Cys Phe Gly Trp Cys Leu Thr Gln Glu Val Phe Ala Val
            20                  25                  30

Leu Gly Asp Leu Val Asn Ser Ala Asp Gln Val Asn Arg Leu Phe Phe
        35                  40                  45

Xaa


<210> 97
<211> 57
<212> PRT
<213> Homo sapiens

<220>
<221> SITE
<222> (57)
<223> Xaa equals stop translation

<400> 97
Met Arg Ser Ser Phe Leu Tyr Ala Ile Pro Ala Val Phe Phe Phe Leu

1                   5                   10                  15

Thr Gly Pro Cys Leu Arg Ile Asn Lys Ser Val Met Ser Glu Thr Lys
            20                  25                  30

Val Tyr Ser Ser Val Cys Arg Cys Val Ala Pro Pro Phe Ser Pro Ala
        35                  40                  45

Ala Pro His Ile Gln Ser Arg Ser Xaa
    50                  55

<210> 98
<211> 70
<212> PRT
<213> Homo sapiens

<220>
<221> SITE
<222> (70)
<223> Xaa equals stop translation

<400> 98
Met Ala Cys Arg Ser Trp Cys Phe Thr Leu Leu Ala Asn Val Ser Phe
 1               5                  10                  15

Thr Leu Leu Leu Pro Val His Trp Gly Ser Ala Glu Ala Val Phe Ser
            20                  25                  30

Val Ser Ile Thr Leu Gly Cys Arg Pro Pro Ser Ser Leu Ser Val Pro
        35                  40                  45

Leu Ser Arg Gly Arg Arg Asp Leu Gly Ser His Val Leu Ala Leu Val
    50                  55                  60

Ala Ser Leu Trp Lys Xaa
65                  70

<210> 99
<211> 83
<212> PRT
<213> Homo sapiens

<220>
<221> SITE
<222> (83)
<223> Xaa equals stop translation

<400> 99
Met Ala Glu Thr Arg Gly Leu Cys Ser Val Cys Phe Cys Ala Leu Cys
 1               5                  10                  15

Leu Tyr Gly Ser Tyr Ala Ala Cys Pro Pro Cys Phe Ser Arg Glu Pro
            20                  25                  30

Arg Gln Arg Arg His His Gly Asn Asp Trp Val Arg Trp Lys Phe Arg
        35                  40                  45

Gly Pro Ala Leu Val Gly Arg Glu Ala Trp Leu Thr Ser Gln Ala Gln
50                  55                  60

His Val Cys Gly Ser Leu Leu Cys Thr Val Ser Ser Ser Pro Lys Trp
65                  70                  75                  80

Glu Ser Xaa

<210> 100
<211> 43
<212> PRT
<213> Homo sapiens

<220>
<221> SITE
<222> (43)
<223> Xaa equals stop translation

<400> 100
Met Ser Ser Pro Cys Leu Phe Leu Ser Leu Thr Glu Asn Ile Phe Met
1               5                   10                  15

Ser Phe Leu Ile Ala Gly Phe Gly Leu Phe Ile Ile Met Phe Ile Asn
            20                  25                  30

Thr Phe Asp Ser Thr Val Arg Asn Val Gly Xaa
        35                  40

<210> 101
<211> 49
<212> PRT
<213> Homo sapiens

<220>
<221> SITE
<222> (49)
<223> Xaa equals stop translation

<400> 101
Met Leu Leu Phe Phe Val Ala Ala Ala Ala Leu Ala Leu Gly Ala Glu
1               5                   10                  15

Pro Glu Gly Arg Arg Trp Arg Asp Asp Cys Arg Val Gly Glu Gln Arg
            20                  25                  30

Ser Gly Ala Arg Leu Val Ser Gln His Pro Glu Cys Gly Phe Leu Leu
        35                  40                  45

Xaa

<210> 102
<211> 46

<212> PRT
<213> Homo sapiens

<220>
<221> SITE
<222> (46)
<223> Xaa equals stop translation

<400> 102
Met Leu Leu Gln Phe Ser Ile Phe Phe Ala Pro Val Val Cys Leu Pro
1               5                   10                  15

Lys Tyr Ser Pro Phe Met Lys Glu Glu Cys Lys Ala Asp Pro Thr Arg
            20                  25                  30

Asp Tyr Lys Phe Leu Tyr Ile Tyr Ile Glu Arg Gly Thr Xaa
        35                  40                  45


<210> 103
<211> 49
<212> PRT
<213> Homo sapiens

<220>
<221> SITE
<222> (49)
<223> Xaa equals stop translation

<400> 103
Met Cys Gly Ile Phe Ser Ile Leu Cys Ile Lys Ile Phe Phe Leu Ile
1               5                   10                  15

Leu Gln Leu Phe Phe Tyr Phe Pro Leu Tyr Asn Cys Ile Phe Asn Thr
            20                  25                  30

Ser Ile Ser Ile Leu Asn Arg Val Leu Val Lys Lys Arg Ser Thr Phe
        35                  40                  45

Xaa


<210> 104
<211> 66
<212> PRT
<213> Homo sapiens

<220>
<221> SITE
<222> (66)
<223> Xaa equals stop translation

<400> 104
Met Tyr Leu Leu His Ser Ile Leu Phe Met Leu Cys Leu Val Gly Met
1               5                   10                  15

Val Glu Phe Asn Lys Ser Thr Arg Glu Cys Ile Leu Phe Lys Thr Leu

20                  25                  30

Trp Leu Ile Pro Leu Phe Thr Tyr Lys Leu Ala Tyr Leu Cys Glu Lys
        35                  40                  45

Leu Lys Phe Val Lys Phe Cys Ala Ser Leu Leu Ile Ala Val Phe Asp
    50                  55                  60

His Xaa
65

<210> 105
<211> 46
<212> PRT
<213> Homo sapiens

<220>
<221> SITE
<222> (46)
<223> Xaa equals stop translation

<400> 105
Met Thr Ala Phe Ile Thr Tyr Pro Leu Leu Phe Ile Cys Leu Pro Ser
  1               5                  10                  15

Val Ser His Phe Leu Pro Val Pro Thr Cys Leu Phe Pro Cys Glu Gly
             20                  25                  30

Leu Asn Cys Glu Pro Leu Arg Phe Asn Val Arg Ser Pro Xaa
        35                  40                  45

<210> 106
<211> 74
<212> PRT
<213> Homo sapiens

<220>
<221> SITE
<222> (74)
<223> Xaa equals stop translation

<400> 106
Met Pro His Leu Asn His Ser Leu Phe Leu Phe Leu Ser Val Gly Cys
  1               5                  10                  15

Ala Leu Ser Ala Gln Met Ala Phe His Gln Leu Asp Leu Glu Gln Pro
             20                  25                  30

Glu Asp Ala Thr Leu Pro Ser Glu Pro Phe Phe His His Thr Val Val
        35                  40                  45

Pro Gln Arg Ser Phe Ser Arg Ile Leu Val Asn Met Gly Gln Leu Ser
    50                  55                  60

Glu Thr Leu Ala Glu Gln Gly Tyr Ile Xaa
 65                  70

<210> 107
<211> 50
<212> PRT
<213> Homo sapiens

<220>
<221> SITE
<222> (50)
<223> Xaa equals stop translation

<400> 107
Met Phe Pro Trp Cys Val Cys Val Ile Ala Cys Ile Ser Ala Val Thr
1               5                   10                  15

Pro Leu Ile Gln Gly Phe Thr Phe Cys Ser Phe Ser Tyr Pro Gln Tyr
            20                  25                  30

Ser Thr Val Arg Tyr Phe Glu Arg Glu Thr Thr Leu Thr Leu Leu Leu
        35                  40                  45

Leu Xaa
    50


<210> 108
<211> 228
<212> PRT
<213> Homo sapiens

<220>
<221> SITE
<222> (228)
<223> Xaa equals stop translation

<400> 108
Met Ala Ala Pro Ile Ile Gly Val Thr Pro Met Phe Ala Val Cys Phe
1               5                   10                  15

Phe Gly Phe Gly Leu Gly Lys Lys Leu Gln Gln Lys His Pro Glu Asp
            20                  25                  30

Val Leu Ser Tyr Pro Gln Leu Phe Ala Ala Gly Met Leu Ser Gly Val
        35                  40                  45

Phe Thr Thr Gly Ile Met Thr Pro Gly Glu Arg Ile Lys Cys Leu Leu
    50                  55                  60

Gln Ile Gln Ala Ser Ser Gly Glu Ser Lys Tyr Thr Gly Thr Leu Asp
65                  70                  75                  80

Cys Ala Lys Lys Leu Tyr Gln Glu Phe Gly Ile Arg Gly Ile Tyr Lys
                85                  90                  95

Gly Thr Val Leu Thr Leu Met Arg Asp Val Pro Ala Ser Gly Met Tyr
            100                 105                 110

Phe Met Thr Tyr Glu Trp Leu Lys Asn Ile Phe Thr Pro Glu Gly Lys
        115                 120                 125

Arg Val Ser Glu Leu Ser Ala Pro Arg Ile Leu Val Ala Gly Gly Ile
    130                 135                 140

Ala Gly Ile Phe Asn Trp Ala Val Ala Ile Pro Pro Asp Val Leu Lys
145                 150                 155                 160

Ser Arg Phe Gln Thr Ala Pro Pro Gly Lys Tyr Pro Asn Gly Phe Arg
                165                 170                 175

Asp Val Leu Arg Glu Leu Ile Arg Asp Glu Gly Val Thr Ser Leu Tyr
            180                 185                 190

Lys Gly Phe Asn Ala Val Met Ile Arg Ala Phe Pro Ala Asn Ala Ala
        195                 200                 205

Cys Phe Leu Gly Phe Glu Val Ala Met Lys Phe Leu Asn Trp Ala Thr
    210                 215                 220

Pro Asn Leu Xaa
225

<210> 109
<211> 74
<212> PRT
<213> Homo sapiens

<220>
<221> SITE
<222> (74)
<223> Xaa equals stop translation

<400> 109
Met Thr Arg Ala Thr Thr Glu Phe Pro Ser Pro Lys Phe Ser Thr Leu
1               5                   10                  15

Leu Val Leu Val Leu Ser Leu Leu Arg Ala His Ile Leu Ile Pro Lys
            20                  25                  30

Glu Pro Leu Gln Ser Ser Cys Leu Leu Lys Thr Leu Tyr Trp Ala Cys
        35                  40                  45

Ser Cys Asn Ser Asp Phe Ile Arg Cys Ile Leu Arg Glu Val Ser Gly
    50                  55                  60

Lys Ile Trp Arg Phe Ser Lys Thr Leu Xaa
65                  70

<210> 110
<211> 43
<212> PRT
<213> Homo sapiens

<220>

<221> SITE
<222> (43)
<223> Xaa equals stop translation

<400> 110
Met Ile Tyr Phe Leu Cys Leu Ala Tyr Cys Lys Phe Phe Ile Leu Ile
1               5                   10                  15

His Ser Ser Asn Ile Ile Ala Thr Lys Lys Cys Leu Tyr Leu Asp Gln
            20                  25                  30

Arg Gln Asp Phe Leu Cys Val Cys Phe Ala Xaa
        35                  40


<210> 111
<211> 180
<212> PRT
<213> Homo sapiens

<220>
<221> SITE
<222> (180)
<223> Xaa equals stop translation

<400> 111
Met Ala Cys Lys Gly Leu Leu Gln Gln Val Gln Gly Pro Arg Leu Pro
1               5                   10                  15

Trp Thr Arg Leu Leu Leu Leu Leu Leu Val Phe Ala Val Gly Phe Leu
            20                  25                  30

Cys His Asp Leu Arg Ser His Ser Ser Phe Gln Ala Ser Leu Thr Gly
        35                  40                  45

Arg Leu Leu Arg Ser Ser Gly Phe Leu Pro Ala Ser Gln Gln Ala Cys
    50                  55                  60

Ala Lys Leu Tyr Ser Tyr Ser Leu Gln Gly Tyr Ser Trp Leu Gly Glu
65                  70                  75                  80

Thr Leu Pro Leu Trp Gly Ser His Leu Leu Thr Val Val Arg Pro Ser
                85                  90                  95

Leu Gln Leu Ala Trp Ala His Thr Asn Ala Thr Val Ser Phe Leu Ser
            100                 105                 110

Ala His Cys Ala Ser His Leu Ala Trp Phe Gly Asp Ser Leu Thr Ser
        115                 120                 125

Leu Ser Gln Arg Leu Gln Ile Gln Leu Pro Asp Ser Val Asn Gln Leu
    130                 135                 140

Leu Arg Tyr Leu Arg Glu Leu Pro Leu Leu Phe His Gln Asn Val Leu
145                 150                 155                 160

Leu Pro Leu Trp His Leu Leu Leu Glu Ala Leu Ala Trp Ala Gln Gly
                165                 170                 175

Ala Leu Pro Xaa
            180

<210> 112
<211> 47
<212> PRT
<213> Homo sapiens

<220>
<221> SITE
<222> (47)
<223> Xaa equals stop translation

<400> 112
Met Val Trp Phe Ile Tyr Phe Val Leu Gln Gly Leu Phe Cys Pro Lys
  1               5                  10                  15

Asn Glu Gly Ala Ser Pro Gly Leu Gln Phe Pro Thr Leu Ser Leu Ala
             20                  25                  30

Gly His Ala Ser Pro Ala Leu Val Pro His Gly Met Gly Gly Xaa
         35                  40                  45

<210> 113
<211> 81
<212> PRT
<213> Homo sapiens

<220>
<221> SITE
<222> (34)
<223> Xaa equals any of the naturally occurring L-amino acids

<220>
<221> SITE
<222> (81)
<223> Xaa equals stop translation

<400> 113
Met Asn Val Thr Ser Val Ile Leu Val Leu Ile Leu Trp Asn Val Ile
  1               5                  10                  15

Gly Val Ala Thr Trp Val His Gln Asn Thr Phe Leu Tyr Lys Arg Gln
             20                  25                  30

Met Xaa Glu Leu Lys Arg Leu Lys Asp Arg Val Phe Cys Phe Phe Val
         35                  40                  45

Leu Ile Trp Leu Leu Gly Ile Lys Ile Arg Pro Arg Ser Leu Lys Ile
     50                  55                  60

Ser Asn Arg Gly Arg Pro Leu Ile Asp Leu Lys Ser Val Asn Ser Leu
 65                  70                  75                  80

Xaa

<210> 114
<211> 68
<212> PRT
<213> Homo sapiens

<220>
<221> SITE
<222> (68)
<223> Xaa equals stop translation

<400> 114
Met Gln Pro Ala Cys Leu Ala Pro Cys Leu Asp Ala Leu Thr Ser Phe
 1               5                  10                  15

Cys Leu Gly Leu Leu Lys Leu Thr Phe Cys Leu Ala Phe Phe Pro Ser
            20                  25                  30

Gly Val Leu Glu Gly Glu Cys Ser Phe Phe Thr Met Ser Arg Ser Leu
        35                  40                  45

Ser His Pro Arg Thr Leu His Arg Tyr Thr Thr Glu Arg Pro Ala His
    50                  55                  60

Ser Arg His Xaa
65


<210> 115
<211> 43
<212> PRT
<213> Homo sapiens

<220>
<221> SITE
<222> (43)
<223> Xaa equals stop translation

<400> 115
Met Phe Leu Val Phe Trp Leu Leu Gly Ile Tyr Phe Cys His Leu Leu
 1               5                  10                  15

Val Ile Thr Val Leu Thr Lys Trp Ile Leu Ala Pro Pro Tyr Leu Met
            20                  25                  30

Ala Gln Thr Thr Thr Pro Gln Ser Leu Tyr Xaa
        35                  40


<210> 116
<211> 212
<212> PRT
<213> Homo sapiens

<220>
<221> SITE

<222> (212)
<223> Xaa equals stop translation

<400> 116
Met Ile Ser Leu Pro Gly Pro Leu Val Thr Asn Leu Leu Arg Phe Leu
1               5                   10                  15

Phe Leu Gly Leu Ser Ala Leu Asp Val Ile Arg Gly Ser Leu Ser Leu
            20                  25                  30

Thr Asn Leu Ser Ser Ser Met Ala Gly Val Tyr Val Cys Lys Ala His
        35                  40                  45

Asn Glu Val Gly Thr Ala Gln Cys Asn Val Thr Leu Glu Val Ser Thr
    50                  55                  60

Gly Pro Gly Ala Ala Val Val Ala Gly Ala Val Val Gly Thr Leu Val
65                  70                  75                  80

Gly Leu Gly Leu Leu Ala Gly Leu Val Leu Leu Tyr His Arg Arg Gly
                85                  90                  95

Lys Ala Leu Glu Glu Pro Ala Asn Asp Ile Lys Glu Asp Ala Ile Ala
            100                 105                 110

Pro Arg Thr Leu Pro Trp Pro Lys Ser Ser Asp Thr Ile Ser Lys Asn
        115                 120                 125

Gly Thr Leu Ser Ser Val Thr Ser Ala Arg Ala Leu Arg Pro Pro His
    130                 135                 140

Gly Pro Pro Arg Pro Gly Ala Leu Thr Pro Thr Pro Ser Leu Ser Ser
145                 150                 155                 160

Gln Ala Leu Pro Ser Pro Arg Leu Pro Thr Thr Asp Gly Ala His Pro
                165                 170                 175

Gln Pro Ile Ser Pro Ile Pro Gly Gly Val Ser Ser Ser Gly Leu Ser
            180                 185                 190

Arg Met Gly Ala Val Pro Val Met Val Pro Ala Gln Ser Gln Ala Gly
        195                 200                 205

Ser Leu Val Xaa
    210


<210> 117
<211> 44
<212> PRT
<213> Homo sapiens

<220>
<221> SITE
<222> (44)
<223> Xaa equals stop translation

<400> 117

Met Lys Leu Pro Trp Asn Ile Val Asn Ile Leu Lys Ala Ser Ala Leu
1               5                   10                  15

Tyr Ala Leu Lys Trp Leu Leu Leu Ile Leu Tyr Tyr Val Ile Phe Thr
            20                  25                  30

Leu Lys Lys Glu Lys Ile Ala Leu Leu Tyr Thr Xaa
        35                  40


<210> 118
<211> 127
<212> PRT
<213> Homo sapiens

<220>
<221> SITE
<222> (127)
<223> Xaa equals stop translation

<400> 118
Met Gly Thr Ser Ala Leu Trp Pro Phe Leu Pro Leu Leu Phe Leu Leu
1               5                   10                  15

Gly Phe Leu Phe Ser Ser Cys Gly Phe Pro Glu Ala Ser Phe Gly Pro
            20                  25                  30

Trp Val Val Val Arg Ala Glu Leu Trp Gly Cys Val Val Gly Ala Ala
        35                  40                  45

Cys Val Leu Gly Leu Tyr Trp Gln Val Gly Gln Ser Ser Leu Asn Thr
    50                  55                  60

Leu Ala Arg Ser Gln Lys Pro Gly Leu Arg Val Gln Pro Gly Lys Pro
65                  70                  75                  80

Gly Lys Leu Leu Pro Val Thr Phe Gln Met Leu Pro Pro Pro Cys Gly
                85                  90                  95

Gly Cys Cys Ser Pro Leu Gly Leu Cys Pro Ser Ser Gly Gly Ser Arg
            100                 105                 110

Met Trp Arg Arg Thr Trp Val Gly Ala Arg Ala Leu His Pro Xaa
        115                 120                 125


<210> 119
<211> 57
<212> PRT
<213> Homo sapiens

<220>
<221> SITE
<222> (57)
<223> Xaa equals stop translation

<400> 119
Met Phe Leu Lys Val Leu Val Phe Leu Ile Phe Phe Ser Pro Phe Ser

1               5                   10                  15

Ser Ser Leu Phe Ser Gly Glu Ala Val Arg Gly Arg Gly Ala Gly Leu
            20                  25                  30

Gly Leu Gly Ile Gly Arg Gly Trp Thr Ser Cys Leu Ser Val Leu Asn
        35                  40                  45

Gly Cys Asp Gly Ala Arg Ser His Xaa
    50                  55


<210> 120
<211> 46
<212> PRT
<213> Homo sapiens

<220>
<221> SITE
<222> (46)
<223> Xaa equals stop translation

<400> 120
Met Trp Ser Ile Lys Leu Thr Cys Arg Leu Arg Gly Phe Trp Phe Trp
 1               5                  10                  15

Phe Trp Val Leu Phe Phe Cys Gly Gly Gly Ala Gly Ile Trp Lys Asn
            20                  25                  30

Leu Ala Leu Tyr Val Thr Glu Ile Phe Phe Ala Arg Thr Xaa
        35                  40                  45


<210> 121
<211> 58
<212> PRT
<213> Homo sapiens

<220>
<221> SITE
<222> (47)
<223> Xaa equals any of the naturally occurring L-amino acids

<400> 121
Met Arg Leu Ile Leu Ile Ile Gly Arg Leu Ala Leu Asp Ser Ile Ala
 1               5                  10                  15

Gln Asn Ser Gln Asn Val Ser Gln Ser Ser Gln Gly Ser Tyr His His
            20                  25                  30

Gly Ser Ser Pro Pro Arg Pro Val Arg Pro Leu Pro Gly Pro Xaa Arg
        35                  40                  45

Arg Arg Asp Pro Ser Leu Asp Cys Cys Ser
    50                  55


<210> 122

<211> 57
<212> PRT
<213> Homo sapiens

<220>
<221> SITE
<222> (57)
<223> Xaa equals stop translation

<400> 122
Met Lys Ala Met Leu Gln Cys Phe Arg Phe Tyr Phe Met Arg Leu Phe
1               5                   10                  15

Val Phe Leu Leu Thr Ser Gly Lys Met Ile Asp Ser Asp Ser Thr Met
            20                  25                  30

Gln Gly Cys Trp Tyr Gln Pro Glu Pro Tyr Arg Trp Gln Ser Leu Glu
        35                  40                  45

Lys Trp Ser Gln Lys Met Glu Leu Xaa
    50                  55


<210> 123
<211> 273
<212> PRT
<213> Homo sapiens

<220>
<221> SITE
<222> (273)
<223> Xaa equals stop translation

<400> 123
Met Trp Gly Asn Lys Phe Gly Val Leu Leu Phe Leu Tyr Ser Val Leu
1               5                   10                  15

Leu Thr Lys Gly Ile Glu Asn Ile Lys Asn Glu Ile Glu Asp Ala Ser
            20                  25                  30

Glu Pro Leu Ile Asp Pro Val Tyr Gly His Gly Ser Gln Ser Leu Ile
        35                  40                  45

Asn Leu Leu Leu Thr Gly His Ala Val Ser Asn Val Trp Asp Gly Asp
    50                  55                  60

Arg Glu Cys Ser Gly Met Lys Leu Leu Gly Ile His Glu Gln Ala Ala
65                  70                  75                  80

Val Gly Phe Leu Thr Leu Met Glu Ala Leu Arg Tyr Cys Lys Val Gly
                85                  90                  95

Ser Tyr Leu Lys Ser Pro Lys Phe Pro Ile Trp Ile Val Gly Ser Glu
            100                 105                 110

Thr His Leu Thr Val Phe Phe Ala Lys Asp Met Ala Leu Val Ala Pro
        115                 120                 125

Glu Ala Pro Ser Glu Gln Ala Arg Arg Val Phe Gln Thr Tyr Asp Pro
    130                 135                 140

Glu Asp Asn Gly Phe Ile Pro Asp Ser Leu Leu Glu Asp Val Met Lys
145                 150                 155                 160

Ala Leu Asp Leu Val Ser Asp Pro Glu Tyr Ile Asn Leu Met Lys Asn
                165                 170                 175

Lys Leu Asp Pro Glu Gly Leu Gly Ile Ile Leu Leu Gly Pro Phe Leu
            180                 185                 190

Gln Glu Phe Phe Pro Asp Gln Gly Ser Ser Gly Pro Glu Ser Phe Thr
        195                 200                 205

Val Tyr His Tyr Asn Gly Leu Lys Gln Ser Asn Tyr Asn Glu Lys Val
    210                 215                 220

Met Tyr Val Glu Gly Thr Ala Val Val Met Gly Phe Glu Asp Pro Met
225                 230                 235                 240

Leu Gln Thr Asp Asp Thr Pro Ile Lys Arg Cys Leu Gln Thr Lys Trp
                245                 250                 255

Pro Tyr Ile Glu Leu Leu Trp Thr Thr Asp Arg Ser Pro Ser Leu Asn
            260                 265                 270

Xaa

<210> 124
<211> 281
<212> PRT
<213> Homo sapiens

<220>
<221> SITE
<222> (281)
<223> Xaa equals stop translation

<400> 124
Met Ala Pro Ser Gly Ser Leu Ala Val Pro Leu Ala Val Leu Val Leu
  1               5                  10                  15

Leu Leu Trp Gly Ala Pro Trp Thr His Gly Arg Arg Ser Asn Val Arg
             20                  25                  30

Val Ile Thr Asp Glu Asn Trp Arg Glu Leu Leu Glu Gly Asp Trp Met
         35                  40                  45

Ile Glu Phe Tyr Ala Pro Trp Cys Pro Ala Cys Gln Asn Leu Gln Pro
     50                  55                  60

Glu Trp Glu Ser Phe Ala Glu Trp Gly Glu Asp Leu Glu Val Asn Ile
 65                  70                  75                  80

Ala Lys Val Asp Val Thr Glu Gln Pro Gly Leu Ser Gly Arg Phe Ile

85                  90                  95

Ile Thr Ala Leu Pro Thr Ile Tyr His Cys Lys Asp Gly Glu Phe Arg
            100                 105                 110

Arg Tyr Gln Gly Pro Arg Thr Lys Lys Asp Phe Ile Asn Phe Ile Ser
        115                 120                 125

Asp Lys Glu Trp Lys Ser Ile Glu Pro Val Ser Ser Trp Phe Gly Pro
    130                 135                 140

Gly Ser Val Leu Met Ser Ser Met Ser Ala Leu Phe Gln Leu Ser Met
145                 150                 155                 160

Trp Ile Arg Thr Cys His Asn Tyr Phe Ile Glu Asp Leu Gly Leu Pro
                165                 170                 175

Val Trp Gly Ser Tyr Thr Val Phe Ala Leu Ala Thr Leu Phe Ser Gly
            180                 185                 190

Leu Leu Leu Gly Leu Cys Met Ile Phe Val Ala Asp Cys Leu Cys Pro
        195                 200                 205

Ser Lys Arg Arg Arg Pro Gln Pro Tyr Pro Tyr Pro Ser Lys Lys Leu
    210                 215                 220

Leu Ser Glu Ser Ala Gln Pro Leu Lys Lys Val Glu Glu Glu Gln Glu
225                 230                 235                 240

Ala Asp Glu Glu Asp Val Ser Glu Glu Glu Ala Glu Ser Lys Glu Gly
                245                 250                 255

Thr Asn Lys Asp Phe Pro Gln Asn Ala Ile Arg Gln Arg Ser Leu Gly
            260                 265                 270

Pro Ser Leu Ala Thr Asp Lys Ser Xaa
        275                 280


<210> 125
<211> 92
<212> PRT
<213> Homo sapiens

<220>
<221> SITE
<222> (84)
<223> Xaa equals any of the naturally occurring L-amino acids

<220>
<221> SITE
<222> (92)
<223> Xaa equals stop translation

<400> 125
Met Tyr Gly Lys Ser Ser Thr Arg Ala Val Leu Leu Leu Leu Gly Ile
  1               5                  10                  15

Gln Leu Thr Ala Leu Trp Pro Ile Ala Ala Val Glu Ile Tyr Thr Ser
            20                  25                  30

Arg Val Leu Glu Ala Val Asn Gly Thr Asp Ala Arg Leu Lys Cys Thr
        35                  40                  45

Phe Ser Ser Phe Ala Pro Val Gly Asp Ala Leu Thr Val Thr Trp Asn
    50                  55                  60

Phe Arg Pro Leu Asp Gly Gly Pro Glu Gln Phe Val Phe Tyr Tyr His
65                  70                  75                  80

Ile Asp Pro Xaa Pro Thr His Glu Trp Ala Val Xaa
                85                  90

<210> 126
<211> 295
<212> PRT
<213> Homo sapiens

<220>
<221> SITE
<222> (188)
<223> Xaa equals any of the naturally occurring L-amino acids

<220>
<221> SITE
<222> (211)
<223> Xaa equals any of the naturally occurring L-amino acids

<220>
<221> SITE
<222> (295)
<223> Xaa equals stop translation

<400> 126
Met Pro Arg Gly Asp Ser Glu Gln Val Arg Tyr Cys Ala Arg Phe Ser
  1               5                  10                  15

Tyr Leu Trp Leu Lys Phe Ser Leu Ile Ile Tyr Ser Thr Val Phe Trp
            20                  25                  30

Leu Ile Gly Ala Leu Val Leu Ser Val Gly Ile Tyr Ala Glu Val Glu
        35                  40                  45

Arg Gln Lys Tyr Lys Thr Leu Glu Ser Ala Phe Leu Ala Pro Ala Ile
    50                  55                  60

Ile Leu Ile Leu Leu Gly Val Val Met Phe Met Val Ser Phe Ile Gly
65                  70                  75                  80

Val Leu Ala Ser Leu Arg Asp Asn Leu Tyr Leu Leu Gln Ala Phe Met
                85                  90                  95

Tyr Ile Leu Gly Ile Cys Leu Ile Met Glu Leu Ile Gly Gly Val Val
            100                 105                 110

Ala Leu Thr Phe Arg Asn Gln Thr Ile Asp Phe Leu Asn Asp Asn Ile
        115                 120                 125

Arg Arg Gly Ile Glu Asn Tyr Tyr Asp Asp Leu Asp Phe Lys Asn Ile
    130                 135                 140

Met Asp Phe Val Gln Lys Lys Phe Lys Cys Cys Gly Gly Glu Asp Tyr
145                 150                 155                 160

Arg Asp Trp Ser Lys Asn Gln Tyr His Asp Cys Ser Ala Pro Gly Pro
                165                 170                 175

Leu Ala Cys Gly Val Pro Tyr Thr Cys Cys Ile Xaa Asn Thr Thr Glu
            180                 185                 190

Val Val Asn Thr Met Cys Gly Tyr Lys Thr Ile Asp Lys Glu Arg Phe
        195                 200                 205

Ser Val Xaa Asp Val Ile Tyr Val Arg Gly Cys Thr Asn Ala Val Ile
    210                 215                 220

Ile Trp Phe Met Asp Asn Tyr Thr Ile Met Ala Gly Ile Leu Leu Gly
225                 230                 235                 240

Ile Leu Leu Pro Gln Phe Leu Gly Val Leu Leu Thr Leu Leu Tyr Ile
                245                 250                 255

Thr Arg Val Glu Asp Ile Ile Met Glu His Ser Val Thr Asp Gly Leu
            260                 265                 270

Leu Gly Pro Gly Ala Lys Pro Ser Val Glu Ala Ala Gly Thr Gly Cys
        275                 280                 285

Cys Leu Cys Tyr Pro Asn Xaa
    290                 295

<210> 127
<211> 43
<212> PRT
<213> Homo sapiens

<220>
<221> SITE
<222> (43)
<223> Xaa equals stop translation

<400> 127
Met Tyr Asn Lys Leu Leu Leu Thr Val Val Thr Leu Phe Cys Tyr Gln
  1               5                  10                  15

Ile Val Asp Phe Ile Tyr Ser Asn Tyr Ile Phe Ile Ser Ile Asn His
             20                  25                  30

Pro Pro His Pro Pro Asn Ile Leu Val Phe Xaa
         35                  40

<210> 128
<211> 73
<212> PRT
<213> Homo sapiens

<220>
<221> SITE
<222> (73)
<223> Xaa equals stop translation

<400> 128
Met Gly Asn Phe Thr Ser Tyr Leu Phe Leu Phe Ala Phe Ser Gly Ile
1               5                   10                  15

Ile Leu Ala Phe Ile Lys Asn Gly Leu Ala Ala Glu Ile Val Leu Ile
            20                  25                  30

Leu Ser Glu Ala Gly Cys Ser Gln Asp Lys Ser Lys Met Val Tyr Leu
        35                  40                  45

Ser Pro Gly Glu Gly Lys Leu Ile Lys Ile Ser Tyr Phe Cys Leu Val
    50                  55                  60

Trp Phe Cys Phe Phe Leu Leu Leu Xaa
65                  70


<210> 129
<211> 427
<212> PRT
<213> Homo sapiens

<220>
<221> SITE
<222> (427)
<223> Xaa equals stop translation

<400> 129
Met Ile Val Phe Gly Trp Ala Val Phe Leu Ala Ser Arg Ser Leu Gly
1               5                   10                  15

Gln Gly Leu Leu Leu Thr Leu Glu Glu His Ile Ala His Phe Leu Gly
            20                  25                  30

Thr Gly Gly Ala Ala Thr Thr Met Gly Asn Ser Cys Ile Cys Arg Asp
        35                  40                  45

Asp Ser Gly Thr Asp Asp Ser Val Asp Thr Gln Gln Gln Gln Ala Glu
    50                  55                  60

Asn Ser Ala Val Pro Thr Ala Asp Thr Arg Ser Gln Pro Arg Asp Pro
65                  70                  75                  80

Val Arg Pro Pro Arg Arg Gly Arg Gly Pro His Glu Pro Arg Arg Lys
                85                  90                  95

Lys Gln Asn Val Asp Gly Leu Val Leu Asp Thr Leu Ala Val Ile Arg
            100                 105                 110

Thr Leu Val Asp Asn Asp Gln Glu Pro Tyr Ser Met Ile Thr Leu His
        115                 120                 125

Glu Met Ala Glu Thr Asp Glu Gly Trp Leu Asp Val Val Gln Ser Leu
    130                 135                 140

Ile Arg Val Ile Pro Leu Glu Asp Pro Leu Gly Pro Ala Val Ile Thr
145                 150                 155                 160

Leu Leu Leu Asp Glu Cys Pro Leu Pro Thr Lys Asp Ala Leu Gln Lys
                165                 170                 175

Leu Thr Glu Ile Leu Asn Leu Asn Gly Glu Val Ala Cys Gln Asp Ser
            180                 185                 190

Ser His Pro Ala Lys His Arg Asn Thr Ser Ala Val Leu Gly Cys Leu
        195                 200                 205

Ala Glu Lys Leu Ala Gly Pro Ala Ser Ile Gly Leu Leu Ser Pro Gly
    210                 215                 220

Ile Leu Glu Tyr Leu Leu Gln Cys Leu Lys Leu Gln Ser His Pro Thr
225                 230                 235                 240

Val Met Leu Phe Ala Leu Ile Ala Leu Glu Lys Phe Ala Gln Thr Ser
                245                 250                 255

Glu Asn Lys Leu Thr Ile Ser Glu Ser Ser Ile Ser Asp Arg Leu Val
            260                 265                 270

Thr Leu Glu Ser Trp Ala Asn Asp Pro Asp Tyr Leu Lys Arg Gln Val
        275                 280                 285

Gly Phe Cys Ala Gln Trp Ser Leu Asp Asn Leu Phe Leu Lys Glu Gly
    290                 295                 300

Arg Gln Leu Thr Tyr Glu Lys Val Asn Leu Ser Ser Ile Arg Ala Met
305                 310                 315                 320

Leu Asn Ser Asn Asp Val Ser Glu Tyr Leu Lys Ile Ser Pro His Gly
                325                 330                 335

Leu Glu Ala Arg Cys Asp Ala Ser Ser Phe Glu Ser Val Arg Cys Thr
            340                 345                 350

Phe Cys Val Asp Ala Gly Val Trp Tyr Tyr Glu Val Thr Val Val Thr
        355                 360                 365

Ser Gly Val Met Gln Ile Gly Trp Val Thr Arg Asp Ser Lys Phe Leu
    370                 375                 380

Asn His Glu Gly Tyr Gly Ile Gly Asp Asp Glu Tyr Ser Cys Ala Tyr
385                 390                 395                 400

Asp Gly Cys Arg Gln Leu Ile Trp Tyr Asn Ala Arg Ser Ser Leu Thr
                405                 410                 415

Tyr Thr His Ala Gly Lys Lys Glu Ile Gln Xaa
            420                 425

<210> 130
<211> 323
<212> PRT
<213> Homo sapiens

<220>
<221> SITE
<222> (323)
<223> Xaa equals stop translation

<400> 130
Met Pro Pro Arg Gly Pro Ala Ser Glu Leu Leu Leu Leu Arg Leu Leu
  1               5                  10                  15

Leu Leu Gly Ala Ala Thr Ala Ala Pro Leu Ala Pro Arg Pro Ser Lys
             20                  25                  30

Glu Glu Leu Thr Arg Cys Leu Ala Glu Val Val Thr Glu Val Leu Thr
         35                  40                  45

Val Gly Gln Val Gln Arg Gly Pro Cys Thr Ala Leu Leu His Lys Glu
     50                  55                  60

Leu Cys Gly Thr Glu Pro His Gly Cys Ala Ser Thr Glu Glu Lys Gly
 65                  70                  75                  80

Leu Leu Leu Gly Asp Phe Lys Lys Gln Glu Ala Gly Lys Met Arg Ser
                 85                  90                  95

Ser Gln Glu Val Arg Asp Glu Glu Glu Glu Glu Val Ala Glu Arg Thr
            100                 105                 110

His Lys Ser Glu Val Gln Glu Gln Ala Ile Arg Met Gln Gly His Arg
        115                 120                 125

Gln Leu His Gln Glu Glu Asp Glu Glu Glu Glu Lys Glu Glu Arg Lys
    130                 135                 140

Arg Gly Pro Met Glu Thr Phe Glu Asp Leu Trp Gln Arg His Leu Glu
145                 150                 155                 160

Asn Gly Gly Asp Leu Gln Lys Arg Val Ala Glu Lys Ala Ser Asp Lys
                165                 170                 175

Glu Thr Ala Gln Phe Gln Ala Glu Glu Lys Gly Val Arg Val Leu Gly
            180                 185                 190

Gly Asp Arg Ser Leu Trp Gln Gly Ala Glu Arg Gly Gly Gly Glu Arg
        195                 200                 205

Arg Glu Asp Leu Pro His His His His His His His Gln Pro Glu Ala
    210                 215                 220

Glu Pro Arg Gln Glu Lys Glu Glu Ala Ser Glu Arg Glu Val Ser Arg

225                 230                 235                 240

Gly Met Lys Glu Glu His Gln His Ser Leu Glu Ala Gly Leu Met Met
                245                 250                 255

Val Ser Gly Val Thr Thr His Ser His Arg Cys Trp Pro Cys Thr Thr
            260                 265                 270

Arg Ser Ile Thr Ser Gly Ser Gln Trp Pro Arg Leu Thr Pro Arg Leu
        275                 280                 285

Ala Asn Asn Phe Arg Ala Arg Pro Leu Pro Tyr Thr Ser Thr Leu Leu
    290                 295                 300

Tyr Gly Leu Gln Gln Pro Arg Trp His His Cys Thr Glu Ala Ser His
305                 310                 315                 320

His His Xaa

<210> 131
<211> 56
<212> PRT
<213> Homo sapiens

<220>
<221> SITE
<222> (56)
<223> Xaa equals stop translation

<400> 131
Met Leu Phe Leu Arg Ser Ile Leu Trp Leu Ser Ser Leu Phe Phe Cys
  1               5                  10                  15

His Phe Val Pro Thr Ser His Ser Leu Gly Phe Gln Asn Ile Thr Ser
             20                  25                  30

Val Tyr Asn Ala Thr Leu Gln Gln Thr Val Phe Gln His Asp Ser Lys
         35                  40                  45

Thr Val Thr Thr Cys Phe Thr Xaa
     50                  55

<210> 132
<211> 76
<212> PRT
<213> Homo sapiens

<220>
<221> SITE
<222> (76)
<223> Xaa equals stop translation

<400> 132
Met Phe Cys Val Phe Ile Leu Thr Phe Phe Met Val Phe Asn Leu Trp
  1               5                  10                  15

Leu Ala Ala Thr Val Tyr His Val Tyr Gly Thr Cys Lys Lys Val Leu
                20                  25                  30

Asp Ile Gln Ile Leu Arg Asp Glu Ile Thr Phe Thr Tyr Lys Asn His
            35                  40                  45

Phe Tyr Cys Gly Leu Thr Ala Leu Ser Ser Arg Ile Leu Asn Asp Ile
    50                  55                  60

Thr Asn Ile Leu His Val Ile Cys Ser Phe Glu Xaa
65                  70                  75

<210> 133
<211> 185
<212> PRT
<213> Homo sapiens

<220>
<221> SITE
<222> (185)
<223> Xaa equals stop translation

<400> 133
Met Leu Phe Leu Phe Ser Met Ala Thr Leu Leu Arg Thr Ser Phe Ser
  1               5                  10                  15

Asp Pro Gly Val Ile Pro Arg Ala Leu Pro Asp Glu Ala Ala Phe Ile
                20                  25                  30

Glu Met Glu Ile Glu Ala Thr Asn Gly Ala Val Pro Gln Gly Gln Arg
            35                  40                  45

Pro Pro Pro Arg Ile Lys Asn Phe Gln Ile Asn Asn Gln Ile Val Lys
    50                  55                  60

Leu Lys Tyr Cys Tyr Thr Cys Lys Ile Phe Arg Pro Pro Arg Ala Ser
65                  70                  75                  80

His Cys Ser Ile Cys Asp Asn Cys Val Glu Arg Phe Asp His His Cys
                85                  90                  95

Pro Trp Val Gly Asn Cys Val Gly Lys Arg Asn Tyr Arg Tyr Phe Tyr
            100                 105                 110

Leu Phe Ile Leu Ser Leu Ser Leu Leu Thr Ile Tyr Val Phe Ala Phe
        115                 120                 125

Asn Ile Val Tyr Val Ala Leu Lys Ser Leu Lys Ile Gly Phe Leu Glu
    130                 135                 140

Thr Leu Lys Gly Asn Ser Trp Asn Cys Ser Arg Ser Pro His Leu Leu
145                 150                 155                 160

Leu Tyr Thr Leu Val Arg Arg Gly Thr Asp Trp Ile Ser Tyr Phe Pro
                165                 170                 175

Arg Gly Ser Gln Pro Asp Asn Gln Xaa
            180                 185

<210> 134
<211> 66
<212> PRT
<213> Homo sapiens

<220>
<221> SITE
<222> (66)
<223> Xaa equals stop translation

<400> 134
Met Phe His Cys Trp Ser Leu Phe Leu Tyr Tyr Phe Ser Leu Ser Leu
  1               5                  10                  15

Ser Ser Tyr His Arg Lys Cys Ile Leu Leu Arg Met Lys Ile Lys Glu
            20                  25                  30

Gln Ser Arg Asp Val Pro Cys Gln Gly Ala Gln Gln Ser His Pro Lys
        35                  40                  45

Phe His Leu Asp His His Leu Pro Asp Tyr Pro His Thr Asn Leu Leu
    50                  55                  60

Pro Xaa
65

<210> 135
<211> 63
<212> PRT
<213> Homo sapiens

<220>
<221> SITE
<222> (63)
<223> Xaa equals stop translation

<400> 135
Met Ala Val Arg Cys Ile Leu Ala Gly Gly Cys Leu Pro Ala Val Arg
  1               5                  10                  15

Gly Thr Phe Ser Val Leu Leu Lys Gly Met Tyr Lys Pro Met Gly Asp
            20                  25                  30

Leu Ile Ser Cys Val Phe Arg Cys Val Ala Gly Gly Leu Gly Trp Gly
        35                  40                  45

Gly Gly Ala Ser Glu Gln Cys Val Glu Ser Leu Val Val Thr Xaa
    50                  55                  60

<210> 136
<211> 379
<212> PRT

<213> Homo sapiens

<220>
<221> SITE
<222> (379)
<223> Xaa equals stop translation

<400> 136
Met Ser Lys Glu Pro Leu Ile Leu Trp Leu Met Ile Glu Phe Trp Trp
1               5                   10                  15

Leu Tyr Leu Thr Pro Val Thr Ser Glu Thr Val Val Thr Glu Val Leu
            20                  25                  30

Gly His Arg Val Thr Leu Pro Cys Leu Tyr Ser Ser Trp Ser His Asn
        35                  40                  45

Ser Asn Ser Met Cys Trp Gly Lys Asp Gln Cys Pro Tyr Ser Gly Cys
    50                  55                  60

Lys Glu Ala Leu Ile Arg Thr Asp Gly Met Arg Val Thr Ser Arg Lys
65                  70                  75                  80

Ser Ala Lys Tyr Arg Leu Gln Gly Thr Ile Pro Arg Gly Asp Val Ser
                85                  90                  95

Leu Thr Ile Leu Asn Pro Ser Glu Ser Asp Ser Gly Val Tyr Cys Cys
            100                 105                 110

Arg Ile Glu Val Pro Gly Trp Phe Asn Asp Val Lys Ile Asn Val Arg
        115                 120                 125

Leu Asn Leu Gln Arg Ala Ser Thr Thr Thr His Arg Thr Ala Thr Thr
    130                 135                 140

Thr Thr Arg Arg Thr Thr Thr Thr Ser Pro Thr Thr Thr Arg Gln Met
145                 150                 155                 160

Thr Thr Thr Pro Ala Ala Leu Pro Thr Thr Val Val Thr Thr Pro Asp
                165                 170                 175

Leu Thr Thr Gly Thr Pro Leu Gln Met Thr Thr Ile Ala Val Phe Thr
            180                 185                 190

Thr Ala Asn Thr Cys Leu Ser Leu Thr Pro Ser Thr Leu Pro Glu Glu
        195                 200                 205

Ala Thr Gly Leu Leu Thr Pro Glu Pro Ser Lys Glu Gly Pro Ile Leu
    210                 215                 220

Thr Ala Glu Ser Glu Thr Val Leu Pro Ser Asp Ser Trp Ser Ser Ala
225                 230                 235                 240

Glu Ser Thr Ser Ala Asp Thr Val Leu Leu Thr Ser Lys Glu Ser Lys
                245                 250                 255

Val Trp Asp Leu Pro Ser Thr Ser His Val Ser Met Trp Lys Thr Ser
            260                 265                 270

Asp Ser Val Ser Ser Pro Gln Pro Gly Ala Ser Asp Thr Ala Val Pro
        275                 280                 285

Glu Gln Asn Lys Thr Thr Lys Thr Gly Gln Met Asp Gly Ile Pro Met
    290                 295                 300

Ser Met Lys Asn Glu Met Pro Ile Ser Gln Leu Leu Met Ile Ile Ala
305                 310                 315                 320

Pro Ser Leu Gly Phe Val Leu Phe Ala Leu Phe Val Ala Phe Leu Leu
                325                 330                 335

Arg Gly Lys Leu Met Glu Thr Tyr Cys Ser Gln Lys His Thr Arg Leu
            340                 345                 350

Asp Tyr Ile Gly Asp Ser Lys Asn Val Leu Asn Asp Val Gln His Gly
        355                 360                 365

Arg Glu Asp Glu Asp Gly Leu Phe Thr Leu Xaa
    370                 375

<210> 137
<211> 47
<212> PRT
<213> Homo sapiens

<220>
<221> SITE
<222> (47)
<223> Xaa equals stop translation

<400> 137
Met Ile His Arg Ala Arg Ser Leu Ala Ala Leu Ser Ser Leu Met Leu
  1               5                  10                  15

Tyr Thr Lys Leu Val Gln Pro Val Ala Cys Ile Ser His Val Ala Gln
             20                  25                  30

Asp Gly Phe Glu Tyr Gly Pro Thr Gln Ile His Lys Leu Ser Xaa
         35                  40                  45

<210> 138
<211> 206
<212> PRT
<213> Homo sapiens

<220>
<221> SITE
<222> (206)
<223> Xaa equals stop translation

<400> 138
Met Lys Thr Gly Leu Val Leu Val Val Leu Gly His Val Ser Phe Ile
  1               5                  10                  15

Thr Ala Ala Leu Phe His Gly Thr Val Leu Arg Tyr Val Gly Thr Pro
            20                  25                  30

Gln Asp Ala Val Ala Leu Gln Tyr Cys Val Val Asn Ile Leu Ser Val
        35                  40                  45

Thr Ser Ala Ile Val Val Ile Thr Ser Gly Ile Ala Ala Ile Val Leu
    50                  55                  60

Ser Arg Tyr Leu Pro Ser Thr Pro Leu Arg Trp Thr Val Phe Ser Ser
65                  70                  75                  80

Ser Val Ala Cys Ala Leu Leu Ser Leu Thr Cys Ala Leu Gly Leu Leu
                85                  90                  95

Ala Ser Ile Ala Met Thr Phe Ala Thr Gln Gly Lys Ala Leu Leu Ala
            100                 105                 110

Ala Cys Thr Phe Gly Ser Ser Glu Leu Leu Ala Leu Ala Pro Asp Cys
        115                 120                 125

Pro Phe Asp Pro Thr Arg Ile Tyr Ser Ser Ser Leu Cys Leu Trp Gly
    130                 135                 140

Ile Ala Leu Val Leu Cys Val Ala Glu Asn Val Phe Ala Val Arg Cys
145                 150                 155                 160

Ala Gln Leu Thr His Gln Leu Leu Glu Leu Arg Pro Trp Trp Gly Lys
                165                 170                 175

Ser Ser His His Met Met Arg Glu Asn Pro Glu Leu Val Glu Gly Arg
            180                 185                 190

Asp Leu Leu Ser Cys Thr Ser Ser Glu Pro Leu Thr Leu Xaa
        195                 200                 205

<210> 139
<211> 221
<212> PRT
<213> Homo sapiens

<220>
<221> SITE
<222> (221)
<223> Xaa equals stop translation

<400> 139
Met Pro Pro Arg Arg Pro Trp Asp Arg Glu Ala Gly Thr Leu Gln Val
1               5                   10                  15

Leu Gly Ala Leu Ala Val Leu Trp Leu Gly Ser Val Ala Leu Ile Cys
            20                  25                  30

Leu Leu Trp Gln Val Pro Arg Pro Pro Thr Trp Gly Gln Val Gln Pro
        35                  40                  45

Lys Asp Val Pro Arg Ser Trp Glu His Gly Phe Gln Pro Ser Leu Gly

50                  55                  60

Ala Pro Gly Ser Arg Gly Pro Gly Ser Arg Gly Thr Pro Ala Ser Leu
65                  70                  75                  80

Ser Leu Trp Lys Ala Ser Pro Arg Thr Cys His Leu Gln Pro Ala Ala
                85                  90                  95

Pro Leu Pro Ser Leu Trp Ala Arg Pro Gly Cys Ser Cys Trp Thr Leu
            100                 105                 110

Pro Arg Arg Ala Ser Thr Trp Leu His Thr Thr Gly Pro Ser Gln Gly
        115                 120                 125

Leu Thr Ser Gly Ser Thr Thr Arg Leu Pro Ser Trp Glu Arg Leu Phe
    130                 135                 140

Cys Arg Ser Cys Ser Ser Cys Trp Ala Gly Thr Phe Pro Trp Leu Trp
145                 150                 155                 160

Pro Pro Ala Ala Arg His Trp Pro Gly His Pro Pro Thr Cys Arg Phe
                165                 170                 175

Trp Leu Pro Glu Val Pro Met Tyr Asp Arg Cys Pro Trp Gly Gly Ser
            180                 185                 190

Pro Trp Val Phe Cys Thr Pro Asn Ser Gly Leu Trp Met Asp Gly Thr
        195                 200                 205

Tyr Thr Trp Ala Val Pro Thr Trp Thr Gly Gly Leu Xaa
    210                 215                 220


<210> 140
<211> 60
<212> PRT
<213> Homo sapiens

<220>
<221> SITE
<222> (60)
<223> Xaa equals stop translation

<400> 140
Met Leu Leu Cys Ile Leu Ile Phe Lys Val His Leu Leu Leu Phe Cys
 1               5                  10                  15

Arg Ser Phe Ser Ala Phe Leu Asn Leu Lys Glu Arg Phe Leu Phe Leu
            20                  25                  30

Ile Leu Val Trp Ile Phe Val Ala Phe Tyr Gly Cys Lys Tyr Ser Pro
        35                  40                  45

Leu Ser Phe Asp Ser Phe Lys Ser Leu Gly Ser Xaa
    50                  55                  60


<210> 141

<211> 67
<212> PRT
<213> Homo sapiens

<220>
<221> SITE
<222> (67)
<223> Xaa equals stop translation

<400> 141
Met Leu Leu Ile Ser Ala Val Gln Val Phe Ile Leu Leu Ser Pro Ser
1               5                   10                  15

Phe Tyr Leu Ile Leu Tyr Leu Leu Arg Pro Gly Gly Thr Gly Arg Gly
            20                  25                  30

Leu Glu Pro Ile Cys Pro Ala Ala Glu Trp Gly Gly Trp Arg Asp Gly
        35                  40                  45

Tyr Leu Trp Leu Gln Tyr Gln Glu Pro Thr Val Ser Leu Asp Asn Trp
    50                  55                  60

Gly Asn Xaa
65


<210> 142
<211> 59
<212> PRT
<213> Homo sapiens

<220>
<221> SITE
<222> (59)
<223> Xaa equals stop translation

<400> 142
Met Val Ile Ser Ile Phe Phe Ser Leu Pro Phe Ser Thr Ser Ala Tyr
1               5                   10                  15

Thr Leu Ile Ala Pro Asn Ile Asn Arg Arg Asn Glu Ile Gln Arg Ile
            20                  25                  30

Ala Asp Arg Ser Trp Pro Thr Trp Arg Ser Gly Arg Ser Arg Thr Glu
        35                  40                  45

Leu Asn Arg Phe Thr Trp Cys Pro Asp Gly Xaa
    50                  55


<210> 143
<211> 68
<212> PRT
<213> Homo sapiens

<220>
<221> SITE
<222> (68)

<223> Xaa equals stop translation

<400> 143
Met Lys Gln His Gln Lys Leu Trp Arg Leu Gly Phe Leu Leu Cys Phe
1               5                   10                  15

Asn Leu Val Phe Cys Val Leu Gly Arg Arg His Pro Trp Pro Trp Ala
            20                  25                  30

Val Arg Pro Leu Met Cys Val Tyr Ala Asp Arg Glu Leu Leu Gly Trp
        35                  40                  45

Leu Leu Arg Trp Val Val Leu Leu Val Phe Ser Val Leu Lys Leu Ile
    50                  55                  60

Phe Arg Leu Xaa
65


<210> 144
<211> 177
<212> PRT
<213> Homo sapiens

<220>
<221> SITE
<222> (177)
<223> Xaa equals stop translation

<400> 144
Met Ala Ser Val Phe Val Cys Leu Leu Leu Ser Gly Leu Ala Val Phe
1               5                   10                  15

Phe Leu Phe Pro Arg Ser Ile Asp Val Lys Tyr Ile Gly Val Lys Ser
            20                  25                  30

Ala Tyr Val Ser Tyr Asp Val Gln Lys Arg Thr Ile Tyr Leu Asn Ile
        35                  40                  45

Thr Asn Thr Leu Asn Ile Thr Asn Asn Asn Tyr Tyr Ser Val Glu Val
    50                  55                  60

Glu Asn Ile Thr Ala Gln Val Gln Phe Ser Lys Thr Val Ile Gly Lys
65                  70                  75                  80

Ala Arg Leu Asn Asn Ile Ser Ile Ile Gly Pro Leu Asp Met Lys Gln
                85                  90                  95

Ile Asp Tyr Thr Val Pro Thr Val Ile Ala Glu Glu Met Ser Tyr Met
            100                 105                 110

Tyr Asp Phe Cys Thr Leu Ile Ser Ile Lys Val His Asn Ile Val Leu
        115                 120                 125

Met Met Gln Val Thr Val Thr Thr Thr Tyr Phe Gly His Ser Glu Gln
    130                 135                 140

Ile Ser Gln Glu Arg Tyr Gln Tyr Val Asp Cys Gly Arg Asn Thr Thr

145                 150                 155                 160

Tyr Gln Leu Gly Gln Ser Glu Tyr Leu Asn Val Leu Gln Pro Gln Gln
                165                 170                 175

Xaa

<210> 145
<211> 120
<212> PRT
<213> Homo sapiens

<220>
<221> SITE
<222> (120)
<223> Xaa equals stop translation

<400> 145
Met Arg Arg Leu Leu Leu Val Thr Ser Leu Val Val Val Leu Leu Trp
  1               5                  10                  15

Glu Ala Gly Ala Val Pro Ala Pro Lys Val Pro Ile Lys Met Gln Val
             20                  25                  30

Lys His Trp Pro Ser Glu Gln Asp Pro Glu Lys Ala Trp Gly Ala Arg
         35                  40                  45

Val Val Glu Pro Pro Glu Lys Asp Asp Gln Leu Val Val Leu Phe Pro
     50                  55                  60

Val Gln Lys Pro Lys Leu Leu Thr Thr Glu Glu Lys Pro Arg Gly Thr
 65                  70                  75                  80

Lys Ala Trp Met Glu Thr Glu Asp Thr Leu Gly Arg Val Leu Ser Pro
                 85                  90                  95

Glu Pro Asp His Asp Ser Leu Tyr His Pro Pro Pro Glu Glu Asp Gln
            100                 105                 110

Gly Glu Glu Arg Pro Arg Leu Xaa
        115                 120

<210> 146
<211> 265
<212> PRT
<213> Homo sapiens

<220>
<221> SITE
<222> (265)
<223> Xaa equals stop translation

<400> 146
Met Pro Phe Arg Leu Leu Ile Pro Leu Gly Leu Leu Cys Ala Leu Leu
  1               5                  10                  15

Pro Gln His His Gly Ala Pro Gly Pro Asp Gly Ser Ala Pro Asp Pro
            20                  25                  30

Ala His Tyr Arg Glu Arg Val Lys Ala Met Phe Tyr His Ala Tyr Asp
        35                  40                  45

Ser Tyr Leu Glu Asn Ala Phe Pro Phe Asp Glu Leu Arg Pro Leu Thr
    50                  55                  60

Cys Asp Gly His Asp Thr Trp Gly Ser Phe Ser Leu Thr Leu Ile Asp
65                  70                  75                  80

Ala Leu Asp Thr Leu Leu Ile Leu Gly Asn Val Ser Glu Phe Gln Arg
                85                  90                  95

Val Val Glu Val Leu Gln Asp Ser Val Asp Phe Asp Ile Asp Val Asn
            100                 105                 110

Ala Ser Val Phe Glu Thr Asn Ile Arg Val Val Gly Gly Leu Leu Ser
        115                 120                 125

Ala His Leu Leu Ser Lys Lys Ala Gly Val Glu Val Glu Ala Gly Trp
    130                 135                 140

Pro Cys Ser Gly Pro Leu Leu Arg Met Ala Glu Glu Ala Ala Arg Lys
145                 150                 155                 160

Leu Leu Pro Ala Phe Gln Thr Pro Thr Gly Met Pro Tyr Gly Thr Val
                165                 170                 175

Asn Leu Leu His Gly Val Asn Pro Gly Glu Thr Pro Val Thr Cys Thr
            180                 185                 190

Ala Gly Ile Gly Thr Phe Ile Val Glu Phe Ala Thr Leu Ser Ser Leu
        195                 200                 205

Thr Gly Asp Pro Val Phe Glu Asp Val Ala Arg Val Ala Leu Met Arg
    210                 215                 220

Leu Trp Glu Ser Arg Ser Asp Ile Gly Leu Val Gly Asn His Ile Asp
225                 230                 235                 240

Val Leu Thr Gly Lys Gly Trp Pro Arg Thr Gln Ala Ser Gly Leu Ala
                245                 250                 255

Trp Thr Pro Thr Leu Ser Thr Trp Xaa
            260                 265

<210> 147
<211> 21
<212> PRT
<213> Homo sapiens

<400> 147
Gly Ser Phe Leu Gly Ser Thr Asn Arg Asp Arg Glu Ser Leu Ala Phe
1               5                   10                  15

Gln Phe Cys Ala Gly
            20

<210> 148
<211> 19
<212> PRT
<213> Homo sapiens

<400> 148
His Glu Val Glu Glu Lys Phe Asn Ser Pro Leu Met Gln Thr Glu Gly
 1               5                  10                  15

Asp Ile Gln

<210> 149
<211> 423
<212> PRT
<213> Homo sapiens

<220>
<221> SITE
<222> (193)
<223> Xaa equals any of the naturally occurring L-amino acids

<220>
<221> SITE
<222> (215)
<223> Xaa equals any of the naturally occurring L-amino acids

<220>
<221> SITE
<222> (242)
<223> Xaa equals any of the naturally occurring L-amino acids

<220>
<221> SITE
<222> (361)
<223> Xaa equals any of the naturally occurring L-amino acids

<220>
<221> SITE
<222> (378)
<223> Xaa equals any of the naturally occurring L-amino acids

<400> 149
Ile Asn Phe Ser Glu Met Thr Leu Gln Glu Leu Val His Lys Ala Ala
 1               5                  10                  15

Ser Cys Tyr Met Asp Arg Val Ala Val Cys Phe Asp Glu Cys Asn Asn
            20                  25                  30

Gln Leu Pro Val Tyr Tyr Thr Tyr Lys Thr Val Val Asn Ala Ala Ser
        35                  40                  45

Glu Leu Ser Asn Phe Leu Leu Leu His Cys Asp Phe Gln Gly Ile Arg
    50                  55                  60

Glu Ile Gly Leu Tyr Cys Gln Pro Gly Ile Asp Leu Pro Ser Trp Ile
65                  70                  75                  80

Leu Gly Ile Leu Gln Val Pro Ala Ala Tyr Val Pro Ile Glu Pro Asp
                85                  90                  95

Ser Pro Pro Ser Leu Ser Thr His Phe Met Lys Lys Cys Asn Leu Lys
            100                 105                 110

Tyr Ile Leu Val Glu Lys Lys Gln Ile Asn Lys Phe Lys Ser Phe His
        115                 120                 125

Glu Thr Leu Leu Asn Tyr Asp Thr Phe Thr Val Glu His Asn Asp Leu
    130                 135                 140

Val Leu Phe Arg Leu His Trp Lys Asn Thr Glu Val Asn Leu Met Leu
145                 150                 155                 160

Asn Asp Gly Lys Glu Lys Tyr Glu Lys Glu Lys Ile Lys Ser Ile Ser
                165                 170                 175

Ser Glu His Val Asn Glu Glu Lys Ala Glu Glu His Met Asp Leu Arg
            180                 185                 190

Xaa Lys His Cys Leu Ala Tyr Val Leu His Thr Ser Gly Thr Thr Gly
        195                 200                 205

Ile Pro Lys Ile Val Arg Xaa Pro His Lys Cys Ile Val Pro Asn Ile
    210                 215                 220

Gln His Phe Arg Val Leu Phe Asp Ile Thr Gln Glu Asp Val Leu Phe
225                 230                 235                 240

Leu Xaa Ser Pro Leu Thr Phe Asp Pro Ser Val Val Glu Ile Phe Leu
                245                 250                 255

Ala Leu Ser Ser Gly Ala Ser Leu Leu Ile Val Pro Thr Ser Val Lys
            260                 265                 270

Leu Leu Pro Ser Lys Leu Ala Ser Val Leu Phe Ser His His Arg Val
        275                 280                 285

Thr Val Leu Gln Ala Thr Pro Thr Leu Leu Arg Arg Phe Gly Ser Gln
    290                 295                 300

Leu Ile Lys Ser Thr Val Leu Ser Ala Thr Thr Ser Leu Arg Val Leu
305                 310                 315                 320

Ala Leu Gly Gly Glu Ala Phe Pro Ser Leu Thr Val Leu Arg Ser Trp
                325                 330                 335

Arg Gly Glu Gly Asn Lys Thr Gln Ile Phe Asn Val Tyr Gly Ile Thr
            340                 345                 350

Glu Val Ser Ser Trp Ala Thr Ile Xaa Arg Ile Pro Glu Lys Thr Leu

355                 360                 365

Asn Ser Thr Leu Lys Cys Glu Leu Pro Xaa Gln Leu Gly Phe Pro Leu
    370                 375                 380

Leu Gly Thr Val Val Glu Val Arg Asp Thr Asn Gly Phe Thr Ile Gln
385                 390                 395                 400

Glu Gly Ser Gly Gln Val Phe Leu Gly Cys Phe Ile Phe Val Asp Trp
                405                 410                 415

Glu Phe Phe Phe Gln Glu Lys
            420

<210> 150
<211> 44
<212> PRT
<213> Homo sapiens

<400> 150
Ile Asn Phe Ser Glu Met Thr Leu Gln Glu Leu Val His Lys Ala Ala
 1               5                  10                  15

Ser Cys Tyr Met Asp Arg Val Ala Val Cys Phe Asp Glu Cys Asn Asn
            20                  25                  30

Gln Leu Pro Val Tyr Tyr Thr Tyr Lys Thr Val Val
        35                  40

<210> 151
<211> 47
<212> PRT
<213> Homo sapiens

<400> 151
Asn Ala Ala Ser Glu Leu Ser Asn Phe Leu Leu Leu His Cys Asp Phe
 1               5                  10                  15

Gln Gly Ile Arg Glu Ile Gly Leu Tyr Cys Gln Pro Gly Ile Asp Leu
            20                  25                  30

Pro Ser Trp Ile Leu Gly Ile Leu Gln Val Pro Ala Ala Tyr Val
        35                  40                  45

<210> 152
<211> 46
<212> PRT
<213> Homo sapiens

<400> 152
Pro Ile Glu Pro Asp Ser Pro Pro Ser Leu Ser Thr His Phe Met Lys
 1               5                  10                  15

Lys Cys Asn Leu Lys Tyr Ile Leu Val Glu Lys Lys Gln Ile Asn Lys
            20                  25                  30

Phe Lys Ser Phe His Glu Thr Leu Leu Asn Tyr Asp Thr Phe
        35                  40                  45

<210> 153
<211> 47
<212> PRT
<213> Homo sapiens

<400> 153
Thr Val Glu His Asn Asp Leu Val Leu Phe Arg Leu His Trp Lys Asn
 1               5                  10                  15

Thr Glu Val Asn Leu Met Leu Asn Asp Gly Lys Glu Lys Tyr Glu Lys
            20                  25                  30

Glu Lys Ile Lys Ser Ile Ser Ser Glu His Val Asn Glu Glu Lys
        35                  40                  45

<210> 154
<211> 46
<212> PRT
<213> Homo sapiens

<220>
<221> SITE
<222> (9)
<223> Xaa equals any of the naturally occurring L-amino acids

<220>
<221> SITE
<222> (31)
<223> Xaa equals any of the naturally occurring L-amino acids

<400> 154
Ala Glu Glu His Met Asp Leu Arg Xaa Lys His Cys Leu Ala Tyr Val
 1               5                  10                  15

Leu His Thr Ser Gly Thr Thr Gly Ile Pro Lys Ile Val Arg Xaa Pro
            20                  25                  30

His Lys Cys Ile Val Pro Asn Ile Gln His Phe Arg Val Leu
        35                  40                  45

<210> 155
<211> 48
<212> PRT
<213> Homo sapiens

<220>
<221> SITE
<222> (12)
<223> Xaa equals any of the naturally occurring L-amino acids

<400> 155

Phe Asp Ile Thr Gln Glu Asp Val Leu Phe Leu Xaa Ser Pro Leu Thr
1               5                   10                  15

Phe Asp Pro Ser Val Val Glu Ile Phe Leu Ala Leu Ser Ser Gly Ala
            20                  25                  30

Ser Leu Leu Ile Val Pro Thr Ser Val Lys Leu Leu Pro Ser Lys Leu
        35                  40                  45

<210> 156
<211> 46
<212> PRT
<213> Homo sapiens

<400> 156
Ala Ser Val Leu Phe Ser His His Arg Val Thr Val Leu Gln Ala Thr
1               5                   10                  15

Pro Thr Leu Leu Arg Arg Phe Gly Ser Gln Leu Ile Lys Ser Thr Val
            20                  25                  30

Leu Ser Ala Thr Thr Ser Leu Arg Val Leu Ala Leu Gly Gly
        35                  40                  45

<210> 157
<211> 47
<212> PRT
<213> Homo sapiens

<220>
<221> SITE
<222> (37)
<223> Xaa equals any of the naturally occurring L-amino acids

<400> 157
Glu Ala Phe Pro Ser Leu Thr Val Leu Arg Ser Trp Arg Gly Glu Gly
1               5                   10                  15

Asn Lys Thr Gln Ile Phe Asn Val Tyr Gly Ile Thr Glu Val Ser Ser
            20                  25                  30

Trp Ala Thr Ile Xaa Arg Ile Pro Glu Lys Thr Leu Asn Ser Thr
        35                  40                  45

<210> 158
<211> 52
<212> PRT
<213> Homo sapiens

<220>
<221> SITE
<222> (7)

<223> Xaa equals any of the naturally occurring L-amino acids

<400> 158
Leu Lys Cys Glu Leu Pro Xaa Gln Leu Gly Phe Pro Leu Leu Gly Thr
1               5                   10                  15

Val Val Glu Val Arg Asp Thr Asn Gly Phe Thr Ile Gln Glu Gly Ser
            20                  25                  30

Gly Gln Val Phe Leu Gly Cys Phe Ile Phe Val Asp Trp Glu Phe Phe
        35                  40                  45

Phe Gln Glu Lys
    50


<210> 159
<211> 43
<212> PRT
<213> Homo sapiens

<400> 159
Glu Ala Lys Ala Gln Phe Trp Leu Leu His Ser Tyr Leu Phe Cys His
1               5                   10                  15

Ser Ser Asn Val Pro Asp Leu Leu Arg Pro Arg Met Thr Asn Asp Ser
            20                  25                  30

Glu Gly Lys Met Gly Phe Lys His Pro Lys Ile
        35                  40


<210> 160
<211> 40
<212> PRT
<213> Homo sapiens

<400> 160
Gly Thr Ser Gly Asp Gly Ala Lys Met Ile Ser Gly His Leu Leu Gln
1               5                   10                  15

Glu Pro Thr Gly Ser Pro Val Val Ser Glu Glu Pro Leu Asp Leu Leu
            20                  25                  30

Pro Thr Leu Asp Leu Arg Gln Glu
        35                  40


<210> 161
<211> 396
<212> PRT
<213> Homo sapiens

<220>
<221> SITE
<222> (6)
<223> Xaa equals any of the naturally occurring L-amino acids

<220>
<221> SITE
<222> (56)
<223> Xaa equals any of the naturally occurring L-amino acids

<220>
<221> SITE
<222> (67)
<223> Xaa equals any of the naturally occurring L-amino acids

<220>
<221> SITE
<222> (113)
<223> Xaa equals any of the naturally occurring L-amino acids

<220>
<221> SITE
<222> (130)
<223> Xaa equals any of the naturally occurring L-amino acids

<220>
<221> SITE
<222> (137)
<223> Xaa equals any of the naturally occurring L-amino acids

<220>
<221> SITE
<222> (139)
<223> Xaa equals any of the naturally occurring L-amino acids

<220>
<221> SITE
<222> (211)
<223> Xaa equals any of the naturally occurring L-amino acids

<220>
<221> SITE
<222> (222)
<223> Xaa equals any of the naturally occurring L-amino acids

<220>
<221> SITE
<222> (224)
<223> Xaa equals any of the naturally occurring L-amino acids

<220>
<221> SITE
<222> (227)
<223> Xaa equals any of the naturally occurring L-amino acids

<220>
<221> SITE
<222> (280)
<223> Xaa equals any of the naturally occurring L-amino acids

<400> 161
Leu Thr Thr Glu Glu Xaa Cys Met Leu Gly Ser Ala Leu Cys Pro Phe
1               5                   10                  15

Gln Gly Asn Phe Thr Ile Ile Leu Tyr Gly Arg Ala Asp Glu Gly Ile
            20                  25                  30

Gln Pro Asp Pro Tyr Tyr Gly Leu Lys Tyr Ile Gly Val Gly Lys Gly
        35                  40                  45

Gly Ala Leu Glu Leu His Gly Xaa Lys Lys Leu Ser Trp Thr Phe Leu
    50                  55                  60

Asn Lys Xaa Leu His Pro Gly Gly Met Ala Glu Gly Gly Tyr Phe Phe
65                  70                  75                  80

Glu Arg Ser Trp Gly His Arg Gly Val Ile Val His Val Ile Asp Pro
                85                  90                  95

Lys Ser Gly Thr Val Ile His Ser Asp Arg Phe Asp Thr Tyr Arg Ser
            100                 105                 110

Xaa Lys Glu Ser Glu Arg Leu Val Gln Tyr Leu Asn Ala Val Pro Asp
        115                 120                 125

Gly Xaa Ile Leu Ser Val Ala Val Xaa Asp Xaa Gly Ser Arg Asn Leu
    130                 135                 140

Asp Asp Met Ala Arg Lys Ala Met Thr Lys Leu Gly Ser Lys His Phe
145                 150                 155                 160

Leu His Leu Gly Phe Arg His Pro Trp Ser Phe Leu Thr Val Lys Gly
                165                 170                 175

Asn Pro Ser Ser Ser Val Glu Asp His Ile Glu Tyr His Gly His Arg
            180                 185                 190

Gly Ser Ala Ala Ala Arg Val Phe Lys Leu Phe Gln Thr Glu His Gly
        195                 200                 205

Glu Tyr Xaa Asn Val Ser Leu Ser Ser Glu Trp Val Gln Xaa Val Xaa
    210                 215                 220

Trp Thr Xaa Trp Phe Asp His Asp Lys Val Ser Gln Thr Lys Gly Gly
225                 230                 235                 240

Glu Lys Ile Ser Asp Leu Trp Lys Ala His Pro Gly Lys Ile Cys Asn
                245                 250                 255

Arg Pro Ile Asp Ile Gln Ala Thr Thr Met Asp Gly Val Asn Leu Ser
            260                 265                 270

Thr Glu Val Val Tyr Lys Lys Xaa Gln Asp Tyr Arg Phe Ala Cys Tyr
        275                 280                 285

Asp Arg Gly Arg Ala Cys Arg Ser Tyr Arg Val Arg Phe Leu Cys Gly
    290                 295                 300

Lys Pro Val Arg Pro Lys Leu Thr Val Thr Ile Asp Thr Asn Val Asn
305                 310                 315                 320

Ser Thr Ile Leu Asn Leu Glu Asp Asn Val Gln Ser Trp Lys Pro Gly
                325                 330                 335

Asp Thr Leu Val Ile Ala Ser Thr Asp Tyr Ser Met Tyr Gln Ala Glu
            340                 345                 350

Glu Phe Gln Val Leu Pro Cys Arg Ser Cys Ala Pro Asn Gln Val Lys
        355                 360                 365

Val Ala Gly Lys Pro Met Tyr Leu His Ile Gly Gly Arg Arg Gly Arg
    370                 375                 380

Glu Ser Arg Val Asp Glu Leu Thr Ser Arg Arg Pro
385                 390                 395

<210> 162
<211> 44
<212> PRT
<213> Homo sapiens

<220>
<221> SITE
<222> (6)
<223> Xaa equals any of the naturally occurring L-amino acids

<400> 162
Leu Thr Thr Glu Glu Xaa Cys Met Leu Gly Ser Ala Leu Cys Pro Phe
  1               5                  10                  15

Gln Gly Asn Phe Thr Ile Ile Leu Tyr Gly Arg Ala Asp Glu Gly Ile
             20                  25                  30

Gln Pro Asp Pro Tyr Tyr Gly Leu Lys Tyr Ile Gly
         35                  40

<210> 163
<211> 42
<212> PRT
<213> Homo sapiens

<220>
<221> SITE
<222> (12)
<223> Xaa equals any of the naturally occurring L-amino acids

<220>
<221> SITE
<222> (23)
<223> Xaa equals any of the naturally occurring L-amino acids

<400> 163
Val Gly Lys Gly Gly Ala Leu Glu Leu His Gly Xaa Lys Lys Leu Ser
  1               5                  10                  15

Trp Thr Phe Leu Asn Lys Xaa Leu His Pro Gly Gly Met Ala Glu Gly
             20                  25                  30

Gly Tyr Phe Phe Glu Arg Ser Trp Gly His
        35                  40

<210> 164
<211> 46
<212> PRT
<213> Homo sapiens

<220>
<221> SITE
<222> (27)
<223> Xaa equals any of the naturally occurring L-amino acids

<220>
<221> SITE
<222> (44)
<223> Xaa equals any of the naturally occurring L-amino acids

<400> 164
Arg Gly Val Ile Val His Val Ile Asp Pro Lys Ser Gly Thr Val Ile
  1               5                  10                  15

His Ser Asp Arg Phe Asp Thr Tyr Arg Ser Xaa Lys Glu Ser Glu Arg
            20                  25                  30

Leu Val Gln Tyr Leu Asn Ala Val Pro Asp Gly Xaa Ile Leu
        35                  40                  45

<210> 165
<211> 41
<212> PRT
<213> Homo sapiens

<220>
<221> SITE
<222> (5)
<223> Xaa equals any of the naturally occurring L-amino acids

<220>
<221> SITE
<222> (7)
<223> Xaa equals any of the naturally occurring L-amino acids

<400> 165
Ser Val Ala Val Xaa Asp Xaa Gly Ser Arg Asn Leu Asp Asp Met Ala
  1               5                  10                  15

Arg Lys Ala Met Thr Lys Leu Gly Ser Lys His Phe Leu His Leu Gly
            20                  25                  30

Phe Arg His Pro Trp Ser Phe Leu Thr
        35                  40

<210> 166

<211> 44
<212> PRT
<213> Homo sapiens

<220>
<221> SITE
<222> (38)
<223> Xaa equals any of the naturally occurring L-amino acids

<400> 166
Val Lys Gly Asn Pro Ser Ser Ser Val Glu Asp His Ile Glu Tyr His
1               5                   10                  15

Gly His Arg Gly Ser Ala Ala Ala Arg Val Phe Lys Leu Phe Gln Thr
            20                  25                  30

Glu His Gly Glu Tyr Xaa Asn Val Ser Leu Ser Ser
        35                  40


<210> 167
<211> 43
<212> PRT
<213> Homo sapiens

<220>
<221> SITE
<222> (5)
<223> Xaa equals any of the naturally occurring L-amino acids

<220>
<221> SITE
<222> (7)
<223> Xaa equals any of the naturally occurring L-amino acids

<220>
<221> SITE
<222> (10)
<223> Xaa equals any of the naturally occurring L-amino acids

<400> 167
Glu Trp Val Gln Xaa Val Xaa Trp Thr Xaa Trp Phe Asp His Asp Lys
1               5                   10                  15

Val Ser Gln Thr Lys Gly Gly Glu Lys Ile Ser Asp Leu Trp Lys Ala
            20                  25                  30

His Pro Gly Lys Ile Cys Asn Arg Pro Ile Asp
        35                  40


<210> 168
<211> 43
<212> PRT
<213> Homo sapiens

<220>
<221> SITE

<222> (20)
<223> Xaa equals any of the naturally occurring L-amino acids

<400> 168
Ile Gln Ala Thr Thr Met Asp Gly Val Asn Leu Ser Thr Glu Val Val
1               5                   10                  15

Tyr Lys Lys Xaa Gln Asp Tyr Arg Phe Ala Cys Tyr Asp Arg Gly Arg
            20                  25                  30

Ala Cys Arg Ser Tyr Arg Val Arg Phe Leu Cys
        35                  40


<210> 169
<211> 45
<212> PRT
<213> Homo sapiens

<400> 169
Gly Lys Pro Val Arg Pro Lys Leu Thr Val Thr Ile Asp Thr Asn Val
1               5                   10                  15

Asn Ser Thr Ile Leu Asn Leu Glu Asp Asn Val Gln Ser Trp Lys Pro
            20                  25                  30

Gly Asp Thr Leu Val Ile Ala Ser Thr Asp Tyr Ser Met
        35                  40                  45


<210> 170
<211> 48
<212> PRT
<213> Homo sapiens

<400> 170
Tyr Gln Ala Glu Glu Phe Gln Val Leu Pro Cys Arg Ser Cys Ala Pro
1               5                   10                  15

Asn Gln Val Lys Val Ala Gly Lys Pro Met Tyr Leu His Ile Gly Gly
            20                  25                  30

Arg Arg Gly Arg Glu Ser Arg Val Asp Glu Leu Thr Ser Arg Arg Pro
        35                  40                  45


<210> 171
<211> 24
<212> PRT
<213> Homo sapiens

<400> 171
Gly Thr Arg Asn Gly Trp Val Phe Phe Lys Gln Leu Leu Pro Gln His
1               5                   10                  15

Phe Asp Ile Arg Tyr Ala Asn Leu
            20

<210> 172
<211> 39
<212> PRT
<213> Homo sapiens

<400> 172
Gly Glu Val Glu Ala Gly Gln Gly Lys Arg Arg Val Ser Leu Gly Glu
  1               5                  10                  15

Ser Thr Leu Gly Pro Pro Cys Arg Gly Thr Pro Ser Thr Leu Arg Pro
            20                  25                  30

Ala Ala Gln Gln Ala Arg Arg
        35

<210> 173
<211> 25
<212> PRT
<213> Homo sapiens

<400> 173
Gln Ser Lys Thr Pro Asp Pro Val Ser Lys Lys Lys Phe Pro Ser Ser
  1               5                  10                  15

Gln Gly Val Val Glu Ala Glu Ser Val
            20                  25

<210> 174
<211> 348
<212> PRT
<213> Homo sapiens

<220>
<221> SITE
<222> (309)
<223> Xaa equals any of the naturally occurring L-amino acids

<220>
<221> SITE
<222> (341)
<223> Xaa equals any of the naturally occurring L-amino acids

<400> 174
Cys Phe Cys Phe Leu Leu Pro Leu Leu Pro Ser Arg Trp Glu Pro Ser
  1               5                  10                  15

Arg Arg Glu Gly Gly Gly Glu Met Ile Ala Glu Leu Val Ser Ser Ala
            20                  25                  30

Leu Gly Leu Ala Leu Tyr Leu Asn Thr Leu Ser Ala Asp Phe Cys Tyr
        35                  40                  45

Asp Asp Ser Arg Ala Ile Lys Thr Asn Gln Asp Leu Leu Pro Glu Thr
        50                  55                  60

Pro Trp Thr His Ile Phe Tyr Asn Asp Phe Trp Gly Thr Leu Leu Thr
65                  70                  75                  80

His Ser Gly Ser His Lys Ser Tyr Arg Pro Leu Cys Thr Leu Ser Phe
                85                  90                  95

Arg Leu Asn His Ala Ile Gly Gly Leu Asn Pro Trp Ser Tyr His Leu
            100                 105                 110

Val Asn Val Leu Leu His Ala Ala Val Thr Gly Leu Phe Thr Ser Phe
        115                 120                 125

Ser Lys Ile Leu Leu Gly Asp Gly Tyr Trp Thr Phe Met Ala Gly Leu
    130                 135                 140

Met Phe Ala Ser His Pro Ile His Thr Glu Ala Val Ala Gly Ile Val
145                 150                 155                 160

Gly Arg Ala Asp Val Gly Ala Ser Leu Phe Phe Leu Leu Ser Leu Leu
                165                 170                 175

Cys Tyr Ile Lys His Cys Ser Thr Arg Gly Tyr Ser Ala Arg Thr Trp
            180                 185                 190

Gly Trp Phe Leu Gly Ser Gly Leu Cys Ala Gly Cys Ser Met Leu Trp
        195                 200                 205

Lys Glu Gln Gly Val Thr Val Leu Ala Val Ser Ala Val Tyr Asp Val
    210                 215                 220

Phe Val Phe His Arg Leu Lys Ile Lys Gln Ile Leu Pro Thr Ile Tyr
225                 230                 235                 240

Lys Arg Lys Asn Leu Ser Leu Phe Leu Ser Ile Ser Leu Leu Ile Phe
                245                 250                 255

Trp Gly Ser Ser Leu Leu Gly Ala Arg Leu Tyr Trp Met Gly Asn Lys
            260                 265                 270

Pro Pro Ser Phe Ser Asn Ser Asp Asn Pro Ala Ala Asp Ser Asp Ser
        275                 280                 285

Leu Leu Thr Arg Thr Leu Thr Phe Phe Tyr Leu Pro Thr Lys Asn Leu
    290                 295                 300

Trp Leu Leu Leu Xaa Pro Asp Thr Leu Ser Phe Glu Trp Ser Met Asp
305                 310                 315                 320

Ala Val Pro Leu Leu Lys Thr Val Cys Asp Trp Arg Asn Leu His Thr
                325                 330                 335

Val Gly Leu Leu Xaa Trp Asp Ser Phe Ser Leu Ala
            340                 345

<210> 175
<211> 43
<212> PRT
<213> Homo sapiens

<400> 175
Cys Phe Cys Phe Leu Leu Pro Leu Leu Pro Ser Arg Trp Glu Pro Ser
1               5                   10                  15

Arg Arg Glu Gly Gly Gly Glu Met Ile Ala Glu Leu Val Ser Ser Ala
            20                  25                  30

Leu Gly Leu Ala Leu Tyr Leu Asn Thr Leu Ser
        35                  40


<210> 176
<211> 44
<212> PRT
<213> Homo sapiens

<400> 176
Ala Asp Phe Cys Tyr Asp Asp Ser Arg Ala Ile Lys Thr Asn Gln Asp
1               5                   10                  15

Leu Leu Pro Glu Thr Pro Trp Thr His Ile Phe Tyr Asn Asp Phe Trp
            20                  25                  30

Gly Thr Leu Leu Thr His Ser Gly Ser His Lys Ser
        35                  40


<210> 177
<211> 43
<212> PRT
<213> Homo sapiens

<400> 177
Tyr Arg Pro Leu Cys Thr Leu Ser Phe Arg Leu Asn His Ala Ile Gly
1               5                   10                  15

Gly Leu Asn Pro Trp Ser Tyr His Leu Val Asn Val Leu Leu His Ala
            20                  25                  30

Ala Val Thr Gly Leu Phe Thr Ser Phe Ser Lys
        35                  40


<210> 178
<211> 44
<212> PRT
<213> Homo sapiens

<400> 178
Ile Leu Leu Gly Asp Gly Tyr Trp Thr Phe Met Ala Gly Leu Met Phe
1               5                   10                  15

Ala Ser His Pro Ile His Thr Glu Ala Val Ala Gly Ile Val Gly Arg

20                  25                  30

Ala Asp Val Gly Ala Ser Leu Phe Phe Leu Leu Ser
        35                  40


<210> 179
<211> 43
<212> PRT
<213> Homo sapiens

<400> 179
Leu Leu Cys Tyr Ile Lys His Cys Ser Thr Arg Gly Tyr Ser Ala Arg
1               5                   10                  15

Thr Trp Gly Trp Phe Leu Gly Ser Gly Leu Cys Ala Gly Cys Ser Met
            20                  25                  30

Leu Trp Lys Glu Gln Gly Val Thr Val Leu Ala
        35                  40


<210> 180
<211> 47
<212> PRT
<213> Homo sapiens

<400> 180
Val Ser Ala Val Tyr Asp Val Phe Val Phe His Arg Leu Lys Ile Lys
1               5                   10                  15

Gln Ile Leu Pro Thr Ile Tyr Lys Arg Lys Asn Leu Ser Leu Phe Leu
            20                  25                  30

Ser Ile Ser Leu Leu Ile Phe Trp Gly Ser Ser Leu Leu Gly Ala
        35                  40                  45


<210> 181
<211> 43
<212> PRT
<213> Homo sapiens

<400> 181
Arg Leu Tyr Trp Met Gly Asn Lys Pro Pro Ser Phe Ser Asn Ser Asp
1               5                   10                  15

Asn Pro Ala Ala Asp Ser Asp Ser Leu Leu Thr Arg Thr Leu Thr Phe
            20                  25                  30

Phe Tyr Leu Pro Thr Lys Asn Leu Trp Leu Leu
        35                  40


<210> 182
<211> 41
<212> PRT
<213> Homo sapiens

<220>
<221> SITE
<222> (2)
<223> Xaa equals any of the naturally occurring L-amino acids

<220>
<221> SITE
<222> (34)
<223> Xaa equals any of the naturally occurring L-amino acids

<400> 182
Leu Xaa Pro Asp Thr Leu Ser Phe Glu Trp Ser Met Asp Ala Val Pro
1               5                   10                  15

Leu Leu Lys Thr Val Cys Asp Trp Arg Asn Leu His Thr Val Gly Leu
            20                  25                  30

Leu Xaa Trp Asp Ser Phe Ser Leu Ala
        35                  40


<210> 183
<211> 24
<212> PRT
<213> Homo sapiens

<400> 183
His Asn Val Phe Lys Val Tyr Ser Cys Cys Ser Lys Val Arg Asn Cys
1               5                   10                  15

Phe Ser Phe Lys Glu Lys Val Ser
            20


<210> 184
<211> 11
<212> PRT
<213> Homo sapiens

<400> 184
Asn Cys Met His Gly Lys Ile Thr Pro Phe Gln
1               5                   10


<210> 185
<211> 40
<212> PRT
<213> Homo sapiens

<400> 185
Glu Gln Ile Pro Lys Lys Val Gln Lys Ser Leu Gln Glu Thr Ile Gln
1               5                   10                  15

Ser Leu Lys Leu Thr Asn Gln Glu Leu Leu Arg Lys Gly Ser Ser Asn
            20                  25                  30

Asn Gln Asp Val Val Ser Cys Asp

35                  40

<210> 186
<211> 23
<212> PRT
<213> Homo sapiens

<400> 186
Gly Thr Ser Phe Cys Ser His Leu Pro Ser Gln Arg Pro Leu His Leu
1               5                   10                  15

Ser Gly Ser Ser Cys Leu Val
            20

<210> 187
<211> 58
<212> PRT
<213> Homo sapiens

<400> 187
Phe Cys Ile Gln Val Pro Gly Phe Val Ser Cys Trp Tyr Ala Ser Pro
1               5                   10                  15

Asp Arg Pro Ser Cys Ile His Val Thr Arg Leu Tyr Leu Leu Gly Leu
            20                  25                  30

Ser Gln Ile Leu Ala Ser Tyr Ser Ser Ser Cys Pro Asn Ser Ile Leu
        35                  40                  45

Ser Leu Arg Asn Gly Gly Lys Ile Leu Arg
    50                  55

<210> 188
<211> 40
<212> PRT
<213> Homo sapiens

<400> 188
Pro Arg Val Arg Ser Ala Ala Arg Leu Pro Arg Thr Leu Arg Pro Ser
1               5                   10                  15

Arg Thr Ser Ala Pro Ala Gly Pro Cys Val Pro Arg Leu Ala Pro Leu
            20                  25                  30

Thr Pro Ser Arg Pro Gly Arg Ala
        35                  40

<210> 189
<211> 460
<212> PRT
<213> Homo sapiens

<220>
<221> SITE

<222> (236)
<223> Xaa equals any of the naturally occurring L-amino acids

<220>
<221> SITE
<222> (324)
<223> Xaa equals any of the naturally occurring L-amino acids

<400> 189
Ser Val Leu Trp Gly Gly Ser Lys Gly Pro Trp Ser Trp Pro Arg Pro
1               5                   10                  15

Arg His Arg Glu Arg Leu Asp Phe Leu Ser Leu Cys Ala Glu Trp Leu
            20                  25                  30

Arg Trp Arg Pro Leu Ser Leu Thr Gln Gln Leu Lys His Thr Ile Ser
        35                  40                  45

Gly Ser Asn Trp Leu Pro His Pro Leu Pro Cys Pro Leu Gly Ser Ala
    50                  55                  60

Glu Asn Asn Gly Asn Ala Asn Ile Leu Ile Ala Ala Asn Gly Thr Lys
65                  70                  75                  80

Arg Lys Ala Ile Ala Ala Glu Asp Pro Ser Leu Asp Phe Arg Asn Asn
                85                  90                  95

Pro Thr Lys Glu Asp Leu Gly Lys Leu Gln Pro Leu Val Ala Ser Tyr
            100                 105                 110

Leu Cys Ser Asp Val Thr Ser Val Pro Ser Lys Glu Ser Leu Lys Leu
        115                 120                 125

Gln Gly Val Phe Ser Lys Gln Thr Val Leu Lys Ser His Pro Leu Leu
    130                 135                 140

Ser Gln Ser Tyr Glu Leu Arg Ala Glu Leu Leu Gly Arg Gln Pro Val
145                 150                 155                 160

Leu Glu Phe Ser Leu Glu Asn Leu Arg Thr Met Asn Thr Ser Gly Gln
                165                 170                 175

Thr Ala Leu Pro Gln Ala Pro Val Asn Gly Leu Ala Lys Lys Leu Thr
            180                 185                 190

Lys Ser Ser Thr His Ser Asp His Asp Asn Ser Thr Ser Leu Asn Gly
        195                 200                 205

Gly Lys Arg Ala Leu Thr Ser Ser Ala Leu His Gly Gly Glu Met Gly
    210                 215                 220

Gly Ser Glu Ser Gly Asp Leu Lys Gly Gly Met Xaa Asn Cys Thr Leu
225                 230                 235                 240

Pro His Arg Ser Leu Asp Val Glu His Thr Ile Leu Tyr Ser Asn Asn
                245                 250                 255

Ser Thr Ala Asn Lys Ser Ser Val Asn Ser Met Glu Gln Pro Ala Leu

            260                 265                 270

Gln Gly Ser Ser Arg Leu Ser Pro Gly Thr Asp Ser Ser Ser Asn Leu
        275                 280                 285

Gly Gly Val Lys Leu Glu Gly Lys Lys Ser Pro Leu Ser Ser Ile Leu
    290                 295                 300

Phe Ser Ala Leu Asp Ser Asp Thr Arg Ile Thr Ala Leu Leu Arg Arg
305                 310                 315                 320

Gln Ala Asp Xaa Glu Ser Arg Ala Arg Arg Leu Gln Lys Arg Leu Gln
                325                 330                 335

Val Val Gln Ala Lys Gln Val Glu Arg His Ile Gln His Gln Leu Gly
            340                 345                 350

Gly Phe Leu Glu Lys Thr Leu Ser Lys Leu Pro Asn Leu Glu Ser Leu
        355                 360                 365

Arg Pro Arg Ser Gln Leu Met Leu Thr Arg Lys Ala Glu Ala Ala Leu
    370                 375                 380

Arg Lys Ala Ala Ser Glu Thr Thr Thr Ser Glu Gly Leu Ser Asn Phe
385                 390                 395                 400

Leu Lys Ser Asn Ser Ile Ser Glu Glu Leu Glu Arg Phe Thr Ala Ser
                405                 410                 415

Gly Ile Ala Asn Leu Arg Cys Ser Glu Gln Ala Phe Asp Ser Asp Val
            420                 425                 430

Thr Asp Ser Ser Ser Gly Gly Glu Ser Asp Ile Glu Glu Glu Glu Leu
        435                 440                 445

Thr Arg Ala Asp Pro Glu Gln Arg His Val Pro Leu
    450                 455                 460

<210> 190
<211> 43
<212> PRT
<213> Homo sapiens

<400> 190
Ser Val Leu Trp Gly Gly Ser Lys Gly Pro Trp Ser Trp Pro Arg Pro
1               5                   10                  15

Arg His Arg Glu Arg Leu Asp Phe Leu Ser Leu Cys Ala Glu Trp Leu
            20                  25                  30

Arg Trp Arg Pro Leu Ser Leu Thr Gln Gln Leu
        35                  40

<210> 191
<211> 45
<212> PRT

<213> Homo sapiens

<400> 191
Lys His Thr Ile Ser Gly Ser Asn Trp Leu Pro His Pro Leu Pro Cys
1               5                   10                  15

Pro Leu Gly Ser Ala Glu Asn Asn Gly Asn Ala Asn Ile Leu Ile Ala
            20                  25                  30

Ala Asn Gly Thr Lys Arg Lys Ala Ile Ala Ala Glu Asp
        35                  40                  45


<210> 192
<211> 45
<212> PRT
<213> Homo sapiens

<400> 192
Pro Ser Leu Asp Phe Arg Asn Asn Pro Thr Lys Glu Asp Leu Gly Lys
1               5                   10                  15

Leu Gln Pro Leu Val Ala Ser Tyr Leu Cys Ser Asp Val Thr Ser Val
            20                  25                  30

Pro Ser Lys Glu Ser Leu Lys Leu Gln Gly Val Phe Ser
        35                  40                  45


<210> 193
<211> 46
<212> PRT
<213> Homo sapiens

<400> 193
Lys Gln Thr Val Leu Lys Ser His Pro Leu Leu Ser Gln Ser Tyr Glu
1               5                   10                  15

Leu Arg Ala Glu Leu Leu Gly Arg Gln Pro Val Leu Glu Phe Ser Leu
            20                  25                  30

Glu Asn Leu Arg Thr Met Asn Thr Ser Gly Gln Thr Ala Leu
        35                  40                  45


<210> 194
<211> 44
<212> PRT
<213> Homo sapiens

<400> 194
Pro Gln Ala Pro Val Asn Gly Leu Ala Lys Lys Leu Thr Lys Ser Ser
1               5                   10                  15

Thr His Ser Asp His Asp Asn Ser Thr Ser Leu Asn Gly Gly Lys Arg
            20                  25                  30

Ala Leu Thr Ser Ser Ala Leu His Gly Gly Glu Met

35                  40

<210> 195
<211> 45
<212> PRT
<213> Homo sapiens

<220>
<221> SITE
<222> (13)
<223> Xaa equals any of the naturally occurring L-amino acids

<400> 195
Gly Gly Ser Glu Ser Gly Asp Leu Lys Gly Gly Met Xaa Asn Cys Thr
1               5                   10                  15

Leu Pro His Arg Ser Leu Asp Val Glu His Thr Ile Leu Tyr Ser Asn
            20                  25                  30

Asn Ser Thr Ala Asn Lys Ser Ser Val Asn Ser Met Glu
        35                  40                  45

<210> 196
<211> 47
<212> PRT
<213> Homo sapiens

<400> 196
Gln Pro Ala Leu Gln Gly Ser Ser Arg Leu Ser Pro Gly Thr Asp Ser
1               5                   10                  15

Ser Ser Asn Leu Gly Gly Val Lys Leu Glu Gly Lys Lys Ser Pro Leu
            20                  25                  30

Ser Ser Ile Leu Phe Ser Ala Leu Asp Ser Asp Thr Arg Ile Thr
        35                  40                  45

<210> 197
<211> 47
<212> PRT
<213> Homo sapiens

<220>
<221> SITE
<222> (9)
<223> Xaa equals any of the naturally occurring L-amino acids

<400> 197
Ala Leu Leu Arg Arg Gln Ala Asp Xaa Glu Ser Arg Ala Arg Arg Leu
1               5                   10                  15

Gln Lys Arg Leu Gln Val Val Gln Ala Lys Gln Val Glu Arg His Ile
            20                  25                  30

Gln His Gln Leu Gly Gly Phe Leu Glu Lys Thr Leu Ser Lys Leu

35                  40                  45

<210> 198
<211> 47
<212> PRT
<213> Homo sapiens

<400> 198
Pro Asn Leu Glu Ser Leu Arg Pro Arg Ser Gln Leu Met Leu Thr Arg
1               5                   10                  15

Lys Ala Glu Ala Ala Leu Arg Lys Ala Ala Ser Glu Thr Thr Thr Ser
            20                  25                  30

Glu Gly Leu Ser Asn Phe Leu Lys Ser Asn Ser Ile Ser Glu Glu
        35                  40                  45

<210> 199
<211> 51
<212> PRT
<213> Homo sapiens

<400> 199
Leu Glu Arg Phe Thr Ala Ser Gly Ile Ala Asn Leu Arg Cys Ser Glu
1               5                   10                  15

Gln Ala Phe Asp Ser Asp Val Thr Asp Ser Ser Ser Gly Gly Glu Ser
            20                  25                  30

Asp Ile Glu Glu Glu Glu Leu Thr Arg Ala Asp Pro Glu Gln Arg His
        35                  40                  45

Val Pro Leu
    50

<210> 200
<211> 16
<212> PRT
<213> Homo sapiens

<400> 200
Ala Lys Val Val Ser Trp Pro Ser Gln Glu Thr Cys Gly Ile Arg Thr
1               5                   10                  15

<210> 201
<211> 26
<212> PRT
<213> Homo sapiens

<400> 201
Leu Pro Ser Gly Thr Phe Leu Lys Arg Ser Phe Arg Ser Leu Pro Glu

1                 5                   10                  15

Leu Lys Asp Ala Val Leu Asp Gln Tyr Ser
            20                  25


<210> 202
<211> 21
<212> PRT
<213> Homo sapiens

<400> 202
Gly Thr Arg Arg Ala Glu Val Gly Ala Ala Thr Ala Leu Pro Val Arg
1               5                   10                  15

Trp Ala Ser Gly Glu
            20


<210> 203
<211> 48
<212> PRT
<213> Homo sapiens

<400> 203
Val Thr Gly Thr Gly Glu Glu Leu Asn Ser Asn Ser Ser Leu Trp Glu
1               5                   10                  15

Asn Ala Val Leu Ala Pro Pro Gly Val Ala Leu Ala Gly Cys Trp Ser
            20                  25                  30

Pro Arg Ser Ala Pro Ser Gly Leu Trp Gly Gln Gly Trp Val Ser Leu
        35                  40                  45


<210> 204
<211> 28
<212> PRT
<213> Homo sapiens

<400> 204
Ser Asn Ser Ser Leu Trp Glu Asn Ala Val Leu Ala Pro Pro Gly Val
1               5                   10                  15

Ala Leu Ala Gly Cys Trp Ser Pro Arg Ser Ala Pro
            20                  25


<210> 205
<211> 134
<212> PRT
<213> Homo sapiens

<220>
<221> SITE

<222> (56)
<223> Xaa equals any of the naturally occurring L-amino acids

<400> 205
Ile Pro Phe Gln Pro Met Ser Gly Arg Phe Lys Asp Arg Val Ser Trp
1               5                   10                  15

Asp Gly Asn Pro Glu Arg Tyr Asp Ala Ser Ile Leu Leu Trp Lys Leu
            20                  25                  30

Gln Phe Asp Asp Asn Gly Thr Tyr Thr Cys Gln Val Lys Asn Pro Pro
        35                  40                  45

Asp Val Asp Gly Val Ile Gly Xaa Ile Arg Leu Ser Val Val His Thr
    50                  55                  60

Val Arg Phe Ser Glu Ile His Phe Leu Ala Leu Ala Ile Gly Ser Ala
65                  70                  75                  80

Cys Ala Leu Met Ile Ile Ile Val Ile Val Val Val Leu Phe Gln His
                85                  90                  95

Tyr Arg Lys Lys Arg Trp Ala Glu Arg Ala His Lys Val Val Glu Ile
            100                 105                 110

Lys Ser Lys Glu Glu Glu Arg Leu Asn Gln Glu Lys Lys Val Ser Val
        115                 120                 125

Tyr Leu Glu Asp Thr Asp
    130


<210> 206
<211> 29
<212> PRT
<213> Homo sapiens

<400> 206
Arg Val Ser Trp Asp Gly Asn Pro Glu Arg Tyr Asp Ala Ser Ile Leu
1               5                   10                  15

Leu Trp Lys Leu Gln Phe Asp Asp Asn Gly Thr Tyr Thr
            20                  25


<210> 207
<211> 24
<212> PRT
<213> Homo sapiens

<220>
<221> SITE
<222> (9)
<223> Xaa equals any of the naturally occurring L-amino acids

<400> 207
Pro Asp Val Asp Gly Val Ile Gly Xaa Ile Arg Leu Ser Val Val His
1               5                   10                  15

Thr Val Arg Phe Ser Glu Ile His
            20

<210> 208
<211> 28
<212> PRT
<213> Homo sapiens

<400> 208
Met Ile Ile Ile Val Ile Val Val Val Leu Phe Gln His Tyr Arg Lys
1               5                   10                  15

Lys Arg Trp Ala Glu Arg Ala His Lys Val Val Glu
            20                  25

<210> 209
<211> 7
<212> PRT
<213> Homo sapiens

<400> 209
Pro Ala Arg Gly Ala Pro Arg
1               5

<210> 210
<211> 6
<212> PRT
<213> Homo sapiens

<400> 210
Ala Arg Val Tyr Phe Lys
1               5

<210> 211
<211> 7
<212> PRT
<213> Homo sapiens

<400> 211
Thr Lys Leu Phe His Asp Lys
1               5

<210> 212
<211> 161
<212> PRT
<213> Homo sapiens

<400> 212
Pro His Ile His Pro Cys Trp Lys Glu Gly Asp Thr Val Gly Phe Leu
1               5                   10                  15

Leu Asp Leu Asn Glu Lys Gln Met Ile Phe Phe Leu Asn Gly Asn Gln

20                  25                  30

Leu Pro Pro Glu Lys Gln Val Phe Ser Ser Thr Val Ser Gly Phe Phe
        35                  40                  45

Ala Ala Ala Ser Phe Met Ser Tyr Gln Gln Cys Glu Phe Asn Phe Gly
    50                  55                  60

Ala Lys Pro Phe Lys Tyr Pro Pro Ser Met Lys Phe Ser Thr Phe Asn
65                  70                  75                  80

Asp Tyr Ala Phe Leu Thr Ala Glu Glu Lys Ile Ile Leu Pro Arg His
                85                  90                  95

Arg Arg Leu Ala Leu Leu Lys Gln Val Ser Ile Arg Glu Asn Cys Cys
            100                 105                 110

Ser Leu Cys Cys Asp Glu Val Ala Asp Thr Gln Leu Lys Pro Cys Gly
        115                 120                 125

His Ser Asp Leu Cys Met Asp Cys Ala Leu Gln Leu Glu Thr Cys Pro
    130                 135                 140

Leu Cys Arg Lys Glu Ile Val Ser Arg Ile Arg Gln Ile Ser His Ile
145                 150                 155                 160

Ser

<210> 213
<211> 31
<212> PRT
<213> Homo sapiens

<400> 213
Asn Glu Lys Gln Met Ile Phe Phe Leu Asn Gly Asn Gln Leu Pro Pro
1               5                   10                  15

Glu Lys Gln Val Phe Ser Ser Thr Val Ser Gly Phe Phe Ala Ala
            20                  25                  30

<210> 214
<211> 27
<212> PRT
<213> Homo sapiens

<400> 214
Ser Tyr Gln Gln Cys Glu Phe Asn Phe Gly Ala Lys Pro Phe Lys Tyr
1               5                   10                  15

Pro Pro Ser Met Lys Phe Ser Thr Phe Asn Asp
            20                  25

<210> 215
<211> 29

<212> PRT
<213> Homo sapiens

<400> 215
Glu Glu Lys Ile Ile Leu Pro Arg His Arg Arg Leu Ala Leu Leu Lys
1               5                   10                  15

Gln Val Ser Ile Arg Glu Asn Cys Cys Ser Leu Cys Cys
            20                  25


<210> 216
<211> 30
<212> PRT
<213> Homo sapiens

<400> 216
Thr Gln Leu Lys Pro Cys Gly His Ser Asp Leu Cys Met Asp Cys Ala
1               5                   10                  15

Leu Gln Leu Glu Thr Cys Pro Leu Cys Arg Lys Glu Ile Val
            20                  25                  30


<210> 217
<211> 8
<212> PRT
<213> Homo sapiens

<400> 217
Ala Leu Glu Lys Phe Ala Gln Thr
1               5


<210> 218
<211> 6
<212> PRT
<213> Homo sapiens

<400> 218
Gly Phe Cys Ala Gln Trp
1               5


<210> 219
<211> 8
<212> PRT
<213> Homo sapiens

<400> 219
Asp Val Ser Glu Tyr Leu Lys Ile
1               5


<210> 220
<211> 7
<212> PRT
<213> Homo sapiens

<400> 220
Gly Leu Glu Ala Arg Cys Asp
1               5


<210> 221
<211> 8
<212> PRT
<213> Homo sapiens

<400> 221
Phe Glu Ser Val Arg Cys Thr Phe
1               5


<210> 222
<211> 6
<212> PRT
<213> Homo sapiens

<400> 222
Gly Val Trp Tyr Tyr Glu
1               5


<210> 223
<211> 8
<212> PRT
<213> Homo sapiens

<400> 223
Thr Ser Gly Val Met Gln Ile Gly
1               5


<210> 224
<211> 12
<212> PRT
<213> Homo sapiens

<400> 224
Phe Leu Asn His Glu Gly Tyr Gly Ile Gly Asp Asp
1               5                   10


<210> 225
<211> 7
<212> PRT
<213> Homo sapiens

<400> 225
Ala Tyr Asp Gly Cys Arg Gln
1               5


<210> 226
<211> 15

<212> PRT
<213> Homo sapiens

<400> 226
His Ala Ser Ala Asp Gly Gly Arg Thr Arg Gly Trp Thr Pro Thr
1               5                   10                  15


<210> 227
<211> 23
<212> PRT
<213> Homo sapiens

<400> 227
Ala Phe Asp Glu Gly Asn Lys Met Glu Leu Arg Lys Asn Thr Ile Leu
1               5                   10                  15

Ile Ile Tyr Tyr Ile Ser Arg
            20


<210> 228
<211> 25
<212> PRT
<213> Homo sapiens

<400> 228
Gly Thr Arg Trp Lys Leu Phe Gln Gln Arg Phe Leu Tyr Arg Gly Asn
1               5                   10                  15

Arg Glu Phe Gln Asn Lys Lys Leu Ser
            20                  25


<210> 229
<211> 10
<212> PRT
<213> Homo sapiens

<400> 229
Gly Thr Ser Ala Ile Pro Val Phe Ala Ala
1               5                   10


<210> 230
<211> 122
<212> PRT
<213> Homo sapiens

<400> 230
Leu Asp Phe Ile Leu Ser Ser Trp Leu Ser Thr Arg Gln Pro Met Lys
1               5                   10                  15

Asp Ile Lys Gly Ser Trp Thr Gly Lys Asn Arg Val Gln Asn Pro Tyr
            20                  25                  30

Ser His Gly Asn Ile Val Lys Asn Cys Cys Glu Val Leu Cys Gly Pro
        35                  40                  45

Leu Pro Pro Ser Val Leu Asp Arg Arg Gly Ile Leu Pro Leu Glu Glu
    50                  55                  60

Ser Gly Ser Arg Pro Pro Ser Thr Gln Glu Thr Ser Ser Ser Leu Leu
65                  70                  75                  80

Pro Gln Ser Pro Ala Pro Thr Glu His Leu Asn Ser Asn Glu Met Pro
                85                  90                  95

Glu Asp Ser Ser Thr Pro Glu Glu Met Pro Pro Pro Glu Pro Pro Glu
            100                 105                 110

Pro Pro Gln Glu Ala Ala Glu Ala Glu Lys
        115                 120


<210> 231
<211> 27
<212> PRT
<213> Homo sapiens

<400> 231
Lys Gly Ser Trp Thr Gly Lys Asn Arg Val Gln Asn Pro Tyr Ser His
1               5                   10                  15

Gly Asn Ile Val Lys Asn Cys Cys Glu Val Leu
            20                  25


<210> 232
<211> 25
<212> PRT
<213> Homo sapiens

<400> 232
Asp Arg Arg Gly Ile Leu Pro Leu Glu Glu Ser Gly Ser Arg Pro Pro
1               5                   10                  15

Ser Thr Gln Glu Thr Ser Ser Ser Leu
            20                  25


<210> 233
<211> 17
<212> PRT
<213> Homo sapiens

<400> 233
Pro Glu Asp Ser Ser Thr Pro Glu Glu Met Pro Pro Pro Glu Pro Pro
1               5                   10                  15

Glu


<210> 234
<211> 8

<212> PRT
<213> Homo sapiens

<400> 234
Tyr Leu Leu Gln Glu Asn Asn Leu
1               5


<210> 235
<211> 12
<212> PRT
<213> Homo sapiens

<400> 235
Val Arg Leu Leu Gly Leu Cys Ile Ala Gln Gly His
1               5                   10


<210> 236
<211> 188
<212> PRT
<213> Homo sapiens

<220>
<221> SITE
<222> (185)
<223> Xaa equals any of the naturally occurring L-amino acids

<400> 236
Met Arg Val Gly Arg Arg Pro Lys Ala Gln Arg Val Gln Gly Gln Asn
1               5                   10                  15

Gly Asn His Ser Ser Asp Ser Glu Gly Ser Phe Ser Leu Leu Cys Leu
            20                  25                  30

Gln Leu Phe Ser Lys Phe Ala Val Val Ser Ile Leu Leu Leu Leu Leu
        35                  40                  45

Leu Leu Phe Asn Thr Ser Lys Lys Lys Leu Met Thr Phe Ser Leu Asp
    50                  55                  60

Ser Leu Leu Ser Pro Ile Ser Ile Pro Thr Ala Leu Leu Phe Gly Ser
65                  70                  75                  80

Pro Pro Pro Pro Pro Ser His Arg Gly Tyr Gly Val Gly Ser Ala Pro
                85                  90                  95

Leu Lys Glu Lys Gln Met Lys Glu Leu Val Pro Pro Arg Arg Glu Cys
            100                 105                 110

Thr Val Gln Gly Gln Pro Trp Gln Gly Pro Ser Leu Pro Gly Pro Ala
        115                 120                 125

Glu Leu Gly His Arg Pro Gly Thr Arg Leu Gly Val Glu Cys Asp Gly
    130                 135                 140

Glu Trp Cys Pro Arg Ser Cys Phe Trp Glu Leu Leu Gly Pro Pro Tyr
145                 150                 155                 160

Leu Lys Cys Ser Gln Pro Ser Pro Ile Pro Pro Leu Asp Gly Thr Gln
                165                 170                 175

Thr Ser Ala Glu Arg Gly Arg Gly Xaa Ala Leu Lys
            180                 185

<210> 237
<211> 35
<212> PRT
<213> Homo sapiens

<400> 237
Pro Lys Ala Gln Arg Val Gln Gly Gln Asn Gly Asn His Ser Ser Asp
 1               5                  10                  15

Ser Glu Gly Ser Phe Ser Leu Leu Cys Leu Gln Leu Phe Ser Lys Phe
            20                  25                  30

Ala Val Val
        35

<210> 238
<211> 22
<212> PRT
<213> Homo sapiens

<400> 238
Leu Asp Ser Leu Leu Ser Pro Ile Ser Ile Pro Thr Ala Leu Leu Phe
 1               5                  10                  15

Gly Ser Pro Pro Pro Pro
            20

<210> 239
<211> 24
<212> PRT
<213> Homo sapiens

<400> 239
Glu Leu Val Pro Pro Arg Arg Glu Cys Thr Val Gln Gly Gln Pro Trp
 1               5                  10                  15

Gln Gly Pro Ser Leu Pro Gly Pro
            20

<210> 240
<211> 25
<212> PRT
<213> Homo sapiens

<400> 240
Arg Leu Gly Val Glu Cys Asp Gly Glu Trp Cys Pro Arg Ser Cys Phe
 1               5                  10                  15

Trp Glu Leu Leu Gly Pro Pro Tyr Leu
            20                  25

<210> 241
<211> 9
<212> PRT
<213> Homo sapiens

<400> 241
Trp His Ile Ser Glu Pro Asn Gly Gln
  1               5

<210> 242
<211> 36
<212> PRT
<213> Homo sapiens

<400> 242
Arg Pro Ser Arg Leu Arg Arg Arg Leu Lys Ala Pro Phe Ser Ala Trp
  1               5                  10                  15

Lys Thr Arg Leu Ala Gly Ala Lys Gly Gly Leu Ser Val Gly Asp Phe
            20                  25                  30

Arg Lys Val Leu
        35

<210> 243
<211> 53
<212> PRT
<213> Homo sapiens

<400> 243
Trp Pro Ser Gly Leu Gly Arg Thr Ser Ser Leu Arg Gly Ser Glu Ala
  1               5                  10                  15

Gln Ser Trp Cys Ser Ser Ala Gly His Gly Pro Pro Pro Ala Leu Gly
            20                  25                  30

Ser Pro Ala Ser Cys Gly Gly Cys Phe Ser Pro Thr Arg Ala Ser Ala
        35                  40                  45

Pro Ala Ala Gly Gly
     50

<210> 244
<211> 29
<212> PRT
<213> Homo sapiens

<400> 244
Ser Leu Arg Gly Ser Glu Ala Gln Ser Trp Cys Ser Ser Ala Gly His
  1               5                  10                  15

Gly Pro Pro Pro Ala Leu Gly Ser Pro Ala Ser Cys Gly
            20                  25

<210> 245
<211> 102
<212> PRT
<213> Homo sapiens

<400> 245
Lys Pro His Leu Gly Pro Arg Gly Ser Ile Glu Pro Ser Gln Ala Ser
1               5                   10                  15

Ser Arg Asn Pro Gly Leu Val Thr Glu Gln Ser Cys Leu Gln Gly Pro
            20                  25                  30

Ser Gly His Arg Ala Trp Ala Gly His His Leu Ser Glu Gly Gln Arg
        35                  40                  45

Leu Arg Ala Gly Ala Ala Gln Gln Val Thr Ala Leu His Gln Leu Trp
    50                  55                  60

Val Leu Pro His His Val Val Ala Ala Phe Pro Pro Pro Gly Pro Gln
65                  70                  75                  80

Leu Gln Gln Leu Val Gly Glu Leu Ser Thr Ala Tyr Ser Lys His Val
                85                  90                  95

Leu Arg His Ala Glu His
            100

<210> 246
<211> 30
<212> PRT
<213> Homo sapiens

<400> 246
Ser Arg Asn Pro Gly Leu Val Thr Glu Gln Ser Cys Leu Gln Gly Pro
1               5                   10                  15

Ser Gly His Arg Ala Trp Ala Gly His His Leu Ser Glu Gly
            20                  25                  30

<210> 247
<211> 33
<212> PRT
<213> Homo sapiens

<400> 247
Thr Ala Leu His Gln Leu Trp Val Leu Pro His His Val Val Ala Ala
1               5                   10                  15

Phe Pro Pro Pro Gly Pro Gln Leu Gln Gln Leu Val Gly Glu Leu Ser
            20                  25                  30

Thr

<210> 248
<211> 37
<212> PRT
<213> Homo sapiens

<400> 248
Ala Glu Gly Leu Gln Ser Ala Ala Gly Ile Arg Ile Asp Thr Lys Ala
1               5                   10                  15

Gly Pro Pro Glu Met Leu Lys Pro Leu Trp Lys Ala Ala Val Ala Pro
            20                  25                  30

Thr Trp Pro Cys Ser
        35

<210> 249
<211> 525
<212> PRT
<213> Homo sapiens

<400> 249


<210> 250
<211> 24
<212> PRT
<213> Homo sapiens

<400> 250
Gln Gly Arg Thr Pro Arg Asp Ala Glu Ala Ser Leu Glu Ser Ser Ser
1               5                   10                  15

Gly Pro His Met Ala Met Leu His
            20


<210> 251
<211> 23
<212> PRT
<213> Homo sapiens

<400> 251
Gly Ser Ala Gly Cys Ala Val Ala Gly Leu Arg Gly Ser Tyr Leu Pro
1               5                   10                  15

Pro Val Ala Ser Ala Pro Ser
            20


<210> 252
<211> 29
<212> PRT
<213> Homo sapiens

<400> 252
Ala Gln Gly Arg Ala Gln Val Leu Gly Ala Trp Leu Pro Ala Gln Leu
1               5                   10                  15

Gly Ser Pro Trp Lys Gln Arg Ala Arg Gln Gln Arg Asp
            20                  25


<210> 253
<211> 21
<212> PRT
<213> Homo sapiens

<400> 253
Pro Ser Ala Ala Gly Ser Pro Ser Ala Gln Pro Leu Gly Gln Ala Trp
1               5                   10                  15

Leu Gln Leu Leu Asp
            20

<210> 254
<211> 26
<212> PRT
<213> Homo sapiens

<400> 254
Val Ala Ser Tyr Tyr Trp Ser Leu Thr Gly Pro Asp Ile Gly Val Asn
1               5                   10                  15

Asp Ser Ser Ser Gln Leu Gly Glu Ala Leu
            20                  25

<210> 255
<211> 25
<212> PRT
<213> Homo sapiens

<400> 255
Ser Leu Ala Val Ala Thr Ser Ser Pro Thr Leu Ala Arg Thr Ser Thr
1               5                   10                  15

Asp Leu Gln Val Leu Ala Ala Arg Gly
            20                  25

<210> 256
<211> 26
<212> PRT
<213> Homo sapiens

<400> 256
Pro Gln Asn Phe Ser Ser His Phe Asn Arg Phe Gln Pro Phe His Gly
1               5                   10                  15

Leu Phe Asp Gly Val Pro Thr Thr Ala Tyr
            20                  25

<210> 257
<211> 27
<212> PRT
<213> Homo sapiens

<400> 257
Pro Gln Gly Arg Thr Arg Asp Leu Glu Ala Leu Leu Ala Val Met Gly
1               5                   10                  15

Ser Ala Gln Glu Phe Ile Tyr Ala Ser Val Met
            20                  25

<210> 258
<211> 24

<212> PRT
<213> Homo sapiens

<400> 258
Ser His Pro Pro Arg Tyr Trp Pro Val Leu Asp Asn Ala Leu Arg Ala
1               5                   10                  15

Ala Ala Phe Gly Lys Gly Val Arg
            20


<210> 259
<211> 29
<212> PRT
<213> Homo sapiens

<400> 259
Thr Asp Pro Thr Met Phe Pro Tyr Leu Arg Ser Leu Gln Ala Leu Ser
1               5                   10                  15

Asn Pro Ala Ala Asn Val Ser Val Asp Val Lys Val Phe
            20                  25


<210> 260
<211> 31
<212> PRT
<213> Homo sapiens

<400> 260
Asp Val Lys Val Phe Ile Val Pro Val Gly Asn His Ser Asn Ile Pro
1               5                   10                  15

Phe Ser Arg Val Asn His Ser Lys Phe Met Val Thr Glu Lys Ala
            20                  25                  30


<210> 261
<211> 24
<212> PRT
<213> Homo sapiens

<400> 261
Gln Leu Arg Gln Leu Phe Glu Arg Asp Trp Ser Ser Arg Tyr Ala Val
1               5                   10                  15

Gly Leu Asp Gly Gln Ala Pro Gly
            20


<210> 262
<211> 10
<212> PRT
<213> Homo sapiens

<400> 262
Lys Gln Pro Arg Gln Leu Phe Asn Ser Leu
1               5                   10

<210> 263
<211> 34
<212> PRT
<213> Homo sapiens

<400> 263
Thr Gln Ser Thr Gly Leu Glu Ser Ser Cys Ser Glu Ala Pro Gly Leu
1               5                   10                  15

Pro Leu Thr Phe Leu Val Ala Ala Thr Gln Arg Ala Leu Glu Trp Thr
            20                  25                  30

Gln Gly

<210> 264
<211> 228
<212> PRT
<213> Homo sapiens

<400> 264
Asp Thr Lys Asn Cys Gly Gln Glu Leu Ala Asn Leu Glu Lys Trp Lys
1               5                   10                  15

Glu Gln Asn Arg Ala Lys Pro Val His Leu Val Pro Arg Arg Leu Gly
            20                  25                  30

Gly Ser Gln Ser Glu Thr Glu Val Arg Gln Lys Gln Gln Leu Gln Leu
        35                  40                  45

Met Gln Ser Lys Tyr Lys Gln Lys Leu Lys Arg Glu Glu Ser Val Arg
    50                  55                  60

Ile Lys Lys Glu Ala Glu Glu Ala Glu Leu Gln Lys Met Lys Ala Ile
65                  70                  75                  80

Gln Arg Glu Lys Ser Asn Lys Leu Glu Glu Lys Lys Arg Leu Gln Glu
                85                  90                  95

Asn Leu Arg Arg Glu Ala Phe Arg Glu His Gln Gln Tyr Lys Thr Ala
            100                 105                 110

Glu Phe Leu Ser Lys Leu Asn Thr Glu Ser Pro Asp Arg Ser Ala Cys
        115                 120                 125

Gln Ser Ala Val Cys Gly Pro Gln Ser Ser Thr Trp Ala Arg Ser Trp
    130                 135                 140

Ala Tyr Arg Asp Ser Leu Lys Ala Glu Glu Asn Arg Lys Leu Gln Lys
145                 150                 155                 160

Met Lys Asp Glu Gln His Gln Lys Ser Glu Leu Leu Glu Leu Lys Arg
                165                 170                 175

Gln Gln Gln Glu Gln Glu Arg Ala Lys Ile His Gln Thr Glu His Arg

180                 185                 190

Arg Val Asn Asn Ala Phe Leu Asp Arg Leu Gln Gly Lys Ser Gln Pro
        195                 200                 205

Gly Gly Leu Glu Gln Ser Gly Gly Cys Trp Asn Met Asn Ser Gly Asn
    210                 215                 220

Ser Trp Gly Ile
225


<210> 265
<211> 21
<212> PRT
<213> Homo sapiens

<400> 265
Gly Gln Glu Leu Ala Asn Leu Glu Lys Trp Lys Glu Gln Asn Arg Ala
1               5                   10                  15

Lys Pro Val His Leu
            20


<210> 266
<211> 26
<212> PRT
<213> Homo sapiens

<400> 266
Arg Arg Leu Gly Gly Ser Gln Ser Glu Thr Glu Val Arg Gln Lys Gln
1               5                   10                  15

Gln Leu Gln Leu Met Gln Ser Lys Tyr Lys
            20                  25


<210> 267
<211> 21
<212> PRT
<213> Homo sapiens

<400> 267
Glu Glu Ala Glu Leu Gln Lys Met Lys Ala Ile Gln Arg Glu Lys Ser
1               5                   10                  15

Asn Lys Leu Glu Glu
            20


<210> 268
<211> 22
<212> PRT
<213> Homo sapiens

<400> 268
His Gln Gln Tyr Lys Thr Ala Glu Phe Leu Ser Lys Leu Asn Thr Glu

1               5                   10                  15

Ser Pro Asp Arg Ser Ala
            20



<210> 269
<211> 23
<212> PRT
<213> Homo sapiens

<400> 269
Leu Leu Glu Leu Lys Arg Gln Gln Gln Glu Gln Glu Arg Ala Lys Ile
1               5                   10                  15

His Gln Thr Glu His Arg Arg
            20



<210> 270
<211> 22
<212> PRT
<213> Homo sapiens

<400> 270
Leu Asp Arg Leu Gln Gly Lys Ser Gln Pro Gly Gly Leu Glu Gln Ser
1               5                   10                  15

Gly Gly Cys Trp Asn Met
            20



<210> 271
<211> 13
<212> PRT
<213> Homo sapiens

<400> 271
Leu Phe Ser Gly Glu Cys Leu Gln Arg Leu Trp Val Arg
1               5                   10



<210> 272
<211> 79
<212> PRT
<213> Homo sapiens

<400> 272
Arg His Glu Leu Val Pro Leu Val Pro Gly Leu Val Asn Ser Glu Val
1               5                   10                  15

His Asn Glu Asp Gly Arg Asn Gly Asp Val Ser Gln Phe Pro Tyr Val
            20                  25                  30

Glu Phe Thr Gly Arg Asp Ser Val Thr Cys Pro Thr Cys Gln Gly Thr
        35                  40                  45

Gly Arg Ile Pro Arg Gly Gln Glu Asn Gln Leu Val Ala Leu Ile Pro

50                  55                  60

Tyr Ser Asp Gln Arg Leu Arg Pro Arg Arg Thr Lys Leu Tyr Val
65                  70                  75

<210> 273
<211> 23
<212> PRT
<213> Homo sapiens

<400> 273
Pro Gly Leu Val Asn Ser Glu Val His Asn Glu Asp Gly Arg Asn Gly
1               5                   10                  15

Asp Val Ser Gln Phe Pro Tyr
            20

<210> 274
<211> 26
<212> PRT
<213> Homo sapiens

<400> 274
Thr Cys Pro Thr Cys Gln Gly Thr Gly Arg Ile Pro Arg Gly Gln Glu
1               5                   10                  15

Asn Gln Leu Val Ala Leu Ile Pro Tyr Ser
            20                  25

<210> 275
<211> 10
<212> PRT
<213> Homo sapiens

<400> 275
Ala Leu Ser Thr Glu Thr Arg Thr Pro Asp
1               5                   10